# EUROPEAN PATENT SPECIFICATION

(11) **EP 3 035 955 B1**
(45) Date of publication and mention of the grant of the patent: **11.09.2019**
(21) Application number: 14755334.1
(22) Date of filing: 21.08.2014
(51) Int. Cl.: A61K 39/00

(54) **COMPOSITION AND VACCINE FOR TREATING LUNG CANCER**
ZUSAMMENSETZUNG UND IMPFSTOFF ZUR BEHANDLUNG VON LUNGENKREBS
COMPOSITION ET VACCIN POUR LE TRAITEMENT DU CANCER DU POUMON

(30) Priority: 21.08.2013 WO PCT/EP2013/002514
(43) Date of publication of application: 29.06.2016
(60) Divisional application: 19172853.4
(73) Proprietor: CureVac AG, 72076 Tübingen (DE)
(72) Inventor: KALLEN, Karl-Josef, 50226 Königsdorf (DE); FOTIN-MLECZEK, Mariola, 71065 Sindelfingen (DE); GNAD-VOGT, Ulrike, 64367 Mühltal (DE)
(74) Representative: Graf von Stosch, Andreas
(86) International application number: PCT/EP2014/002299
(87) International publication number: WO 2015/024666

(56) References cited:
- WO-A1-2009/046739
- WO-A1-2011/067161
- WO-A1-2012/019630
- WO-A1-2012/116714
- WO-A1-2012/116715
- WO-A2-2006/015789
- WO-A2-2009/046974
- BENJAMIN PETSCH ET AL: "Protective efficacy of in vitro synthesized, specific mRNA vaccines against influenza A virus infection", NATURE BIOTECHNOLOGY, vol. 30, no. 12, December 2012 (2012-12), pages 1210-1216, XP055051005, ISSN: 1087-0156, DOI: 10.1038/nbt.2436
- THOMAS SCHLAKE ET AL: "Developing mRNA-vaccine technologies", RNA BIOLOGY, vol. 9, no. 11, 1 November 2012 (2012-11-01), pages 1319-1330, XP055098782, ISSN: 1547-6286, DOI: 10.4161/rna.22269
- FOTIN-MLECZEK MARIOLA ET AL: "Highly potent mRNA based cancer vaccines represent an attractive platform for combination therapies supporting an improved therapeutic effect", THE JOURNAL OF GENE MEDICINE, vol. 14, no. 6, 1 June 2012 (2012-06-01), pages 428-439, XP002719717, ISSN: 1521-2254
- BAUER ASLI PETRA ET AL: "The impact of intragenic CpG content on gene expression", NUCLEIC ACIDS RESEARCH, vol. 38, no. 12, 1 July 2010 (2010-07-01), pages 3891-3908, XP002719718, OXFORD UNIVERSITY PRESS, GB ISSN: 1362-4962
- J Greenhalgh ET AL: "Pemetrexed for the maintenance treatment of locally advanced or metastatic non-small cell lung cancer", HEALTH TECHNOLOGY ASSESSMENT, vol. 14, no. Suppl. 2, 1 October 2010 (2010-10-01), XP055523683, ISSN: 1366-5278, DOI: 10.3310/hta14suppl2/05

### SEQUENCE LISTING

<110> CureVac GmbH
<120> Composition and Vaccine for Treating Lung Cancer
<130> CU01P148WO1EP
<160> 83
<170> PatentIn version 3.5
<210> 1
   <211> 1467
   <212> RNA
   <213> Artificial Sequence
<220>
   <223> 5T4 (GC)-muag-A64-C30
<400> 1
   gggagaaagc uuaccaugcc cggcgggugc agccggggcc cggccgccgg ggacggccgc     60
   cugcggcucg cgcgccuggc ccuggugcuc cugggguggg ucuccagcuc cagccccacc     120
   uccagcgccu ccagcuucuc cagcuccgcc cccuuccugg ccagcgcggu guccgcccag    180
   cccccgcucc ccgaccagug ccccgcccug ugcgagugca gcgaggccgc gcggaccgug    240
   aagugcguca accgcaaccu gacggaggug cccaccgacc ucccggccua cgugcggaac    300
   cuguuccuga ccggcaacca gcucgccguc cugcccgccg gcgccuucgc gcgccggccg    360
   ccccuggccg agcucgccgc ccugaaccug uccgggagcc gccucgacga ggugcgggcc    420
   ggcgcguucg agcaccugcc gucccugcgc cagcucgacc ugagccacaa cccccuggcc    480
   gaccucuccc ccuucgccuu cagcgggagc aacgccuccg ugagcgcccc cuccccgcug    540
   gucgagcuga uccucaacca caucgugccc cccgaggacg agcggcagaa ccgcagcuuc    600
   gagggcaugg uggucgcggc ccugcuggcc gggcgggccc uccagggccu gcgccggcug    660
   gagcucgccu ccaaccacuu ccuguaccug ccccgcgacg ugcucgcgca gcugccgagc    720
   cugcggcacc ucgaccuguc caacaacagc cugguguccc ucaccuacgu cagcuuccgc     780
   aaccugacgc accuggaguc ccuccaccug gaggacaacg cccugaaggu gcugcacaac    840
   ggcacccucg ccgagcugca ggggcugccc cacauccggg uguuccucga caacaacccc    900
   ugggucugcg acugccacau ggccgacaug gugaccuggc ugaaggagac cgaggugguc    960
   cagggcaagg accgccugac gugcgcguac cccgagaaga ugcggaaccg ggugcuccug   1020
   gagcugaaca gcgccgaccu cgacugcgac ccgauccugc cccccucccu gcagaccagc   1080
   uacguguucc ucgggaucgu ccuggcccug aucggcgcca ucuuccuccu ggugcuguac   1140
   cucaaccgca agggcaucaa gaaguggaug cacaacaucc gggacgccug ccgcgaccac   1200
   auggaggggu accacuaccg guacgagauc aacgcggacc cccgccugac caaccugucc   1260
   agcaacuccg acgucugacc acuaguuaua agacugacua gcccgauggg ccucccaacg   1320
   ggcccuccuc cccuccuugc accgagauua auaaaaaaaa aaaaaaaaaa aaaaaaaaaa   1380
   aaaaaaaaaa aaaaaaaaaa aaaaaaaaaa aaaaaauauu cccccccccc cccccccccc   1440
   cccccccccc ucuagacaau uggaauu                                       1467
<210> 2
   <211> 1263
   <212> RNA
   <213> Artificial Sequence
<220>
   <223> 5T4 CDS wild type
<400> 2
   augccugggg ggugcucccg gggccccgcc gccggggacg ggcgucugcg gcuggcgcga      60
   cuagcgcugg uacuccuggg cugggucucc ucgucuucuc ccaccuccuc ggcauccucc     120
   uucuccuccu cggcgccguu ccuggcuucc gccguguccg cccagccccc gcugccggac     180
   cagugccccg cgcugugcga gugcuccgag gcagcgcgca cagucaagug cguuaaccgc     240
   aaucugaccg aggugcccac ggaccugccc gccuacgugc gcaaccucuu ccuuaccggc     300
   aaccagcugg ccgugcuccc ugccggcgcc uucgcccgcc ggccgccgcu ggcggagcug     360
   gccgcgcuca accucagcgg cagccgccug gacgaggugc gcgcgggcgc cuucgagcau     420
   cugcccagcc ugcgccagcu cgaccucagc cacaacccac uggccgaccu cagucccuuc     480
   gcuuucucgg gcagcaaugc cagcgucucg gcccccaguc cccuugugga acugauccug     540
   aaccacaucg ugcccccuga agaugagcgg cagaaccgga gcuucgaggg caugguggug     600
   gcggcccugc uggcgggccg ugcacugcag gggcuccgcc gcuuggagcu ggccagcaac     660
   cacuuccuuu accugccgcg ggaugugcug gcccaacugc ccagccucag gcaccuggac     720
   uuaaguaaua auucgcuggu gagccugacc uacguguccu uccgcaaccu gacacaucua     780
   gaaagccucc accuggagga caaugcccuc aagguccuuc acaauggcac ccuggcugag     840
   uugcaagguc uaccccacau uaggguuuuc cuggacaaca aucccugggu cugcgacugc     900
   cacauggcag acauggugac cuggcucaag gaaacagagg uagugcaggg caaagaccgg     960
   cucaccugug cauauccgga aaaaaugagg aaucgggucc ucuuggaacu caacagugcu    1020
   gaccuggacu gugacccgau ucuuccccca ucccugcaaa ccucuuaugu cuuccugggu    1080
   auuguuuuag cccugauagg cgcuauuuuc cuccugguuu uguauuugaa ccgcaagggg    1140
   auaaaaaagu ggaugcauaa caucagagau gccugcaggg aucacaugga aggguaucau    1200
   uacagauaug aaaucaaugc ggaccccaga uuaacgaacc ucaguucuaa cucggauguc    1260
   uga                                                                  1263
<210> 3
   <211> 1263
   <212> RNA
   <213> Artificial Sequence
<220>
   <223> 5T4 CDS GC-optimized
<400> 3
   augcccggcg ggugcagccg gggcccggcc gccggggacg gccgccugcg gcucgcgcgc 60
   cuggcccugg ugcuccuggg gugggucucc agcuccagcc ccaccuccag cgccuccagc 120
   uucuccagcu ccgcccccuu ccuggccagc gcgguguccg cccagccccc gcuccccgac 180
   cagugccccg cccugugcga gugcagcgag gccgcgcgga ccgugaagug cgucaaccgc 240
   aaccugacgg aggugcccac cgaccucccg gccuacgugc ggaaccuguu ccugaccggc 300
   aaccagcucg ccguccugcc cgccggcgcc uucgcgcgcc ggccgccccu ggccgagcuc 360
   gccgcccuga accuguccgg gagccgccuc gacgaggugc gggccggcgc guucgagcac 420
   cugccguccc ugcgccagcu cgaccugagc cacaaccccc uggccgaccu cucccccuuc 480
   gccuucagcg ggagcaacgc cuccgugagc gcccccuccc cgcuggucga gcugauccuc 540
   aaccacaucg ugccccccga ggacgagcgg cagaaccgca gcuucgaggg cauggugguc 600
   gcggcccugc uggccgggcg ggcccuccag ggccugcgcc ggcuggagcu cgccuccaac 660
   cacuuccugu accugccccg cgacgugcuc gcgcagcugc cgagccugcg gcaccucgac 720
   cuguccaaca acagccuggu gucccucacc uacgucagcu uccgcaaccu gacgcaccug 780
   gagucccucc accuggagga caacgcccug aaggugcugc acaacggcac ccucgccgag 840
   cugcaggggc ugccccacau ccggguguuc cucgacaaca accccugggu cugcgacugc 900
   cacauggccg acauggugac cuggcugaag gagaccgagg ugguccaggg caaggaccgc 960
   cugacgugcg cguaccccga gaagaugcgg aaccgggugc uccuggagcu gaacagcgcc 1020
   gaccucgacu gcgacccgau ccugcccccc ucccugcaga ccagcuacgu guuccucggg 1080
   aucguccugg cccugaucgg cgccaucuuc cuccuggugc uguaccucaa ccgcaagggc 1140
   aucaagaagu ggaugcacaa cauccgggac gccugccgcg accacaugga gggguaccac 1200
   uaccgguacg agaucaacgc ggacccccgc cugaccaacc uguccagcaa cuccgacguc 1260
   uga 1263
<210> 4
   <211> 633
   <212> RNA
   <213> Artificial Sequence
<220>
   <223> Survivin (GC)-muag-A64-C30
<400> 4
   gggagaaagc uuaccauggg cgcccccacc cugccgccgg ccuggcagcc guuccucaag      60
   gaccaccgca ucucgaccuu caagaacugg ccguuccugg agggcugcgc gugcaccccg     120
   gagcggaugg ccgaggccgg cuucauccac ugccccaccg agaacgagcc ggaccuggcc     180
   cagugcuucu ucugcuucaa ggagcuggag ggcugggagc cggacgacga cccgaucgag     240
   gagcacaaga agcacagcag cggcugcgcc uuccugagcg ugaagaagca guucgaggag     300
   cugacgcucg ggaguucccu gaagcuggac cgggagcggg ccaagaacaa gaucgcgaag      360
   gagaccaaca acaagaagaa ggaguucgag gagaccgcca agaaggugcg gcgggccauc     420
   gagcagcugg ccgccaugga cugaccacua guuauaagac ugacuagccc gaugggccuc     480
   ccaacgggcc cuccuccccu ccuugcaccg agauuaauaa aaaaaaaaaa aaaaaaaaaa     540
   aaaaaaaaaa aaaaaaaaaa aaaaaaaaaa aaaaaaaaaa aauauucccc cccccccccc     600
   cccccccccc ccccccucua gacaauugga auu                                   633
<210> 5
   <211> 429
   <212> RNA
   <213> Artificial Sequence
<220>
   <223> Survivin CDS wild type
<400> 5
   augggugccc cgacguugcc cccugccugg cagcccuuuc ucaaggacca ccgcaucucu      60
   acauucaaga acuggcccuu cuuggagggc ugcgccugca ccccggagcg gauggccgag     120
   gcuggcuuca uccacugccc cacugagaac gagccagacu uggcccagug uuucuucugc     180
   uucaaggagc uggaaggcug ggagccagau gacgacccca uagaggaaca uaaaaagcau     240
   ucguccgguu gcgcuuuccu uucugucaag aagcaguuug aagaauuaac ccuuggugaa     300
   uuuuugaaac uggacagaga aagagccaag aacaaaauug caaaggaaac caacaauaag     360
   aagaaagaau uugaggaaac ugcgaagaaa gugcgccgug ccaucgagca gcuggcugcc     420
   auggauuga                                                            429
<210> 6
   <211> 429
   <212> RNA
   <213> Artificial Sequence
<220>
   <223> Survivin CDS GC-optimized
<400> 6
   augggcgccc ccacccugcc gccggccugg cagccguucc ucaaggacca ccgcaucucg        60
   accuucaaga acuggccguu ccuggagggc ugcgcgugca ccccggagcg gauggccgag       120
   gccggcuuca uccacugccc caccgagaac gagccggacc uggcccagug cuucuucugc       180
   uucaaggagc uggagggcug ggagccggac gacgacccga ucgaggagca caagaagcac       240
   agcagcggcu gcgccuuccu gagcgugaag aagcaguucg aggagcugac gcucggggag       300
   uuccugaagc uggaccggga gcgggccaag aacaagaucg cgaaggagac caacaacaag       360
   aagaaggagu ucgaggagac cgccaagaag gugcggcggg ccaucgagca gcuggccgcc       420
   auggacuga                                                               429
<210> 7
   <211> 747
   <212> RNA
   <213> Artificial Sequence
<220>
   <223> NY-ESO-1 (GC)-MUAG-A64-C30
<400> 7
   gggagaaagc uuaccaugca ggccgagggc cgcggcaccg gcggcucgac cggcgacgcc        60
   gacgggcccg gcggcccggg caucccggac ggcccgggcg ggaacgcggg cggcccgggc       120
   gaggccggcg ccaccggcgg gcggggcccg cggggcgccg gcgccgcccg ggcgagcggc       180
   cccggcgggg gcgccccgcg gggcccgcac ggcggcgccg ccagcggccu gaacgggugc       240
   ugccggugcg gcgcccgcgg cccggagagc cggcuccugg aguucuaccu ggccaugccg       300
   uucgcgaccc cgauggaggc cgagcuggcc cggcggagcc uggcccagga cgccccgccg       360
   cugcccgugc cgggcgugcu ccugaaggag uucacgguga gcggcaacau ccugaccauc       420
   cggcugaccg ccgcggacca ccggcagcug cagcugucga ucagcagcug ccuccagcag       480
   cugagccugc ugauguggau cacccagugc uuccugccgg uguuccuggc ccagccgccc       540
   agcggccagc gccggugacc acuaguuaua agacugacua gcccgauggg ccucccaacg       600
   ggcccuccuc cccuccuugc accgagauua auaaaaaaaa aaaaaaaaaa aaaaaaaaaa       660
   aaaaaaaaaa aaaaaaaaaa aaaaaaaaaa aaaaaauauu cccccccccc cccccccccc       720
   cccccccccc ucuagacaau uggaauu                                           747
<210> 8
   <211> 543
   <212> RNA
   <213> Artificial Sequence
<220>
   <223> NY-ESO-1 CDS wild type
<400> 8
   augcaggccg aaggccgggg cacagggggu ucgacgggcg augcugaugg cccaggaggc      60
   ccuggcauuc cugauggccc agggggcaau gcuggcggcc caggagaggc gggugccacg     120
   ggcggcagag guccccgggg cgcaggggca gcaagggccu cggggccggg aggaggcgcc     180
   ccgcgggguc cgcauggcgg cgcggcuuca gggcugaaug gaugcugcag augcggggcc    240
   agggggccgg agagccgccu gcuugaguuc uaccucgcca ugccuuucgc gacacccaug     300
   gaagcagagc uggcccgcag gagccuggcc caggaugccc caccgcuucc cgugccaggg     360
   gugcuucuga aggaguucac uguguccggc aacauacuga cuauccgacu gacugcugca     420
   gaccaccgcc aacugcagcu cuccaucagc uccugucucc agcagcuuuc ccuguugaug     480
   uggaucacgc agugcuuucu gcccguguuu uuggcucagc cucccucagg gcagaggcgc     540
   uaa                                                                   543
<210> 9
   <211> 543
   <212> RNA
   <213> Artificial Sequence
<220>
   <223> NY-ESO-1 CDS GC-optimized
<400> 9
   augcaggccg agggccgcgg caccggcggc ucgaccggcg acgccgacgg gcccggcggc      60
   ccgggcaucc cggacggccc gggcgggaac gcgggcggcc cgggcgaggc cggcgccacc     120
   ggcgggcggg gcccgcgggg cgccggcgcc gcccgggcga gcggccccgg cgggggcgcc     180
   ccgcggggcc cgcacggcgg cgccgccagc ggccugaacg ggugcugccg gugcggcgcc     240
   cgcggcccgg agagccggcu ccuggaguuc uaccuggcca ugccguucgc gaccccgaug     300
   gaggccgagc uggcccggcg gagccuggcc caggacgccc cgccgcugcc cgugccgggc     360
   gugcuccuga aggaguucac ggugagcggc aacauccuga ccauccggcu gaccgccgcg     420
   gaccaccggc agcugcagcu gucgaucagc agcugccucc agcagcugag ccugcugaug     480
   uggaucaccc agugcuuccu gccgguguuc cuggcccagc cgcccagcgg ccagcgccgg     540
   uga                                                                   543
<210> 10
   <211> 1800
   <212> RNA
   <213> Artificial Sequence
<220>
   <223> MAGE-C1 (aa 613-1142) (GC)-muag-A64-C30
<400> 10
   gggagaaagc uuaccaugca guccccgcug cagggcgagg aguuccagag cucccugcag      60
   agccccgugu ccaucugcag cuccagcacc cccuccagcc ucccgcagag cuuccccgag     120
   uccagccagu ccccccccga gggcccgguc cagagccccc ugcacucccc gcagagcccc     180
   ccggagggga ugcacuccca gagcccccug cagucccccg agagcgcccc cgagggcgag      240
   gacucccuca gcccgcugca gaucccccag uccccgcugg agggggagga cagccucucc     300
   agccugcacu ucccccaguc cccgcccgag ugggaggaca gccugagccc ccuccacuuc     360
   ccccaguucc cgccccaggg cgaggacuuc caguccagcc ugcagucccc cgugagcauc      420
   ugcuccagcu ccacgagccu gucccucccc cagagcuucc cggagucccc ccagagcccg      480
   cccgaggggc cggcgcaguc cccccugcag cgccccguga gcuccuucuu cagcuacacc     540
   cuggccuccc uccugcagag cucccacgag agcccgcaga gcccgcccga gggccccgcc     600
   caguccccgc ugcagagccc cgucuccagc uuccccucca gcaccuccag cucccucagc     660
   caguccagcc ccguguccag cuucccgucc agcaccucca gcucccugag caagagcucc     720
   cccgagagcc cccugcaguc ccccgugauc agcuucucca gcuccacgag ccucuccccg      780
   uucagcgagg aguccagcuc ccccgucgac gaguacacca gcuccagcga cacccugcug     840
   gaguccgaca gccucaccga cuccgagagc cugaucgaga gcgagccccu guucaccuac      900
   acgcucgacg agaaggugga cgagcuggcc cgguuccugc uccugaagua ccaggugaag     960
   cagcccauca ccaaggccga gaugcugacc aacgucaucu cccgcuacac cggcuacuuc    1020
   ccggugaucu uccggaaggc gcgcgaguuc aucgagaucc ucuucgggau cagccugcgg    1080
   gagguggacc ccgacgacuc cuacgucuuc gugaacacgc uggaccucac cagcgagggc    1140
   ugccuguccg acgagcaggg gaugagccag aaccgccugc ucauccugau ccuguccauc    1200
   aucuucauca agggcaccua cgccagcgag gaggucaucu gggacgugcu cuccgggauc    1260
   ggcgugcggg ccggccgcga gcacuucgcc uucggggagc cccgggagcu gcugaccaag    1320
   gucugggugc aggagcacua ccucgaguac cgcgaggugc ccaacagcuc cccgccccgg      1380
   uacgaguucc uguggggccc ccgcgcccac agcgagguca ucaagcggaa ggugguggag    1440
   uuccuggcga ugcucaagaa cacggucccc aucaccuucc cguccagcua caaggacgcc    1500
   cugaaggacg uggaggagcg ggcccaggcc aucaucgaca ccaccgacga cuccacggcc    1560
   accgagagcg cguccagcuc cgugaugagc cccagcuucu ccagcgagug accacuaguu    1620
   auaagacuga cuagcccgau gggccuccca acgggcccuc cuccccuccu ugcaccgaga    1680
   uuaauaaaaa aaaaaaaaaa aaaaaaaaaa aaaaaaaaaa aaaaaaaaaa aaaaaaaaaa    1740
   aaaaaaaaau auuccccccc cccccccccc cccccccccc cccucuagac aauuggaauu     1800
<210> 11
   <211> 3429
   <212> RNA
   <213> Artificial Sequence
<220>
   <223> MAGE-C1 full-length CDS wild type
<400> 11
   augggggaca aggauaugcc uacugcuggg augccgaguc uucuccagag uuccucugag 60
   aguccucaga guuguccuga gggggaggac ucccagucuc cucuccagau uccccagagu 120
   ucuccugaga gcgacgacac ccuguauccu cuccagaguc cucagagucg uucugagggg 180
   gaggacuccu cggauccucu ccagagaccu ccugagggga aggacuccca gucuccucuc 240
   cagauucccc agaguucucc ugagggcgac gacacccagu cuccucucca gaauucucag 300
   aguucuccug aggggaagga cucccugucu ccucuagaga uuucucagag cccuccugag 360
   ggugaggaug uccagucucc ucugcagaau ccugcgaguu ccuucuucuc cucugcuuua 420
   uugaguauuu uccagaguuc cccugagagu acucaaaguc cuuuugaggg uuuuccccag 480
   ucuguucucc agauuccugu gagcgccgcc uccuccucca cuuuagugag uauuuuccag 540
   aguuccccug agaguacuca aaguccuuuu gaggguuuuc cccagucucc acuccagauu 600
   ccugugagcc gcuccuucuc cuccacuuua uugaguauuu uccagaguuc cccugagaga 660
   acucagagua cuuuugaggg uuuugcccag ucuccucucc agauuccugu gagccccucc 720
   uccuccucca cuuuacugag ucuuuuccag aguuucucug agagaacuca gaguacuuuu 780
   gaggguuuug cccagucuuc ucuccagauu ccugugagcc ccuccuucuc cuccacuuua 840
   gugagucuuu uccagaguuc cccugagaga acucagagua cuuuugaggg uuuuccccag 900
   ucuccucucc agauuccugu gagcuccucc uccuccucca cuuuauugag ucuuuuccag 960
   aguuccccug agagaacuca caguacuuuu gaggguuuuc cccagucucu ucuccagauu 1020
   ccuaugaccu ccuccuucuc cucuacuuua uugaguauuu uccagaguuc uccugagagu 1080
   gcucaaagua cuuuugaggg uuuuccccag ucuccucucc agauuccugg gagccccucc 1140
   uucuccucca cuuuacugag ucuuuuccag aguuccccug agagaacuca caguacuuuu 1200
   gaggguuuuc cccagucucc ucuccagauu ccuaugaccu ccuccuucuc cucuacuuua 1260
   uugaguauuu uacagaguuc uccugagagu gcucaaagug cuuuugaggg uuuuccccag 1320
   ucuccucucc agauuccugu gagcuccucu uucuccuaca cuuuauugag ucuuuuccag 1380
   aguuccccug agagaacuca caguacuuuu gaggguuuuc cccagucucc ucuccagauu 1440
   ccugugagcu ccuccuccuc cuccuccacu uuauugaguc uuuuccagag uuccccugag 1500
   uguacucaaa guacuuuuga ggguuuuccc cagucuccuc uccagauucc ucagaguccu 1560
   ccugaagggg agaauaccca uucuccucuc cagauuguuc caagucuucc ugagugggag 1620
   gacucccugu cuccucacua cuuuccucag agcccuccuc agggggagga cucccuaucu 1680
   ccucacuacu uuccucagag cccuccucag ggggaggacu cccugucucc ucacuacuuu 1740
   ccucagagcc cucaggggga ggacucccug ucuccucacu acuuuccuca gagcccuccu 1800
   cagggggagg acuccauguc uccucucuac uuuccucaga guccucuuca gggggaggaa 1860
   uuccagucuu cucuccagag cccugugagc aucugcuccu ccuccacucc auccagucuu 1920
   ccccagaguu ucccugagag uucucagagu ccuccugagg ggccugucca gucuccucuc 1980
   cauaguccuc agagcccucc ugaggggaug cacucccaau cuccucucca gaguccugag 2040
   agugcuccug agggggagga uucccugucu ccucuccaaa uuccucagag uccucuugag 2100
   ggagaggacu cccugucuuc ucuccauuuu ccucagaguc cuccugagug ggaggacucc 2160
   cucucuccuc uccacuuucc ucaguuuccu ccucaggggg aggacuucca gucuucucuc 2220
   cagaguccug ugaguaucug cuccuccucc acuucuuuga gucuucccca gaguuucccu 2280
   gagaguccuc agaguccucc ugaggggccu gcucagucuc cucuccagag accugucagc 2340
   uccuucuucu ccuacacuuu agcgagucuu cuccaaaguu cccaugagag uccucagagu 2400
   ccuccugagg ggccugccca gucuccucuc cagaguccug ugagcuccuu ccccuccucc 2460
   acuucaucga gucuuuccca gaguucuccu gugagcuccu uccccuccuc cacuucaucg 2520
   agucuuucca agaguucccc ugagaguccu cuccagaguc cugugaucuc cuucuccucc 2580
   uccacuucau ugagcccauu cagugaagag uccagcagcc caguagauga auauacaagu 2640
   uccucagaca ccuugcuaga gagugauucc uugacagaca gcgaguccuu gauagagagc 2700
   gagcccuugu ucacuuauac acuggaugaa aagguggacg aguuggcgcg guuucuucuc 2760
   cucaaauauc aagugaagca gccuaucaca aaggcagaga ugcugacgaa ugucaucagc 2820
   agguacacgg gcuacuuucc ugugaucuuc aggaaagccc gugaguucau agagauacuu 2880
   uuuggcauuu cccugagaga aguggacccu gaugacuccu augucuuugu aaacacauua 2940
   gaccucaccu cugaggggug ucugagugau gagcagggca ugucccagaa ccgccuccug 3000
   auucuuauuc ugaguaucau cuucauaaag ggcaccuaug ccucugagga ggucaucugg 3060
   gaugugcuga guggaauagg ggugcgugcu gggagggagc acuuugccuu uggggagccc 3120
   agggagcucc ucacuaaagu uugggugcag gaacauuacc uagaguaccg ggaggugccc 3180
   aacucuucuc cuccucguua cgaauuccug ugggguccaa gagcucauuc agaagucauu 3240
   aagaggaaag uaguagaguu uuuggccaug cuaaagaaua ccgucccuau uaccuuucca 3300
   uccucuuaca aggaugcuuu gaaagaugug gaagagagag cccaggccau aauugacacc 3360
   acagaugauu cgacugccac agaaagugca agcuccagug ucaugucccc cagcuucucu 3420
   ucugaguga 3429
<210> 12
   <211> 3429
   <212> RNA
   <213> Artificial Sequence
<220>
   <223> MAGE-C1 full-length CDS GC-optimized
<400> 12
   augggcgaca aggacaugcc caccgccggg augccgagcc ugcuccaguc cagcuccgag 60
   agcccccagu ccugccccga gggcgaggac agccaguccc cccugcagau cccgcagagc 120
   ucccccgaga gcgacgacac ccuguacccc cuccaguccc cgcagagccg guccgagggg 180
   gaggacagcu ccgacccgcu gcagcgcccc cccgagggca aggacagcca guccccgcug 240
   cagaucccgc agagcucccc cgagggggac gacacgcaga gcccccucca gaacagccag 300
   uccagccccg agggcaagga cucccugagc ccgcuggaga ucucccagag cccccccgag 360
   ggcgaggacg ugcagucccc gcuccagaac ccggccagcu ccuucuucag cuccgcgcug 420
   cugagcaucu uccaguccag ccccgagucc acccagagcc ccuucgaggg guucccccag 480
   uccguccucc agaucccggu gagcgccgcc uccagcagca cccugguguc caucuuccag 540
   agcucccccg agagcaccca gucccccuuc gagggcuucc cccagagccc gcugcagauc 600
   cccguguccc ggagcuucuc cagcacgcuc cuguccaucu uccagagcuc ccccgagcgc 660
   acccagagca ccuucgaggg guucgcccag uccccgcugc agauccccgu gagccccucc 720
   agcagcucca cccuccugag ccuguuccag uccuucagcg agcggacgca guccaccuuc 780
   gagggcuucg cccagagcuc ccuccagauc cccgugagcc cguccuucag cuccacccug 840
   gucagccugu uccaguccag ccccgagcgc acccagucca cguucgaggg guucccccag 900
   agcccccucc agaucccggu guccagcucc agcagcucca cccugcugag ccucuuccag 960
   uccagccccg agcggaccca cuccaccuuc gagggcuucc cccagagccu gcugcagauc 1020
   cccaugacgu ccagcuucuc cagcacccuc cuguccaucu uccagagcuc cccggagagc 1080
   gcgcagucca ccuucgaggg cuucccccag agcccccugc agauccccgg guccccgagc 1140
   uucuccagca cccuccugag ccuguuccag uccagccccg agcgcacgca cuccaccuuc 1200
   gagggcuucc cccagagccc ccuccagauc ccgaugaccu ccagcuucuc cagcacccug 1260
   cuguccaucc uccagagcuc ccccgagagc gcccaguccg ccuucgaggg guucccccag 1320
   agcccccugc agaucccggu guccagcucc uucagcuaca cgcugcucuc ccuguuccag 1380
   agcagccccg agcggaccca cuccaccuuc gagggcuucc cccagagccc gcugcagauc 1440
   cccgugucca gcuccagcuc cagcuccacc cuccugagcc uguuccaguc cagccccgag 1500
   ugcacgcagu ccaccuucga gggcuucccc cagagcccgc ugcagauccc ccaguccccc 1560
   cccgaggggg agaacaccca cagcccgcuc cagaucgugc ccucccugcc cgagugggag 1620
   gacagccugu ccccgcacua cuucccgcag agccccccgc agggcgagga cagccucucc 1680
   ccccacuacu ucccgcagag cccgccccag ggggaggacu cccugagccc ccacuacuuc 1740
   ccgcaguccc cccagggcga ggacagccug uccccgcacu acuuccccca gagcccgccc 1800
   cagggggagg acuccaugag cccccucuac uucccccagu ccccgcugca gggcgaggag 1860
   uuccagagcu cccugcagag ccccgugucc aucugcagcu ccagcacccc cuccagccuc 1920
   ccgcagagcu uccccgaguc cagccagucc ccccccgagg gcccggucca gagcccccug 1980
   cacuccccgc agagcccccc ggaggggaug cacucccaga gcccccugca gucccccgag 2040
   agcgcccccg agggcgagga cucccucagc ccgcugcaga ucccccaguc cccgcuggag 2100
   ggggaggaca gccucuccag ccugcacuuc ccccaguccc cgcccgagug ggaggacagc 2160
   cugagccccc uccacuuccc ccaguucccg ccccagggcg aggacuucca guccagccug 2220
   cagucccccg ugagcaucug cuccagcucc acgagccugu cccuccccca gagcuucccg 2280
   gagucccccc agagcccgcc cgaggggccg gcgcaguccc cccugcagcg ccccgugagc 2340
   uccuucuuca gcuacacccu ggccucccuc cugcagagcu cccacgagag cccgcagagc 2400
   ccgcccgagg gccccgccca guccccgcug cagagccccg uguccagcuu ccccuccagc 2460
   accuccagcu cccucagcca guccagcccc guguccagcu ucccguccag caccuccagc 2520
   ucccugagca agagcucccc cgagagcccc cugcaguccc ccgugaucag cuucuccagc 2580
   uccacgagcc ucuccccguu cagcgaggag uccagcuccc ccgucgacga guacaccagc 2640
   uccagcgaca cccugcugga guccgacagc cucaccgacu ccgagagccu gaucgagagc 2700
   gagccccugu ucaccuacac gcucgacgag aagguggacg agcuggcccg guuccugcuc 2760
   cugaaguacc aggugaagca gcccaucacc aaggccgaga ugcugaccaa cgucaucucc 2820
   cgcuacaccg gcuacuuccc ggugaucuuc cggaaggcgc gcgaguucau cgagauccuc 2880
   uucgggauca gccugcggga gguggacccc gacgacuccu acgucuucgu gaacacgcug 2940
   gaccucacca gcgagggcug ccuguccgac gagcagggga ugagccagaa ccgccugcuc 3000
   auccugaucc uguccaucau cuucaucaag ggcaccuacg ccagcgagga ggucaucugg 3060
   gacgugcucu ccgggaucgg cgugcgggcc ggccgcgagc acuucgccuu cggggagccc 3120
   cgggagcugc ugaccaaggu cugggugcag gagcacuacc ucgaguaccg cgaggugccc 3180
   aacagcuccc cgccccggua cgaguuccug uggggccccc gcgcccacag cgaggucauc 3240
   aagcggaagg ugguggaguu ccuggcgaug cucaagaaca cgguccccau caccuucccg 3300
   uccagcuaca aggacgcccu gaaggacgug gaggagcggg cccaggccau caucgacacc 3360
   accgacgacu ccacggccac cgagagcgcg uccagcuccg ugaugagccc cagcuucucc 3420
   agcgaguga 3429
<210> 13
   <211> 1326
   <212> RNA
   <213> Artificial Sequence
<220>
   <223> MAGE-C2 (GC)-muag-A64-C30
<400> 13
   gggagaaagc uuaccaugcc cccggugccc ggcguccccu uccggaacgu ggacaacgac 60
   agccccaccu ccguggagcu ggaggacugg gucgacgccc agcacccgac cgacgaggag 120
   gaggaggagg ccagcuccgc gagcuccacg cucuaccugg uguucagccc cuccagcuuc 180
   uccaccagcu ccagccugau ccucgggggc cccgaggagg aggaggugcc cuccgggguc 240
   aucccgaacc ugaccgagag cauccccucc agccccccgc agggcccgcc ccaggggccc 300
   ucccagagcc cccuguccag cugcugcagc uccuucagcu gguccagcuu cuccgaggag 360
   agcuccagcc agaagggcga ggacaccggc acgugccagg ggcucccgga cuccgagagc 420
   uccuucaccu acacccugga cgagaaggug gccgagcugg uggaguuccu ccugcugaag 480
   uacgaggccg aggagcccgu caccgaggcc gagaugcuca ugaucgugau caaguacaag 540
   gacuacuucc ccgugauccu gaagcgcgcc cgggaguuca uggagcugcu cuucggccug 600
   gcgcugaucg aggucgggcc cgaccacuuc ugcguguucg ccaacacggu gggccucacc 660
   gacgagggga gcgacgacga gggcaugccg gagaacuccc ugcugaucau cauccucagc 720
   gucaucuuca ucaagggcaa cugcgccucc gaggagguga ucugggaggu gcugaacgcc 780
   gucggggugu acgcgggccg cgagcacuuc guguacgggg agccccggga gcugcucacc 840
   aaggucuggg ugcagggcca cuaccuggag uaccgcgagg ugccgcacag cucccccccg 900
   uacuacgagu uccugugggg cccccgggcc cacagcgagu ccaucaagaa gaagguccuc 960
   gaguuccugg ccaagcugaa caacaccgug cccagcagcu uccccuccug guacaaggac 1020
   gcccucaagg acgucgagga gcgcgugcag gccacgaucg acaccgcgga cgacgccacc 1080
   gugauggcca gcgagucccu gagcgucaug uccagcaacg uguccuucag cgagugacca 1140
   cuaguuauaa gacugacuag cccgaugggc cucccaacgg gcccuccucc ccuccuugca 1200
   ccgagauuaa uaaaaaaaaa aaaaaaaaaa aaaaaaaaaa aaaaaaaaaa aaaaaaaaaa 1260
   aaaaaaaaaa aaaaauauuc cccccccccc cccccccccc cccccccccu cuagacaauu 1320
   ggaauu 1326
<210> 14
   <211> 1122
   <212> RNA
   <213> Artificial Sequence
<220>
   <223> MAGE-C2 CDS wild type
<400> 14
   augccucccg uuccaggcgu uccauuccgc aacguugaca acgacucccc gaccucaguu     60
   gaguuagaag acuggguaga ugcacagcau cccacagaug aggaagagga ggaagccucc    120
   uccgccucuu ccacuuugua cuuaguauuu ucccccucuu cuuucuccac auccucuucu    180
   cugauucuug gugguccuga ggaggaggag gugcccucug gugugauacc aaaucuuacc    240
   gagagcauuc ccaguagucc uccacagggu ccuccacagg guccuuccca gaguccucug    300
   agcuccugcu gcuccucuuu uucauggagc ucauucagug aggaguccag cagccagaaa    360
   ggggaggaua caggcaccug ucagggccug ccagacagug aguccucuuu cacauauaca    420
   cuagaugaaa agguggccga guuaguggag uuccugcucc ucaaauacga agcagaggag    480
   ccuguaacag aggcagagau gcugaugauu gucaucaagu acaaagauua cuuuccugug    540
   auacucaaga gagcccguga guucauggag cuucuuuuug gccuugcccu gauagaagug    600
   ggcccugacc acuucugugu guuugcaaac acaguaggcc ucaccgauga ggguagugau    660
   gaugagggca ugcccgagaa cagccuccug auuauuauuc ugagugugau cuucauaaag    720
   ggcaacugug ccucugagga ggucaucugg gaagugcuga augcaguagg gguauaugcu    780
   gggagggagc acuucgucua uggggagccu agggagcucc ucacuaaagu uugggugcag    840
   ggacauuacc uggaguaucg ggaggugccc cacaguucuc cuccauauua ugaauuccug    900
   ugggguccaa gagcccauuc agaaagcauc aagaagaaag uacuagaguu uuuagccaag    960
   cugaacaaca cuguuccuag uuccuuucca uccugguaca aggaugcuuu gaaagaugug   1020
   gaagagagag uccaggccac aauugauacc gcagaugaug ccacugucau ggccagugaa   1080
   agccucagug ucauguccag caacgucucc uuuucugagu ga                      1122
<210> 15
   <211> 1122
   <212> RNA
   <213> Artificial Sequence
<220>
   <223> MAGE-C2 CDS GC-optimized
<400> 15
   augcccccgg ugcccggcgu ccccuuccgg aacguggaca acgacagccc caccuccgug      60
   gagcuggagg acugggucga cgcccagcac ccgaccgacg aggaggagga ggaggccagc     120
   uccgcgagcu ccacgcucua ccugguguuc agccccucca gcuucuccac cagcuccagc     180
   cugauccucg ggggccccga ggaggaggag gugcccuccg gggucauccc gaaccugacc     240
   gagagcaucc ccuccagccc cccgcagggc ccgccccagg ggcccuccca gagcccccug     300
   uccagcugcu gcagcuccuu cagcuggucc agcuucuccg aggagagcuc cagccagaag     360
   ggcgaggaca ccggcacgug ccaggggcuc ccggacuccg agagcuccuu caccuacacc     420
   cuggacgaga agguggccga gcugguggag uuccuccugc ugaaguacga ggccgaggag     480
   cccgucaccg aggccgagau gcucaugauc gugaucaagu acaaggacua cuuccccgug     540
   auccugaagc gcgcccggga guucauggag cugcucuucg gccuggcgcu gaucgagguc     600
   gggcccgacc acuucugcgu guucgccaac acggugggcc ucaccgacga ggggagcgac     660
   gacgagggca ugccggagaa cucccugcug aucaucaucc ucagcgucau cuucaucaag     720
   ggcaacugcg ccuccgagga ggugaucugg gaggugcuga acgccgucgg gguguacgcg     780
   ggccgcgagc acuucgugua cggggagccc cgggagcugc ucaccaaggu cugggugcag     840
   ggccacuacc uggaguaccg cgaggugccg cacagcuccc ccccguacua cgaguuccug     900
   uggggccccc gggcccacag cgaguccauc aagaagaagg uccucgaguu ccuggccaag     960
   cugaacaaca ccgugcccag cagcuucccc uccugguaca aggacgcccu caaggacguc    1020
   gaggagcgcg ugcaggccac gaucgacacc gcggacgacg ccaccgugau ggccagcgag    1080
   ucccugagcg ucauguccag caacgugucc uucagcgagu ga                       1122
<210> 16
   <211> 1860
   <212> RNA
   <213> Artificial Sequence
<220>
   <223> MUC1 5 VNTR (GC)-muag-A64-C30
<400> 16
   gggagaaagc uuaccaugac ccccggcacc cagagcccgu ucuuccugcu ccugcugcuc 60
   acggugcuga ccgucgugac cggguccggc cacgccagcu ccacccccgg gggcgagaag 120
   gagacgagcg ccacccagcg guccagcgug cccuccagca ccgagaagaa cgcggucucc 180
   augaccagcu ccgugcugag cucccacagc cccggguccg gcagcuccac gacccagggc 240
   caggacguga cccucgcccc ggccaccgag cccgccagcg gguccgccgc gacguggggc 300
   caggacguca ccagcgugcc cgugacccgc cccgcccugg ggagcaccac gccgcccgcc 360
   cacgacguca ccuccgcccc cgacaacaag cccgcgccgg gcagcaccgc cccccccgcc 420
   cacgggguga ccuccgcccc cgacacgcgg ccggcccccg gcagcaccgc gccccccgcc 480
   cacggcguga ccuccgcccc ggacacccgc cccgcccccg ggagcacggc cccgccggcg 540
   cacggcguca ccuccgcccc cgacacccgg cccgcccccg ggagcaccgc cccgcccgcc 600
   cacggcguga cguccgcgcc cgacacccgc ccggcccccg gcagcaccgc cccccccgcc 660
   cacgggguga ccuccgcccc ggacacgcgg cccgcgcccg gcagcaccgc cccgccggcc 720
   cacgggguca ccuccgcgcc cgacaaccgc cccgcccugg ggagcaccgc cccgcccgug 780
   cacaacguga ccuccgccag cggcuccgcg agcggguccg ccagcacccu cguccacaac 840
   ggcacguccg cccgggccac caccaccccc gccagcaagu ccacgcccuu cagcaucccg 900
   ucccaccaca gcgacacccc caccacccug gcgucccaca gcacgaagac cgacgccucc 960
   agcacccacc acuccagcgu gcccccgcug accagcucca accacagcac guccccgcag 1020
   cucagcaccg ggguguccuu cuucuuccug agcuuccaca ucuccaaccu gcaguucaac 1080
   agcucccucg aggaccccag caccgacuac uaccaggagc ugcagcggga caucuccgag 1140
   auguuccugc agaucuacaa gcagggcggc uuccucgggc ugagcaacau caaguuccgc 1200
   cccggcuccg ucguggugca gcugacccuc gccuuccggg aggggacgau caacguccac 1260
   gacguggaga cccaguucaa ccaguacaag accgaggccg ccagccgcua caaccugacc 1320
   aucuccgacg ugagcgucuc cgacgugccc uucccguuca gcgcgcaguc cggcgccggc 1380
   gugcccgggu ggggcaucgc ccugcucguc cuggugugcg ugcuggucgc ccucgccauc 1440
   guguaccuga ucgcgcuggc cgugugccag ugccggcgca agaacuacgg gcagcucgac 1500
   aucuuccccg cccgggacac guaccacccg augagcgagu acccgaccua ccacacccac 1560
   ggccgcuacg ucccccccag cuccaccgac cggagccccu acgagaaggu guccgccggg 1620
   aacggcggca gcucccugag cuacaccaac ccggcggugg ccgccgccuc cgccaaccug 1680
   ugaccacuag uuauaagacu gacuagcccg augggccucc caacgggccc uccuccccuc 1740
   cuugcaccga gauuaauaaa aaaaaaaaaa aaaaaaaaaa aaaaaaaaaa aaaaaaaaaa 1800
   aaaaaaaaaa aaaaaaaaaa auauuccccc cccccccccc cccccccccc cccccucuag 1860
<210> 17
   <211> 1668
   <212> RNA
   <213> Artificial Sequence
<220>
   <223> MUC1 5 VNTR CDS wild type
<400> 17
   augacaccgg gcacccaguc uccuuucuuc cugcugcugc uccucacagu gcuuacaguu 60
   guuacagguu cuggucaugc aagcucuacc ccagguggag aaaaggagac uucggcuacc 120
   cagagaaguu cagugcccag cucuacugag aagaaugcug ugaguaugac cagcagcgua 180
   cucuccagcc acagccccgg uucaggcucc uccaccacuc agggacagga ugucacucug 240
   gccccggcca cggaaccagc uucagguuca gcugccaccu ggggacagga ugucaccucg 300
   gucccaguca ccaggccagc ccugggcucc accaccccgc cagcccacga ugucaccuca 360
   gccccggaca acaagccagc cccgggcucc accgcccccc cagcccacgg ugucaccucg 420
   gccccggaca ccaggccggc cccgggcucc accgcccccc cagcccacgg ugucaccucg 480
   gccccggaca ccaggccggc cccgggcucc accgcccccc cagcccacgg ugucaccucg 540
   gccccggaca ccaggccggc cccgggcucc accgcccccc cagcccacgg ugucaccucg 600
   gccccggaca ccaggccggc cccgggcucc accgcccccc cagcccacgg ugucaccucg 660
   gccccggaca ccaggccggc cccgggcucc accgcccccc cagcccacgg ugucaccucg 720
   gccccggaca acaggcccgc cuugggcucc accgccccuc caguccacaa ugucaccucg 780
   gccucaggcu cugcaucagg cucagcuucu acucuggugc acaacggcac cucugccagg 840
   gcuaccacaa ccccagccag caagagcacu ccauucucaa uucccagcca ccacucugau 900
   acuccuacca cccuugccag ccauagcacc aagacugaug ccaguagcac ucaccauagc     960
   ucgguaccuc cucucaccuc cuccaaucac agcacuucuc cccaguuguc uacugggguc   1020
   ucuuucuuuu uccugucuuu ucacauuuca aaccuccagu uuaauuccuc ucuggaagau    1080
   cccagcaccg acuacuacca agagcugcag agagacauuu cugaaauguu uuugcagauu    1140
   uauaaacaag gggguuuucu gggccucucc aauauuaagu ucaggccagg aucuguggug    1200
   guacaauuga cucuggccuu ccgagaaggu accaucaaug uccacgacgu ggagacacag    1260
   uucaaucagu auaaaacgga agcagccucu cgauauaacc ugacgaucuc agacgucagc    1320
   gugagugaug ugccauuucc uuucucugcc cagucugggg cuggggugcc aggcuggggc    1380
   aucgcgcugc uggugcuggu cuguguucug guugcgcugg ccauugucua ucucauugcc    1440
   uuggcugucu gucagugccg ccgaaagaac uacgggcagc uggacaucuu uccagcccgg    1500
   gauaccuacc auccuaugag cgaguacccc accuaccaca cccaugggcg cuaugugccc    1560
   ccuagcagua ccgaucguag ccccuaugag aagguuucug cagguaacgg uggcagcagc    1620
   cucucuuaca caaacccagc aguggcagcc gcuucugcca acuuguag                 1668
<210> 18
   <211> 1668
   <212> RNA
   <213> Artificial Sequence
<220>
   <223> MUC1 5 VNTR CDS GC-optimized
<400> 18
   augacccccg gcacccagag cccguucuuc cugcuccugc ugcucacggu gcugaccguc 60
   gugaccgggu ccggccacgc cagcuccacc cccgggggcg agaaggagac gagcgccacc 120
   cagcggucca gcgugcccuc cagcaccgag aagaacgcgg ucuccaugac cagcuccgug 180
   cugagcuccc acagccccgg guccggcagc uccacgaccc agggccagga cgugacccuc 240
   gccccggcca ccgagcccgc cagcgggucc gccgcgacgu ggggccagga cgucaccagc 300
   gugcccguga cccgccccgc ccuggggagc accacgccgc ccgcccacga cgucaccucc 360
   gcccccgaca acaagcccgc gccgggcagc accgcccccc ccgcccacgg ggugaccucc 420
   gcccccgaca cgcggccggc ccccggcagc accgcgcccc ccgcccacgg cgugaccucc 480
   gccccggaca cccgccccgc ccccgggagc acggccccgc cggcgcacgg cgucaccucc 540
   gcccccgaca cccggcccgc ccccgggagc accgccccgc ccgcccacgg cgugacgucc 600
   gcgcccgaca cccgcccggc ccccggcagc accgcccccc ccgcccacgg ggugaccucc 660
   gccccggaca cgcggcccgc gcccggcagc accgccccgc cggcccacgg ggucaccucc 720
   gcgcccgaca accgccccgc ccuggggagc accgccccgc ccgugcacaa cgugaccucc 780
   gccagcggcu ccgcgagcgg guccgccagc acccucgucc acaacggcac guccgcccgg 840
   gccaccacca cccccgccag caaguccacg cccuucagca ucccguccca ccacagcgac 900
   acccccacca cccuggcguc ccacagcacg aagaccgacg ccuccagcac ccaccacucc 960
   agcgugcccc cgcugaccag cuccaaccac agcacguccc cgcagcucag caccggggug 1020
   uccuucuucu uccugagcuu ccacaucucc aaccugcagu ucaacagcuc ccucgaggac 1080
   cccagcaccg acuacuacca ggagcugcag cgggacaucu ccgagauguu ccugcagauc 1140
   uacaagcagg gcggcuuccu cgggcugagc aacaucaagu uccgccccgg cuccgucgug 1200
   gugcagcuga cccucgccuu ccgggagggg acgaucaacg uccacgacgu ggagacccag 1260
   uucaaccagu acaagaccga ggccgccagc cgcuacaacc ugaccaucuc cgacgugagc 1320
   gucuccgacg ugcccuuccc guucagcgcg caguccggcg ccggcgugcc cgggnggggc 1380
   aucgcccugc ucguccuggu gugcgugcug gucgcccucg ccaucgugua ccugaucgcg 1440
   cuggccgugu gccagugccg gcgcaagaac uacgggcagc ucgacaucuu ccccgcccgg 1500
   gacacguacc acccgaugag cgaguacccg accuaccaca cccacggccg cuacgucccc 1560
   cccagcucca ccgaccggag ccccuacgag aaggugnccg ccgggaacgg cggcagcucc 1620
   cugagcuaca ccaacccggc gguggccgcc gccuccgcca accuguga 1668
<210> 19
   <211> 1480
   <212> RNA
   <213> Artificial Sequence
<220>
   <223> 5T4 (GC)-muag-A64-C30-HistoneSL
<400> 19
   gggagaaagc uuaccaugcc cggcgggugc agccggggcc cggccgccgg ggacggccgc      60
   cugcggcucg cgcgccuggc ccuggugcuc cugggguggg ucuccagcuc cagccccacc      120
   uccagcgccu ccagcuucuc cagcuccgcc cccuuccugg ccagcgcggu guccgcccag     180
   cccccgcucc ccgaccagug ccccgcccug ugcgagugca gcgaggccgc gcggaccgug     240
   aagugcguca accgcaaccu gacggaggug cccaccgacc ucccggccua cgugcggaac     300
   cuguuccuga ccggcaacca gcucgccguc cugcccgccg gcgccuucgc gcgccggccg     360
   ccccuggccg agcucgccgc ccugaaccug uccgggagcc gccucgacga ggugcgggcc     420
   ggcgcguucg agcaccugcc gucccugcgc cagcucgacc ugagccacaa cccccuggcc     480
   gaccucuccc ccuucgccuu cagcgggagc aacgccuccg ugagcgcccc cuccccgcug     540
   gucgagcuga uccucaacca caucgugccc cccgaggacg agcggcagaa ccgcagcuuc     600
   gagggcaugg uggucgcggc ccugcuggcc gggcgggccc uccagggccu gcgccggcug     660
   gagcucgccu ccaaccacuu ccuguaccug ccccgcgacg ugcucgcgca gcugccgagc     720
   cugcggcacc ucgaccuguc caacaacagc cuggaguccc ucaccuacgu cagcuuccgc     780
   aaccugacgc accuggaguc ccuccaccug gaggacaacg cccugaaggu gcugcacaac     840
   ggcacccucg ccgagcugca ggggcugccc cacauccggg uguuccucga caacaacccc     900
   ugggucugcg acugccacau ggccgacaug gugaccuggc ugaaggagac cgaggugguc     960
   cagggcaagg accgccugac gugcgcguac cccgagaaga ugcggaaccg ggugcuccug    1020
   gagcugaaca gcgccgaccu cgacugcgac ccgauccugc cccccucccu gcagaccagc    1080
   uacguguucc ucgggaucgu ccuggcccug aucggcgcca ucuuccuccu ggugcuguac    1140
   cucaaccgca agggcaucaa gaaguggaug cacaacaucc gggacgccug ccgcgaccac    1200
   auggaggggu accacuaccg guacgagauc aacgcggacc cccgccugac caaccugucc    1260
   agcaacuccg acgucugacc acuaguuaua agacugacua gcccgauggg ccucccaacg    1320
   ggcccuccuc cccuccuugc accgagauua auaaaaaaaa aaaaaaaaaa aaaaaaaaaa    1380
   aaaaaaaaaa aaaaaaaaaa aaaaaaaaaa aaaaaaugca uccccccccc cccccccccc    1440
   cccccccccc ccaaaggcuc uuuucagagc caccagaauu                          1480
<210> 20
   <211> 646
   <212> RNA
   <213> Artificial Sequence
<220>
   <223> Survivin (GC)-muag-A64-C30-HistoneSL
<400> 20
   gggagaaagc uuaccauggg cgcccccacc cugccgccgg ccuggcagcc guuccucaag 60
   gaccaccgca ucucgaccuu caagaacugg ccguuccugg agggcugcgc gugcaccccg 120
   gagcggaugg ccgaggccgg cuucauccac ugccccaccg agaacgagcc ggaccuggcc 180
   cagugcuucu ucugcuucaa ggagcuggag ggcugggagc cggacgacga cccgaucgag 240
   gagcacaaga agcacagcag cggcugcgcc uuccugagcg ugaagaagca guucgaggag 300
   cugacgcucg gggaguuccu gaagcuggac cgggagcggg ccaagaacaa gaucgcgaag 360
   gagaccaaca acaagaagaa ggaguucgag gagaccgcca agaaggugcg gcgggccauc 420
   gagcagcugg ccgccaugga cugaccacua guuauaagac ugacuagccc gaugggccuc 480
   ccaacgggcc cuccuccccu ccuugcaccg agauuaauaa aaaaaaaaaa aaaaaaaaaa 540
   aaaaaaaaaa aaaaaaaaaa aaaaaaaaaa aaaaaaaaaa aaugcauccc cccccccccc 600
   cccccccccc ccccccccaa aggcucuuuu cagagccacc agaauu 646
<210> 21
   <211> 760
   <212> RNA
   <213> Artificial Sequence
<220>
   <223> NY-ESO-1 (GC)-muag-A64-C30-histone SL
<400> 21
   gggagaaagc uuaccaugca ggccgagggc cgcggcaccg gcggcucgac cggcgacgcc 60
   gacgggcccg gcggcccggg caucccggac ggcccgggcg ggaacgcggg cggcccgggc 120
   gaggccggcg ccaccggcgg gcggggcccg cggggcgccg gcgccgcccg ggcgagcggc 180
   cccggcgggg gcgccccgcg gggcccgcac ggcggcgccg ccagcggccu gaacgggugc 240
   ugccggugcg gcgcccgcgg cccggagagc cggcuccugg aguucuaccu ggccaugccg 300
   uucgcgaccc cgauggaggc cgagcuggcc cggcggagcc uggcccagga cgccccgccg 360
   cugcccgugc cgggcgugcu ccugaaggag uucacgguga gcggcaacau ccugaccauc 420
   cggcugaccg ccgcggacca ccggcagcug cagcugucga ucagcagcug ccuccagcag 480
   cugagccugc ugauguggau cacccagugc uuccugccgg uguuccuggc ccagccgccc 540
   agcggccagc gccggugacc acuaguuaua agacugacua gcccgauggg ccucccaacg 600
   ggcccuccuc cccuccuugc accgagauua auaaaaaaaa aaaaaaaaaa aaaaaaaaaa 660
   aaaaaaaaaa aaaaaaaaaa aaaaaaaaaa aaaaaaugca uccccccccc cccccccccc 720
   cccccccccc ccaaaggcuc uuuucagagc caccagaauu 760
<210> 22
   <211> 1813
   <212> RNA
   <213> Artificial Sequence
<220>
   <223> MAGE-C1 (aa 613-1142) (GC)-muag-A64-C30-histoneSL
<400> 22
   gggagaaagc uuaccaugca guccccgcug cagggcgagg aguuccagag cucccugcag 60
   agccccgugu ccaucugcag cuccagcacc cccuccagcc ucccgcagag cuuccccgag 120
   uccagccagu ccccccccga gggcccgguc cagagccccc ugcacucccc gcagagcccc 180
   ccggagggga ugcacuccca gagcccccug cagucccccg agagcgcccc cgagggcgag 240
   gacucccuca gcccgcugca gaucccccag uccccgcugg agggggagga cagccucucc 300
   agccugcacu ucccccaguc cccgcccgag ugggaggaca gccugagccc ccuccacuuc 360
   ccccaguucc cgccccaggg cgaggacuuc caguccagcc ugcagucccc cgugagcauc 420
   ugcuccagcu ccacgagccu gucccucccc cagagcuucc cggagucccc ccagagcccg 480
   cccgaggggc cggcgcaguc cccccugcag cgccccguga gcuccuucuu cagcuacacc 540
   cuggccuccc uccugcagag cucccacgag agcccgcaga gcccgcccga gggccccgcc 600
   caguccccgc ugcagagccc cgucuccagc uuccccucca gcaccuccag cucccucagc 660
   caguccagcc ccguguccag cuucccgucc agcaccucca gcucccugag caagagcucc 720
   cccgagagcc cccugcaguc ccccgugauc agcuucucca gcuccacgag ccucuccccg 780
   uucagcgagg aguccagcuc ccccgucgac gaguacacca gcuccagcga cacccugcug 840
   gaguccgaca gccucaccga cuccgagagc cugaucgaga gcgagccccu guucaccuac 900
   acgcucgacg agaaggugga cgagcuggcc cgguuccugc uccugaagua ccaggugaag 960
   cagcccauca ccaaggccga gaugcugacc aacgucaucu cccgcuacac cggcuacuuc 1020
   ccggugaucu uccggaaggc gcgcgaguuc aucgagaucc ucuucgggau cagccugcgg 1080
   gagguggacc ccgacgacuc cuacgucuuc gugaacacgc uggaccucac cagcgagggc 1140
   ugccuguccg acgagcaggg gaugagccag aaccgccugc ucauccugau ccuguccauc 1200
   aucuucauca agggcaccua cgccagcgag gaggucaucu gggacgugcu cuccgggauc 1260
   ggcgugcggg ccggccgcga gcacuucgcc uucggggagc cccgggagcu gcugaccaag 1320
   gucugggugc aggagcacua ccucgaguac cgcgaggugc ccaacagcuc cccgccccgg 1380
   uacgaguucc uguggggccc ccgcgcccac agcgagguca ucaagcggaa ggugguggag 1440
   uuccuggcga ugcucaagaa cacggucccc aucaccuucc cguccagcua caaggacgcc 1500
   cugaaggacg uggaggagcg ggcccaggcc aucaucgaca ccaccgacga cuccacggcc 1560
   accgagagcg cguccagcuc cgugaugagc cccagcuucu ccagcgagug accacuaguu 1620
   auaagacuga cuagcccgau gggccuccca acgggcccuc cuccccuccu ugcaccgaga 1680
   uuaauaaaaa aaaaaaaaaa aaaaaaaaaa aaaaaaaaaa aaaaaaaaaa aaaaaaaaaa 1740
   aaaaaaaaau gcaucccccc cccccccccc cccccccccc cccccaaagg cucuuuucag 1800
   agccaccaga auu 1813
<210> 23
   <211> 1339
   <212> RNA
   <213> Artificial Sequence
<220>
   <223> MAGE-C2 (GC)-muag-A64-C30-histoneSL
<400> 23
   gggagaaagc uuaccaugcc cccggugccc ggcguccccu uccggaacgu ggacaacgac      60
   agccccaccu ccguggagcu ggaggacugg gucgacgccc agcacccgac cgacgaggag     120
   gaggaggagg ccagcuccgc gagcuccacg cucuaccugg uguucagccc cuccagcuuc     180
   uccaccagcu ccagccugau ccucgggggc cccgaggagg aggaggugcc cuccgggguc    240
   aucccgaacc ugaccgagag cauccccucc agccccccgc agggcccgcc ccaggggccc      300
   ucccagagcc cccuguccag cugcugcagc uccuucagcu gguccagcuu cuccgaggag     360
   agcuccagcc agaagggcga ggacaccggc acgugccagg ggcucccgga cuccgagagc     420
   uccuucaccu acacccugga cgagaaggug gccgagcugg uggaguuccu ccugcugaag     480
   uacgaggccg aggagcccgu caccgaggcc gagaugcuca ugaucgugau caaguacaag     540
   gacuacuucc ccgugauccu gaagcgcgcc cgggaguuca uggagcugcu cuucggccug     600
   gcgcugaucg aggucgggcc cgaccacuuc ugcguguucg ccaacacggu gggccucacc     660
   gacgagggga gcgacgacga gggcaugccg gagaacuccc ugcugaucau cauccucagc     720
   gucaucuuca ucaagggcaa cugcgccucc gaggagguga ucugggaggu gcugaacgcc     780
   gucggggugu acgcgggccg cgagcacuuc guguacgggg agccccggga gcugcucacc     840
   aaggucuggg ugcagggcca cuaccuggag uaccgcgagg ugccgcacag cucccccccg      900
   uacuacgagu uccugugggg cccccgggcc cacagcgagu ccaucaagaa gaagguccuc     960
   gaguuccugg ccaagcugaa caacaccgug cccagcagcu uccccuccug guacaaggac    1020
   gcccucaagg acgucgagga gcgcgugcag gccacgaucg acaccgcgga cgacgccacc    1080
   gugauggcca gcgagucccu gagcgucaug uccagcaacg uguccuucag cgagugacca    1140
   cuaguuauaa gacugacuag cccgaugggc cucccaacgg gcccuccucc ccuccuugca    1200
   ccgagauuaa uaaaaaaaaa aaaaaaaaaa aaaaaaaaaa aaaaaaaaaa aaaaaaaaaa    1260
   aaaaaaaaaa aaaaaugcau cccccccccc cccccccccc cccccccccc caaaggcucu    1320
   uuucagagcc accagaauu                                                 1339
<210> 24
   <211> 1885
   <212> RNA
   <213> Artificial Sequence
<220>
   <223> MUC1 5 VNTR (GC)-muag-A64-C30-histoneSL
<400> 24
   gggagaaagc uuaccaugac ccccggcacc cagagcccgu ucuuccugcu ccugcugcuc        60
   acggugcuga ccgucgugac cggguccggc cacgccagcu ccacccccgg gggcgagaag       120
   gagacgagcg ccacccagcg guccagcgug cccuccagca ccgagaagaa cgcggucucc       180
   augaccagcu ccgugcugag cucccacagc cccggguccg gcagcuccac gacccagggc       240
   caggacguga cccucgcccc ggccaccgag cccgccagcg gguccgccgc gacguggggc       300
   caggacguca ccagcgugcc cgugacccgc cccgcccugg ggagcaccac gccgcccgcc       360
   cacgacguca ccuccgcccc cgacaacaag cccgcgccgg gcagcaccgc cccccccgcc        420
   cacgggguga ccuccgcccc cgacacgcgg ccggcccccg gcagcaccgc gccccccgcc        480
   cacggcguga ccuccgcccc ggacacccgc cccgcccccg ggagcacggc cccgccggcg       540
   cacggcguca ccuccgcccc cgacacccgg cccgcccccg ggagcaccgc cccgcccgcc       600
   cacggcguga cguccgcgcc cgacacccgc ccggcccccg gcagcaccgc cccccccgcc        660
   cacgggguga ccuccgcccc ggacacgcgg cccgcgcccg gcagcaccgc cccgccggcc        720
   cacgggguca ccuccgcgcc cgacaaccgc cccgcccugg ggagcaccgc cccgcccgug        780
   cacaacguga ccuccgccag cggcuccgcg agcggguccg ccagcacccu cguccacaac        840
   ggcacguccg cccgggccac caccaccccc gccagcaagu ccacgcccuu cagcaucccg        900
   ucccaccaca gcgacacccc caccacccug gcgucccaca gcacgaagac cgacgccucc        960
   agcacccacc acuccagcgu gcccccgcug accagcucca accacagcac guccccgcag       1020
   cucagcaccg ggguguccuu cuucuuccug agcuuccaca ucuccaaccu gcaguucaac       1080
   agcucccucg aggaccccag caccgacuac uaccaggagc ugcagcggga caucuccgag       1140
   auguuccugc agaucuacaa gcagggcggc uuccucgggc ugagcaacau caaguuccgc       1200
   cccggcuccg ucguggugca gcugacccuc gccuuccggg aggggacgau caacguccac       1260
   gacguggaga cccaguucaa ccaguacaag accgaggccg ccagccgcua caaccugacc       1320
   aucuccgacg ugagcgucuc cgacgugccc uucccguuca gcgcgcaguc cggcgccggc       1380
   gugcccgggu ggggcaucgc ccugcucguc cuggugugcg ugcuggucgc ccucgccauc       1440
   guguaccuga ucgcgcuggc cgugugccag ugccggcgca agaacuacgg gcagcucgac       1500
   aucuuccccg cccgggacac guaccacccg augagcgagu acccgaccua ccacacccac       1560
   ggccgcuacg ucccccccag cuccaccgac cggagccccu acgagaaggu guccgccggg       1620
   aacggcggca gcucccugag cuacaccaac ccggcggugg ccgccgccuc cgccaaccug       1680
   ugaccacuag uuauaagacu gacuagcccg augggccucc caacgggccc uccuccccuc       1740
   cuugcaccga gauuaauaaa aaaaaaaaaa aaaaaaaaaa aaaaaaaaaa aaaaaaaaaa       1800
   aaaaaaaaaa aaaaaaaaaa augcaucccc cccccccccc cccccccccc cccccccaaa       1860
   ggcucuuuuc agagccacca gaauu                                              1885
<210> 25
   <211> 1596
   <212> RNA
   <213> Artificial Sequence
<220>
   <223> MAGE-C1 (aa 613-1142) CDS GC-optimized
<400> 25
   augcaguccc cgcugcaggg cgaggaguuc cagagcuccc ugcagagccc cguguccauc      60
   ugcagcucca gcacccccuc cagccucccg cagagcuucc ccgaguccag ccaguccccc     120
   cccgagggcc cgguccagag cccccugcac uccccgcaga gccccccgga ggggaugcac    180
   ucccagagcc cccugcaguc ccccgagagc gcccccgagg gcgaggacuc ccucagcccg     240
   cugcagaucc cccagucccc gcuggagggg gaggacagcc ucuccagccu gcacuucccc     300
   caguccccgc ccgaguggga ggacagccug agcccccucc acuuccccca guucccgccc     360
   cagggcgagg acuuccaguc cagccugcag ucccccguga gcaucugcuc cagcuccacg     420
   agccuguccc ucccccagag cuucccggag uccccccaga gcccgcccga ggggccggcg     480
   cagucccccc ugcagcgccc cgugagcucc uucuucagcu acacccuggc cucccuccug     540
   cagagcuccc acgagagccc gcagagcccg cccgagggcc ccgcccaguc cccgcugcag     600
   agccccgugu ccagcuuccc cuccagcacc uccagcuccc ucagccaguc cagccccgug     660
   uccagcuucc cguccagcac cuccagcucc cugagcaaga gcucccccga gagcccccug      720
   cagucccccg ugaucagcuu cuccagcucc acgagccucu ccccguucag cgaggagucc      780
   agcucccccg ucgacgagua caccagcucc agcgacaccc ugcuggaguc cgacagccuc      840
   accgacuccg agagccugau cgagagcgag ccccuguuca ccuacacgcu cgacgagaag     900
   guggacgagc uggcccgguu ccugcuccug aaguaccagg ugaagcagcc caucaccaag     960
   gccgagaugc ugaccaacgu caucucccgc uacaccggcu acuucccggu gaucuuccgg    1020
   aaggcgcgcg aguucaucga gauccucuuc gggaucagcc ugcgggaggu ggaccccgac    1080
   gacuccuacg ucuucgugaa cacgcuggac cucaccagcg agggcugccu guccgacgag    1140
   caggggauga gccagaaccg ccugcucauc cugauccugu ccaucaucuu caucaagggc    1200
   accuacgcca gcgaggaggu caucugggac gugcucuccg ggaucggcgu gcgggccggc    1260
   cgcgagcacu ucgccuucgg ggagccccgg gagcugcuga ccaaggucug ggugcaggag    1320
   cacuaccucg aguaccgcga ggugcccaac agcuccccgc cccgguacga guuccugugg    1380
   ggcccccgcg cccacagcga ggucaucaag cggaaggugg uggaguuccu ggcgaugcuc    1440
   aagaacacgg uccccaucac cuucccgucc agcuacaagg acgcccugaa ggacguggag    1500
   gagcgggccc aggccaucau cgacaccacc gacgacucca cggccaccga gagcgcgucc    1560
   agcuccguga ugagccccag cuucuccagc gaguga                              1596
<210> 26
   <211> 16
   <212> RNA
   <213> Artificial Sequence
<220>
   <223> Sequence according to formula (Ic)
<220>
   <221> misc_feature
   <222> (1)..(1)
   <223> n is a, u, t, g, and c, or a nucleotide analogue thereof
<220>
   <221> misc_feature
   <222> (3)..(8)
   <223> n is a, u, t, g, and c, or a nucleotide analogue thereof
<220>
   <221> misc_feature
   <222> (10)..(14)
   <223> n is a, u, t, g, and c, or a nucleotide analogue thereof
<220>
   <221> misc_feature
   <222> (16)..(16)
   <223> n is a, u, t, g, and c, or a nucleotide analogue thereof
<400> 26
   ngnnnnnnun nnnncn 16
<210> 27
   <211> 26
   <212> RNA
   <213> Artificial Sequence
<220>
   <223> Sequence according to formula (IIc)
<220>
   <221> misc_feature
   <222> (1)..(2)
   <223> n is a, u, t, g, and c, or a nucleotide analogue thereof; may be present or not
<220>
   <221> misc_feature
   <222> (3)..(6)
   <223> n is a, u, t, g, and c, or a nucleotide analogue thereof
<220>
   <221> misc_feature
   <222> (8)..(13)
   <223> n is a, u, t, g, and c, or a nucleotide analogue thereof
<220>
   <221> misc_feature
   <222> (15)..(19)
   <223> n is a, u, t, g, and c, or a nucleotide analogue thereof
<220>
   <221> misc_feature
   <222> (21)..(24)
   <223> n is a, u, t, g, and c, or a nucleotide analogue thereof
<220>
   <221> misc_feature
   <222> (25)..(26)
   <223> n is a, u, t, g, and c, or a nucleotide analogue thereof; may be present or not.
<400> 27
   nnnnnngnnn nnnunnnnnc nnnnnn 26
<210> 28
   <211> 16
   <212> RNA
   <213> Artificial Sequence
<220>
   <223> Sequence according to formula (Id)
<220>
   <221> misc_feature
   <222> (1)..(1)
   <223> n is a, u, t, g, and c, or a nucleotide analogue thereof
<220>
   <221> misc_feature
   <222> (3)..(8)
   <223> n is a, u, t, g, and c, or a nucleotide analogue thereof
<220>
   <221> misc_feature
   <222> (10)..(14)
   <223> n is a, u, t, g, and c, or a nucleotide analogue thereof
<220>
   <221> misc_feature
   <222> (16)..(16)
   <223> n is a, u, t, g, and c, or a nucleotide analogue thereof
<400> 28
   ncnnnnnnun nnnngn 16
<210> 29
   <211> 26
   <212> RNA
   <213> Artificial Sequence
<220>
   <223> Sequence according to formula (IId)
<220>
   <221> misc_feature
   <222> (1)..(2)
   <223> n is a, u, t, g, and c, or a nucleotide analogue thereof; may be present or not.
<220>
   <221> misc_feature
   <222> (3)..(6)
   <223> n is a, u, t, g, and c, or a nucleotide analogue thereof
<220>
   <221> misc_feature
   <222> (8)..(13)
   <223> n is a, u, t, g, and c, or a nucleotide analogue thereof
<220>
   <221> misc_feature
   <222> (15)..(19)
   <223> n is a, u, t, g, and c, or a nucleotide analogue thereof
<220>
   <221> misc_feature
   <222> (21)..(23)
   <223> n is a, u, t, g, and c, or a nucleotide analogue thereof
<220>
   <221> misc_feature
   <222> (24)..(26)
   <223> n is a, u, t, g, and c, or a nucleotide analogue thereof; may be present or not
<400> 29
   nnnnnncnnn nnnunnnnng nnnnnn 26
<210> 30
   <211> 16
   <212> RNA
   <213> Artificial Sequence
<220>
   <223> Sequence according to formula (Ie)
<220>
   <221> misc_feature
   <222> (3)..(8)
   <223> n is a, u, t, g, and c, or a nucleotide analogue thereof
<220>
   <221> misc_feature
   <222> (10)..(14)
   <223> n is a, u, t, g, and c, or a nucleotide analogue thereof
<400> 30
   dgnnnnnnun nnnnch 16
<210> 31
   <211> 26
   <212> RNA
   <213> Artificial Sequence
<220>
   <223> Sequence according to formula (IIe)
<220>
   <221> misc_feature
   <222> (1)..(2)
   <223> n is a, u, t, g, and c, or a nucleotide analogue thereof; may be present or not
<220>
   <221> misc_feature
   <222> (3)..(5)
   <223> n is a, u, t, g, and c, or a nucleotide analogue thereof
<220>
   <221> misc_feature
   <222> (8)..(13)
   <223> n is a, u, t, g, and c, or a nucleotide analogue thereof
<220>
   <221> misc_feature
   <222> (15)..(19)
   <223> n is a, u, t, g, and c, or a nucleotide analogue thereof
<220>
   <221> misc_feature
   <222> (22)..(23)
   <223> n is a, u, t, g, and c, or a nucleotide analogue thereof
<220>
   <221> misc_feature
   <222> (24)..(26)
   <223> n is a, u, t, g, and c, or a nucleotide analogue thereof; may be present or not
<400> 31
   nnnnndgnnn nnnunnnnnc hnnnnn 26
<210> 32
   <211> 16
   <212> RNA
   <213> Artificial Sequence
<220>
   <223> Sequence according to formula (If)
<220>
   <221> misc_feature
   <222> (1)..(1)
   <223> n is a, u, t, g, and c, or a nucleotide analogue thereof
<220>
   <221> misc_feature
   <222> (3)..(3)
   <223> n is a, u, t, g, and c, or a nucleotide analogue thereof
<220>
   <221> misc_feature
   <222> (7)..(8)
   <223> n is a, u, t, g, and c, or a nucleotide analogue thereof
<220>
   <221> misc_feature
   <222> (10)..(10)
   <223> n is a, u, t, g, and c, or a nucleotide analogue thereof
<220>
   <221> misc_feature
   <222> (12)..(12)
   <223> n is a, u, t, g, and c, or a nucleotide analogue thereof
<220>
   <221> misc_feature
   <222> (14)..(14)
   <223> n is a, u, t, g, and c, or a nucleotide analogue thereof
<220>
   <221> misc_feature
   <222> (16)..(16)
   <223> n is a, u, t, g, and c, or a nucleotide analogue thereof
<400> 32
   ngnbyynnun vndncn 16
<210> 33
   <211> 26
   <212> RNA
   <213> Artificial Sequence
<220>
   <223> Sequence according to formula (IIf)
<220>
   <221> misc_feature
   <222> (1)..(2)
   <223> n is a, u, t, g, and c, or a nucleotide analogue thereof; may be present or not
<220>
   <221> misc_feature
   <222> (3)..(6)
   <223> n is a, u, t, g, and c, or a nucleotide analogue thereof
<220>
   <221> misc_feature
   <222> (8)..(8)
   <223> n is a, u, t, g, and c, or a nucleotide analogue thereof
<220>
   <221> misc_feature
   <222> (12)..(13)
   <223> n is a, u, t, g, and c, or a nucleotide analogue thereof
<220>
   <221> misc_feature
   <222> (15)..(15)
   <223> n is a, u, t, g, and c, or a nucleotide analogue thereof
<220>
   <221> misc_feature
   <222> (17)..(17)
   <223> n is a, u, t, g, and c, or a nucleotide analogue thereof
<220>
   <221> misc_feature
   <222> (19)..(19)
   <223> n is a, u, t, g, and c, or a nucleotide analogue thereof
<220>
   <221> misc_feature
   <222> (21)..(23)
   <223> n is a, u, t, g, and c, or a nucleotide analogue thereof
<220>
   <221> misc_feature
   <222> (24)..(26)
   <223> n is a, u, t, g, and c, or a nucleotide analogue thereof; may be present or not
<400> 33
   nnnnnngnby ynnunvndnc nnnnnn 26
<210> 34
   <211> 16
   <212> RNA
   <213> Artificial Sequence
<220>
   <223> Sequence according to formula (Ig)
<220>
   <221> misc_feature
   <222> (1)..(1)
   <223> n is a, u, t, g, and c, or a nucleotide analogue thereof
<220>
   <221> misc_feature
   <222> (8)..(8)
   <223> n is a, u, t, g, and c, or a nucleotide analogue thereof
<220>
   <221> misc_feature
   <222> (16)..(16)
   <223> n is a, u, t, g, and c, or a nucleotide analogue thereof
<400> 34
   nghyyydnuh abrdcn 16
<210> 35
   <211> 26
   <212> RNA
   <213> Artificial Sequence
<220>
   <223> Sequence according to formula (IIg)
<220>
   <221> misc_feature
   <222> (1)..(2)
   <223> n is a, u, t, g, and c, or a nucleotide analogue thereof; may be present or not
<220>
   <221> misc_feature
   <222> (4)..(6)
   <223> n is a, u, t, g, and c, or a nucleotide analogue thereof
<220>
   <221> misc_feature
   <222> (13)..(13)
   <223> n is a, u, t, g, and c, or a nucleotide analogue thereof
<220>
   <221> misc_feature
   <222> (21)..(23)
   <223> n is a, u, t, g, and c, or a nucleotide analogue thereof
<220>
   <221> misc_feature
   <222> (24)..(25)
   <223> n is a, u, t, g, and c, or a nucleotide analogue thereof; may be present or not
<220>
   <221> misc_feature
   <222> (26).. (26)
   <223> may be present or not
<400> 35
   nnhnnnghyy ydnuhabrdc nnnnnh 26
<210> 36
   <211> 16
   <212> RNA
   <213> Artificial Sequence
<220>
   <223> Sequence according to formula (Ih)
<400> 36
   dghycudyuh asrrcc 16
<210> 37
   <211> 26
   <212> RNA
   <213> Artificial Sequence
<220>
   <223> Sequence according to formula (IIh)
<220>
   <221> misc_feature
   <222> (1)..(1)
   <223> n is a, u, t, g, and c, or a nucleotide analogue thereof; may be present or not
<220>
   <221> misc_feature
   <222> (2)..(2)
   <223> may be present or not
<220>
   <221> misc_feature
   <222> (25)..(25)
   <223> n is a, u, t, g, and c, or a nucleotide analogue thereof; may be present or not
<220>
   <221> misc_feature
   <222> (26)..(26)
   <223> may be present or not
<400> 37
   nhaahdghyc udyuhasrrc cvhbnh 26
<210> 38
   <211> 16
   <212> DNA
   <213> Artificial Sequence
<220>
   <223> Sequence according to formula (Ic)
<400> 38
   vgyyyyhhth rvvrcb 16
<210> 39
   <211> 16
   <212> DNA
   <213> Artificial Sequence
<220>
   <223> Sequence according to formula (Ic)
<400> 39
   sgyyyttytm arrrcs 16
<210> 40
   <211> 16
   <212> DNA
   <213> Artificial Sequence
<220>
   <223> Sequence according to formula (Ic)
<400> 40
   sgyycttttm agrrcs 16
<210> 41
   <211> 16
   <212> DNA
   <213> Artificial Sequence
<220>
   <223> Sequence according to formula (Ie)
<220>
   <221> misc_feature
   <222> (3)..(5)
   <223> n is a, u, t, g, and c, or a nucleotide analogue thereof
<220>
   <221> misc_feature
   <222> (7)..(8)
   <223> n is a, u, t, g, and c, or a nucleotide analogue thereof
<220>
   <221> misc_feature
   <222> (12)..(14)
   <223> n is a, u, t, g, and c, or a nucleotide analogue thereof
<400> 41
   dgnnnbnnth vnnnch 16
<210> 42
   <211> 16
   <212> DNA
   <213> Artificial Sequence
<220>
   <223> Sequence according to formula (Ie)
<220>
   <221> misc_feature
   <222> (3)..(5)
   <223> n is a, u, t, g, and c, or a nucleotide analogue thereof
<220>
   <221> misc_feature
   <222> (13)..(14)
   <223> n is a, u, t, g, and c, or a nucleotide analogue thereof
<400> 42
   rgnnnyhbth rdnncy 16
<210> 43
   <211> 16
   <212> DNA
   <213> Artificial Sequence
<220>
   <223> Sequence according to formula (Ie)
<220>
   <221> misc_feature
   <222> (3)..(3)
   <223> n is a, u, t, g, and c, or a nucleotide analogue thereof
<220>
   <221> misc_feature
   <222> (14)..(14)
   <223> n is a, u, t, g, and c, or a nucleotide analogue thereof
<400> 43
   rgndbyhyth rdhncy 16
<210> 44
   <211> 16
   <212> DNA
   <213> Artificial Sequence
<220>
   <223> Sequence according to formula (If)
<400> 44
   vgyyytyhth rvrrcb 16
<210> 45
   <211> 16
   <212> DNA
   <213> Artificial Sequence
<220>
   <223> Sequence according to formula (If)
<400> 45
   sgyycttytm agrrcs 16
<210> 46
   <211> 16
   <212> DNA
   <213> Artificial Sequence
<220>
   <223> Sequence according to formula (If)
<400> 46
   sgyycttttm agrrcs 16
<210> 47
   <211> 16
   <212> DNA
   <213> Artificial Sequence
<220>
   <223> Sequence according to formula (Ig)
<400> 47
   ggyycttyth agrrcc 16
<210> 48
   <211> 16
   <212> DNA
   <213> Artificial Sequence
<220>
   <223> Sequence according to formula (Ig)
<400> 48
   ggcycttytm agrgcc 16
<210> 49
   <211> 16
   <212> DNA
   <213> Artificial Sequence
<220>
   <223> Sequence according to formula (Ig)
<400> 49
   ggctcttttm agrgcc 16
<210> 50
   <211> 16
   <212> DNA
   <213> Artificial Sequence
<220>
   <223> Sequence according to formula (Ih)
<400> 50
   dghyctdyth asrrcc 16
<210> 51
   <211> 16
   <212> DNA
   <213> Artificial Sequence
<220>
   <223> Sequence according to formula (Ih)
<400> 51
   ggcyctttth agrgcc 16
<210> 52
   <211> 16
   <212> DNA
   <213> Artificial Sequence
<220>
   <223> Sequence according to formula (Ih)
<400> 52
   ggcycttttm agrgcc 16
<210> 53
   <211> 26
   <212> DNA
   <213> Artificial Sequence
<220>
   <223> Sequence according to formula (IIc)
<220>
   <221> misc_feature
   <222> (1)..(2)
   <223> may be present or not
<220>
   <221> misc_feature
   <222> (25)..(26)
   <223> n is a, u, t, g, and c, or a nucleotide analogue thereof; may be present or not
<400> 53
   hhhhwgyyy yhhthrvvrc bvhhnn 26
<210> 54
   <211> 26
   <212> DNA
   <213> Artificial Sequence
<220>
   <223> Sequence according to formula (IIc)
<220>
   <221> misc_feature
   <222> (1)..(2)
   <223> may be present or not
<220>
   <221> misc_feature
   <222> (25)..(26)
   <223> may be present or not
<400> 54
   mhmhmsgyyy ttytmarrrc smchhh 26
<210> 55
   <211> 26
   <212> DNA
   <213> Artificial Sequence
<220>
   <223> Sequence according to formula (IIc)
<220>
   <221> misc_feature
   <222> (1)..(2)
   <223> may be present or not
<220>
   <221> misc_feature
   <222> (25)..(26)
   <223> may be present or not
<400> 55
   mmmmmsgyyc ttttmagrrc sachmh 26
<210> 56
   <211> 26
   <212> DNA
   <213> Artificial Sequence
<220>
   <223> Sequence according to formula (IIe)
<220>
   <221> misc_feature
   <222> (1)..(2)
   <223> n is a, u, t, g, and c, or a nucleotide analogue thereof; may be present or not
<220>
   <221> misc_feature
   <222> (3)..(5)
   <223> n is a, u, t, g, and c, or a nucleotide analogue thereof
<220>
   <221> misc_feature
   <222> (8)..(10)
   <223> n is a, u, t, g, and c, or a nucleotide analogue thereof
<220>
   <221> misc_feature
   <222> (12)..(13)
   <223> n is a, u, t, g, and c, or a nucleotide analogue thereof
<220>
   <221> misc_feature
   <222> (17)..(19)
   <223> n is a, u, t, g, and c, or a nucleotide analogue thereof
<220>
   <221> misc_feature
   <222> (22)..(22)
   <223> n is a, u, t, g, and c, or a nucleotide analogue thereof
<220>
   <221> misc_feature
   <222> (24)..(26)
   <223> n is a, u, t, g, and c, or a nucleotide analogue thereof; may be present or not
<400> 56
   nnnnndgnnn bnnthvnnnc hnhnnn 26
<210> 57
   <211> 26
   <212> DNA
   <213> Artificial Sequence
<220>
   <223> Sequence according to formula (IIe)
<220>
   <221> misc_feature
   <222> (1)..(1)
   <223> n is a, u, t, g, and c, or a nucleotide analogue thereof; may be present or not
<220>
   <221> misc_feature
   <222> (2)..(2)
   <223> n is a, u, t, g, and c, or a nucleotide analogue thereof; may be present or not
<220>
   <221> misc_feature
   <222> (5)..(5)
   <223> n is a, u, t, g, and c, or a nucleotide analogue thereof
<220>
   <221> misc_feature
   <222> (8)..(10)
   <223> n is a, u, t, g, and c, or a nucleotide analogue thereof
<220>
   <221> misc_feature
   <222> (18)..(19)
   <223> n is a, u, t, g, and c, or a nucleotide analogue thereof
<220>
   <221> misc_feature
   <222> (24)..(24)
   <223> may be present or not
<220>
   <221> misc_feature
   <222> (25)..(26)
   <223> n is a, u, t, g, and c, or a nucleotide analogue thereof; may be present or not
<400> 57
   nnhhnrgnnn yhbthrdnnc ydhhnn 26
<210> 58
   <211> 26
   <212> DNA
   <213> Artificial Sequence
<220>
   <223> Sequence according to formula (IIe)
<220>
   <221> misc_feature
   <222> (1)..(1)
   <223> n is a, u, t, g, and c, or a nucleotide analogue thereof; may be present or not
<220>
   <221> misc_feature
   <222> (2)..(2)
   <223> may be present or not
<220>
   <221> misc_feature
   <222> (8)..(8)
   <223> n is a, u, t, g, and c, or a nucleotide analogue thereof
<220>
   <221> misc_feature
   <222> (19)..(19)
   <223> n is a, u, t, g, and c, or a nucleotide analogue thereof
<220>
   <221> misc_feature
   <222> (24)..(26)
   <223> may be present or not
<400> 58
   nhhhvrgndb yhythrdhnc yrhhhh 26
<210> 59
   <211> 26
   <212> DNA
   <213> Artificial Sequence
<220>
   <223> Sequence according to formula (IIf)
<220>
   <221> misc_feature
   <222> (1)..(2)
   <223> may be present or not
<220>
   <221> misc_feature
   <222> (25)..(25)
   <223> may be present or not
<220>
   <221> misc_feature
   <222> (26).. (26)
   <223> n is a, u, t, g, and c, or a nucleotide analogue thereof; may be present or not
<400> 59
   hhmhmvgyyy tyhthrvrrc bvmhhn 26
<210> 60
   <211> 26
   <212> DNA
   <213> Artificial Sequence
<220>
   <223> Sequence according to formula (IIf)
<220>
   <221> misc_feature
   <222> (1)..(2)
   <223> may be present or not
<220>
   <221> misc_feature
   <222> (25)..(26)
   <223> may be present or not
<400> 60
   mmmmmsgyyc ttytmagrrc smchhh 26
<210> 61
   <211> 26
   <212> DNA
   <213> Artificial Sequence
<220>
   <223> Sequence according to formula (IIf)
<220>
   <221> misc_feature
   <222> (1)..(2)
   <223> may be present or not
<220>
   <221> misc_feature
   <222> (25)..(26)
   <223> may be present or not
<400> 61
   mmmmmsgyyc ttttmagrrc sachmh 26
<210> 62
   <211> 26
   <212> DNA
   <213> Artificial Sequence
<220>
   <223> Sequence according to formula (IIg)
<220>
   <221> misc_feature
   <222> (1)..(2)
   <223> may be present or not
<220>
   <221> misc_feature
   <222> (24)..(25)
   <223> n is a, u, t, g, and c, or a nucleotide analogue thereof; may be present or not
<220>
   <221> misc_feature
   <222> (26)..(26)
   <223> may be present or not
<400> 62
   hhmamggyyc ttythagrrc cvhnnm 26
<210> 63
   <211> 26
   <212> DNA
   <213> Artificial Sequence
<220>
   <223> Sequence according to formula (IIg)
<220>
   <221> misc_feature
   <222> (1)..(2)
   <223> may be present or not
<220>
   <221> misc_feature
   <222> (25)..(26)
   <223> may be present or not
<400> 63
   hhaamggcyc ttytmagrgc cvchhm 26
<210> 64
   <211> 26
   <212> DNA
   <213> Artificial Sequence
<220>
   <223> Sequence according to formula (IIg)
<220>
   <221> misc_feature
   <222> (1)..(2)
   <223> may be present or not
<220>
   <221> misc_feature
   <222> (24)..(26)
   <223> may be present or not
<400> 64
   mmaamggctc ttttmagrgc cmcymm 26
<210> 65
   <211> 26
   <212> DNA
   <213> Artificial Sequence
<220>
   <223> Sequence according to formula (IIh)
<220>
   <221> misc_feature
   <222> (1)..(1)
   <223> n is a, u, t, g, and c, or a nucleotide analogue thereof; may be present or not
<220>
   <221> misc_feature
   <222> (2)..(2)
   <223> may be present or not
<220>
   <221> misc_feature
   <222> (24)..(24)
   <223> may be present or not
<220>
   <221> misc_feature
   <222> (25)..(25)
   <223> n is a, u, t, g, and c, or a nucleotide analogue thereof; may be present or not
<220>
   <221> misc_feature
   <222> (26)..(26)
   <223> may be present or not
<400> 65
   nhaahdghyc tdythasrrc cvhbnh 26
<210> 66
   <211> 26
   <212> DNA
   <213> Artificial Sequence
<220>
   <223> Sequence according to formula (IIh)
<220>
   <221> misc_feature
   <222> (1)..(2)
   <223> may be present or not
<220>
   <221> misc_feature
   <222> (24)..(24)
   <223> may be present or not
<220>
   <221> misc_feature
   <222> (25)..(25)
   <223> n is a, u, t, g, and c, or a nucleotide analogue thereof; may be present or not
<220>
   <221> misc_feature
   <222> (26)..(26)
   <223> may be present or not
<400> 66
   hhaamggcyc tttthagrgc cvmynm 26
<210> 67
   <211> 26
   <212> DNA
   <213> Artificial Sequence
<220>
   <223> Sequence according to formula (IIh)
<220>
   <221> misc_feature
   <222> (1)..(2)
   <223> may be present or not
<220>
   <221> misc_feature
   <222> (24)..(26)
   <223> may be present or not
<400> 67
   hmaaaggcyc ttttmagrgc crmyhm 26
<210> 68
   <211> 13
   <212> RNA
   <213> Artificial Sequence
<220>
   <223> Kozak sequence
<400> 68
   gccgccacca ugg 13
<210> 69
   <211> 15
   <212> RNA
   <213> Artificial Sequence
<220>
   <223> general formula of a 3-UTR stabilizing sequence
<220>
   <221> misc_feature
   <222> (1)..(1)
   <223> n is c or u
<220>
   <221> repeat_unit
   <222> (5)..(5)
   <223> n is a, u, t, g, or c; may be present or not
<220>
   <221> misc_feature
   <222> (5)..(5)
   <223> n is a, u, t, g, or c
<220>
   <221> misc_feature
   <222> (9)..(9)
   <223> n is a, or u
<220>
   <221> repeat_unit
   <222> (10)..(10)
   <223> n is c or u, may be present or not
<220>
   <221> misc_feature
   <222> (10)..(10)
   <223> n is c or u
<220>
   <221> misc_feature
   <222> (13)..(13)
   <223> n is c or u
<400> 69
   nccancccnn ucncc 15
<210> 70
   <211> 44
   <212> RNA
   <213> Artificial Sequence
<220>
   <223> 3'-UTR of an alpha-globin gene (muag)
<400> 70
   gcccgauggg ccucccaacg ggcccuccuc cccuccuugc accg 44
<210> 71
   <211> 24
   <212> DNA
   <213> Artificial Sequence
<220>
   <223> Specific histone stem-loop sequence
<400> 71
   caaaggctct tttcagagcc acca 24
<210> 72
   <211> 24
   <212> RNA
   <213> Artificial Sequence
<220>
   <223> Specific histone stem-loop sequence
<400> 72
   caaaggcucu uuucagagcc acca 24
<210> 73
   <211> 9
   <212> PRT
   <213> Artificial Sequence
<220>
   <223> MUC1-derived peptide
<400> 73
   Ser Ala Pro Asp Asn Arg Pro Ala Leu
   1 5
<210> 74
   <211> 8
   <212> PRT
   <213> Artificial Sequence
<220>
   <223> Connexin-derived peptide
<400> 74
   Phe Glu Gln Asn Thr Ala Gln Pro
   1 5
<210> 75
   <211> 420
   <212> PRT
   <213> Artificial Sequence
<220>
   <223> 5T4 Protein NP_001159864.1
<400> 75
   Met Pro Gly Gly Cys Ser Arg Gly Pro Ala Ala Gly Asp Gly Arg Leu
   1 5 10 15
   Arg Leu Ala Arg Leu Ala Leu Val Leu Leu Gly Trp Val Ser Ser Ser
   20 25 30
   Ser Pro Thr Ser Ser Ala Ser Ser Phe Ser Ser Ser Ala Pro Phe Leu
   35 40 45
   Ala Ser Ala Val Ser Ala Gln Pro Pro Leu Pro Asp Gln Cys Pro Ala
   50 55 60
   Leu Cys Glu Cys Ser Glu Ala Ala Arg Thr Val Lys Cys Val Asn Arg
   65 70 75 80
   Asn Leu Thr Glu Val Pro Thr Asp Leu Pro Ala Tyr Val Arg Asn Leu
   85 90 95
   Phe Leu Thr Gly Asn Gln Leu Ala Val Leu Pro Ala Gly Ala Phe Ala
   100 105 110
   Arg Arg Pro Pro Leu Ala Glu Leu Ala Ala Leu Asn Leu Ser Gly Ser
   115 120 125
   Arg Leu Asp Glu Val Arg Ala Gly Ala Phe Glu His Leu Pro Ser Leu
   130 135 140
   Arg Gln Leu Asp Leu Ser His Asn Pro Leu Ala Asp Leu Ser Pro Phe
   145 150 155 160
   Ala Phe Ser Gly Ser Asn Ala Ser Val Ser Ala Pro Ser Pro Leu Val
   165 170 175
   Glu Leu Ile Leu Asn His Ile Val Pro Pro Glu Asp Glu Arg Gln Asn
   180 185 190
   Arg Ser Phe Glu Gly Met Val Val Ala Ala Leu Leu Ala Gly Arg Ala
   195 200 205
   Leu Gln Gly Leu Arg Arg Leu Glu Leu Ala Ser Asn His Phe Leu Tyr
   210 215 220
   Leu Pro Arg Asp Val Leu Ala Gln Leu Pro Ser Leu Arg His Leu Asp
   225 230 235 240
   Leu Ser Asn Asn Ser Leu Val Ser Leu Thr Tyr Val Ser Phe Arg Asn
   245 250 255
   Leu Thr His Leu Glu Ser Leu His Leu Glu Asp Asn Ala Leu Lys Val
   260 265 270
   Leu His Asn Gly Thr Leu Ala Glu Leu Gln Gly Leu Pro His Ile Arg
   275 280 285
   Val Phe Leu Asp Asn Asn Pro Trp Val Cys Asp Cys His Met Ala Asp
   290 295 300
   Met Val Thr Trp Leu Lys Glu Thr Glu Val Val Gln Gly Lys Asp Arg
   305 310 315 320
   Leu Thr Cys Ala Tyr Pro Glu Lys Met Arg Asn Arg Val Leu Leu Glu
   325 330 335
   Leu Asn Ser Ala Asp Leu Asp Cys Asp Pro Ile Leu Pro Pro Ser Leu
   340 345 350
   Gln Thr Ser Tyr Val Phe Leu Gly Ile Val Leu Ala Leu Ile Gly Ala
   355 360 365
   Ile Phe Leu Leu Val Leu Tyr Leu Asn Arg Lys Gly Ile Lys Lys Trp
   370 375 380
   Met His Asn Ile Arg Asp Ala Cys Arg Asp His Met Glu Gly Tyr His
   385 390 395 400
   Tyr Arg Tyr Glu Ile Asn Ala Asp Pro Arg Leu Thr Asn Leu Ser Ser
   405 410 415
   Asn Ser Asp Val
   420
<210> 76
   <211> 142
   <212> PRT
   <213> Artificial Sequence
<220>
   <223> Survivin (BIRC5) Protein 015392
<400> 76
   Met Gly Ala Pro Thr Leu Pro Pro Ala Trp Gln Pro Phe Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Ala
   115 120 125
   Lys Lys Val Arg Arg Ala Ile Glu Gln Leu Ala Ala Met Asp
   130 135 140
<210> 77
   <211> 142
   <212> PRT
   <213> Artificial Sequence
<220>
   <223> Survivin (BIRC5) Protein NP_001159.2
<400> 77
   Met Gly Ala Pro Thr Leu Pro Pro Ala Trp Gln Pro Phe Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Ala
   115 120 125
   Glu Lys Val Arg Arg Ala Ile Glu Gln Leu Ala Ala Met Asp
   130 135 140
<210> 78
   <211> 180
   <212> PRT
   <213> Artificial Sequence
<220>
   <223> NY-ESO-1 Protein NP_001318.1
<400> 78
   Met Gln Ala Glu Gly Arg Gly Thr Gly Gly Ser Thr Gly Asp Ala Asp
   1               5                   10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Arg
               180
<210> 79
   <211> 1142
   <212> PRT
   <213> Artificial Sequence
<220>
   <223> MAGE-C1 Protein NP_005453.2
<400> 79
   Met Gly Asp Lys Asp Met Pro Thr Ala Gly Met Pro Ser Leu Leu Gln
   1 5 10 15
   Ser Ser Ser Glu Ser Pro Gln Ser Cys Pro Glu Gly Glu Asp Ser Gln
   20 25 30
   Ser Pro Leu Gln Ile Pro Gln Ser Ser Pro Glu Ser Asp Asp Thr Leu
   35 40 45
   Tyr Pro Leu Gln Ser Pro Gln Ser Arg Ser Glu Gly Glu Asp Ser Ser
   50 55 60
   Asp Pro Leu Gln Arg Pro Pro Glu Gly Lys Asp Ser Gln Ser Pro Leu
   65 70 75 80
   Gln Ile Pro Gln Ser Ser Pro Glu Gly Asp Asp Thr Gln Ser Pro Leu
   85 90 95
   Gln Asn Ser Gln Ser Ser Pro Glu Gly Lys Asp Ser Leu Ser Pro Leu
   100 105 110
   Glu Ile Ser Gln Ser Pro Pro Glu Gly Glu Asp Val Gln Ser Pro Leu
   115 120 125
   Gln Asn Pro Ala Ser Ser Phe Phe Ser Ser Ala Leu Leu Ser Ile Phe
   130 135 140
   Gln Ser Ser Pro Glu Ser Thr Gln Ser Pro Phe Glu Gly Phe Pro Gln
   145 150 155 160
   Ser Val Leu Gln Ile Pro Val Ser Ala Ala Ser Ser Ser Thr Leu Val
   165 170 175
   Ser Ile Phe Gln Ser Ser Pro Glu Ser Thr Gln Ser Pro Phe Glu Gly
   180 185 190
   Phe Pro Gln Ser Pro Leu Gln Ile Pro Val Ser Arg Ser Phe Ser Ser
   195 200 205
   Thr Leu Leu Ser Ile Phe Gln Ser Ser Pro Glu Arg Thr Gln Ser Thr
   210 215 220
   Phe Glu Gly Phe Ala Gln Ser Pro Leu Gln Ile Pro Val Ser Pro Ser
   225 230 235 240
   Ser Ser Ser Thr Leu Leu Ser Leu Phe Gln Ser Phe Ser Glu Arg Thr
   245 250 255
   Gln Ser Thr Phe Glu Gly Phe Ala Gln Ser Ser Leu Gln Ile Pro Val
               260                 265                 270
   Ser Pro Ser Phe Ser Ser Thr Leu Val Ser Leu Phe Gln Ser Ser Pro
           275                 280                 285
   Glu Arg Thr Gln Ser Thr Phe Glu Gly Phe Pro Gln Ser Pro Leu Gln
       290                 295                 300
   Ile Pro Val Ser Ser Ser Ser Ser Ser Thr Leu Leu Ser Leu Phe Gln
   305                 310                 315                 320
   Ser Ser Pro Glu Arg Thr His Ser Thr Phe Glu Gly Phe Pro Gln Ser
                   325                 330                 335
   Leu Leu Gln Ile Pro Met Thr Ser Ser Phe Ser Ser Thr Leu Leu Ser
               340                 345                 350
   Ile Phe Gln Ser Ser Pro Glu Ser Ala Gln Ser Thr Phe Glu Gly Phe
           355                 360                 365
   Pro Gln Ser Pro Leu Gln Ile Pro Gly Ser Pro Ser Phe Ser Ser Thr
       370                 375                 380
   Leu Leu Ser Leu Phe Gln Ser Ser Pro Glu Arg Thr His Ser Thr Phe
   385                 390                 395                 400
   Glu Gly Phe Pro Gln Ser Pro Leu Gln Ile Pro Met Thr Ser Ser Phe
                   405                 410                 415
   Ser Ser Thr Leu Leu Ser Ile Leu Gln Ser Ser Pro Glu Ser Ala Gln
               420                 425                 430
   Ser Ala Phe Glu Gly Phe Pro Gln Ser Pro Leu Gln Ile Pro Val Ser
           435                 440                 445
   Ser Ser Phe Ser Tyr Thr Leu Leu Ser Leu Phe Gln Ser Ser Pro Glu
       450                 455                 460
   Arg Thr His Ser Thr Phe Glu Gly Phe Pro Gln Ser Pro Leu Gln Ile
   465                 470                 475                 480
   Pro Val Ser Ser Ser Ser Ser Ser Ser Thr Leu Leu Ser Leu Phe Gln
                   485                 490                 495
   Ser Ser Pro Glu Cys Thr Gln Ser Thr Phe Glu Gly Phe Pro Gln Ser
               500                 505                 510
   Pro Leu Gln Ile Pro Gln Ser Pro Pro Glu Gly Glu Asn Thr His Ser
           515                 520                 525
   Pro Leu Gln Ile Val Pro Ser Leu Pro Glu Trp Glu Asp Ser Leu Ser
       530                 535                 540
   Pro His Tyr Phe Pro Gln Ser Pro Pro Gln Gly Glu Asp Ser Leu Ser
   545                 550                 555                 560
   Pro His Tyr Phe Pro Gln Ser Pro Pro Gln Gly Glu Asp Ser Leu Ser
                   565                 570                 575
   Pro His Tyr Phe Pro Gln Ser Pro Gln Gly Glu Asp Ser Leu Ser Pro
               580                 585                 590
   His Tyr Phe Pro Gln Ser Pro Pro Gln Gly Glu Asp Ser Met Ser Pro
           595                 600                 605
   Leu Tyr Phe Pro Gln Ser Pro Leu Gln Gly Glu Glu Phe Gln Ser Ser
       610                 615                 620
   Leu Gln Ser Pro Val Ser Ile Cys Ser Ser Ser Thr Pro Ser Ser Leu
   625                 630                 635                 640
   Pro Gln Ser Phe Pro Glu Ser Ser Gln Ser Pro Pro Glu Gly Pro Val
                   645                 650                 655
   Gln Ser Pro Leu His Ser Pro Gln Ser Pro Pro Glu Gly Met His Ser
               660                 665                 670
   Gln Ser Pro Leu Gln Ser Pro Glu Ser Ala Pro Glu Gly Glu Asp Ser
           675                 680                 685
   Leu Ser Pro Leu Gln Ile Pro Gln Ser Pro Leu Glu Gly Glu Asp Ser
       690                 695                 700
   Leu Ser Ser Leu His Phe Pro Gln Ser Pro Pro Glu Trp Glu Asp Ser
   705                 710                 715                 720
   Leu Ser Pro Leu His Phe Pro Gln Phe Pro Pro Gln Gly Glu Asp Phe
                   725                 730                 735
   Gln Ser Ser Leu Gln Ser Pro Val Ser Ile Cys Ser Ser Ser Thr Ser
               740                 745                 750
   Leu Ser Leu Pro Gln Ser Phe Pro Glu Ser Pro Gln Ser Pro Pro Glu
           755                 760                 765
   Gly Pro Ala Gln Ser Pro Leu Gln Arg Pro Val Ser Ser Phe Phe Ser
       770                 775                 780
   Tyr Thr Leu Ala Ser Leu Leu Gln Ser Ser His Glu Ser Pro Gln Ser
   785                 790                 795                 800
   Pro Pro Glu Gly Pro Ala Gln Ser Pro Leu Gln Ser Pro Val Ser Ser
                   805                 810                 815
   Phe Pro Ser Ser Thr Ser Ser Ser Leu Ser Gln Ser Ser Pro Val Ser
               820                 825                 830
   Ser Phe Pro Ser Ser Thr Ser Ser Ser Leu Ser Lys Ser Ser Pro Glu
           835                 840                 845
   Ser Pro Leu Gln Ser Pro Val Ile Ser Phe Ser Ser Ser Thr Ser Leu
       850                 855                 860
   Ser Pro Phe Ser Glu Glu Ser Ser Ser Pro Val Asp Glu Tyr Thr Ser
   865                 870                 875                 880
   Ser Ser Asp Thr Leu Leu Glu Ser Asp Ser Leu Thr Asp Ser Glu Ser
                   885                 890                 895
   Leu Ile Glu Ser Glu Pro Leu Phe Thr Tyr Thr Leu Asp Glu Lys Val
               900                 905                 910
   Asp Glu Leu Ala Arg Phe Leu Leu Leu Lys Tyr Gln Val Lys Gln Pro
           915                 920                 925
   Ile Thr Lys Ala Glu Met Leu Thr Asn Val Ile Ser Arg Tyr Thr Gly
       930                 935                 940
   Tyr Phe Pro Val Ile Phe Arg Lys Ala Arg Glu Phe Ile Glu Ile Leu
   945                 950                 955                 960
   Phe Gly Ile Ser Leu Arg Glu Val Asp Pro Asp Asp Ser Tyr Val Phe
                   965                 970                 975
   Val Asn Thr Leu Asp Leu Thr Ser Glu Gly Cys Leu Ser Asp Glu Gln
               980                 985                 990
   Gly Met Ser Gln Asn Arg Leu Leu  Ile Leu Ile Leu Ser  Ile Ile Phe
           995                 1000                1005
   Ile Lys Gly Thr Tyr Ala Ser Glu Glu Val Ile Trp Asp Val Leu
   1010 1015 1020
   Ser Gly Ile Gly Val Arg Ala Gly Arg Glu His Phe Ala Phe Gly
   1025 1030 1035
   Glu Pro Arg Glu Leu Leu Thr Lys Val Trp Val Gln Glu His Tyr
   1040 1045 1050
   Leu Glu Tyr Arg Glu Val Pro Asn Ser Ser Pro Pro Arg Tyr Glu
   1055 1060 1065
   Phe Leu Trp Gly Pro Arg Ala His Ser Glu Val Ile Lys Arg Lys
   1070 1075 1080
   Val Val Glu Phe Leu Ala Met Leu Lys Asn Thr Val Pro Ile Thr
   1085 1090 1095
   Phe Pro Ser Ser Tyr Lys Asp Ala Leu Lys Asp Val Glu Glu Arg
   1100 1105 1110
   Ala Gln Ala Ile Ile Asp Thr Thr Asp Asp Ser Thr Ala Thr Glu
   1115 1120 1125
   Ser Ala Ser Ser Ser Val Met Ser Pro Ser Phe Ser Ser Glu
   1130 1135 1140
<210> 80
   <211> 373
   <212> PRT
   <213> Artificial Sequence
<220>
   <223> MAGE-C2 Protein NP_057333.1
<400> 80
   Met Pro Pro Val Pro Gly Val Pro Phe Arg Asn Val Asp Asn Asp Ser
   1               5                   10                  15
   Pro Thr Ser Val Glu Leu Glu Asp Trp Val Asp Ala Gln His Pro Thr
               20                  25                  30
   Asp Glu Glu Glu Glu Glu Ala Ser Ser Ala Ser Ser Thr Leu Tyr Leu
           35                  40                  45
   Val Phe Ser Pro Ser Ser Phe Ser Thr Ser Ser Ser Leu Ile Leu Gly
       50                  55                  60
   Gly Pro Glu Glu Glu Glu Val Pro Ser Gly Val Ile Pro Asn Leu Thr
   65 70 75 80
   Glu Ser Ile Pro Ser Ser Pro Pro Gln Gly Pro Pro Gln Gly Pro Ser
   85 90 95
   Gln Ser Pro Leu Ser Ser Cys Cys Ser Ser Phe Ser Trp Ser Ser Phe
   100 105 110
   Ser Glu Glu Ser Ser Ser Gln Lys Gly Glu Asp Thr Gly Thr Cys Gln
   115 120 125
   Gly Leu Pro Asp Ser Glu Ser Ser Phe Thr Tyr Thr Leu Asp Glu Lys
   130 135 140
   Val Ala Glu Leu Val Glu Phe Leu Leu Leu Lys Tyr Glu Ala Glu Glu
   145 150 155 160
   Pro Val Thr Glu Ala Glu Met Leu Met Ile Val Ile Lys Tyr Lys Asp
   165 170 175
   Tyr Phe Pro Val Ile Leu Lys Arg Ala Arg Glu Phe Met Glu Leu Leu
   180 185 190
   Phe Gly Leu Ala Leu Ile Glu Val Gly Pro Asp His Phe Cys Val Phe
   195 200 205
   Ala Asn Thr Val Gly Leu Thr Asp Glu Gly Ser Asp Asp Glu Gly Met
   210 215 220
   Pro Glu Asn Ser Leu Leu Ile Ile Ile Leu Ser Val Ile Phe Ile Lys
   225 230 235 240
   Gly Asn Cys Ala Ser Glu Glu Val Ile Trp Glu Val Leu Asn Ala Val
   245 250 255
   Gly Val Tyr Ala Gly Arg Glu His Phe Val Tyr Gly Glu Pro Arg Glu
   260 265 270
   Leu Leu Thr Lys Val Trp Val Gln Gly His Tyr Leu Glu Tyr Arg Glu
   275 280 285
   Val Pro His Ser Ser Pro Pro Tyr Tyr Glu Phe Leu Trp Gly Pro Arg
   290 295 300
   Ala His Ser Glu Ser Ile Lys Lys Lys Val Leu Glu Phe Leu Ala Lys
   305 310 315 320
   Leu Asn Asn Thr Val Pro Ser Ser Phe Pro Ser Trp Tyr Lys Asp Ala
   325 330 335
   Leu Lys Asp Val Glu Glu Arg Val Gln Ala Thr Ile Asp Thr Ala Asp
   340 345 350
   Asp Ala Thr Val Met Ala Ser Glu Ser Leu Ser Val Met Ser Ser Asn
   355 360 365
   Val Ser Phe Ser Glu
   370
<210> 81
   <211> 1255
   <212> PRT
   <213> Artificial Sequence
<220>
   <223> MUC1 Protein J05582.1
<400> 81
   Met Thr Pro Gly Thr Gln Ser Pro Phe Phe Leu Leu Leu Leu Leu Thr
   1               5                   10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Val Ser Met Thr Ser Ser Val Leu Ser Ser His
       50                  55                  60
   Ser Pro Gly Ser Gly Ser Ser Thr Thr Gln Gly Gln Asp Val Thr Leu
   65                  70                  75                  80
   Ala Pro Ala Thr Glu Pro Ala Ser Gly Ser Ala Ala Thr Trp Gly Gln
                   85                  90                  95
   Asp Val Thr Ser Val Pro Val Thr Arg Pro Ala Leu Gly Ser Thr Thr
               100                 105                 110
   Pro Pro Ala His Asp Val Thr Ser Ala Pro Asp Asn Lys Pro Ala Pro
           115                 120                 125
   Gly Ser Thr Ala Pro Pro Ala His Gly Val Thr Ser Ala Pro Asp Thr
       130                 135                 140
   Arg Pro Ala Pro Gly Ser Thr Ala Pro Pro Ala His Gly Val Thr Ser
   145 150 155 160
   Ala Pro Asp Thr Arg Pro Ala Pro Gly Ser Thr Ala Pro Pro Ala His
   165 170 175
   Gly Val Thr Ser Ala Pro Asp Thr Arg Pro Ala Pro Gly Ser Thr Ala
   180 185 190
   Pro Pro Ala His Gly Val Thr Ser Ala Pro Asp Thr Arg Pro Ala Pro
   195 200 205
   Gly Ser Thr Ala Pro Pro Ala His Gly Val Thr Ser Ala Pro Asp Thr
   210 215 220
   Arg Pro Ala Pro Gly Ser Thr Ala Pro Pro Ala His Gly Val Thr Ser
   225 230 235 240
   Ala Pro Asp Thr Arg Pro Ala Pro Gly Ser Thr Ala Pro Pro Ala His
   245 250 255
   Gly Val Thr Ser Ala Pro Asp Thr Arg Pro Ala Pro Gly Ser Thr Ala
   260 265 270
   Pro Pro Ala His Gly Val Thr Ser Ala Pro Asp Thr Arg Pro Ala Pro
   275 280 285
   Gly Ser Thr Ala Pro Pro Ala His Gly Val Thr Ser Ala Pro Asp Thr
   290 295 300
   Arg Pro Ala Pro Gly Ser Thr Ala Pro Pro Ala His Gly Val Thr Ser
   305 310 315 320
   Ala Pro Asp Thr Arg Pro Ala Pro Gly Ser Thr Ala Pro Pro Ala His
   325 330 335
   Gly Val Thr Ser Ala Pro Asp Thr Arg Pro Ala Pro Gly Ser Thr Ala
   340 345 350
   Pro Pro Ala His Gly Val Thr Ser Ala Pro Asp Thr Arg Pro Ala Pro
   355 360 365
   Gly Ser Thr Ala Pro Pro Ala His Gly Val Thr Ser Ala Pro Asp Thr
   370 375 380
   Arg Pro Ala Pro Gly Ser Thr Ala Pro Pro Ala His Gly Val Thr Ser
   385 390 395 400
   Ala Pro Asp Thr Arg Pro Ala Pro Gly Ser Thr Ala Pro Pro Ala His
                   405                 410                 415
   Gly Val Thr Ser Ala Pro Asp Thr Arg Pro Ala Pro Gly Ser Thr Ala
               420                 425                 430
   Pro Pro Ala His Gly Val Thr Ser Ala Pro Asp Thr Arg Pro Ala Pro
           435                 440                 445
   Gly Ser Thr Ala Pro Pro Ala His Gly Val Thr Ser Ala Pro Asp Thr
       450                 455                 460
   Arg Pro Ala Pro Gly Ser Thr Ala Pro Pro Ala His Gly Val Thr Ser
   465                 470                 475                 480
   Ala Pro Asp Thr Arg Pro Ala Pro Gly Ser Thr Ala Pro Pro Ala His
                   485                 490                 495
   Gly Val Thr Ser Ala Pro Asp Thr Arg Pro Ala Pro Gly Ser Thr Ala
               500                 505                 510
   Pro Pro Ala His Gly Val Thr Ser Ala Pro Asp Thr Arg Pro Ala Pro
           515                 520                 525
   Gly Ser Thr Ala Pro Pro Ala His Gly Val Thr Ser Ala Pro Asp Thr
       530                 535                 540
   Arg Pro Ala Pro Gly Ser Thr Ala Pro Pro Ala His Gly Val Thr Ser
   545                 550                 555                 560
   Ala Pro Asp Thr Arg Pro Ala Pro Gly Ser Thr Ala Pro Pro Ala His
                   565                 570                 575
   Gly Val Thr Ser Ala Pro Asp Thr Arg Pro Ala Pro Gly Ser Thr Ala
               580                 585                 590
   Pro Pro Ala His Gly Val Thr Ser Ala Pro Asp Thr Arg Pro Ala Pro
           595                 600                 605
   Gly Ser Thr Ala Pro Pro Ala His Gly Val Thr Ser Ala Pro Asp Thr
       610                 615                 620
   Arg Pro Ala Pro Gly Ser Thr Ala Pro Pro Ala His Gly Val Thr Ser
   625                 630                 635                 640
   Ala Pro Asp Thr Arg Pro Ala Pro Gly Ser Thr Ala Pro Pro Ala His
                   645                 650                 655
   Gly Val Thr Ser Ala Pro Asp Thr Arg Pro Ala Pro Gly Ser Thr Ala
               660                 665                 670
   Pro Pro Ala His Gly Val Thr Ser Ala Pro Asp Thr Arg Pro Ala Pro
           675                 680                 685
   Gly Ser Thr Ala Pro Pro Ala His Gly Val Thr Ser Ala Pro Asp Thr
       690                 695                 700
   Arg Pro Ala Pro Gly Ser Thr Ala Pro Pro Ala His Gly Val Thr Ser
   705                 710                 715                 720
   Ala Pro Asp Thr Arg Pro Ala Pro Gly Ser Thr Ala Pro Pro Ala His
                   725                 730                 735
   Gly Val Thr Ser Ala Pro Asp Thr Arg Pro Ala Pro Gly Ser Thr Ala
               740                 745                 750
   Pro Pro Ala His Gly Val Thr Ser Ala Pro Asp Thr Arg Pro Ala Pro
           755                 760                 765
   Gly Ser Thr Ala Pro Pro Ala His Gly Val Thr Ser Ala Pro Asp Thr
       770                 775                 780
   Arg Pro Ala Pro Gly Ser Thr Ala Pro Pro Ala His Gly Val Thr Ser
   785                 790                 795                 800
   Ala Pro Asp Thr Arg Pro Ala Pro Gly Ser Thr Ala Pro Pro Ala His
                   805                 810                 815
   Gly Val Thr Ser Ala Pro Asp Thr Arg Pro Ala Pro Gly Ser Thr Ala
               820                 825                 830
   Pro Pro Ala His Gly Val Thr Ser Ala Pro Asp Thr Arg Pro Ala Pro
           835                 840                 845
   Gly Ser Thr Ala Pro Pro Ala His Gly Val Thr Ser Ala Pro Asp Thr
       850                 855                 860
   Arg Pro Ala Pro Gly Ser Thr Ala Pro Pro Ala His Gly Val Thr Ser
   865                 870                 875                 880
   Ala Pro Asp Thr Arg Pro Ala Pro Gly Ser Thr Ala Pro Pro Ala His
                   885                 890                 895
   Gly Val Thr Ser Ala Pro Asp Thr Arg Pro Ala Pro Gly Ser Thr Ala
               900                 905                 910
   Pro Pro Ala His Gly Val Thr Ser Ala Pro Asp Thr Arg Pro Ala Pro
   915 920 925
   Gly Ser Thr Ala Pro Pro Ala His Gly Val Thr Ser Ala Pro Asp Asn
   930 935 940
   Arg Pro Ala Leu Gly Ser Thr Ala Pro Pro Val His Asn Val Thr Ser
   945 950 955 960
   Ala Ser Gly Ser Ala Ser Gly Ser Ala Ser Thr Leu Val His Asn Gly
   965 970 975
   Thr Ser Ala Arg Ala Thr Thr Thr Pro Ala Ser Lys Ser Thr Pro Phe
   980 985 990
   Ser Ile Pro Ser His His Ser Asp Thr Pro Thr Thr Leu Ala Ser His
   995 1000 1005
   Ser Thr Lys Thr Asp Ala Ser Ser Thr His His Ser Ser Val Pro
   1010 1015 1020
   Pro Leu Thr Ser Ser Asn His Ser Thr Ser Pro Gln Leu Ser Thr
   1025 1030 1035
   Gly Val Ser Phe Phe Phe Leu Ser Phe His Ile Ser Asn Leu Gln
   1040 1045 1050
   Phe Asn Ser Ser Leu Glu Asp Pro Ser Thr Asp Tyr Tyr Gln Glu
   1055 1060 1065
   Leu Gln Arg Asp Ile Ser Glu Met Phe Leu Gln Ile Tyr Lys Gln
   1070 1075 1080
   Gly Gly Phe Leu Gly Leu Ser Asn Ile Lys Phe Arg Pro Gly Ser
   1085 1090 1095
   Val Val Val Gln Leu Thr Leu Ala Phe Arg Glu Gly Thr Ile Asn
   1100 1105 1110
   Val His Asp Val Glu Thr Gln Phe Asn Gln Tyr Lys Thr Glu Ala
   1115 1120 1125
   Ala Ser Arg Tyr Asn Leu Thr Ile Ser Asp Val Ser Val Ser Asp
   1130 1135 1140
   Val Pro Phe Pro Phe Ser Ala Gln Ser Gly Ala Gly Val Pro Gly
   1145 1150 1155
   Trp Gly Ile Ala Leu Leu Val Leu Val Cys Val Leu Val Ala Leu
   1160 1165 1170
   Ala Ile Val Tyr Leu Ile Ala Leu Ala Val Cys Gln Cys Arg Arg
   1175 1180 1185
   Lys Asn Tyr Gly Gln Leu Asp Ile Phe Pro Ala Arg Asp Thr Tyr
   1190 1195 1200
   His Pro Met Ser Glu Tyr Pro Thr Tyr His Thr His Gly Arg Tyr
   1205 1210 1215
   Val Pro Pro Ser Ser Thr Asp Arg Ser Pro Tyr Glu Lys Val Ser
   1220 1225 1230
   Ala Gly Asn Gly Gly Ser Ser Leu Ser Tyr Thr Asn Pro Ala Val
   1235 1240 1245
   Ala Ala Ala Ser Ala Asn Leu
   1250 1255
<210> 82
   <211> 555
   <212> PRT
   <213> Artificial Sequence
<220>
   <223> MUC1 Protein 5xVNTR
<400> 82
   Met Thr Pro Gly Thr Gln Ser Pro Phe Phe Leu Leu Leu Leu Leu Thr
   1               5                   10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Val Ser Met Thr Ser Ser Val Leu Ser Ser His
       50                  55                  60
   Ser Pro Gly Ser Gly Ser Ser Thr Thr Gln Gly Gln Asp Val Thr Leu
   65                  70                  75                  80
   Ala Pro Ala Thr Glu Pro Ala Ser Gly Ser Ala Ala Thr Trp Gly Gln
                   85                  90                  95
   Asp Val Thr Ser Val Pro Val Thr Arg Pro Ala Leu Gly Ser Thr Thr
   100 105 110
   Pro Pro Ala His Asp Val Thr Ser Ala Pro Asp Asn Lys Pro Ala Pro
   115 120 125
   Gly Ser Thr Ala Pro Pro Ala His Gly Val Thr Ser Ala Pro Asp Thr
   130 135 140
   Arg Pro Ala Pro Gly Ser Thr Ala Pro Pro Ala His Gly Val Thr Ser
   145 150 155 160
   Ala Pro Asp Thr Arg Pro Ala Pro Gly Ser Thr Ala Pro Pro Ala His
   165 170 175
   Gly Val Thr Ser Ala Pro Asp Thr Arg Pro Ala Pro Gly Ser Thr Ala
   180 185 190
   Pro Pro Ala His Gly Val Thr Ser Ala Pro Asp Thr Arg Pro Ala Pro
   195 200 205
   Gly Ser Thr Ala Pro Pro Ala His Gly Val Thr Ser Ala Pro Asp Thr
   210 215 220
   Arg Pro Ala Pro Gly Ser Thr Ala Pro Pro Ala His Gly Val Thr Ser
   225 230 235 240
   Ala Pro Asp Asn Arg Pro Ala Leu Gly Ser Thr Ala Pro Pro Val His
   245 250 255
   Asn Val Thr Ser Ala Ser Gly Ser Ala Ser Gly Ser Ala Ser Thr Leu
   260 265 270
   Val His Asn Gly Thr Ser Ala Arg Ala Thr Thr Thr Pro Ala Ser Lys
   275 280 285
   Ser Thr Pro Phe Ser Ile Pro Ser His His Ser Asp Thr Pro Thr Thr
   290 295 300
   Leu Ala Ser His Ser Thr Lys Thr Asp Ala Ser Ser Thr His His Ser
   305 310 315 320
   Ser Val Pro Pro Leu Thr Ser Ser Asn His Ser Thr Ser Pro Gln Leu
   325 330 335
   Ser Thr Gly Val Ser Phe Phe Phe Leu Ser Phe His Ile Ser Asn Leu
                   340                 345                 350
   Gln Phe Asn Ser Ser Leu Glu Asp Pro Ser Thr Asp Tyr Tyr Gln Glu
               355                 360                 365
   Leu Gln Arg Asp Ile Ser Glu Met Phe Leu Gln Ile Tyr Lys Gln Gly
       370                 375                 380
   Gly Phe Leu Gly Leu Ser Asn Ile Lys Phe Arg Pro Gly Ser Val Val
   385                 390                 395                 400
   Val Gln Leu Thr Leu Ala Phe Arg Glu Gly Thr Ile Asn Val His Asp
                   405                 410                 415
   Val Glu Thr Gln Phe Asn Gln Tyr Lys Thr Glu Ala Ala Ser Arg Tyr
               420                 425                 430
   Asn Leu Thr Ile Ser Asp Val Ser Val Ser Asp Val Pro Phe Pro Phe
           435                 440                 445
   Ser Ala Gln Ser Gly Ala Gly Val Pro Gly Trp Gly Ile Ala Leu Leu
       450                 455                 460
   Val Leu Val Cys Val Leu Val Ala Leu Ala Ile Val Tyr Leu Ile Ala
   465                 470                 475                 480
   Leu Ala Val Cys Gln Cys Arg Arg Lys Asn Tyr Gly Gln Leu Asp Ile
                   485                 490                 495
   Phe Pro Ala Arg Asp Thr Tyr His Pro Met Ser Glu Tyr Pro Thr Tyr
               500                 505                 510
   His Thr His Gly Arg Tyr Val Pro Pro Ser Ser Thr Asp Arg Ser Pro
           515                 520                 525
   Tyr Glu Lys Val Ser Ala Gly Asn Gly Gly Ser Ser Leu Ser Tyr Thr
       530                 535                 540
   Asn Pro Ala Val Ala Ala Ala Ser Ala Asn Leu
   545                 550                 555
<210> 83
   <211> 3768
   <212> PRT
   <213> Artificial Sequence
<220>
   <223> MUC1 CDS wild type
<400> 83
   Ala Thr Gly Ala Cys Ala Cys Cys Gly Gly Gly Cys Ala Cys Cys Cys
   1               5                   10                  15
   Ala Gly Thr Cys Thr Cys Cys Thr Thr Thr Cys Thr Thr Cys Cys Thr
               20                  25                  30
   Gly Cys Thr Gly Cys Thr Gly Cys Thr Cys Cys Thr Cys Ala Cys Ala
           35                  40                  45
   Gly Thr Gly Cys Thr Thr Ala Cys Ala Gly Thr Thr Gly Thr Thr Ala
       50                  55                  60
   Cys Ala Gly Gly Thr Thr Cys Thr Gly Gly Thr Cys Ala Thr Gly Cys
   65                  70                  75                  80
   Ala Ala Gly Cys Thr Cys Thr Ala Cys Cys Cys Cys Ala Gly Gly Thr
                   85                  90                  95
   Gly Gly Ala Gly Ala Ala Ala Ala Gly Gly Ala Gly Ala Cys Thr Thr
               100                 105                 110
   Cys Gly Gly Cys Thr Ala Cys Cys Cys Ala Gly Ala Gly Ala Ala Gly
           115                 120                 125
   Thr Thr Cys Ala Gly Thr Gly Cys Cys Cys Ala Gly Cys Thr Cys Thr
       130                 135                 140
   Ala Cys Thr Gly Ala Gly Ala Ala Gly Ala Ala Thr Gly Cys Thr Gly
   145                 150                 155                 160
   Thr Gly Ala Gly Thr Ala Thr Gly Ala Cys Cys Ala Gly Cys Ala Gly
                   165                 170                 175
   Cys Gly Thr Ala Cys Thr Cys Thr Cys Cys Ala Gly Cys Cys Ala Cys
               180                 185                 190
   Ala Gly Cys Cys Cys Cys Gly Gly Thr Thr Cys Ala Gly Gly Cys Thr
           195                 200                 205
   Cys Cys Thr Cys Cys Ala Cys Cys Ala Cys Thr Cys Ala Gly Gly Gly
       210                 215                 220
   Ala Cys Ala Gly Gly Ala Thr Gly Thr Cys Ala Cys Thr Cys Thr Gly
   225                 230                 235                 240
   Gly Cys Cys Cys Cys Gly Gly Cys Cys Ala Cys Gly Gly Ala Ala Cys
   245 250 255
   Cys Ala Gly Cys Thr Thr Cys Ala Gly Gly Thr Thr Cys Ala Gly Cys
   260 265 270
   Thr Gly Cys Cys Ala Cys Cys Thr Gly Gly Gly Gly Ala Cys Ala Gly
   275 280 285
   Gly Ala Thr Gly Thr Cys Ala Cys Cys Thr Cys Gly Gly Thr Cys Cys
   290 295 300
   Cys Ala Gly Thr Cys Ala Cys Cys Ala Gly Gly Cys Cys Ala Gly Cys
   305 310 315 320
   Cys Cys Thr Gly Gly Gly Cys Thr Cys Cys Ala Cys Cys Ala Cys Cys
   325 330 335
   Cys Cys Gly Cys Cys Ala Gly Cys Cys Cys Ala Cys Gly Ala Thr Gly
   340 345 350
   Thr Cys Ala Cys Cys Thr Cys Ala Gly Cys Cys Cys Cys Gly Gly Ala
   355 360 365
   Cys Ala Ala Cys Ala Ala Gly Cys Cys Ala Gly Cys Cys Cys Cys Gly
   370 375 380
   Gly Gly Cys Thr Cys Cys Ala Cys Cys Gly Cys Cys Cys Cys Cys Cys
   385 390 395 400
   Cys Ala Gly Cys Cys Cys Ala Cys Gly Gly Thr Gly Thr Cys Ala Cys
   405 410 415
   Cys Thr Cys Gly Gly Cys Cys Cys Cys Gly Gly Ala Cys Ala Cys Cys
   420 425 430
   Ala Gly Gly Cys Cys Gly Gly Cys Cys Cys Cys Gly Gly Gly Cys Thr
   435 440 445
   Cys Cys Ala Cys Cys Gly Cys Cys Cys Cys Cys Cys Cys Ala Gly Cys
   450 455 460
   Cys Cys Ala Cys Gly Gly Thr Gly Thr Cys Ala Cys Cys Thr Cys Gly
   465 470 475 480
   Gly Cys Cys Cys Cys Gly Gly Ala Cys Ala Cys Cys Ala Gly Gly Cys
   485 490 495
   Cys Gly Gly Cys Cys Cys Cys Gly Gly Gly Cys Thr Cys Cys Ala Cys
               500                 505                 510
   Cys Gly Cys Cys Cys Cys Cys Cys Cys Ala Gly Cys Cys Cys Ala Cys
           515                 520                 525
   Gly Gly Thr Gly Thr Cys Ala Cys Cys Thr Cys Gly Gly Cys Cys Cys
       530                 535                 540
   Cys Gly Gly Ala Cys Ala Cys Cys Ala Gly Gly Cys Cys Gly Gly Cys
   545                 550                 555                 560
   Cys Cys Cys Gly Gly Gly Cys Thr Cys Cys Ala Cys Cys Gly Cys Cys
                   565                 570                 575
   Cys Cys Cys Cys Cys Ala Gly Cys Cys Cys Ala Cys Gly Gly Thr Gly
               580                 585                 590
   Thr Cys Ala Cys Cys Thr Cys Gly Gly Cys Cys Cys Cys Gly Gly Ala
           595                 600                 605
   Cys Ala Cys Cys Ala Gly Gly Cys Cys Gly Gly Cys Cys Cys Cys Gly
       610                 615                 620
   Gly Gly Cys Thr Cys Cys Ala Cys Cys Gly Cys Cys Cys Cys Cys Cys
   625                 630                 635                 640
   Cys Ala Gly Cys Cys Cys Ala Cys Gly Gly Thr Gly Thr Cys Ala Cys
                   645                 650                 655
   Cys Thr Cys Gly Gly Cys Cys Cys Cys Gly Gly Ala Cys Ala Cys Cys
               660                 665                 670
   Ala Gly Gly Cys Cys Gly Gly Cys Cys Cys Cys Gly Gly Gly Cys Thr
           675                 680                 685
   Cys Cys Ala Cys Cys Gly Cys Cys Cys Cys Cys Cys Cys Ala Gly Cys
       690                 695                 700
   Cys Cys Ala Cys Gly Gly Thr Gly Thr Cys Ala Cys Cys Thr Cys Gly
   705                 710                 715                 720
   Gly Cys Cys Cys Cys Gly Gly Ala Cys Ala Cys Cys Ala Gly Gly Cys
                   725                 730                 735
   Cys Gly Gly Cys Cys Cys Cys Gly Gly Gly Cys Thr Cys Cys Ala Cys
               740                 745                 750
   Cys Gly Cys Cys Cys Cys Cys Cys Cys Ala Gly Cys Cys Cys Ala Cys
           755                 760                 765
   Gly Gly Thr Gly Thr Cys Ala Cys Cys Thr Cys Gly Gly Cys Cys Cys
       770                 775                 780
   Cys Gly Gly Ala Cys Ala Cys Cys Ala Gly Gly Cys Cys Gly Gly Cys
   785                 790                 795                 800
   Cys Cys Cys Gly Gly Gly Cys Thr Cys Cys Ala Cys Cys Gly Cys Cys
                   805                 810                 815
   Cys Cys Cys Cys Cys Ala Gly Cys Cys Cys Ala Cys Gly Gly Thr Gly
               820                 825                 830
   Thr Cys Ala Cys Cys Thr Cys Gly Gly Cys Cys Cys Cys Gly Gly Ala
           835                 840                 845
   Cys Ala Cys Cys Ala Gly Gly Cys Cys Gly Gly Cys Cys Cys Cys Gly
       850                 855                 860
   Gly Gly Cys Thr Cys Cys Ala Cys Cys Gly Cys Cys Cys Cys Cys Cys
   865                 870                 875                 880
   Cys Ala Gly Cys Cys Cys Ala Cys Gly Gly Thr Gly Thr Cys Ala Cys
                   885                 890                 895
   Cys Thr Cys Gly Gly Cys Cys Cys Cys Gly Gly Ala Cys Ala Cys Cys
               900                 905                 910
   Ala Gly Gly Cys Cys Gly Gly Cys Cys Cys Cys Gly Gly Gly Cys Thr
           915                 920                 925
   Cys Cys Ala Cys Cys Gly Cys Cys Cys Cys Cys Cys Cys Ala Gly Cys
       930                 935                 940
   Cys Cys Ala Cys Gly Gly Thr Gly Thr Cys Ala Cys Cys Thr Cys Gly
   945                 950                 955                 960
   Gly Cys Cys Cys Cys Gly Gly Ala Cys Ala Cys Cys Ala Gly Gly Cys
                   965                 970                 975
   Cys Gly Gly Cys Cys Cys Cys Gly Gly Gly Cys Thr Cys Cys Ala Cys
               980                 985                 990
   Cys Gly Cys Cys Cys Cys Cys Cys  Cys Ala Gly Cys Cys  Cys Ala Cys
           995                 1000                1005
   Gly Gly Thr Gly Thr Cys Ala Cys Cys Thr Cys Gly Gly Cys Cys
   1010 1015 1020
   Cys Cys Gly Gly Ala Cys Ala Cys Cys Ala Gly Gly Cys Cys Gly
   1025 1030 1035
   Gly Cys Cys Cys Cys Gly Gly Gly Cys Thr Cys Cys Ala Cys Cys
   1040 1045 1050
   Gly Cys Cys Cys Cys Cys Cys Cys Ala Gly Cys Cys Cys Ala Cys
   1055 1060 1065
   Gly Gly Thr Gly Thr Cys Ala Cys Cys Thr Cys Gly Gly Cys Cys
   1070 1075 1080
   Cys Cys Gly Gly Ala Cys Ala Cys Cys Ala Gly Gly Cys Cys Gly
   1085 1090 1095
   Gly Cys Cys Cys Cys Gly Gly Gly Cys Thr Cys Cys Ala Cys Cys
   1100 1105 1110
   Gly Cys Cys Cys Cys Cys Cys Cys Ala Gly Cys Cys Cys Ala Cys
   1115 1120 1125
   Gly Gly Thr Gly Thr Cys Ala Cys Cys Thr Cys Gly Gly Cys Cys
   1130 1135 1140
   Cys Cys Gly Gly Ala Cys Ala Cys Cys Ala Gly Gly Cys Cys Gly
   1145 1150 1155
   Gly Cys Cys Cys Cys Gly Gly Gly Cys Thr Cys Cys Ala Cys Cys
   1160 1165 1170
   Gly Cys Cys Cys Cys Cys Cys Cys Ala Gly Cys Cys Cys Ala Cys
   1175 1180 1185
   Gly Gly Thr Gly Thr Cys Ala Cys Cys Thr Cys Gly Gly Cys Cys
   1190 1195 1200
   Cys Cys Gly Gly Ala Cys Ala Cys Cys Ala Gly Gly Cys Cys Gly
   1205 1210 1215
   Gly Cys Cys Cys Cys Gly Gly Gly Cys Thr Cys Cys Ala Cys Cys
   1220 1225 1230
   Gly Cys Cys Cys Cys Cys Cys Cys Ala Gly Cys Cys Cys Ala Cys
   1235 1240 1245
   Gly Gly Thr Gly Thr Cys Ala Cys Cys Thr Cys Gly Gly Cys Cys
   1250 1255 1260
   Cys Cys Gly Gly Ala Cys Ala Cys Cys Ala Gly Gly Cys Cys Gly
   1265 1270 1275
   Gly Cys Cys Cys Cys Gly Gly Gly Cys Thr Cys Cys Ala Cys Cys
   1280 1285 1290
   Gly Cys Cys Cys Cys Cys Cys Cys Ala Gly Cys Cys Cys Ala Cys
   1295 1300 1305
   Gly Gly Thr Gly Thr Cys Ala Cys Cys Thr Cys Gly Gly Cys Cys
   1310 1315 1320
   Cys Cys Gly Gly Ala Cys Ala Cys Cys Ala Gly Gly Cys Cys Gly
   1325 1330 1335
   Gly Cys Cys Cys Cys Gly Gly Gly Cys Thr Cys Cys Ala Cys Cys
   1340 1345 1350
   Gly Cys Cys Cys Cys Cys Cys Cys Ala Gly Cys Cys Cys Ala Cys
   1355 1360 1365
   Gly Gly Thr Gly Thr Cys Ala Cys Cys Thr Cys Gly Gly Cys Cys
   1370 1375 1380
   Cys Cys Gly Gly Ala Cys Ala Cys Cys Ala Gly Gly Cys Cys Gly
   1385 1390 1395
   Gly Cys Cys Cys Cys Gly Gly Gly Cys Thr Cys Cys Ala Cys Cys
   1400 1405 1410
   Gly Cys Cys Cys Cys Cys Cys Cys Ala Gly Cys Cys Cys Ala Cys
   1415 1420 1425
   Gly Gly Thr Gly Thr Cys Ala Cys Cys Thr Cys Gly Gly Cys Cys
   1430 1435 1440
   Cys Cys Gly Gly Ala Cys Ala Cys Cys Ala Gly Gly Cys Cys Gly
   1445 1450 1455
   Gly Cys Cys Cys Cys Gly Gly Gly Cys Thr Cys Cys Ala Cys Cys
   1460 1465 1470
   Gly Cys Cys Cys Cys Cys Cys Cys Ala Gly Cys Cys Cys Ala Cys
   1475 1480 1485
   Gly Gly Thr Gly Thr Cys Ala Cys Cys Thr Cys Gly Gly Cys Cys
   1490 1495 1500
   Cys Cys Gly Gly Ala Cys Ala Cys Cys Ala Gly Gly Cys Cys Gly
   1505 1510 1515
   Gly Cys Cys Cys Cys Gly Gly Gly Cys Thr Cys Cys Ala Cys Cys
   1520 1525 1530
   Gly Cys Cys Cys Cys Cys Cys Cys Ala Gly Cys Cys Cys Ala Cys
   1535 1540 1545
   Gly Gly Thr Gly Thr Cys Ala Cys Cys Thr Cys Gly Gly Cys Cys
   1550 1555 1560
   Cys Cys Gly Gly Ala Cys Ala Cys Cys Ala Gly Gly Cys Cys Gly
   1565 1570 1575
   Gly Cys Cys Cys Cys Gly Gly Gly Cys Thr Cys Cys Ala Cys Cys
   1580 1585 1590
   Gly Cys Cys Cys Cys Cys Cys Cys Ala Gly Cys Cys Cys Ala Cys
   1595 1600 1605
   Gly Gly Thr Gly Thr Cys Ala Cys Cys Thr Cys Gly Gly Cys Cys
   1610 1615 1620
   Cys Cys Gly Gly Ala Cys Ala Cys Cys Ala Gly Gly Cys Cys Gly
   1625 1630 1635
   Gly Cys Cys Cys Cys Gly Gly Gly Cys Thr Cys Cys Ala Cys Cys
   1640 1645 1650
   Gly Cys Cys Cys Cys Cys Cys Cys Ala Gly Cys Cys Cys Ala Cys
   1655 1660 1665
   Gly Gly Thr Gly Thr Cys Ala Cys Cys Thr Cys Gly Gly Cys Cys
   1670 1675 1680
   Cys Cys Gly Gly Ala Cys Ala Cys Cys Ala Gly Gly Cys Cys Gly
   1685 1690 1695
   Gly Cys Cys Cys Cys Gly Gly Gly Cys Thr Cys Cys Ala Cys Cys
   1700 1705 1710
   Gly Cys Cys Cys Cys Cys Cys Cys Ala Gly Cys Cys Cys Ala Cys
   1715 1720 1725
   Gly Gly Thr Gly Thr Cys Ala Cys Cys Thr Cys Gly Gly Cys Cys
   1730 1735 1740
   Cys Cys Gly Gly Ala Cys Ala Cys Cys Ala Gly Gly Cys Cys Gly
   1745 1750 1755
   Gly Cys Cys Cys Cys Gly Gly Gly Cys Thr Cys Cys Ala Cys Cys
   1760 1765 1770
   Gly Cys Cys Cys Cys Cys Cys Cys Ala Gly Cys Cys Cys Ala Cys
   1775 1780 1785
   Gly Gly Thr Gly Thr Cys Ala Cys Cys Thr Cys Gly Gly Cys Cys
   1790 1795 1800
   Cys Cys Gly Gly Ala Cys Ala Cys Cys Ala Gly Gly Cys Cys Gly
   1805 1810 1815
   Gly Cys Cys Cys Cys Gly Gly Gly Cys Thr Cys Cys Ala Cys Cys
   1820 1825 1830
   Gly Cys Cys Cys Cys Cys Cys Cys Ala Gly Cys Cys Cys Ala Cys
   1835 1840 1845
   Gly Gly Thr Gly Thr Cys Ala Cys Cys Thr Cys Gly Gly Cys Cys
   1850 1855 1860
   Cys Cys Gly Gly Ala Cys Ala Cys Cys Ala Gly Gly Cys Cys Gly
   1865 1870 1875
   Gly Cys Cys Cys Cys Gly Gly Gly Cys Thr Cys Cys Ala Cys Cys
   1880 1885 1890
   Gly Cys Cys Cys Cys Cys Cys Cys Ala Gly Cys Cys Cys Ala Cys
   1895 1900 1905
   Gly Gly Thr Gly Thr Cys Ala Cys Cys Thr Cys Gly Gly Cys Cys
   1910 1915 1920
   Cys Cys Gly Gly Ala Cys Ala Cys Cys Ala Gly Gly Cys Cys Gly
   1925 1930 1935
   Gly Cys Cys Cys Cys Gly Gly Gly Cys Thr Cys Cys Ala Cys Cys
   1940 1945 1950
   Gly Cys Cys Cys Cys Cys Cys Cys Ala Gly Cys Cys Cys Ala Cys
   1955 1960 1965
   Gly Gly Thr Gly Thr Cys Ala Cys Cys Thr Cys Gly Gly Cys Cys
   1970 1975 1980
   Cys Cys Gly Gly Ala Cys Ala Cys Cys Ala Gly Gly Cys Cys Gly
   1985 1990 1995
   Gly Cys Cys Cys Cys Gly Gly Gly Cys Thr Cys Cys Ala Cys Cys
   2000 2005 2010
   Gly Cys Cys Cys Cys Cys Cys Cys Ala Gly Cys Cys Cys Ala Cys
   2015 2020 2025
   Gly Gly Thr Gly Thr Cys Ala Cys Cys Thr Cys Gly Gly Cys Cys
   2030 2035 2040
   Cys Cys Gly Gly Ala Cys Ala Cys Cys Ala Gly Gly Cys Cys Gly
   2045 2050 2055
   Gly Cys Cys Cys Cys Gly Gly Gly Cys Thr Cys Cys Ala Cys Cys
   2060 2065 2070
   Gly Cys Cys Cys Cys Cys Cys Cys Ala Gly Cys Cys Cys Ala Cys
   2075 2080 2085
   Gly Gly Thr Gly Thr Cys Ala Cys Cys Thr Cys Gly Gly Cys Cys
   2090 2095 2100
   Cys Cys Gly Gly Ala Cys Ala Cys Cys Ala Gly Gly Cys Cys Gly
   2105 2110 2115
   Gly Cys Cys Cys Cys Gly Gly Gly Cys Thr Cys Cys Ala Cys Cys
   2120 2125 2130
   Gly Cys Cys Cys Cys Cys Cys Cys Ala Gly Cys Cys Cys Ala Cys
   2135 2140 2145
   Gly Gly Thr Gly Thr Cys Ala Cys Cys Thr Cys Gly Gly Cys Cys
   2150 2155 2160
   Cys Cys Gly Gly Ala Cys Ala Cys Cys Ala Gly Gly Cys Cys Gly
   2165 2170 2175
   Gly Cys Cys Cys Cys Gly Gly Gly Cys Thr Cys Cys Ala Cys Cys
       2180                2185                2190
   Gly Cys  Cys Cys Cys Cys Cys  Cys Ala Gly Cys Cys  Cys Ala Cys
       2195                2200                2205
   Gly Gly  Thr Gly Thr Cys Ala  Cys Cys Thr Cys Gly  Gly Cys Cys
       2210                2215                2220
   Cys Cys  Gly Gly Ala Cys Ala  Cys Cys Ala Gly Gly  Cys Cys Gly
       2225                2230                2235
   Gly Cys  Cys Cys Cys Gly Gly  Gly Cys Thr Cys Cys  Ala Cys Cys
       2240                2245                2250
   Gly Cys  Cys Cys Cys Cys Cys  Cys Ala Gly Cys Cys  Cys Ala Cys
       2255                2260                2265
   Gly Gly  Thr Gly Thr Cys Ala  Cys Cys Thr Cys Gly  Gly Cys Cys
       2270                2275                2280
   Cys Cys  Gly Gly Ala Cys Ala  Cys Cys Ala Gly Gly  Cys Cys Gly
       2285                2290                2295
   Gly Cys  Cys Cys Cys Gly Gly  Gly Cys Thr Cys Cys  Ala Cys Cys
       2300                2305                2310
   Gly Cys  Cys Cys Cys Cys Cys  Cys Ala Gly Cys Cys  Cys Ala Cys
       2315                2320                2325
   Gly Gly  Thr Gly Thr Cys Ala  Cys Cys Thr Cys Gly  Gly Cys Cys
       2330                2335                2340
   Cys Cys  Gly Gly Ala Cys Ala  Cys Cys Ala Gly Gly  Cys Cys Gly
       2345                2350                2355
   Gly Cys  Cys Cys Cys Gly Gly  Gly Cys Thr Cys Cys  Ala Cys Cys
       2360                2365                2370
   Gly Cys  Cys Cys Cys Cys Cys  Cys Ala Gly Cys Cys  Cys Ala Cys
       2375                2380                2385
   Gly Gly  Thr Gly Thr Cys Ala  Cys Cys Thr Cys Gly  Gly Cys Cys
       2390                2395                2400
   Cys Cys  Gly Gly Ala Cys Ala  Cys Cys Ala Gly Gly  Cys Cys Gly
       2405                2410                2415
   Gly Cys  Cys Cys Cys Gly Gly  Gly Cys Thr Cys Cys  Ala Cys Cys
       2420                 2425                 2430
   Gly Cys  Cys Cys Cys Cys Cys  Cys Ala Gly Cys Cys  Cys Ala Cys
       2435                 2440                 2445
   Gly Gly  Thr Gly Thr Cys Ala  Cys Cys Thr Cys Gly  Gly Cys Cys
       2450                 2455                 2460
   Cys Cys  Gly Gly Ala Cys Ala  Cys Cys Ala Gly Gly  Cys Cys Gly
       2465                 2470                 2475
   Gly Cys  Cys Cys Cys Gly Gly  Gly Cys Thr Cys Cys  Ala Cys Cys
       2480                 2485                 2490
   Gly Cys  Cys Cys Cys Cys Cys  Cys Ala Gly Cys Cys  Cys Ala Cys
       2495                 2500                 2505
   Gly Gly  Thr Gly Thr Cys Ala  Cys Cys Thr Cys Gly  Gly Cys Cys
       2510                 2515                 2520
   Cys Cys  Gly Gly Ala Cys Ala  Cys Cys Ala Gly Gly  Cys Cys Gly
       2525                 2530                 2535
   Gly Cys  Cys Cys Cys Gly Gly  Gly Cys Thr Cys Cys  Ala Cys Cys
       2540                 2545                 2550
   Gly Cys  Cys Cys Cys Cys Cys  Cys Ala Gly Cys Cys  Cys Ala Cys
       2555                 2560                 2565
   Gly Gly  Thr Gly Thr Cys Ala  Cys Cys Thr Cys Gly  Gly Cys Cys
       2570                 2575                 2580
   Cys Cys  Gly Gly Ala Cys Ala  Cys Cys Ala Gly Gly  Cys Cys Gly
       2585                 2590                 2595
   Gly Cys  Cys Cys Cys Gly Gly  Gly Cys Thr Cys Cys  Ala Cys Cys
       2600                 2605                 2610
   Gly Cys  Cys Cys Cys Cys Cys  Cys Ala Gly Cys Cys  Cys Ala Cys
       2615                 2620                 2625
   Gly Gly  Thr Gly Thr Cys Ala  Cys Cys Thr Cys Gly  Gly Cys Cys
       2630                 2635                 2640
   Cys Cys  Gly Gly Ala Cys Ala  Cys Cys Ala Gly Gly  Cys Cys Gly
       2645                 2650                 2655
   Gly Cys Cys Cys Cys Gly Gly Gly Cys Thr Cys Cys Ala Cys Cys
   2660 2665 2670
   Gly Cys Cys Cys Cys Cys Cys Cys Ala Gly Cys Cys Cys Ala Cys
   2675 2680 2685
   Gly Gly Thr Gly Thr Cys Ala Cys Cys Thr Cys Gly Gly Cys Cys
   2690 2695 2700
   Cys Cys Gly Gly Ala Cys Ala Cys Cys Ala Gly Gly Cys Cys Gly
   2705 2710 2715
   Gly Cys Cys Cys Cys Gly Gly Gly Cys Thr Cys Cys Ala Cys Cys
   2720 2725 2730
   Gly Cys Cys Cys Cys Cys Cys Cys Ala Gly Cys Cys Cys Ala Cys
   2735 2740 2745
   Gly Gly Thr Gly Thr Cys Ala Cys Cys Thr Cys Gly Gly Cys Cys
   2750 2755 2760
   Cys Cys Gly Gly Ala Cys Ala Cys Cys Ala Gly Gly Cys Cys Gly
   2765 2770 2775
   Gly Cys Cys Cys Cys Gly Gly Gly Cys Thr Cys Cys Ala Cys Cys
   2780 2785 2790
   Gly Cys Cys Cys Cys Cys Cys Cys Ala Gly Cys Cys Cys Ala Thr
   2795 2800 2805
   Gly Gly Thr Gly Thr Cys Ala Cys Cys Thr Cys Gly Gly Cys Cys
   2810 2815 2820
   Cys Cys Gly Gly Ala Cys Ala Ala Cys Ala Gly Gly Cys Cys Cys
   2825 2830 2835
   Gly Cys Cys Thr Thr Gly Gly Gly Cys Thr Cys Cys Ala Cys Cys
   2840 2845 2850
   Gly Cys Cys Cys Cys Thr Cys Cys Ala Gly Thr Cys Cys Ala Cys
   2855 2860 2865
   Ala Ala Thr Gly Thr Cys Ala Cys Cys Thr Cys Gly Gly Cys Cys
   2870 2875 2880
   Thr Cys Ala Gly Gly Cys Thr Cys Thr Gly Cys Ala Thr Cys Ala
   2885 2890 2895
   Gly Gly Cys Thr Cys Ala Gly Cys Thr Thr Cys Thr Ala Cys Thr
   2900 2905 2910
   Cys Thr Gly Gly Thr Gly Cys Ala Cys Ala Ala Cys Gly Gly Cys
   2915 2920 2925
   Ala Cys Cys Thr Cys Thr Gly Cys Cys Ala Gly Gly Gly Cys Thr
   2930 2935 2940
   Ala Cys Cys Ala Cys Ala Ala Cys Cys Cys Cys Ala Gly Cys Cys
   2945 2950 2955
   Ala Gly Cys Ala Ala Gly Ala Gly Cys Ala Cys Thr Cys Cys Ala
   2960 2965 2970
   Thr Thr Cys Thr Cys Ala Ala Thr Thr Cys Cys Cys Ala Gly Cys
   2975 2980 2985
   Cys Ala Cys Cys Ala Cys Thr Cys Thr Gly Ala Thr Ala Cys Thr
   2990 2995 3000
   Cys Cys Thr Ala Cys Cys Ala Cys Cys Cys Thr Thr Gly Cys Cys
   3005 3010 3015
   Ala Gly Cys Cys Ala Thr Ala Gly Cys Ala Cys Cys Ala Ala Gly
   3020 3025 3030
   Ala Cys Thr Gly Ala Thr Gly Cys Cys Ala Gly Thr Ala Gly Cys
   3035 3040 3045
   Ala Cys Thr Cys Ala Cys Cys Ala Thr Ala Gly Cys Thr Cys Gly
   3050 3055 3060
   Gly Thr Ala Cys Cys Thr Cys Cys Thr Cys Thr Cys Ala Cys Cys
   3065 3070 3075
   Thr Cys Cys Thr Cys Cys Ala Ala Thr Cys Ala Cys Ala Gly Cys
   3080 3085 3090
   Ala Cys Thr Thr Cys Thr Cys Cys Cys Cys Ala Gly Thr Thr Gly
   3095 3100 3105
   Thr Cys Thr Ala Cys Thr Gly Gly Gly Gly Thr Cys Thr Cys Thr
   3110 3115 3120
   Thr Thr Cys Thr Thr Thr Thr Thr Cys Cys Thr Gly Thr Cys Thr
   3125 3130 3135
   Thr Thr Thr Cys Ala Cys Ala Thr Thr Thr Cys Ala Ala Ala Cys
   3140 3145 3150
   Cys Thr Cys Cys Ala Gly Thr Thr Thr Ala Ala Thr Thr Cys Cys
   3155 3160 3165
   Thr Cys Thr Cys Thr Gly Gly Ala Ala Gly Ala Thr Cys Cys Cys
   3170 3175 3180
   Ala Gly Cys Ala Cys Cys Gly Ala Cys Thr Ala Cys Thr Ala Cys
   3185 3190 3195
   Cys Ala Ala Gly Ala Gly Cys Thr Gly Cys Ala Gly Ala Gly Ala
   3200 3205 3210
   Gly Ala Cys Ala Thr Thr Thr Cys Thr Gly Ala Ala Ala Thr Gly
   3215 3220 3225
   Thr Thr Thr Thr Thr Gly Cys Ala Gly Ala Thr Thr Thr Ala Thr
   3230 3235 3240
   Ala Ala Ala Cys Ala Ala Gly Gly Gly Gly Gly Thr Thr Thr Thr
   3245 3250 3255
   Cys Thr Gly Gly Gly Cys Cys Thr Cys Thr Cys Cys Ala Ala Thr
   3260 3265 3270
   Ala Thr Thr Ala Ala Gly Thr Thr Cys Ala Gly Gly Cys Cys Ala
   3275 3280 3285
   Gly Gly Ala Thr Cys Thr Gly Thr Gly Gly Thr Gly Gly Thr Ala
   3290 3295 3300
   Cys Ala Ala Thr Thr Gly Ala Cys Thr Cys Thr Gly Gly Cys Cys
   3305 3310 3315
   Thr Thr Cys Cys Gly Ala Gly Ala Ala Gly Gly Thr Ala Cys Cys
   3320 3325 3330
   Ala Thr Cys Ala Ala Thr Gly Thr Cys Cys Ala Cys Gly Ala Cys
   3335 3340 3345
   Gly Thr Gly Gly Ala Gly Ala Cys Ala Cys Ala Gly Thr Thr Cys
   3350 3355 3360
   Ala Ala  Thr Cys Ala Gly Thr  Ala Thr Ala Ala Ala  Ala Cys Gly
       3365                 3370                 3375
   Gly Ala  Ala Gly Cys Ala Gly  Cys Cys Thr Cys Thr  Cys Gly Ala
       3380                 3385                 3390
   Thr Ala  Thr Ala Ala Cys Cys  Thr Gly Ala Cys Gly  Ala Thr Cys
       3395                 3400                 3405
   Thr Cys  Ala Gly Ala Cys Gly  Thr Cys Ala Gly Cys  Gly Thr Gly
       3410                 3415                 3420
   Ala Gly  Thr Gly Ala Thr Gly  Thr Gly Cys Cys Ala  Thr Thr Thr
       3425                 3430                 3435
   Cys Cys  Thr Thr Thr Cys Thr  Cys Thr Gly Cys Cys  Cys Ala Gly
       3440                 3445                 3450
   Thr Cys  Thr Gly Gly Gly Gly  Cys Thr Gly Gly Gly  Gly Thr Gly
       3455                 3460                 3465
   Cys Cys  Ala Gly Gly Cys Thr  Gly Gly Gly Gly Cys  Ala Thr Cys
       3470                 3475                 3480
   Gly Cys  Gly Cys Thr Gly Cys  Thr Gly Gly Thr Gly  Cys Thr Gly
       3485                 3490                 3495
   Gly Thr  Cys Thr Gly Thr Gly  Thr Thr Cys Thr Gly  Gly Thr Thr
       3500                 3505                 3510
   Gly Cys  Gly Cys Thr Gly Gly  Cys Cys Ala Thr Thr  Gly Thr Cys
       3515                 3520                 3525
   Thr Ala  Thr Cys Thr Cys Ala  Thr Thr Gly Cys Cys  Thr Thr Gly
       3530                 3535                 3540
   Gly Cys  Thr Gly Thr Cys Thr  Gly Thr Cys Ala Gly  Thr Gly Cys
       3545                 3550                 3555
   Cys Gly  Cys Cys Gly Ala Ala  Ala Gly Ala Ala Cys  Thr Ala Cys
       3560                 3565                 3570
   Gly Gly  Gly Cys Ala Gly Cys  Thr Gly Gly Ala Cys  Ala Thr Cys
       3575                 3580                 3585
   Thr Thr  Thr Cys Cys Ala Gly  Cys Cys Cys Gly Gly  Gly Ala Thr
       3590                 3595                 3600
   Ala Cys  Cys Thr Ala Cys Cys  Ala Thr Cys Cys Thr  Ala Thr Gly
       3605                 3610                 3615
   Ala Gly  Cys Gly Ala Gly Thr  Ala Cys Cys Cys Cys  Ala Cys Cys
       3620                 3625                 3630
   Thr Ala  Cys Cys Ala Cys Ala  Cys Cys Cys Ala Thr  Gly Gly Gly
       3635                 3640                 3645
   Cys Gly  Cys Thr Ala Thr Gly  Thr Gly Cys Cys Cys  Cys Cys Thr
       3650                 3655                 3660
   Ala Gly  Cys Ala Gly Thr Ala  Cys Cys Gly Ala Thr  Cys Gly Thr
       3665                 3670                 3675
   Ala Gly  Cys Cys Cys Cys Thr  Ala Thr Gly Ala Gly  Ala Ala Gly
       3680                 3685                 3690
   Gly Thr  Thr Thr Cys Thr Gly  Cys Ala Gly Gly Thr  Ala Ala Cys
       3695                 3700                 3705
   Gly Gly  Thr Gly Gly Cys Ala  Gly Cys Ala Gly Cys  Cys Thr Cys
       3710                 3715                 3720
   Thr Cys  Thr Thr Ala Cys Ala  Cys Ala Ala Ala Cys  Cys Cys Ala
       3725                 3730                 3735
   Gly Cys  Ala Gly Thr Gly Gly  Cys Ala Gly Cys Cys  Gly Cys Thr
       3740                 3745                 3750
   Thr Cys  Thr Gly Cys Cys Ala  Ala Cys Thr Thr Gly  Thr Ala Gly
       3755                 3760                 3765

## Description

### Field of the Invention

The present invention relates to a composition for use in the treatment of non-small cell lung cancer as defined in the attached claims. In particular, the present invention provides a composition for use in the treatment of non-small cell lung cancer, the composition comprising six mRNAs, wherein each mRNA encodes a different antigen selected from the group consisting of 5T4 (Trophoblast glycoprotein, TPBG); Survivin (Baculoviral IAP repeat-containing protein 5; BIRC5), NY-ESO-1 (New York esophageal squamous cell carcinoma 1; CTAG1B), MAGE-C1 (Melanoma antigen family C1), MAGE-C2 (Melanoma antigen family C2), and MUC1 (Mucin 1), and wherein each mRNA is identical to a different RNA sequence selected from the RNA sequences according to SEQ ID NO: 19, 20, 21, 22, 23 or 24, wherein each mRNA is complexed with a cationic compound selected from the group consisting of a cationic peptide, a cationic protein, a cationic polysaccharide, a cationic polymer and a cationic lipid, and wherein the treatment further comprises administration of a chemotherapeutic agent. The invention furthermore relates to a vaccine for use in the treatment of non-small cell lung cancer, the vaccine comprising the composition, wherein the treatment further comprises administration of a chemotherapeutic agent. Furthermore, the invention concerns a combination of six mRNAs for use in the treatment of non-small cell lung cancer, wherein each mRNA encodes one antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1) and wherein each mRNA is identical to a different RNA sequence selected from the RNA sequences according to SEQ ID NO: 19, 20, 21, 22, 23 or 24, and wherein each mRNA is complexed with a cationic compound selected from the group consisting of a cationic peptide, a cationic protein, a cationic polysaccharide, a cationic polymer and a cationic lipid, and wherein the treatment further comprises administration of a chemotherapeutic agent. Finally, the invention relates to kits, particularly to kits of parts, containing the composition and/or the vaccine for use in the treatment of non-small cell lung cancer as defined in the claims.

### Background of the invention

Of all malignant tumors 25 % are bronchial carcinoma (carcinoma of the lung). Worldwide, it is the most common cause of cancer-related death in men and the second most common in women. In Germany it is the third most abundant sort of carcinoma following carcinoma of the prostata and the colorectal carcinoma. It is responsible for 1.3 million deaths worldwide annually. In Central Europe the incidence is approximately 60 per 100.000 inhabitants and the number of newly people diagnosed with lung cancer is steadily on the rise (in Germany currently being at approximately 50.000 per year). When diagnosed with lung cancer, the avarage overall fife-year survival rate is a mere 5 percent. Nevertheless, the life expectancy of each single patient is wholly dependent on the disease stage (TMN classification) and the subtype of carcinoma (lung cancer) encountered (see below).

The main sub-types of lung cancer categorized by the size and appearance of the malignant cells identified under the microscope are small cell lung cancer (20%) and non-small cell lung cancer (NSCLC) (80%). This classification, although based on simple histological criteria, has very important implications for clinical management and prognosis of the disease, with small cell lung cancer usually being treated by chemotherapy, while non-small cell lung cancer is mostly subject to surgery as a first-line treatment.

The non-small cell lung cancers (NSCLC) are grouped together because their prognosis and management are roughly identical. There are three main sub-types: squamous cell lung carcinoma, adenocarcinoma and large cell lung carcinoma. Surgery is the mainstay of treatment; however, only a quarter of the patients undergo successful resection, with a recurrence rate of 50%. Therapeutic approaches in advanced disease involve - following surgery - both adjuvant chemotherapy and/or adjuvant radiotherapy, whereas chemotherapy as monotherapy (first-line therapy) seems to be an approach associated with relatively poor results. In a comparison of four commonly used combination chemotherapy regimens, none was superior. Response rates varied from 15% to 22%, with 1-year survival rates of 31% to 36% (see e.g. O'Mahony, D., S. Kummar, et al. (2005). "Non-small-cell lung cancer vaccine therapy: a concise review." J Clin Oncol 23(35): 9022-8). Thus, even though preoperative chemotherapy seems to have not resulted in a prolongation of life expectancy, adjuvant chemotherapy - also if combined with radiotherapy - did show a significant increase in life expectancy.

One of the chemotherapeutic approaches used today are combinations of platin-based substances with e.g. Gemcitabin even as first-line-therapy, wheras e.g. Pemetrexed is used as second-line therapy.

Another option used for the treatment of NSCLC is the so-called "Targeted Therapy" trying to enhance success of classical cytotoxic chemotherapy by influencing tumor specific target structures on a molecular level. Substances used include Bevacizumab (an angiogenesis inhibitor) or Erlotinib, which is aimed at the tyrosine kinases of the epidermal growth factor receptor (EGFR).

Even though doubtless there is some improvement in the current therapeutic approaches, treatment of lung cancer, especially of NSCLC, is still an uphill-struggle with - given the high mortality rates - a strong need for further, alternative or improved ways of treatment.

Thus, it is suggested here to use the immune system in an approach for the treatment of the NSCLC. The immune system plays an important role in the treatment and prevention of numerous diseases. According to the present stage of knowledge, various mechanisms are provided by mammalians to protect the organism by identifying and killing e.g. tumor cells. These tumor cells have to be detected and distinguished from the organism's normal cells and tissues.

The immune system of vertebrates such as humans consists of many types of proteins, cells, organs, and tissues, which interact in an elaborate and dynamic network. As part of this more complex immune response, the vertebrate system adapts over time to recognize particular pathogens or tumor cells more efficiently. The adaptation process creates immunological memories and allows even more effective protection during future encounters. This process of adaptive or acquired immunity forms the basis for vaccination strategies.

The adaptive immune system is antigen-specific and requires the recognition of specific "self" or "non-self" antigens during a process called antigen presentation. Antigen specificity allows for the generation of responses that are tailored to specific pathogens or pathogen-infected cells or tumor cells. The ability to mount these tailored responses is maintained in the body by so called "memory cells". Should a pathogen infect the body more than once, these specific memory cells are used to quickly eliminate it. The adaptive immune system thus allows for a stronger immune response as well as for an immunological memory, where each pathogen or tumor cell is "remembered" by one or more signature antigens.

The major components of the adaptive immune system in vertebrates predominantly include lymphocytes on the cellular level and antibodies on the molecular level. Lymphocytes as cellular components of the adaptive immune system include B cells and T cells which are derived from hematopoietic stem cells in the bone marrow. B cells are involved in the humoral response, whereas T cells are involved in cell mediated immune response. Both B cells and T cells carry receptor molecules that recognize specific targets. T cells recognize a "non-self" target, such as a pathogenic target structure, only after antigens (e.g. small fragments of a pathogen) have been processed and presented in combination with a "self" receptor called a major histocompatibility complex (MHC) molecule. In contrast, the B cell antigen-specific receptor is an antibody molecule on the B cell surface, and recognizes pathogens as such when antibodies on its surface bind to a specific foreign antigen. This antigen/antibody complex is taken up by the B cell and processed by proteolysis into peptides. The B cell then displays these antigenic peptides on its surface MHC class II molecules. This combination of MHC and antigen attracts a matching helper T cell, which releases lymphokines and activates the B cell. As the activated B cell then begins to divide, its offspring secretes millions of copies of the antibody that recognizes this antigen. These antibodies circulate in blood plasma and lymph, bind to pathogens or tumor cells expressing the antigen and mark them for destruction by complement activation or for uptake and destruction by phagocytes.

As a cellular component of the adaptive immune system cytotoxic T cells (CD8⁺) may form a CTL-response. Cytotoxic T cells (CD8⁺) can recognize peptides from endogenous pathogens and self-antigens bound by MHC type I molecules. CD8⁺-T cells carry out their killing function by releasing cytotoxic proteins in the cell.

Mechanisms of the immune system form targets for curative treatments. Appropriate methods are typically based on the administration of adjuvants to elicit an innate immune response or on the administration of antigens or immunogens in order to evoke an adaptive immune response. As antigens are typically based on specific components of pathogens (e.g. surface proteins) or fragments thereof, administration of nucleic acids to the patient which is followed by the expression of desired polypeptides, proteins or antigens is envisaged as well.

Hitherto conventional methods for eliciting the immune response, immunization or vaccination are based on the use of DNA molecules in order to incorporate the required genetic information into the cell. Various methods have been developed for introducing DNA into cells, such as calcium phosphate transfection, polyprene transfection, protoplast fusion, electroporation, microinjection and lipofection, lipofection having in particular proven to be a suitable method. DNA viruses may likewise be used as a DNA vehicle. Because of their infectious properties, such viruses achieve a very high transfection rate. The viruses used are genetically modified in such a manner that no functional infectious particles are formed in the transfected cell. Despite these precautions, however, it is not possible to rule out the risk of uncontrolled propagation of the introduced gene and viral genes, for example due to potential recombination events. This also entails the risk of the DNA being inserted into an intact gene of the host cell's genome by e.g. recombination, with the consequence that this gene may be mutated and thus completely or partially inactivated or may give rise to misinformation. In other words, synthesis of a gene product which is vital to the cell may be completely suppressed or alternatively a modified or incorrect gene product is expressed. One particular risk occurs if the DNA is integrated into a gene which is involved in the regulation of cell growth. In this case, the host cell may become degenerate and lead to cancer or tumor formation. Furthermore, if the DNA introduced into the cell is to be expressed, it is necessary for the corresponding DNA vehicle to contain a strong promoter, such as the viral CMV promoter. The integration of such promoters into the genome of the treated cell may result in unwanted alterations of the regulation of gene expression in the cell. Another risk of using DNA as an agent to induce an immune response (e.g. as a vaccine) is the induction of pathogenic anti-DNA antibodies in the patient into whom the foreign DNA has been introduced, so bringing about a (possibly fatal) immune response.

Thus, in order to effectively stimulate the immune system to allow treatment of lung cancer while avoiding the problems of uncontrolled propagation of an introduced gene due to DNA based compositions, RNA based compositions have been developed. WO2009/046738 provides a composition comprising at least one RNA encoding at least one antigen selected from the group consisting of NY-ESO-1, MAGE-C1 and MAGE-C2 and further encoding at least one antigen selected from the group consisting of hTERT, WT1, MAGE-A2, 5T4, MAGE-A3, MUC1, Her-2/neu, NY-ESO-1, CEA, Survivin, MAGE-C1 and/or MAGE-C2. Even though the combination of at least two antigens in said composition represents an important step towards an active immunotherapy against lung cancer, the practitioner is still faced - when looking for treatment options for an individual subject - with the selection of a suitable antigen combination, which is both, effective and well-tolerated. Also WO 2009/046974 concerns RNA compositions for use in lung cancer treatment and describes vaccination of mice with a composition comprising five RNAs encoding NY-ESO-1, MAGE-C2, MAGE-C1, Survivin and 5T4.

Thus overall, there is room and a need for an efficient system, which may be used to effectively stimulate the immune system to allow treatment of lung cancer, especially of non-small cell lung cancer (NSCLC), while avoiding the problems encountered when using compositions that are known in the art.

It is thus an object of the present invention to provide a composition, which a) allows treatment of lung cancer by stimulating the immune system, while b) avoiding the above mentioned disadvantages.

It is thus an object of the present invention to provide a lung cancer vaccine or a composition for treatment of lung cancer by stimulating the immune system.

The object underlying the present invention is solved by the claimed subject matter.

### Summary of the Invention

The invention is set out in the appended set of claims. The embodiments and/or examples of the following description which are not covered by the appended claims are considered as not being part of the present invention. The object mentioned above is solved by the subject matter of the present invention, particularly by a composition for use in the treatment of non-small cell lung cancer, the composition comprising mRNAs, wherein each mRNA encodes a different antigen selected from the group consisting of:
- 5T4 (Trophoblast glycoprotein, TPBG);
- Survivin (Baculoviral IAP repeat-containing protein 5; BIRC5),
- NY-ESO-1 (New York esophageal squamous cell carcinoma 1; CTAG1 B),
- MAGE-C1 (Melanoma antigen family C1);
- MAGE-C2 (Melanoma antigen family C2), and
- MUC1 (Mucin 1),
and wherein each mRNA is identical to a different RNA sequence selected from the RNA sequences according to SEQ ID NO: 19, 20, 21, 22, 23 or 24, wherein each mRNA is complexed with a cationic compound selected from the group consisting of a cationic peptide, a cationic protein, a cationic polysaccharide, a cationic polymer and a cationic lipid, and wherein the treatment further comprises administration of a chemotherapeutic agent.

Surprisingly, it has been found that the specific combination of the antigens, antigenic proteins or antigenic peptides of the afore mentioned group encoded by at least six mRNAs of the composition for use according to the present invention, is capable of effectively stimulating the (adaptive) immune system to allow treatment of non-small cell lung cancers, wherein the treatment further comprises administration of a chemotherapeutic agent. The advantageous effects on the treatment of non-small cell lung cancer are achieved irrespective of whether the combination of antigens as described herein is applied as one single composition or by separate administration of the individual antigens. Accordingly, any combination of antigens described herein, e.g. in the form of six separate mRNA formulations, may fulfil the very same purposes and achieves the desired effect. The number of responders to such vaccination strategy is expected to be significantly increased as compared to other approaches. Herein, the terms antigens, antigenic proteins or antigenic peptides may be used synomously. In the context of the present disclosure, a composition shall be further understood as a composition, which is able to elicit an immune response, preferably an adaptive immune response as defined herein, due to at least one of the component(s) contained in the composition or, rather, due to at least one of the antigens encoded by the at least one component of the composition, i.e. by at least one mRNA encoding the antigens as defined above. The combination of antigens, whether administered separately (e.g. concurrently) or as one single composition, is capable of eliciting the desired immune response. Separate administration may mean that the distinct mRNAs are either essentially simultaneously, e.g. within 10 minutes or time-staggered over an extended period of time, e.g. more than 30 minutes.

In the following, the combination of antigens as employed according to the invention will be illustrated by the description of a composition comprising six mRNAs encoding the combination of antigens. It is understood that the six mRNAs as employed according to the invention are characterized by the features as described herein.

Among the large amount of antigens expressed in lung cancer cells, according to the present invention the six antigens as defined herein, i.e. 5T4, Survivin, NY-ESO-1, MAGE-C1, MAGE-C2 and MUC1 have been selected. These antigens have been identified as potential targets in immunotherapy. According to the invention, each of the above antigens is encoded by the at least one ORF/coding region/coding sequence provided by the at least six mRNAs. In this context, a messenger RNA is typically a single-stranded RNA, which is composed of (at least) several structural elements, e.g. an optional 5' UTR region, an upstream positioned ribosomal binding site followed by a coding region, an optional 3' UTR region, which may be followed by a poly-A tail (and/or a poly-C tail). A coding region for each of the at least six antigens is located on separate mRNAs of the composition. More preferred embodiments for the at least six mRNAs are provided below:
According to the present invention, one mRNA of the composition encodes 5T4. "5T4" is trophoblast glycoprotein. Harrop, Connolly *et al.* (2006) reported that the human oncofetal antigen 5T4 is a 72-kDa leucine-rich membrane glycoprotein which is expressed at high levels on the placenta and also on a wide range of human carcinomas including colorectal, gastric, renal, and ovarian cancers but rarely on normal tissues (see Harrop, R., N. Connolly, et al. (2006). "Vaccination of colorectal cancer patients with modified Vaccinia Ankara delivering the tumor antigen 5T4 (TroVax) induces immune responses which correlate with disease control: a phase I/II trial." Clin Cancer Res 12(11 Pt 1): 3416-24). Overexpression of 5T4 is associated with poor prognosis in patients with colorectal, gastric, and ovarian carcinoma. Despite such compounding factors, 5T4-specific cellular and/or humoral immune responses were induced in the majority of patients (16 of 17; 94%) following TroVax immunization, which was considered encouraging compared with many other cancer immunotherapy trials. In summary, they showed safety and immunogenicity of TroVax delivered via i.m. and i.d. routes of administration. Zhao and Wang (2007) (Zhao, Y. and Y. Wang (2007). "5T4 oncotrophoblast glycoprotein: janus molecule in life and a novel potential target against tumors." Cell Mol Immunol 4(2): 99-104) reported that 5T4 oncotrophoblast glycoprotein is a transmembrane protein expressed on the embryonic tissue and various malignant tumor cell surfaces. It plays a vital role in the multiple biological and pathological processes including massive cellular migration during the embryogenesis, cell invasion associated with implantation, and neoplastic metastasis in the progression of tumorigenesis. According to Kopreski, Benko *et a*/*.* (2001) 5T4 is a trophoblast glycoprotein frequently overexpressed in epithelial malignancies that provides a potential target for cancer therapeutics (see Kopreski, M. S., F. A. Benko, et a/. (2001). "Circulating RNA as a tumor marker: detection of 5T4 mRNA in breast and lung cancer patient serum." Ann N Y Acad Sci 945: 172-8). Serum was collected from 19 patients with advanced breast cancer (5 patients) or non-small-cell lung cancer (14 patients), and from 25 normal control volunteers having amplifiable RNA. RNA extracted from the serum was RT-PCR amplified using heminested, two-stage reactions, with products detected by gel electrophoresis. 5T4 mRNA was reproducibly detected in 8/19 (42%) cancer patient sera, including 2/5 breast cancer patient sera and 6/14 lung cancer patient sera, but in only 3/25 (12%) normal control sera (p = 0.035).

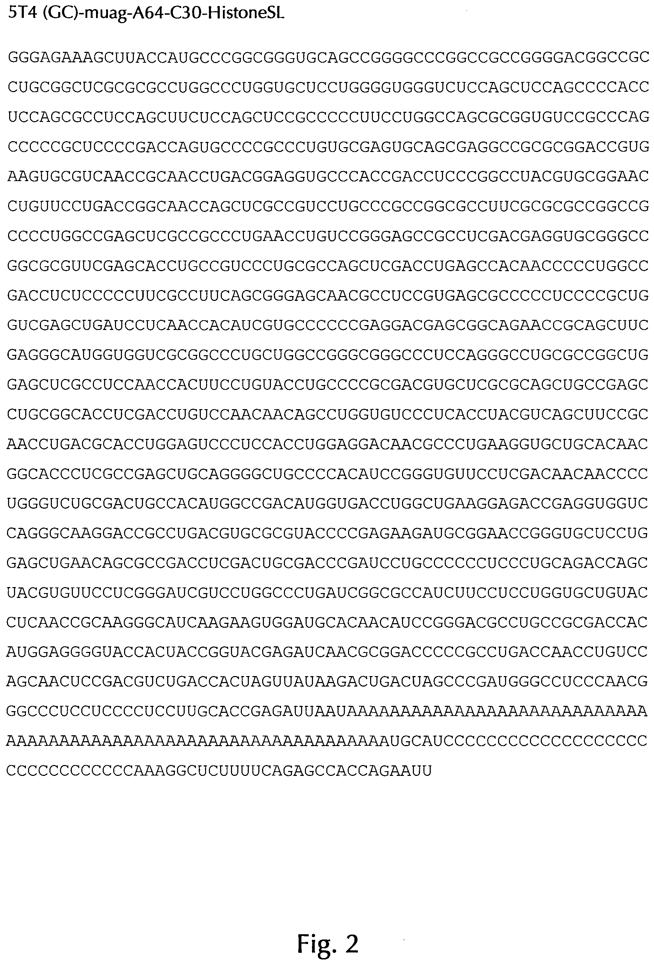

In the context of this invention, the sequence of the mRNA encoding 5T4 antigen consists of a sequence as shown in Fig. 2 (SEQ ID NO: 19).

Furthermore, one mRNA of the composition encodes Survivin. "Survivin" is baculoviral IAP repeat-containing 5 (survivin). Grube, Moritz *et al.* (2007) described Survivin (see Grube, M., S. Moritz, et al. (2007). "CD8+ T cells reactive to survivin antigen in patients with multiple myeloma." Clin Cancer Res 13(3): 1053-60). Survivin is a member of the inhibitors of apoptosis family and is overexpressed in different types of malignancies. Cytotoxic T cells recognizing survivin epitopes can be elicited *in vitro* and by vaccination in patients with leukemia, breast cancer, and melanoma. It was investigated whether survivin-specific CD8+ T cells occur in patients with multiple myeloma and T cells recognizing HLA-A2.1-binding survivin peptide were detected in 9 of 23 patients and in 1 of 21 healthy volunteers. Survivin-reactive T cells were identified as terminally differentiated effector T cells (CD8+, CD45RA+, and CCR7-). Positive survivin expression of myeloma cells in bone marrow specimens was shown in 7 of 11 patients. Survivin is highly expressed in most human cancer cells of epithelial and hematopoietic origin, and overexpression is associated with cancer progression, poor prognosis, resistance, and short patient survival. Duffy, O'Donovan (2007) described that Survivin is a 16.5 kDa protein overexpressed in almost all malignancies but rarely detected in normal differentiated adult tissues (see Duffy, M. J., N. O'Donovan, et al. (2007). "Survivin: a promising tumor biomarker." Cancer Lett 249(1): 49-60). Functionally, survivin has been shown to inhibit apoptosis, promote cell proliferation and enhance angiogenesis. Consistent with its role in these processes, survivin was described as playing a key role in cancer progression. Because of the large difference in expression between normal and malignant tissue and its causal role in cancer progression, survivin is currently undergoing intensive investigation as a potential tumor marker. Emerging data suggests that measurement of survivin can aid the early diagnosis of bladder cancer, determine prognosis in multiple cancer types and predict response to diverse anticancer therapies. Zeis, Siegel *et al.* (2003) demonstrated that human survivin-specific CTLs generated from PBMC by stimulation with autologous dendritic cells transfected with survivin-RNA were cytotoxic for a range of hematopoietic malignant cell lines and primary tumor cells isolated from patients with acute myeloid leukemia (see Zeis, M., S. Siegel, et a/. (2003). "Generation of cytotoxic responses in mice and human individuals against hematological malignancies using survivin-RNA-transfected dendritic cells." J Immunol 170(11): 5391-7). It was also shown that vaccination of mice with survivin-RNA-transfected dendritic cells lead to long term resistance to challenge by a survivin-expressing lymphoma, demonstrating the potential of survivin as a tumor rejection Ag.

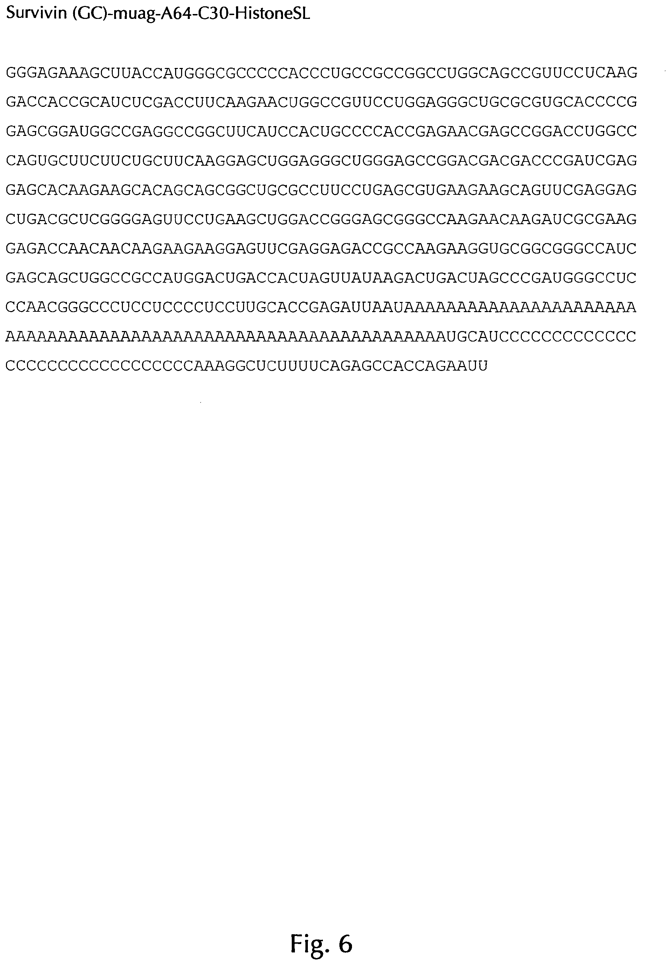

In the context of this invention, the mRNA encoding Survivin consists of a sequence as shown in Fig. 6 (SEQ ID NO: 20).

Furthermore one mRNA of the composition encodes NY-ESO-1. "NY-ESO-1" is cancer/testis antigen 1B. Chen, Scanlan *et al.* (1997) reported the mRNA expression of NY-ESO-1 in various human tumors by RT-PCR finding Melanoma 23/67, Ovarian cancer 2/8, Breast cancer 10/33, Thyroid cancer 2/5, Prostate cancer 4/16, Bladder cancer 4/5, Colon cancer 0/16, Burkitt lymphoma 1/2, Glioma 0/15, Basal cell carcinoma 0/2, Gastric cancer 0/12, Leiomyosarcoma 0/2, Lung cancer 2/12, Other sarcomas 0/2, Renal cancer 0/10, Pancreatic cancer 0/2, Lymphoma 0/10, Seminoma 0/1, Hepatoma 2/7, Spinal cord tumor 0/1 (see Chen, Y. T., M. J. Scanlan, et al. (1997). "A testicular antigen aberrantly expressed in human cancers detected by autologous antibody screening." Proc Natl Acad Sci U S A 94(5): 1914-8). Jager, Karbach *et a*/*.* (2006) reported that NY-ESO-1 is a cancer/testis antigen expressed in a range of human malignancies, and that a number of vaccine strategies targeting NY-ESO-1 were being developed (see Jager, E., J. Karbach, et al. (2006). "Recombinant vaccinia/fowlpox NY-ESO-1 vaccines induce both humoral and cellular NY-ESO-1-specific immune responses in cancer patients." Proc Natl Acad Sci U S A 103(39): 14453-8). In the presented study, the safety and immunogenicity of recombinant vaccinia-NY-ESO-1 and recombinant fowlpox-NY-ESO-1 were analyzed in a series of 36 patients with a range of different tumor types. Each construct was first tested individually at two different dose levels and then in a prime-boost setting with recombinant vaccinia-NY-ESO-1 followed by recombinant fowlpox-NY-ESO-1. The vaccines were well tolerated either individually or together. NY-ESO-1-specific antibody responses and/or specific CD8 and CD4 T cell responses directed against a broad range of NY-ESO-1 epitopes were induced by a course of at least four vaccinations at monthly intervals in a high proportion of patients. CD8 T cell clones derived from five vaccinated patients were shown to lyse NY-ESO-1-expressing melanoma target cells. In several patients with melanoma, there was a strong impression that the natural course of the disease was favorably influenced by vaccination. Davis, Chen *et a*/*.* (2004) reported that HLA-A2-restricted NY-ESO-1 peptides injected intradermally were shown to be safe and immunogenic (Davis, I. D., W. Chen, et al. (2004). "Recombinant NY-ESO-1 protein with ISCOMATRIX adjuvant induces broad integrated antibody and CD4(+) and CD8(+) T cell responses in humans." Proc Natl Acad Sci U S A 101(29): 10697-702). Although these trials were designed only to determine safety and immunogenicity, some patients showed tumor regression or stabilization of disease. It was further expressed by Jager, Gnjatic *et al.* (2000) that a broad NY-ESO-1-specific immune response including antibody and CD4 and CD8 T cell responses was seen after immunization with recombinant NY-ESO-1 protein combined with ISCOMATRIX adjuvant (CSL Ltd., Parkville, Victoria, Australia) in patients with resected NY-ESO-1-expressing melanoma (see Jager, E., S. Gnjatic, et a/. (2000). "Induction of primary NY-ESO-1 immunity: CD8+ T lymphocyte and antibody responses in peptide-vaccinated patients with NY-ESO-1+ cancers." Proc Natl Acad Sci U S A 97(22): 12198-203). This immune response to the vaccine appeared to be associated with long disease-free survival. Furthermore Odunsi, Qian *et a*/*.* (2007) reported that vaccination with an NY-ESO-1 peptide induces integrated humoral and T cell responses in ovarian cancer (see Odunsi, K., F. Qian, et al. (2007). "Vaccination with an NY-ESO-1 peptide of HLA class I/II specificities induces integrated humoral and T cell responses in ovarian cancer." Proc Natl Acad Sci U S A 104(31): 12837-42).

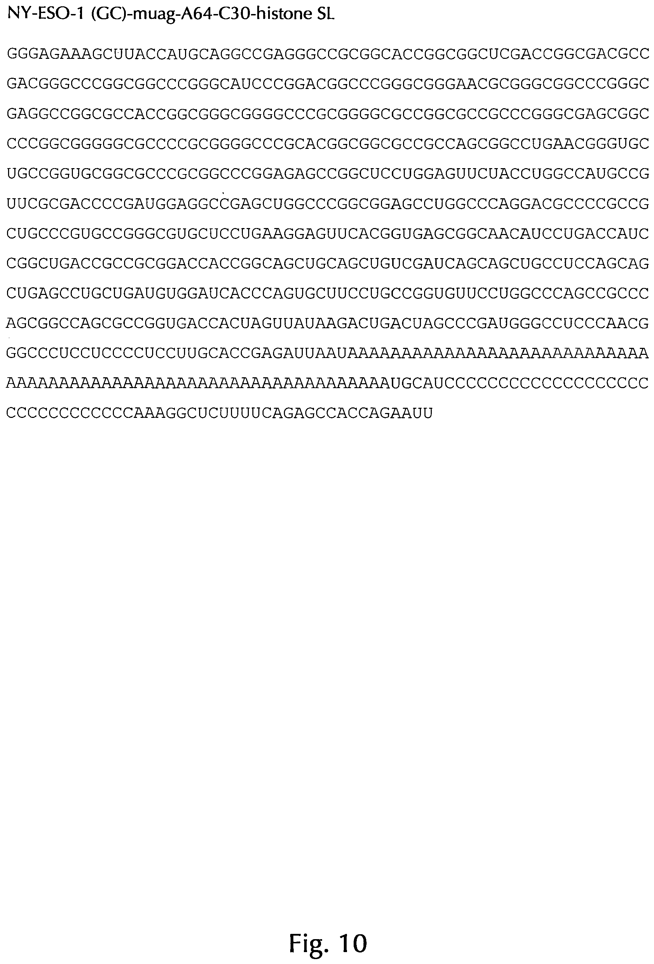

In the context of this invention, the mRNA encoding NY-ESO-1 antigen consists of a sequence as shown in Fig. 10 (SEQ ID NO: 21).

Furthermore, one mRNA of the composition encodes MAGE-C1. "MAGE-C1" is the melanoma antigen family C, 1. Lucas, De Smet *et al.* (1998) recently identified MAGE-C1 by performing RDA (see Lucas, S., C. De Smet, et al. (1998). "Identification of a new MAGE gene with tumor-specific expression by representational difference analysis." Cancer Res 58(4): 743-52). MAGE-C1 was not expressed in a panel of normal tissues tested with the exception of testis. Among tumoral samples, MAGE-C1 was frequently expressed in seminomas, melanomas, and bladder carcinomas. It was also expressed in a significant fraction of head and neck carcinomas, breast carcinomas, non-small lung carcinomas, prostate adenocarcinomas and sarcomas. Jungbluth, Chen *et al.* (2002) described expression in breast cancer, ovary cancer, liver cancer, testis cancer, bladder cancer, melanoma and non-small cell lung cancer (39%) (see Jungbluth, A. A., Y. T. Chen, et a/. (2002). "CT7 (MAGE-C1) antigen expression in normal and neoplastic tissues." Int J Cancer 99(6): 839-45). Gure, Chua *et a*/*.* (2005) analyzed tumors from 523 non-small-cell lung cancer (NSCLC) patients for the expression of cancer-testis antigens (see Gure, A. O., R. Chua, et al. (2005). "Cancer-testis genes are coordinately expressed and are markers of poor outcome in non-small cell lung cancer." Clin Cancer Res 11(22): 8055-62). MAGE-C1 was present in 18,8%. Scanlan, Altorki *et a*/*.* (2000) furthermore reported expression of CT antigens in 33 non-small cell lung cancers: MAGE-C1: 30% (see Scanlan, M. J., N. K. Altorki, et al. (2000). "Expression of cancer-testis antigens in lung cancer: definition of bromodomain testis-specific gene (BRDT) as a new CT gene, CT9." Cancer Lett 150(2): 155-64).

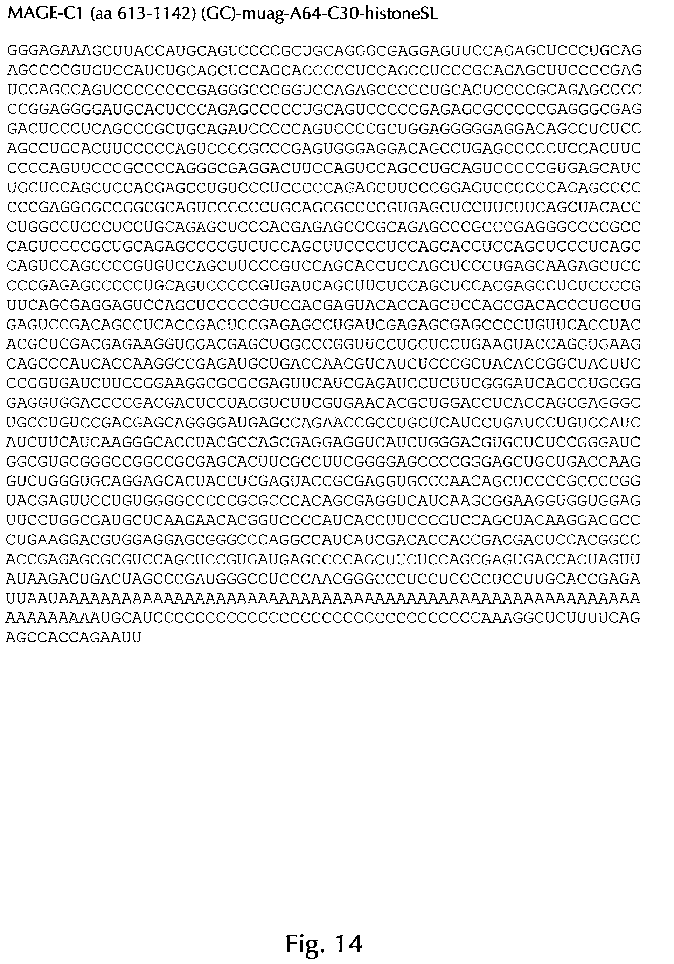

In the context of this invention, the mRNA encoding MAGE-C1 antigen consists of a sequence as shown in Fig. 14 (SEQ ID NO: 22).

Furthermore, one mRNA of the composition encodes MAGE-C2. "MAGE-C2" is the melanoma antigen family C2. Lucas, De Plaen *et al.* (2000) recently identified MAGE-C2 by performing RDA on a melanoma cell line (see Lucas, S., E. De Plaen, et al. (2000). "MAGE-B5, MAGE-B6, MAGE-C2, and MAGE-C3: four new members of the MAGE family with tumor-specific expression." Int J Cancer 87(1): 55-60). MAGE-C2 was not expressed in a panel of normal tissues tested with the exception of testis. Among tumoral samples, MAGE-C2 was frequently expressed in seminomas, melanomas, and bladder carcinomas. It was also expressed in a significant fraction of head and neck carcinomas, breast carcinomas, non-small lung carcinomas and sarcomas. Scanlan, Altorki *et al.* (2000) reported expression of CT antigens in 33 non-small cell lung cancers: MAGE-C2: 30% (see Scanlan, M. J., N. K. Altorki, et al. (2000). "Expression of cancer-testis antigens in lung cancer: definition of bromodomain testis-specific gene (BRDT) as a new CT gene, CT9." Cancer Lett 150(2): 155-64).

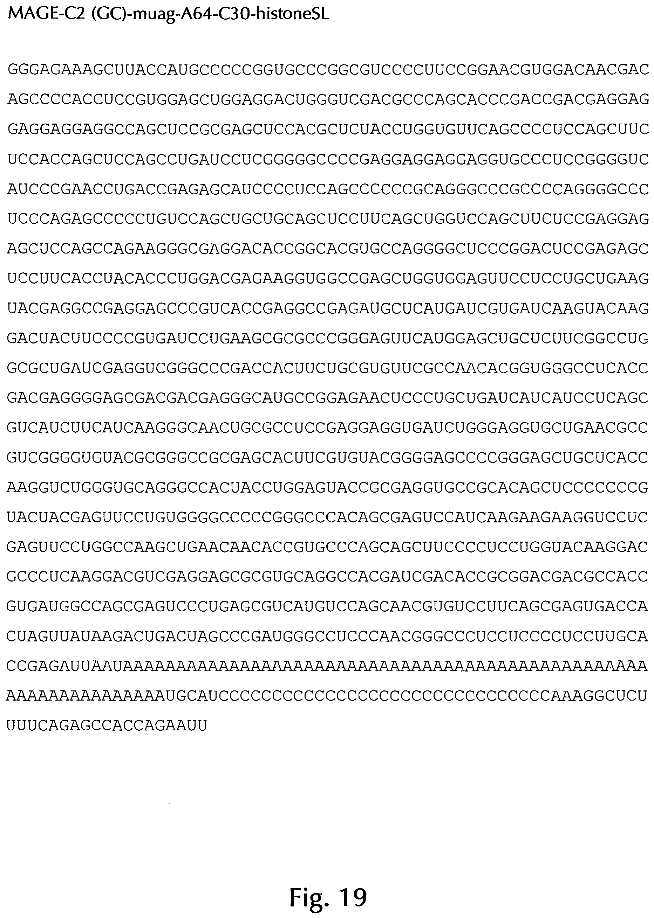

In the context of this invention, the mRNA encoding MAGE-C2 antigen consists of a sequence as shown in Fig. 19 (SEQ ID NO: 23).

Finally, one mRNA of the composition encodes MUC1. "MUC1" is mucin 1. Cancer-associated mucins are thought to promote metastases by facilitating adhesion of malignant cells to the endothelial cell surface. According to Denda-Nagai and Irimura (2000) (Denda-Nagai, K. and T. Irimura (2000). "MUC1 in carcinoma-host interactions." Glycoconj J 17(7-9): 649-58) MUC-1 is overexpressed in 90% of all adenocarcinomas, including breast, lung, pancreas, prostate, stomach, colon and ovary. Kontani, Taguchi *et al.* (2001) found that MUC-1 has been found to be expressed in 60% of lung cancers (see Kontani, K., O. Taguchi, et a/. (2001). "Modulation of MUC1 mucin as an escape mechanism of breast cancer cells from autologous cytotoxic T-lymphocytes." Br J Cancer 84(9): 1258-64), whereas Kontani, Taguchi *et al.* (2003) found in a study analyzing the use of pulsed DCs with MUC1 antigens to elicit cellular immunity in MUC1 positive cancers, that clinically seven of nine MUC-1 positive patients responded to the treatment with either a reduction in tumor marker levels or disappearance of malignant pleural effusion (see Kontani, K., O. Taguchi, et a/. (2003). "Dendritic cell vaccine immunotherapy of cancer targeting MUC1 mucin." Int J Mol Med 12(4): 493-502). Three of these responding patients had NSCLC. Palmer, Parker *et al.* (2001) reported that in a phase I clinical trial using MUC1 peptide in stage III/IV NSCLC, safety and tolerability of this agent was established (see Palmer, M., J. Parker, et al. (2001). "Phase I study of the BLP25 (MUC1 peptide) liposomal vaccine for active specific immunotherapy in stage IIIB/IV non-small-cell lung cancer." Clin Lung Cancer 3(1): 49-57; discussion 58). Five of 12 patients (42%) had immunologic responses, and 4 of 12 patients (33%) achieved stable disease. Wierecky, Mueller *et al.* (2006) further identified two HLA-A2 binding novel 9-mer peptides of the TAA MUC1, which is overexpressed on various hematological and epithelial malignancies (see Wierecky, J., M. Mueller, et al. (2006). "Dendritic cell-based cancer immunotherapy targeting MUC-1." Cancer Immunol Immunother 55(1): 63-7). Cytotoxic T cells generated after pulsing DC with these peptides were able to induce lysis of tumor cells expressing MUC1 in an antigen-specific and HLA-restricted fashion. Within two clinical studies, it was demonstrated that vaccination of patients with advanced cancer using DCs pulsed with MUC1 derived peptides was well tolerated without serious side effects and was able to induce immunological responses. Of 20 patients with metastatic renal cell carcinoma, 6 patients showed regression of metastases with 3 objective responses (1 CR, 2 PR).

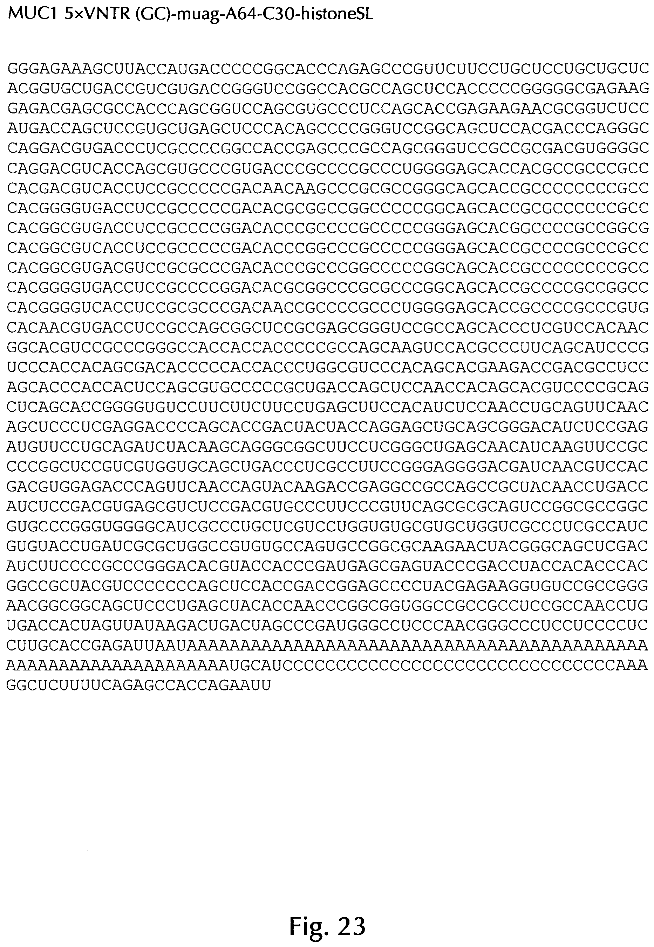

In the context of this invention, the mRNA encoding MUC1 antigen consists of a sequence as shown in Fig. 23 (SEQ ID NO: 24).

Where in the context of the present disclosure, reference is made to antigens or fragments, epitopes or variants thereof, it is understood that the reference concerns the antigen or peptide encoded by one or more of the mRNA sequences provided in the present invention. Further, antigens, antigenic proteins or antigenic peptides as defined above which are encoded by at least one mRNA of the composition disclosed herein, may comprise fragments or variants of those sequences. Such fragments or variants may typically comprise a sequence having a sequence homology with one of the above mentioned antigens, antigenic proteins or antigenic peptides or sequences or their encoding nucleic acid sequences of at least 5%, 10%, 20%, 30%, 40%, 50%, 60%, preferably at least 70%, more preferably at least 80%, equally more preferably at least 85%, even more preferably at least 90% and most preferably at least 95% or even 97%, to the entire wild-type sequence, either on nucleic acid level or on amino acid level.

"Fragments" of antigens, antigenic proteins or antigenic peptides in the context of the present disclosure may comprise a sequence of an antigen, antigenic protein or antigenic peptide as defined above, which is, with regard to its amino acid sequence (or its encoded nucleic acid sequence), N-terminally, C-terminally and/or intrasequentially truncated compared to the amino acid sequence of the original (native) protein (or its encoded nucleic acid sequence). Such truncation may thus occur either on the amino acid level or correspondingly on the nucleic acid level. A sequence homology with respect to such a fragment as defined above may therefore preferably refer to the entire antigen, antigenic protein or antigenic peptide as defined above or to the entire (coding) nucleic acid sequence of such an antigen, antigenic protein or antigenic peptide.

Fragments of antigens, antigenic proteins or antigenic peptides in the context of the present disclosure may furthermore comprise a sequence of an antigen, antigenic protein or antigenic peptide as defined above, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form.

Fragments of antigens, antigenic proteins or antigenic peptides as defined herein may also comprise epitopes of those antigens, antigenic proteins or antigenic peptides. Epitopes (also called "antigen determinants") in the context of the present disclosure are typically fragments located on the outer surface of (native) antigens, antigenic proteins or antigenic peptides as defined herein, preferably having 5 to 15 amino acids, more preferably having 5 to 12 amino acids, even more preferably having 6 to 9 amino acids, which may be recognized by antibodies or B-cell receptors, i.e. in their native form. Such epitopes of antigens, antigenic proteins or antigenic peptides may furthermore be selected from any of the herein mentioned variants of such antigens, antigenic proteins or antigenic peptides. In this context, antigenic determinants can be conformational or discontinous epitopes, which are composed of segments of the antigens, antigenic proteins or antigenic peptides as defined herein that are discontinuous in the amino acid sequence of the antigens, antigenic proteins or antigenic peptides as defined herein but are brought together in the three-dimensional structure or continuous or linear epitopes which are composed of a single polypeptide chain.

"Variants" of antigens, antigenic proteins or antigenic peptides as defined above may be encoded by at least one mRNA of the composition disclosed herein, wherein nucleotides of at least one mRNA, encoding the antigen, antigenic protein or antigenic peptide as defined above, are exchanged. Thereby, an antigen, antigenic protein or antigenic peptide may be generated having an amino acid sequence, which differs from the original sequence in one or more mutation(s), such as one or more substituted, inserted and/or deleted amino acid(s). Preferably, these fragments and/or variants have the same biological function or specific activity compared to the full-length native antigen or antigenic potein, e.g. its specific antigenic property.

At least one mRNA of the composition disclosed herein may also encode an antigen or an antigenic protein as defined above, wherein the encoded amino acid sequence comprises conservative amino acid substitution(s) compared to its physiological sequence. Those encoded amino acid sequences as well as their encoding nucleotide sequences in particular fall under the term variants as defined above. Substitutions in which amino acids which originate from the same class are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions, which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

Furthermore, variants of antigens, antigenic proteins or antigenic peptides as defined above, which may be encoded by at least one mRNA of the composition disclosed herein, may also comprise those sequences, wherein nucleic acids of the at least one mRNA are exchanged according to the degeneration of the genetic code, without leading to an alteration of respective amino acid sequence of the antigen, antigenic protein or antigenic peptide, i.e. the amino acid sequence or at least part thereof may not differ from the original sequence in one or more mutation(s) within the above meaning.

Furthermore, variants of antigens, antigenic proteins or antigenic peptides as defined above, which may be encoded by at least one mRNA of the composition disclosed herein, may also comprise those DNA sequences, which correspond to an RNA sequence as defined herewithin and comprise further RNA sequences, which correspond to DNA sequences as defined herewithin. Those skilled in the art are familiar with the translation of an RNA sequence into a DNA sequence (or vice versa) or with the creation of the complementary strand sequence (i.e. by substitution of U residues with T residues and/or by constructing the complementary strand with respect to a given sequence).

In order to determine the percentage, to which two sequences (nucleic acid sequences, e.g. RNA or mRNA sequences as defined herein, or amino acid sequences, preferably their encoded amino acid sequences, e.g. the amino acid sequences of the antigens, antigenic proteins or antigenic peptides as defined above) are identical, the sequences can be aligned in order to be subsequently compared to one another. Therefore, e.g. gaps can be inserted into the sequence of the first sequence and the component at the corresponding position of the second sequence can be compared. If a position in the first sequence is occupied by the same component as is the case at a position in the second sequence, the two sequences are identical at this position. The percentage to which two sequences are identical is a function of the number of identical positions divided by the total number of positions. The percentage, to which two sequences are identical, can be determined using a mathematical algorithm. A preferred, but not limiting, example of a mathematical algorithm, which can be used, is the algorithm of Karlin et al. (1993), PNAS USA, 90:5873-5877 or Altschul et al. (1997), Nucleic Acids Res., 25:3389-3402. Such an algorithm is integrated in the BLAST program. Sequences, which are identical to the sequences of the present disclosure to a certain extent can be identified by this program.

As used herein, the term "composition" refers to at least six mRNAs as defined by the claims and, optionally, further excipients. The composition contains at least six distinct mRNA species, whereby each mRNA species encodes one of the above antigens. The term "composition" preferably relates to the at least six mRNAs together with at least one other suitable substance. In general, the composition may be a pharmaceutical composition, which is designed for use in the medical field. Accordingly, the composition typically comprises at least one further excipient, which is pharmaceutically acceptable and which may be selected, for example, from carriers, vehicles and the like. The "composition" may be a liquid or a dry composition. If the composition is liquid, it will be preferably an aqueous solution or dispersion of the at least six mRNAs. If the "composition" is a dry composition, it will typically be a lyophilized composition of the at least six mRNAs. The term "composition", as used herewithin, further refers to the at least six mRNAs defined in the claims in combination with a further active ingredient. Preferably, the composition is an immunostimulatory composition, i.e. a composition comprising at least one component, which is able to induce an immune response or from which a component, which is able to induce an immune response, is derivable. In this context, the immune response may be the result of the adaptive and/or of the innate immune system.

The composition for use according to the present invention comprises six mRNAs encoding at least six antigens as defined by the claims, as it was found out that the specific combination of said antigens is capable of effectively stimulating the (adaptive) immune system, thus allowing treatment of non-small cell lung cancer (NSCLC), wherein the treatment further comprises administration of a chemotherapeutic agent.

In summary, the object of the present invention is solved by the provision of a composition comprising six mRNAs coding for a novel combination of antigens as defined herein. The composition comprises the six antigens (5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1)), which are encoded by six monocistronic mRNAs as defined in the claims, each of these mRNAs encoding a different antigen selected from the defined group of antigens. In non-claimed embodiments also disclosed herein, the composition may comprise a combination of monocistronic, bi- and/or multicistronic mRNAs, wherein more than one of the six antigens is encoded by a bi- or multicistronic mRNA. According to non-claimed embodiments disclosed herein, any combination of mono-, bi- or multicistronic mRNAs is envisaged that encode all six antigens as defined herein, e.g. three bicistronic mRNAs, each of which encodes two of the above six antigens or two bicistronic and two monocistronic mRNAs.

According to a non-claimed embodiment, the composition comprises at least one mRNA, which comprises at least one coding sequence selected from RNA sequences being identical or at least 80% identical to the RNA sequence of SEQ ID NOs: 2, 5, 8, 11, 14 or 17. Even more preferably, the composition comprises six mRNAs, wherein the coding sequence in each mRNA is identical or at least 80% identical to one of the RNA sequences according to SEQ ID NOs: 2, 5, 8, 11, 14 and 17.

According to the invention, each of the at least six antigens of the composition for use according to the present invention, is encoded by one (monocistronic) mRNA as defined in the claims. In other words, the composition for use according to the present invention contains six (monocistronic) mRNAs, wherein each of these six (monocistronic) mRNAs encodes just one antigen as defined in the claims.

In a preferred embodiment, the composition comprises six mRNAs as defined by the claims, wherein one mRNA encodes 5T4 (according to SEQ ID NO: 75), one mRNA encodes Survivin (according to SEQ ID NO: 76 or 77), one mRNA encodes NY-ESO-1 (according to SEQ ID NO: 78), one mRNA encodes MAGE-C1 (according to SEQ ID NO: 79), one mRNA encodes MAGE-C2 (according to SEQ ID NO: 80) and one mRNA encodes MUC1 (according to SEQ ID NO: 82), respectively.

In non-claimed embodiment, the composition comprises six mRNAs, wherein one mRNA encodes 5T4 and comprises a coding sequence identical or at least 80% identical to SEQ ID NO: 2, one mRNA encodes Survivin and comprises a coding sequence identical or at least 80% identical to SEQ ID NO: 5, one mRNA encodes NY-ESO-1 and comprises a coding sequence identical or at least 80% identical to SEQ ID NO: 8, one mRNA encodes MAGE-C1 and comprises a coding sequence identical or at least 80% identical to SEQ ID NO: 11, one mRNA encodes MAGE-C2 and comprises a coding sequence identical or at least 80% identical to SEQ ID NO: 14 and one mRNA encodes MUC1 and comprises a coding sequence identical or at least 80% identical to SEQ ID NO:17 (or fragments or variants of each of these sequences) and optionally further excipients.

In a non-claimed embodiment, the composition comprises six mRNAs, wherein one mRNA encodes 5T4 and comprises the coding sequence according to SEQ ID NO: 2, one mRNA encodes Survivin and comprises the coding sequence according to SEQ ID NO: 5, one mRNA encodes NY-ESO-1 and comprises the coding sequence according to SEQ ID NO: 8, one mRNA encodes MAGE-C1 and comprises the coding sequence according to SEQ ID NO: 11, one mRNA encodes MAGE-C2 and comprises the coding sequence according to SEQ ID NO: 14 and one mRNA encodes MUC1 and comprises the coding sequence according to SEQ ID NO:17 or fragments thereof, respectively.

According to an embodiment disclosed herein, at least one mRNA of the composition comprises a histone stem-loop in the 3' UTR region.

According to a non-claimed embodiment, the composition disclosed herein may comprise (at least) one bi- or even multicistronic mRNA, i.e. (at least) one mRNA which carries the coding sequences of two or more of the six antigens according to the present disclosure. Such coding sequences of two or more antigens of the (at least) one bi- or even multicistronic mRNA may be separated by at least one IRES (internal ribosomal entry site) sequence, as defined below. Thus, the term "encoding two or more antigens" may mean, without being limited thereto, that the (at least) one (bi- or even multicistronic) mRNA, may encode e.g. at least two, three, four, five or six (preferably different) antigens of the above mentioned antigens or their fragments or variants within the above definitions. More preferably, without being limited thereto, the (at least) one (bi- or even multicistronic) mRNA, may encode e.g. at least two, three, four, five or six (preferably different) antigens of the above mentioned antigens or their fragments or variants within the above definitions. In this context, a so-called IRES (internal ribosomal entry site) sequence as defined above can function as a sole ribosome binding site, but it can also serve to provide a bi- or even multicistronic mRNA as defined above which codes for several proteins, which are to be translated by the ribosomes independently of one another. Examples of IRES sequences which can be used according to the present disclosure are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to a further particularly preferred embodiment, the composition for use according to the present invention, may comprise a mixture of at least six monocistronic mRNAs as defined in the claims, and at least one bi- or even multicistronic mRNA, as defined above. The at least six monocistronic mRNAs and/or the at least one bi- or even multicistronic mRNA preferably encode different antigens or their fragments or variants within the above definitions. However, the at least six monocistronic mRNAs and the at least one bi- or even multicistronic mRNA may preferably also encode (in part) identical antigens selected from the above mentioned antigens, provided that the composition disclosed herein as a whole provides the six antigens as defined above. By providing multiple copies of one or more of the antigens, the relative protein amounts of said one or more antigens can be increased, i.e. the ratio between the amounts of each of the six antigens can be modulated. Such an embodiment may further be advantageous e.g. for a staggered, e.g. time dependent, administration of the composition disclosed herein to a patient in need thereof. The components of such a composition disclosed herein, particularly the different mRNAs encoding the at least six antigens, may be e.g. contained in (different parts of) a kit of parts composition or may be e.g. administered separately as components of different compositions for use according to the present invention.

Preferably, at least one mRNA of the composition encoding at least one of the six antigens typically comprises a length of about 50 to about 20000, or 100 to about 20000 nucleotides, preferably of about 250 to about 20000 nucleotides, more preferably of about 500 to about 10000, even more preferably of about 500 to about 5000.

According to one embodiment disclosed herein, at least one mRNA of the composition encoding the six antigens, may be in the form of a modified mRNA, wherein any modification, as defined herein, may be introduced into the mRNA of the composition. Modifications as defined herein preferably lead to a stabilized mRNA of the composition.

According to one embodiment disclosed herein, at least one mRNA of the composition may thus be provided as a "stabilized mRNA", that is to say as an mRNA that is essentially resistant to in vivo degradation (e.g. by an exo- or endo-nuclease). Such stabilization can be effected, for example, by a modified phosphate backbone of at least one mRNA of the composition disclosed herein. A backbone modification in connection with the present disclosure is a modification in which phosphates of the backbone of the nucleotides contained in the mRNA are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized mRNAs may further include, for example: nonionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)).

At least one mRNA of the composition disclosed herein may additionally or alternatively also contain sugar modifications. A sugar modification in connection with the present disclosure is a chemical modification of the sugar of the nucleotides of the at least one mRNA and typically includes, without implying any limitation, sugar modifications selected from the group consisting of 2'-deoxy-2'-fluoro-oligoribonucleotide (2'-fluoro-2'-deoxycytidine-5'-triphosphate, 2'-fluoro-2'-deoxyuridine-5'-triphosphate), 2'-deoxy-2'-deamine oligoribonucleotide (2'-amino-2'-deoxycytidine-5'-triphosphate, 2'-amino-2'-deoxyuridine-5'-triphosphate), 2'-O-alkyl oligoribonucleotide, 2'-deoxy-2'-C-alkyl oligoribonucleotide (2'-O-methylcytidine-5'-triphosphate, 2'-methyluridine-5'-triphosphate), 2'-C-alkyl oligoribonucleotide, and isomers thereof (2'-aracytidine-5'-triphosphate, 2'-arauridine-5'-triphosphate), or azidotriphosphate (2'-azido-2'-deoxycytidine-5'-triphosphate, 2'-azido-2'-deoxyuridine-5'-triphosphate).

At least one mRNA of the composition disclosed herein may additionally or alternatively also contain at least one base modification, which is preferably suitable for increasing the expression of the encoded protein as compared with the unaltered, i.e. natural (= native), mRNA sequence. Significant in this case means an increase in the expression of the protein compared with the expression of the native mRNA sequence by at least 20%, preferably at least 30%, 40%, 50% or 60%, more preferably by at least 70%, 80%, 90% or even 100% and most preferably by at least 150%, 200% or even 300% or more. In connection with the present disclosure, a nucleotide having such a base modification is preferably selected from the group of the base-modified nucleotides consisting of 2-amino-6-chloropurineriboside-5'-triphosphate, 2-aminoadenosine-5'-triphosphate, 2-thiocytidine-5'-triphosphate, 2-thiouridine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-iodocytidine-5'-triphosphate, 5-iodouridine-5'-triphosphate, 5-methylcytidine-5'-triphosphate, 5-methyluridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

According to another embodiment disclosed herein, at least one mRNA of the composition can likewise be modified (and preferably stabilized) by introducing further modified nucleotides containing modifications of their ribose or base moieties. Generally, at least one mRNA of the composition disclosed herein may contain any native (= naturally occurring) nucleotide, e.g. guanosine, uridine, adenosine, and/or cytidine or an analogue thereof. In this connection, nucleotide analogues are defined as non-natively occurring variants of naturally occurring nucleotides. Accordingly, analogues are chemically derivatized nucleotides with non-natively occurring functional groups, which are preferably added to or deleted from the naturally occurring nucleotide or which substitute the naturally occurring functional groups of a nucleotide. Accordingly, each component of the naturally occurring nucleotide may be modified, namely the base component, the sugar (ribose) component and/or the phosphate component forming the backbone (see above) of the RNA sequence. Analogues of guanosine, uridine, adenosine, and cytidine include, without implying any limitation, any naturally occurring or non-naturally occurring guanosine, uridine, adenosine, thymidine or cytidine that has been altered chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyl-adenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 3-methyl-cytidine, 4-acetyl-cytidine, 4-Thiouridine, 5-(carboxyhydroxymethyl)-uracil, 5,6-Dihydrouridine, 5-Aminoallylcytidine, 5-Aminoallyldeoxy-uridine, 5-Bromouridine, 5-carboxymehtylaminomethyl-2-thio-uracil, 5-carboxymethylamonomethyl-uracil, 5-Chloro-Ara-cytidine, 5-Fluoro-uridine, 5-lodouridine, 5-methoxycarbonylmethyl-uridine, 5-methoxy-uridine, 5-methyl-2-thio-uridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromo-guanosine, 8-Mercapto-guanosine, 8-Oxoguanosine, Benzimidazole-riboside, Beta-D-mannosyl-queosine, Dihydro-uridine, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given to those analogues that increase the immunogenicity of the mRNA of the composition disclosed herein and/or do not interfere with a further modification of the mRNA that has been introduced.

According to a particular embodiment disclosed herein, at least one mRNA of the composition can contain a lipid modification. Such a lipid-modified mRNA typically comprises an mRNA as defined herein, encoding one of the six antigens as defined above. Such a lipid-modified mRNA typically further comprises at least one linker covalently linked with that mRNA, and at least one lipid covalently linked with the respective linker. Alternatively, the lipid-modified mRNA comprises an (at least one) mRNA as defined herein and at least one (bifunctional) lipid covalently linked (without a linker) with that mRNA. According to a third alternative, the lipid-modified mRNA comprises an mRNA as defined herein, at least one linker covalently linked with that mRNA, and at least one lipid covalently linked with the respective linker, and also at least one (bifunctional) lipid covalently linked (without a linker) with that mRNA.

The lipid contained in at least one mRNA of the composition described herein (complexed or covalently bound thereto) is typically a lipid or a lipophilic residue that preferably is itself biologically active. Such lipids preferably include natural substances or compounds such as, for example, vitamins, e.g. alpha-tocopherol (vitamin E), including RRR-alpha-tocopherol (formerly D-alpha-tocopherol), L-alpha-tocopherol, the racemate D,L-alpha-tocopherol, vitamin E succinate (VES), or vitamin A and its derivatives, e.g. retinoic acid, retinol, vitamin D and its derivatives, e.g. vitamin D and also the ergosterol precursors thereof, vitamin E and its derivatives, vitamin K and its derivatives, e.g. vitamin K and related quinone or phytol compounds, or steroids, such as bile acids, for example cholic acid, deoxycholic acid, dehydrocholic acid, cortisone, digoxygenin, testosterone, cholesterol or thiocholesterol. Further lipids or lipophilic residues within the scope of the present disclosure include, without implying any limitation, polyalkylene glycols (Oberhauser et al., Nucl. Acids Res., 1992, 20, 533), aliphatic groups such as, for example, C1-C20-alkanes, C1-C20-alkenes or C1-C20-alkanol compounds, etc., such as, for example, dodecanediol, hexadecanol or undecyl residues (Saison-Behmoaras et al., EMBO J, 1991, 10, 111; Kabanov et al., FEBS Lett., 1990, 259, 327; Svinarchuk et al., Biochimie, 1993, 75, 49), phospholipids such as, for example, phosphatidylglycerol, diacylphosphatidylglycerol, phosphatidylcholine, dipalmitoylphosphatidylcholine, distearoylphosphatidylcholine, phosphatidylserine, phosphatidylethanolamine, di-hexadecyl-rac-glycerol, sphingolipids, cerebrosides, gangliosides, or triethylammonium 1,2-di-O-hexadecyl-rac-glycero-3-H-phosphonate (Manoharan et al., Tetrahedron Lett., 1995, 36, 3651; Shea et al., Nucl. Acids Res., 1990, 18, 3777), polyamines or polyalkylene glycols, such as, for example, polyethylene glycol (PEG) (Manoharan et al., Nucleosides & Nucleotides, 1995, 14, 969), hexaethylene glycol (HEG), palmitin or palmityl residues (Mishra et al., Biochim. Biophys. Acta, 1995, 1264, 229), octadecylamines or hexylamino-carbonyl-oxycholesterol residues (Crooke et al., J. Pharmacol. Exp. Ther., 1996, 277, 923), and also waxes, terpenes, alicyclic hydrocarbons, saturated and mono- or poly-unsaturated fatty acid residues, etc.

At least one mRNA of the composition disclosed herein may likewise be stabilized in order to prevent degradation of the mRNA in vivo by various approaches. It is known in the art that instability and (fast) degradation of mRNA or of RNA in vivo in general may represent a serious problem in the application of RNA based compositions. This instability of RNA is typically due to RNA-degrading enzymes, "RNases" (ribonucleases), wherein contamination with such ribonucleases may sometimes completely degrade RNA in solution. Accordingly, the natural degradation of mRNA in the cytoplasm of cells is very finely regulated and RNase contaminations may be generally removed by special treatment prior to use of said sompositions, in particular with diethyl pyrocarbonate (DEPC). A number of mechanisms of natural degradation are known in this connection in the prior art, which may be utilized as well. E.g., the terminal structure is typically of critical importance for a mRNA in vivo. As an example, at the 5' end of naturally occurring mRNAs there is usually a so-called "cap structure" (a modified guanosine nucleotide), and at the 3' end is typically a sequence of up to 200 adenosine nucleotides (the so-called poly-A tail).

At least one mRNA of the composition disclosed herein can therefore be stabilized against degradation by RNases by the addition of a so-called "5' cap" structure. Particular preference is given in this connection to an m7G(5')ppp (5'(A,G(5')ppp(5')A or G(5')ppp(5')G as the 5' cap" structure. However, such a modification is introduced only if a modification, for example a lipid modification, has not already been introduced at the 5' end of the mRNA of the composition disclosed herein or if the modification does not interfere with the immunogenic properties of the (unmodified or chemically modified) mRNA.

According to a further preferred embodiment disclosed herein, at least one mRNA of the composition may contain a poly-A tail on the 3' terminus of typically about 10 to 200 adenosine nucleotides, preferably about 10 to 100 adenosine nucleotides, more preferably about 40 to 80 adenosine nucleotides or even more preferably about 50 to 70 adenosine nucleotides.

According to a further preferred embodiment disclosed herein, at least one mRNA of the composition may contain a poly-C tail on the 3' terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

At least one mRNA according to the present disclosure preferably comprises at least one histone stem-loop. In the context of the present disclosure, such a histone stem-loop, in general (irrespective of whether it is a histone stem loop or not), is typically derived from histone genes and comprises an intramolecular base pairing of two neighboring entirely or partially reverse complementary sequences, thereby forming a stem-loop. A stem-loop can occur in single-stranded DNA or, more commonly, in RNA.

In the context of the present disclosure, a histone stem-loop sequence may be described by its DNA or by its corresponding RNA sequence. Thus, any reference - throughout the present disclosure - to histone stem-loop sequences, which are represented herein by DNA sequences (e.g. SEQ ID NO: 38 to 67 and 71), also comprises the corresponding RNA sequence. This applies in particular to histone stem-loop sequences, which are comprised in at least one mRNA as disclosed herein. Accordingly, by reference to a specific DNA sequence that defines a histone stem-loop, the corresponding RNA sequence is defined as well.

The structure is also known as a hairpin or hairpin loop and usually consists of a stem and a (terminal) loop within a consecutive sequence, wherein the stem is formed by two neighbored entirely or partially reverse complementary sequences separated by a short sequence as sort of spacer, which builds the loop of the stem-loop structure. The two neighbored entirely or partially reverse complementary sequences may be defined as e.g. stem loop elements stem1 and stem2. The stem loop is formed when these two neighbored entirely or partially reverse complementary sequences, e.g. stem loop elements stem1 and stem2, form base-pairs with each other, leading to a double stranded nucleic acid sequence stretch comprising an unpaired loop at its terminal ending formed by the short sequence located between stem loop elements stem1 and stem2 on the consecutive sequence. The unpaired loop thereby typically represents a region of the nucleic acid which is not capable of base pairing with either of these stem loop elements. The resulting lollipop-shaped structure is a key building block of many RNA secondary structures. The formation of a stem-loop structure is thus dependent on the stability of the resulting stem and loop regions, wherein the first prerequisite is typically the presence of a sequence that can fold back on itself to form a paired double strand. The stability of paired stem loop elements is determined by the length, the number of mismatches or bulges it contains (a small number of mismatches is typically tolerable, especially in a long double stranded stretch), and the base composition of the paired region. In the context of the present disclosure, a loop length of 3 to 15 bases is conceivable, while a more preferred loop length is 3-10 bases, more preferably 3 to 8, 3 to 7, 3 to 6 or even more preferably 4 to 5 bases, and most preferably 4 bases. The sequence forming the stem region in the histone stem-loop typically has a length of between 5 to 10 bases, more preferably, between 5 to 8 bases, wherein preferably at least one of the bases represents a mismatch, i.e. does not base pair.

In the context of the present disclosure, a histone stem-loop is typically derived from histone genes (e.g. genes from the histone families H1, H2A, H2B, H3, H4) and comprises an intramolecular base pairing of two neighbored entirely or partially reverse complementary sequences, thereby forming a stem-loop. Typically, a histone 3' UTR stem-loop is an RNA element involved in nucleocytoplasmic transport of the histone mRNAs, and in the regulation of stability and of translation efficiency in the cytoplasm. The mRNAs of metazoan histone genes lack polyadenylation and a poly-A tail, instead 3' end processing occurs at a site between this highly conserved stem-loop and a purine rich region around 20 nucleotides downstream (the histone downstream element, or HDE). The histone stem-loop is bound by a 31 kDa stem-loop binding protein (SLBP - also termed the histone hairpin binding protein, or HBP). Such histone stem-loop structures are preferably employed according to the present disclosure in combination with other sequence elements and structures, which do not occur naturally (which means in untransformed living organisms/cells) in histone genes, but are combined - according to the present disclosure - to provide an artificial, heterologous nucleic acid. Accordingly, the present disclosure provides an artificial (non-native) combination of a histone stem-loop structure with other heterologous sequence elements, which do not occur in histone genes or metazoan histone genes and are isolated from operational and/or regulatory sequence regions (influencing transcription and/or translation) of genes coding for proteins other than histones, provide advantageous effects. Accordingly, one embodiment described herein provides the combination of a histone stem-loop structure with a poly(A) sequence or a sequence representing a polyadenylation signal (3'-terminal of a coding region), which does not occur in metazoan histone genes. According to another aspect disclosed herein, a combination of a histone stem-loop structure with a coding region coding for at least one of the antigens as defined above, which does, preferably not occur in metazoan histone genes, is provided herewith (coding region and histone stem loop sequence are heterologous).

A histone stem loop is, therefore, a stem-loop structure as described herein, which, if preferably functionally defined, exhibits/retains the property of binding to its natural binding partner, the stem-loop binding protein (SLBP - also termed the histone hairpin binding protein, or HBP).

In an embodiment disclosed herein, the histone stem loop sequence is not derived from a mouse histone protein. More specifically, the histone stem loop sequence may not be derived from mouse histone gene H2A614. Also, at least one mRNA disclosed herein may neither contain a mouse histone stem loop sequence nor contain mouse histone gene H2A614. Further, at least one mRNA disclosed herein may not contain a stem-loop processing signal, more specifically, a mouse histone processing signal and, most specifically, may not contain mouse stem loop processing signal H2kA614, even if the mRNA contains at least one mammalian histone gene. However, the at least one mammalian histone gene may not be Seq. ID No. 7 of WO 01/12824.

At least one mRNA disclosed herein, may preferably comprise a 5' UTR, a coding region encoding the antigens as defined above or a fragment, variant or derivative thereof; and/or a 3' UTR preferably containing at least one histone stem-loop. When in addition to the antigens defined above, a further peptide or protein is encoded by the mRNA, then the encoded peptide or protein is preferably no histone protein, no reporter protein and/or no marker or selection protein, as defined above. The 3' UTR of the mRNA preferably comprises also a poly(A) and/or a poly(C) sequence as defined herewithin. The single elements of the 3' UTR may occur therein in any order from 5' to 3' along the sequence of the at least one mRNA. In addition, further elements as described herein, may also be contained, such as a stabilizing sequence as defined herewithin (e.g. derived from the UTR of a globin gene), IRES sequences, etc. Each of the elements may also be repeated in the mRNA at least once (particularly in di- or multicistronic constructs), preferably twice or more. As an example, the single elements may be present in the mRNA in the following order:
5' - coding region - histone stem-loop - poly(A)/(C) sequence - 3'; or
5' - coding region - poly(A)/(C) sequence - histone stem-loop - 3'; or
5' - coding region - histone stem-loop - polyadenylation signal - 3'; or
5' - coding region - polyadenylation signal- histone stem-loop - 3'; or
5' - coding region - histone stem-loop - histone stem-loop - poly(A)/(C) sequence - 3'; or
5' - coding region - histone stem-loop - histone stem-loop - polyadenylation signal- 3'; or
5' - coding region - stabilizing sequence - poly(A)/(C) sequence - histone stem-loop - 3'; or
5' - coding region - stabilizing sequence - poly(A)/(C) sequence - poly(A)/(C) sequence - histone stem-loop - 3'; etc.

In this context, it is particularly preferred that - if, in addition to the antigens defined above, a further peptide or protein is encoded by at least one mRNA - the encoded peptide or protein is preferably no histone protein, no reporter protein (e.g. Luciferase, GFP, EGFP, β-Galactosidase, particularly EGFP) and/or no marker or selection protein (e.g. alpha-Globin, Galactokinase and Xanthine:Guanine phosphoribosyl transferase (GPT)).

In an embodiment disclosed herein, the mRNA does not comprise a reporter gene or a marker gene. Preferably, the mRNA does not encode, for instance, luciferase; green fluorescent protein (GFP) and its variants (such as eGFP, RFP or BFP); α-globin; hypoxanthine-guanine phosphoribosyltransferase (HGPRT); β-galactosidase; galactokinase; alkaline phosphatase; secreted embryonic alkaline phosphatase (SEAP)) or a resistance gene (such as a resistance gene against neomycin, puromycin, hygromycin and zeocin). In an embodiment disclosed herein, the mRNA does not encode luciferase. In another embodiment, the mRNA does not encode GFP or a variant thereof.

In a further embodiment disclosed herein, the mRNA does not encode a protein (or a fragment of a protein) derived from a virus, preferably from a virus belonging to the family of Orthomyxoviridae. Preferably the mRNA does not encode a protein that is derived from an influenza virus, more preferably an influenza A virus. Preferably, the mRNA does not encode an influenza A protein selected from the group consisting of hemagglutinin (HA), neuraminidase (NA), nucleoprotein (NP), M1, M2, NS1, NS2 (NEP: nuclear export protein), PA, PB1 (polymerase basic 1), PB1-F2 and PB2. In another embodiment disclosed herein, the mRNA does not encode ovalbumin (OVA) or a fragment thereof. Preferably, the mRNA does not encode an influenza A protein or ovalbumin.

According to one embodiment disclosed herein, at least one mRNA disclosed herein comprises at least one histone stem-loop sequence, preferably according to at least one of the following formulae (I) or (II):
formula (I) (stem-loop sequence without stem bordering elements):
formula (II) (stem-loop sequence with stem bordering elements): wherein:
   - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
   - stem1 [N₀₋₂GN₃₋₅]: is reverse complementary or partially reverse complementary with element stem2, and is a consecutive sequence between of 5 to 7 nucleotides;
   wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
   wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides;
   wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;
   - stem2 [N₃₋₅CN₀₋₂]: is reverse complementary or partially reverse complementary with element stem1, and is a consecutive sequence between of 5 to 7 nucleotides;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
   wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and
   wherein C is cytidine or an analogue thereof, and may be optionally replaced by a guanosine or an analogue thereof provided that its complementary nucleotide guanosine in stem1 is replaced by cytidine;
wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one or more bases in one stem do not have a complementary base in the reverse complementary sequence of the other stem.

In the above context, a wobble base pairing is typically a non-Watson-Crick base pairing between two nucleotides. The four main wobble base pairs in the present context, which may be used, are guanosine-uridine, inosine-uridine, inosine-adenosine, inosine-cytidine (G-U/T, I-U/T, I-A and I-C) and adenosine-cytidine (A-C).

Accordingly, in the context of the present disclosure, a wobble base is a base, which forms a wobble base pair with a further base as described above. Therefore non-Watson-Crick base pairing, e.g. wobble base pairing, may occur in the stem of the histone stem-loop structure in at least one mRNA according to the present disclosure.

In the above context, a partially reverse complementary sequence comprises maximally 2, preferably only one mismatch in the stem-structure of the stem-loop sequence formed by base pairing of stem1 and stem2. In other words, stem1 and stem2 are preferably capable of (full) base pairing with each other throughout the entire sequence of stem1 and stem2 (100% of possible correct Watson-Crick or non-Watson-Crick base pairings), thereby forming a reverse complementary sequence, wherein each base has its correct Watson-Crick or non-Watson-Crick base pendant as a complementary binding partner. Alternatively, stem1 and stem2 are preferably capable of partial base pairing with each other throughout the entire sequence of stem1 and stem2, wherein at least about 70%, 75%, 80%, 85%, 90%, or 95% of the 100% possible correct Watson-Crick or non-Watson-Crick base pairings are occupied with the correct Watson-Crick or non-Watson-Crick base pairings and at most about 30%, 25%, 20%, 15%, 10%, or 5% of the remaining bases are unpaired.

According to an embodiment disclosed herein, the at least one histone stem-loop sequence (with stem bordering elements) of at least one mRNA as defined herein comprises a length of about 15 to about 45 nucleotides, preferably a length of about 15 to about 40 nucleotides, preferably a length of about 15 to about 35 nucleotides, preferably a length of about 15 to about 30 nucleotides and even more preferably a length of about 20 to about 30 and most preferably a length of about 24 to about 28 nucleotides.

According to a further embodiment disclosed herein, the at least one histone stem-loop sequence (without stem bordering elements) of the at least one mRNA as defined herein comprises a length of about 10 to about 30 nucleotides, preferably a length of about 10 to about 20 nucleotides, preferably a length of about 12 to about 20 nucleotides, preferably a length of about 14 to about 20 nucleotides and even more preferably a length of about 16 to about 17 and most preferably a length of about 16 nucleotides.

According to a further embodiment disclosed herein, at least one mRNA disclosed herein may comprise at least one histone stem-loop sequence according to at least one of the following specific formulae (Ia) or (IIa):
formula (la) (stem-loop sequence without stem bordering elements):
formula (IIa) (stem-loop sequence with stem bordering elements): wherein:
   - N, C, G, T and U: are as defined above.

According to a further disclosed embodiment of the first aspect, at least one RNA may comprise or code for at least one histone stem-loop sequence according to at least one of the following specific formulae (Ib) or (IIb):
formula (lb) (stem-loop sequence without stem bordering elements):
formula (IIb) (stem-loop sequence with stem bordering elements): wherein:
   - N, C, G, T and U: are as defined above.

According to an embodiment disclosed herein, at least one mRNA disclosed herein may comprise at least one histone stem-loop sequence according to at least one of the following specific formulae (Ic) to (Ih) or (IIc) to (IIh), shown alternatively in its stem-loop structure and as a linear sequence representing histone stem-loop sequences:
formula (Ic): (metazoan and protozoan histone stem-loop consensus sequence without stem bordering elements):
formula (IIc): (metazoan and protozoan histone stem-loop consensus sequence with stem bordering elements):
formula (Id): (without stem bordering elements)
formula (IId): (with stem bordering elements)
formula (Ie): (protozoan histone stem-loop consensus sequence without stem bordering elements)
formula (IIe): (protozoan histone stem-loop consensus sequence with stem bordering elements)
formula (If): (metazoan histone stem-loop consensus sequence without stem bordering elements)
formula (IIf): (metazoan histone stem-loop consensus sequence with stem bordering elements)
formula (Ig): (vertebrate histone stem-loop consensus sequence without stem bordering elements)
formula (IIg): (vertebrate histone stem-loop consensus sequence with stem bordering elements)
formula (Ih): (human histone stem-loop consensus sequence (Homo sapiens) without stem bordering elements)
formula (IIh): (human histone stem-loop consensus sequence (Homo sapiens) with stem bordering elements) wherein in each of above formulae (Ic) to (Ih) or (IIc) to (IIh):
N, C, G, A, T and U are as defined above;
each U may be replaced by T;
each (highly) conserved G or C in the stem elements 1 and 2 may be replaced by its complementary nucleotide base C or G, provided that its complementary nucleotide in the corresponding stem is replaced by its complementary nucleotide in parallel; and/or
G, A, T, U, C, R, Y, M, K, S, W, H, B, V, D, and N are nucleotide bases as defined in the following Table:

| abbreviation | Nucleotide bases | remark |
|---|---|---|
| G | G | Guanine |
| A | A | Adenine |
| T | T | Thymine |
| U | U | Uracile |
| C | C | Cytosine |
| R | G or A | Purine |
| Y | T/U or C | Pyrimidine |
| M | A or C | Amino |
| K | G or T/U | Keto |
| S | G or C | Strong (3H bonds) |
| W | A or T/U | Weak (2H bonds) |
| H | A or C or T/U | Not G |
| B | G or T/U or C | Not A |
| V | G or C or A | Not T/U |
| D | G or A or T/U | Not C |
| N | G or C or T/U or A | Any base |
| * | Present or not | Base may be present or not |

In this context it is particularly preferred that the histone stem-loop sequence according to at least one of the formulae (I) or (Ia) to (Ih) or (II) or (IIa) to (IIh) above is selected from a naturally occurring histone stem loop sequence, more particularly preferred from protozoan or metazoan histone stem-loop sequences, and even more particularly preferred from vertebrate and mostly preferred from mammalian histone stem-loop sequences especially from human histone stem-loop sequences.

According to an embodiment disclosed herein, the histone stem-loop sequence according to at least one of the specific formulae (I) or (Ia) to (Ih) or (II) or (IIa) to (IIh) is a histone stem-loop sequence comprising at each nucleotide position the most frequently occurring nucleotide, or either the most frequently or the second-most frequently occurring nucleotide of naturally occurring histone stem-loop sequences in metazoa and protozoa, protozoa, metazoa, vertebrates and humans. In this context it is particularly preferred that at least 80%, preferably at least 85%, or most preferably at least 90% of all nucleotides correspond to the most frequently occurring nucleotide of naturally occurring histone stem-loop sequences.

In a further embodiment disclosed herein, the histone stem-loop sequence according to at least one of the specific formulae (I) or (Ia) to (Ih) above is selected from following histone stem-loop sequences (without stem-bordering elements):
VGYYYYHHTHRVVRCB (SEQ ID NO: 38 according to formula (Ic))
SGYYYTTYTMARRRCS (SEQ ID NO: 39 according to formula (Ic))
SGYYCTTTTMAGRRCS (SEQ ID NO: 40 according to formula (Ic))
DGNNNBNNTHVNNNCH (SEQ ID NO: 41 according to formula (Ie))
RGNNNYHBTHRDNNCY (SEQ ID NO: 42 according to formula (Ie))
RGNDBYHYTHRDHNCY (SEQ ID NO: 43 according to formula (Ie))
VGYYYTYHTHRVRRCB (SEQ ID NO: 44 according to formula (If))
SGYYCTTYTMAGRRCS (SEQ ID NO: 45 according to formula (If))
SGYYCTTTTMAGRRCS (SEQ ID NO: 46 according to formula (If))
GGYYCTTYTHAGRRCC (SEQ ID NO: 47 according to formula (Ig))
GGCYCTTYTMAGRGCC (SEQ ID NO: 48 according to formula (Ig))
GGCTCTTTTMAGRGCC (SEQ ID NO: 49 according to formula (Ig))
DGHYCTDYTHASRRCC (SEQ ID NO: 50 according to formula (Ih))
GGCYCTTTTHAGRGCC (SEQ ID NO: 51 according to formula (Ih))
GGCYCTTTTMAGRGCC (SEQ ID NO: 52 according to formula (Ih))

Furthermore in this context, following histone stem-loop sequences (with stem bordering elements) according to one of specific formulae (II) or (IIa) to (IIh) are particularly preferred:
H*H*HHVVGYYYYHHTHRVVRCBVHH*N*N* (SEQ ID NO: 53 according to formula (IIc))
M*H*MHMSGYYYTTYTMARRRCSMCH*H*H* (SEQ ID NO: 54 according to formula (IIc))
M*M*MMMSGYYCTTTTMAGRRCSACH*M*H* (SEQ ID NO: 55 according to formula (IIc))
N*N*NNNDGNNNBNNTHVNNNCHNHN*N*N* (SEQ ID NO: 56 according to formula (IIe))
N*N*HHNRGNNNYHBTHRDNNCYDHH*N*N* (SEQ ID NO: 57 according to formula (IIe))
N*H*HHVRGNDBYHYTHRDHNCYRHH*H*H* (SEQ ID NO: 58 according to formula (IIe))
H*H*MHMVGYYYTYHTHRVRRCBVMH*H*N* (SEQ ID NO: 59 according to formula (IIf))
M*M*MMMSGYYCTTYTMAGRRCSMCH*H*H* (SEQ ID NO: 60 according to formula (IIf))
M*M*MMMSGYYCTTTTMAGRRCSACH*M*H* (SEQ ID NO: 61 according to formula (IIf))
H*H*MAMGGYYCTTYTHAGRRCCVHN*N*M* (SEQ ID NO: 62 according to formula (IIg))
H*H*AAMGGCYCTTYTMAGRGCCVCH*H*M* (SEQ ID NO: 63 according to formula (IIg))
M*M*AAMGGCTCTTTTMAGRGCCMCY*M*M* (SEQ ID NO: 64 according to formula (IIg))
N*H*AAHDGHYCTDYTHASRRCCVHB*N*H* (SEQ ID NO: 65 according to formula (IIh))
H*H*AAMGGCYCTTTTHAGRGCCVMY*N*M* (SEQ ID NO: 66 according to formula (IIh))
H*M*AAAGGCYCTTTTMAGRGCCRMY*H*M* (SEQ ID NO: 67 according to formula (IIh))

According to a further embodiment disclosed herein, at least one mRNA comprises at least one histone stem-loop sequence showing at least about 80%, preferably at least about 85%, more preferably at least about 90%, or even more preferably at least about 95%, sequence identity with the not to 100% conserved nucleotides in the histone stem-loop sequences according to at least one of specific formulae (I) or (Ia) to (Ih) or (II) or (IIa) to (IIh) or with a naturally occurring histone stem-loop sequence.

A particular preferred histone stem-loop sequence is the sequence according to SEQ ID NO: 71 CAAAGGCTCTTTTCAGAGCCACCA or more preferably the corresponding RNA sequence of the nucleic acid sequence according to SEQ ID NO: 71: CAAAGGCUCUUUUCAGAGCCACCA (SEQ ID NO: 72).

In a preferred embodiment, the histone stem loop sequence does not contain the loop sequence 5'-UUUC-3'. More specifically, the histone stem loop does not contain the stem1 sequence 5'-GGCUCU-3' and/or the stem2 sequence 5'-AGAGCC-3', respectively. In another preferred embodiment, the stem loop sequence does not contain the loop sequence 5'-CCUGCCC-3' or the loop sequence 5'-UGAAU-3'. More specifically, the stem loop does not contain the stem1 sequence 5'-CCUGAGC-3' or does not contain the stem1 sequence 5'-ACCUUUCUCCA-3' and/or the stem2 sequence 5'-GCUCAGG-3' or 5'-UGGAGAAAGGU-3', respectively. Also, stem loop sequences are preferably not derived from a mammalian insulin receptor 3'-untranslated region. Also, preferably, at least one mRNA disclosed herein may not contain histone stem loop processing signals, in particular not those derived from mouse histone gene H2A614 gene (H2kA614).

Preferably, at least one mRNA of the composition disclosed herein does not contain one or two or at least one or all but one or all of the components of the group consisting of: a sequence encoding a ribozyme (preferably a self-splicing ribozyme), a viral nucleic acid sequence, a histone stem-loop processing signal, in particular a histone-stem loop processing sequence derived from mouse histone H2A614 gene, a Neo gene, an inactivated promoter sequence and an inactivated enhancer sequence. Even more preferably, at least one mRNA disclosed herein does not contain a ribozyme, preferably a self-splicing ribozyme, and one of the group consisting of: a Neo gene, an inactivated promoter sequence, an inactivated enhancer sequence, a histone stem-loop processing signal, in particular a histone-stem loop processing sequence derived from mouse histone H2A614 gene. Accordingly, the mRNA may in a preferred mode neither contain a ribozyme, preferably a self-splicing ribozyme, nor a Neo gene or, alternatively, neither a ribozyme, preferably a self-splicing ribozyme, nor any resistance gene (e.g. usually applied for selection). In another preferred mode, at least one mRNA disclosed herein may neither contain a ribozyme, preferably a self-splicing ribozyme nor a histone stem-loop processing signal, in particular a histone-stem loop processing sequence derived from mouse histone H2A614 gene.

Alternatively, at least one mRNA of the composition disclosed herein optionally comprises a polyadenylation signal which is defined herein as a signal which conveys polyadenylation to a (transcribed) mRNA by specific protein factors (e.g. cleavage and polyadenylation specificity factor (CPSF), cleavage stimulation factor (CstF), cleavage factors I and II (CF I and CF II), poly(A) polymerase (PAP)). In this context a consensus polyadenylation signal is preferred comprising the NN(U/T)ANA consensus sequence. In a particular preferred aspect the polyadenylation signal comprises one of the following sequences: AA(U/T)AAA or A(U/T)(U/T)AAA (wherein uridine is usually present in RNA and thymidine is usually present in DNA). In some embodiments, the polyadenylation signal used in at least one mRNA disclosed herein does not correspond to the U3 snRNA, U5, the polyadenylation processing signal from human gene G-CSF, or the SV40 polyadenylation signal sequences. In particular, the above polyadenylation signals are not combined with any antibiotics resistance gene (or any other reporter, marker or selection gene), in particular not with the resistance *neo* gene (neomycin phosphotransferase). And any of the above polyadenylation signals are preferably not combined with the histone stem loop or the histone stem loop processing signal from mouse histone gene H2A614 in at least one mRNA disclosed herein.

According to another embodiment disclosed herein, at least one mRNA of the composition disclosed herein may be modified, and thus stabilized, by modifying the G/C content of the mRNA, preferably of the coding region of the mRNA.

In an embodiment described herein, the G/C content of the coding region of at least one mRNA of the composition disclosed herein is modified, particularly increased, compared to the G/C content of the coding region of its particular wild-type mRNA, i.e. the unmodified mRNA. The amino acid sequence encoded by the mRNA is preferably not modified as compared to the amino acid sequence encoded by the particular wild-type mRNA. This modification of at least one mRNA of the composition is based on the fact that the sequence of any mRNA region to be translated is important for efficient translation of that mRNA. Thus, the composition and the sequence of various nucleotides are important. In particular, sequences having an increased G (guanosine)/C (cytidine) content are more stable than sequences having an increased A (adenosine)/U (uridine) content. According to the present disclosure, the codons of the mRNA are therefore varied compared to the respective wild-type mRNA, while retaining the translated amino acid sequence, such that they include an increased amount of G/C nucleotides. In respect to the fact that several codons code for one and the same amino acid (so-called degeneration of the genetic code), the most favorable codons for the stability can be determined (so-called alternative codon usage). Depending on the amino acid to be encoded by the mRNA, there are various possibilities for modification of the mRNA sequence, compared to its wild-type sequence. In the case of amino acids which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons which code for the same amino acids but contain no A and/or U. Examples of these are: the codons for Pro can be modified from CCU or CCA to CCC or CCG; the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC; the codon for His can be modified from CAU to CAC; the codon for Gln can be modified from CAA to CAG; the codons for Ile can be modified from AUU or AUA to AUC; the codons for Thr can be modified from ACU or ACA to ACC or ACG; the codon for Asn can be modified from AAU to AAC; the codon for Lys can be modified from AAA to AAG; the codons for Val can be modified from GUU or GUA to GUC or GUG; the codon for Asp can be modified from GAU to GAC; the codon for Glu can be modified from GAA to GAG; the stop codon UAA can be modified to UAG or UGA. In the case of the codons for Met (AUG) and Trp (UGG), on the other hand, there is no possibility of sequence modification. The substitutions listed above can be used either individually or in all possible combinations to increase the G/C content of at least one mRNA of the composition disclosed herein compared to its particular wild-type mRNA (i.e. the original sequence). Thus, for example, all codons for Thr occurring in the wild-type sequence can be modified to ACC (or ACG). Preferably, however, for example, combinations of the above substitution possibilities are used:
substitution of all codons coding for Thr in the original sequence (wild-type mRNA) to ACC (or ACG) and
substitution of all codons originally coding for Ser to UCC (or UCG or AGC); substitution of all codons coding for Ile in the original sequence to AUC and
substitution of all codons originally coding for Lys to AAG and
substitution of all codons originally coding for Tyr to UAC; substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Arg to CGC (or CGG); substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Gly to GGC (or GGG) and
substitution of all codons originally coding for Asn to AAC; substitution of all codons coding for Val in the original sequence to GUC (or GUG) and
substitution of all codons originally coding for Phe to UUC and
substitution of all codons originally coding for Cys to UGC and
substitution of all codons originally coding for Leu to CUG (or CUC) and
substitution of all codons originally coding for Gln to CAG and
substitution of all codons originally coding for Pro to CCC (or CCG); etc. Preferably, the G/C content of the coding region of at least one mRNA of the composition disclosed herein is increased by at least 7%, more preferably by at least 15%, particularly preferably by at least 20%, compared to the G/C content of the coded region of the wild-type mRNA which codes for an antigen, antigenic protein or antigenic peptide as deinined herein or its fragment or variant thereof. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the region coding for an antigen, antigenic protein or antigenic peptide as defined herein or its fragment or variant thereof or the whole sequence of the wild type mRNA sequence are substituted, thereby increasing the GC/content of said sequence. In this context, it is particularly preferable to increase the G/C content of at least one mRNA of the composition disclosed herein to the maximum (i.e. 100% of the substitutable codons), in particular in the region coding for a protein, compared to the wild-type sequence. According to the present disclosure, a further preferred modification of at least one mRNA of the composition disclosed herein is based on the finding that the translation efficiency is also determined by a different frequency in the occurrence of tRNAs in cells. Thus, if so-called "rare codons" are present in at least one mRNA of the composition disclosed herein to an increased extent, the corresponding modified mRNA sequence is translated to a significantly poorer degree than in the case where codons coding for relatively "frequent" tRNAs are present. According to the the present disclosure, in the modified mRNA of the composition described herein, the region which codes for one of the above defined antigens is modified compared to the corresponding region of the wild-type mRNA such that at least one codon of the wild-type sequence, which codes for a tRNA which is relatively rare in the cell, is exchanged for a codon, which codes for a tRNA which is relatively frequent in the cell and carries the same amino acid as the relatively rare tRNA. By this modification, the sequences of the mRNA of the composition disclosed herein is modified such that codons for which frequently occurring tRNAs are available are inserted. In other words, according to the the present disclosure, by this modification all codons of the wild-type sequence which code for a tRNA which is relatively rare in the cell can in each case be exchanged for a codon which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA, which occurs the most frequently in the (human) cell, are particularly preferred. According to the present disclosure, it is particularly preferable to link the sequential G/C content which is increased, in particular maximized, in the modified mRNA of the composition disclosed herein, with the "frequent" codons without modifying the amino acid sequence of the protein encoded by the coding region of the mRNA. This embodiment allows provision of a particularly efficiently translated and stabilized (modified) mRNA of the composition disclosed herein. The determination of a modified mRNA of the composition as described above (increased G/C content; exchange of tRNAs) can be carried out using the computer program explained in WO 02/098443. Using this computer program, the nucleotide sequence of any desired mRNA can be modified with the aid of the genetic code or the degenerative nature thereof such that a maximum G/C content results, in combination with the use of codons which code for tRNAs occurring as frequently as possible in the cell, the amino acid sequence coded by the modified mRNA preferably not being modified compared to the non-modified sequence. Alternatively, it is also possible to modify only the G/C content or only the codon usage compared to the original sequence. The source code in Visual Basic 6.0 (development environment used: Microsoft Visual Studio Enterprise 6.0 with Servicepack 3) is also described in WO 02/098443. In a further embodiment disclosed herein, the A/U content in the environment of the ribosome binding site of at least one mRNA of the composition disclosed herein is increased compared to the A/U content in the environment of the ribosome binding site of its particular wild-type mRNA. This modification (an increased A/U content around the ribosome binding site) increases the efficiency of ribosome binding to the mRNA. An effective binding of the ribosomes to the ribosome binding site (Kozak sequence: GCCGCCACCAUGG (SEQ ID NO: 68), the AUG forms the start codon) in turn has the effect of an efficient translation of the mRNA. According to a further embodiment disclosed herein, at least one mRNA of the composition disclosed herein may be modified with respect to potentially destabilizing sequence elements. Particularly, the coding region and/or the 5' and/or 3' untranslated region of this mRNA may be modified compared to the particular wild-type mRNA such that it contains no destabilizing sequence elements, the coded amino acid sequence of the modified mRNA preferably not being modified compared to its particular wild-type mRNA. It is known that, for example, in sequences of eukaryotic RNAs destabilizing sequence elements (DSE) occur, to which signal proteins bind and regulate enzymatic degradation of RNA in vivo. For further stabilization of the modified mRNA, optionally in the region which encodes for an antigen, antigenic protein or antigenic peptide as defined herein, one or more such modifications compared to the corresponding region of the wild-type mRNA can therefore be carried out, so that no or substantially no destabilizing sequence elements are contained there. According to the present disclosure, DSE present in the untranslated regions (3'- and/or 5'-UTR) can also be eliminated from at least one mRNA of the composition disclosed herein by such modifications. Such destabilizing sequences are e.g. AU-rich sequences (AURES), which occur in 3'-UTR sections of numerous unstable RNAs (Caput et al., Proc. Natl. Acad. Sci. USA 1986, 83: 1670 to 1674). At least one mRNA of the composition disclosed herein is therefore preferably modified compared to the wild-type mRNA such that the mRNA contains no such destabilizing sequences. This also applies to those sequence motifs which are recognized by possible endonucleases, e.g. the sequence GAACAAG, which is contained in the 3'-UTR segment of the gene which codes for the transferrin receptor (Binder et al., EMBO J. 1994, 13: 1969 to 1980). These sequence motifs are also preferably removed in at least one mRNA of the composition disclosed herein. Also according to the present disclosure, at least one mRNA of the composition has, in a modified form, at least one IRES as defined above and/or at least one 5' and/or 3' stabilizing sequence, in a modified form, e.g. to enhance ribosome binding or to allow expression of different encoded antigens located on an at least one (bi- or even multicistronic) mRNA of the composition disclosed herein.

According to the present disclosure, at least one mRNA of the composition furthermore preferably has at least one 5' and/or 3' stabilizing sequence. These stabilizing sequences in the 5' and/or 3' untranslated regions have the effect of increasing the half-life of the mRNA in the cytosol. These stabilizing sequences can have 100% sequence homology to naturally occurring sequences, which occur in viruses, bacteria and eukaryotes, but can also be partly or completely synthetic. The untranslated sequences (UTR) of the β-globin gene, e.g. from Homo sapiens or Xenopus laevis may be mentioned as an example of stabilizing sequences, which can be used for a stabilized mRNA. Another example of a stabilizing sequence has the general formula (C/U)CCANxCCC(U/A)PyxUC(C/U)CC (SEQ ID NO: 69), which is contained in the 3'UTR of the very stable mRNA which codes for α-globin, α(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art. At least one mRNA of the composition disclosed herein is therefore preferably present as globin UTR (untranslated regions)-stabilized mRNA, in particular as α-globin UTR-stabilized mRNA. Preferably at least one mRNA of the composition comprises a stabilizing sequence in the 3'-UTR derived from the center, α-complex-binding portion of the 3'UTR of an α-globin gene, such as of a human α-globin gene, preferably according to SEQ ID No. 70:
Center, α-complex-binding portion of the 3'UTR of an α-globin gene (also named herein as "muag") GCCCGAUGGGCCUCCCAACGGGCCCUCCUCCCCUCCUUGCACCG (SEQ ID NO. 70).

Nevertheless, substitutions, additions or eliminations of bases are preferably carried out with at least one mRNA of the composition disclosed herein, using a DNA matrix for preparation of the mRNA of the composition disclosed herein by techniques of the well known site-directed mutagenesis or with an oligonucleotide ligation strategy (see e.g. Maniatis et al., Molecular Cloning: A Laboratory Manual, Cold Spring Harbor Laboratory Press, 3rd ed., Cold Spring Harbor, NY, 2001). In such a process, for preparation of the mRNA, a corresponding DNA molecule may be transcribed in vitro. This DNA matrix preferably comprises a suitable promoter, e.g. a T7 or SP6 promoter, for in vitro transcription, which is followed by the desired nucleotide sequence for the mRNA to be prepared and a termination signal for in vitro transcription. The DNA molecule, which forms the matrix of an mRNA of interest, may be prepared by fermentative proliferation and subsequent isolation as part of a plasmid which can be replicated in bacteria. Plasmids, which may be mentioned as suitable for the present invention, are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

According to the present invention, each of the six mRNAs as defined in the claims is complexed with a cationic compound selected from the group consisting of a cationic peptide, a cationic protein, a cationic polysaccharide, a cationic polymer and a cationic lipid.

The stabilization of at least one mRNA of the composition disclosed herein can likewise be carried out by associating or complexing the mRNA with, or binding it to, a cationic compound, in particular a polycationic compound, for example a (poly)cationic peptide or protein. In particular, the use of protamine, nucleoline, spermin or spermidine as the polycationic, nucleic-acid-binding protein to the RNA is particularly effective. Furthermore, the use of other cationic peptides or proteins, such as poly-L-lysine or histones, is likewise possible. This procedure for stabilizing mRNA is described in EP-A-1083232. Further preferred cationic substances, which can be used for stabilizing the mRNA of the composition disclosed herein, include cationic polysaccharides, for example chitosan, polybrene, polyethyleneimine (PEI) or poly-L-lysine (PLL), etc.. Association or complexing of at least one mRNA of the composition disclosed herein with cationic compounds, e.g. cationic proteins or cationic lipids, e.g. oligofectamine as a lipid based complexation reagent) preferably increases the transfer of the mRNA present as a pharmaceutically active component into the cells to be treated or into the organism to be treated. It is also referred to the disclosure herein with regard to the stabilizing effect for at least one mRNA of the composition disclosed herein by complexation, which holds for the stabilization of mRNA as well.

According to another particularly preferred embodiment, at least one mRNA of the composition may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the antigen, antigenic protein or antigenic peptide as encoded by at least one mRNA of the composition into a defined cellular compartiment, preferably the cell surface, the endoplasmic reticulum (ER) or the endosomal-lysosomal compartiment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines as defined herein, signal sequences of the invariant chain of immunoglobulines or antibodies as defined herein, signal sequences of Lamp1, Tapasin, Erp57, Calretikulin, Calnexin, and further membrane associated proteins or of proteins associated with the endoplasmic reticulum (ER) or the endosomal-lysosomal compartiment. Particularly preferably, signal sequences of MHC class I molecule HLA-A*0201 may be used according to the present disclosure.

Any of the above modifications may be applied to at least one mRNA of the composition disclosed herein, and further to any RNA as used in the context of the present disclosure and may be, if suitable or necessary, be combined with each other in any combination, provided, these combinations of modifications do not interfere with each other in the respective mRNA. A person skilled in the art will be able to take his choice accordingly. Each of the six antigens of the composition disclosed herein is encoded by one (monocistronic) mRNA as defined in the claims. In other words, the composition disclosed herein contains six (monocistronic) mRNAs, wherein each of these six (monocistronic) mRNAs, encodes just one antigen as defined above. The composition comprises six mRNAs as defined by the claims, wherein one mRNA encodes 5T4, one mRNA encodes Survivin, one mRNA encodes NY-ESO-1, one mRNA encodes MAGE-C1, one mRNA encodes MAGE-C2 and one mRNA encodes MUC1, respectively.

In a non-claimed embodiment, the composition comprises six mRNAs, wherein one mRNA encodes 5T4 and comprises a coding sequence identical or at least 80% identical to SEQ ID NO: 3, one mRNA encodes Survivin and comprises a coding sequence identical or at least 80% identical to SEQ ID NO: 6, one mRNA encodes NY-ESO-1 and comprises a coding sequence identical or at least 80% identical to SEQ ID NO: 9, one mRNA encodes MAGE-C1 and comprises a coding sequence identical or at least 80% identical to SEQ ID NO: 12 or 25, one mRNA encodes MAGE-C2 and comprises a coding sequence identical or at least 80% identical to SEQ ID NO: 15 and one mRNA encodes MUC1 and comprises a coding sequence identical or at least 80% identical to SEQ ID NO:18 (or fragments or variants of each of these sequences) and optionally further excipients.

In a further non-claimed embodiment, the composition comprises at least one mRNA, which is identical or at least 80% identical to the RNA sequence of SEQ ID NOs: 1, 4, 7, 10, 13 or 16. Even more preferably, the composition comprises six mRNAs, wherein each mRNA is identical or at least 80% identical to one of the RNA sequences according to SEQ ID NOs: 1, 4, 7, 10, 13 or 16.

In a non-claimed embodiment, the composition comprises six mRNAs, wherein one mRNA encodes 5T4 and is identical or at least 80% identical to SEQ ID NO: 1, one mRNA encodes Survivin and is identical or at least 80% identical to SEQ ID NO: 4, one mRNA encodes NY-ESO-1 and is identical or at least 80% identical to SEQ ID NO: 7, one mRNA encodes MAGE-C1 and is identical or at least 80% identical to SEQ ID NO: 10, one mRNA encodes MAGE-C2 and is identical or at least 80% identical to SEQ ID NO: 13 and one mRNA encodes MUC1 and is identical or at least 80% identical to SEQ ID NO:16 (or fragments or variants of each of these sequences) and optionally further excipients. According to the present disclosure, at least one mRNA of the compositions described above comprises a histone stem-loop in the 3' UTR region. According to the present invention, the composition comprises six mRNAs as defined by the claims, wherein each of the mRNAs comprises a histone stem-loop as defined herewithin.

The composition comprises six mRNAs, wherein one mRNA encodes 5T4 and is identical to SEQ ID NO: 19, one mRNA encodes Survivin and is identical to SEQ ID NO: 20, one mRNA encodes NY-ESO-1 and is identical to SEQ ID NO: 21, one mRNA encodes MAGE-C1 and is identical to SEQ ID NO: 22, one mRNA encodes MAGE-C2 and is identical to SEQ ID NO: 23 and one mRNA encodes MUC1 and is identical to SEQ ID NO: 24 (or fragments or variants of each of these sequences) and optionally further excipients.

According to another embodiment, the composition for use according to the invention may comprise an adjuvant in order to enhance the immunostimulatory properties of the composition. In this context, an adjuvant may be understood as any compound, which is suitable to support administration and delivery of the composition for use according to the invention. Furthermore, such an adjuvant may, without being bound thereto, initiate or increase an immune response of the innate immune system, i.e. a non-specific immune response. With other words, when administered, the composition for use according to the invention typically initiates an adaptive immune response due to the at least six antigens encoded by the at least six mRNAs contained in the composition as defined in the claims. Additionally, the composition for use according to the invention may generate an (supportive) innate immune response due to addition of an adjuvant as defined herein to the composition described herein.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMER™ (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE™ (propanediamine); BAY R1005™ ((N-(2-deoxy-2-L-leucylamino-b-D-glucopyranosyl)-N-octadecyl-dodecanoyl-amide hydroacetate); CALCITRIOL™ (1-alpha,25-dihydroxy-vitamin D3); calcium phosphate gel; CAP™ (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTher™ (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMS™; ISCOPREP 7.0.3.™; liposomes; LOXORIBINE™ (7-allyl-8-oxoguanosine); LT oral adjuvant (E.coli labile enterotoxin-protoxin); microspheres and microparticles of any composition; MF59TM; (squalene-water emulsion); MONTANIDE ISA 51™ (purified incomplete Freund's adjuvant); MONTANIDE ISA 720™ (metabolisable oil adjuvant); MPL™ (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE™ (Nac-Mur-L-Ala-D-Gln-OCH3); MURAPALMITINE™ and D-MURAPALMITINE™ (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURAN™ (β-glucan); PLGA, PGA and PLA (homo- and co-polymers of lactic acid and glycolic acid; microspheres/nanospheres); PLURONIC L121™; PMMA (polymethyl methacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON™ (QS-21); Quil-A (Quil-A saponin); S-28463 (4-amino-otec-dimethyl-2-ethoxymethyl-1H-imidazo[4,5 c]quinoline-1-ethanol); SAF-1™ ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Ala-dipalmitoxypropylamide); Theronyl-MDP (Termurtide™ or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the at least six mRNAs of the composition with the cationic or polycationic compound. Association or complexing the mRNA of the composition with cationic or polycationic compounds as defined herein preferably provides adjuvant properties and confers a stabilizing effect to the mRNA of the composition. Particularly such preferred, such cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: 1-(2,3-sioleyloxy)propyl)-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglycylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as -aminoacid- polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing at least one mRNA of the composition, may be selected from following proteins or peptides having the following total formula (III): (Arg)l;(Lys)m;(His)n;(Orn)o;(Xaa)x, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

The ratio of the mRNA to the cationic or polycationic compound in the adjuvant component may be calculated on the basis of the nitrogen/phosphate ratio (N/P-ratio) of the entire mRNA complex, i.e. the ratio of positively charged (nitrogen) atoms of the cationic or polycationic compound to the negatively charged phosphate atoms of the nucleic acids. For example, 1 µg RNA typically contains about 3 nmol phosphate residues, provided the RNA exhibits a statistical distribution of bases. Additionally, 1 µg peptide typically contains about x nmol nitrogen residues, dependent on the molecular weight and the number of basic amino acids. When exemplarily calculated for (Arg)9 (molecular weight 1424 g/mol, 9 nitrogen atoms), 1 µg (Arg)9 contains about 700 pmol (Arg)9 and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 µg RNA, 6 nmol phosphate are to be calulated for the RNA; 1 µg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of RNA:peptide in the complex, and most preferably in the range of about 0.7-1.5.

In a preferred embodiment, the composition for use is obtained in two separate steps in order to obtain both, an efficient immunostimulatory effect and efficient translation of the at least six mRNAs disclosed herein. Therein, a so called "adjuvant component" is prepared by complexing - in a first step - an at least one mRNA of the adjuvant component with a cationic or polycationic compound in a specific ratio to form a stable complex. In this context, it is important, that no free cationic or polycationic compound or only a neglibly small amount remains in the adjuvant component after complexing the mRNA. Accordingly, the ratio of the mRNA and the cationic or polycationic compound in the adjuvant component is typically selected in a range that the mRNA is entirely complexed and no free cationic or polycationic compound or only a neglectably small amount remains in the composition. Preferably the ratio of the adjuvant component, i.e. the ratio of the mRNA to the cationic or polycationic compound is selected from a range of about 6:1 (w/w) to about 0.25:1 (w/w), more preferably from about 5:1 (w/w) to about 0.5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the at least six mRNAs encoding the antigens disclosed herein are added in a second step to the complexed mRNA of the adjuvant component in order to form the (immunostimulatory) composition. Therein, the at least one mRNA is added as free mRNA, i.e. mRNA, which is not complexed by other compounds. Prior to addition, the at least one free mRNA is not complexed and will preferably not undergo any detectable or significant complexation reaction upon the addition of the adjuvant component. This is due to the strong binding of the cationic or polycationic compound to the above described at least one mRNA in the adjuvant component. In other words, when the free mRNA, encoding one of the antigens disclosed herein, is added to the "adjuvant component", preferably no free or substantially no free cationic or polycationic compound is present, which may form a complex with the free mRNA. Accordingly, an efficient translation of the free mRNA of the composition is possible in vivo. Therein, at least one free mRNA may occur as a mono-, di-, or multicistronic mRNA, i.e. an RNA which carries the coding sequences of one or more proteins. Such coding sequences in di-, or even multicistronic mRNA may be separated by at least one IRES sequence, e.g. as defined herein.

In a particularly preferred embodiment, the at least one free mRNA, which is comprised in the composition described herein, may be identical or different to the at least one mRNA of the adjuvant component of the composition described herein, depending on the specific requirements of therapy. Even more preferably, the at least one free mRNA, which is comprised in the composition described herein, is identical to the at least one RNA of the adjuvant component of the composition described herein.

In a particulary preferred embodiment, the composition comprises at least six mRNAs, wherein at least six mRNAs encode the antigens as defined above and wherein said mRNA is present in the composition partially as free mRNA and partially as complexed mRNA. Preferably, the at least six mRNAs each encoding one antigen as defined above are complexed as described above and the same at least six mRNAs are then added as free RNAs, wherein preferably the compound, which is used for complexing the mRNA is not present in free form in the composition at the moment of addition of the free mRNA component.

The ratio of the first component (i.e. the adjuvant component comprising or consisting of at least one mRNA complexed with a cationic or polycationic compound) and the second component (i.e. the at least one free mRNA) may be selected in the composition disclosed herein according to the specific requirements of a particular therapy. Typically, the ratio of the mRNA in the adjuvant component and the at least one free mRNA (mRNA in the adjuvant component: free RNA) of the composition disclosed herein is selected such that a significant stimulation of the innate immune system is elicited due to the adjuvant component. In parallel, the ratio is selected such that a significant amount of the free mRNA can be provided *in vivo* leading to an efficient translation and concentration of the expressed protein *in vivo,* e.g. the six antigens, etc. as defined above. Preferably the ratio of the mRNA in the adjuvant component : free mRNA in the composition disclosed herein is selected from a range of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of mRNA in the adjuvant component : free mRNA in the composition disclosed herein is selected from a ratio of about 1:1 (w/w).

Additionally or alternatively, the ratio of the first component (i.e. the adjuvant component comprising or consisting of at least one mRNA complexed with a cationic or polycationic compound) and the second component (i.e. the at least one free mRNA) may be calculated on the basis of the nitrogen/phosphate ratio (N/P-ratio) of the entire mRNA complex. In the context of the present disclosure, an N/P-ratio is preferably in the range of about 0.1-10, preferably in a range of about 0.3-4 and most preferably in a range of about 0.5-2 or 0.7-2 regarding the ratio of mRNA:peptide in the complex, and most preferably in the range of about 0.7-1.5.

Additionally or alternatively, the ratio of the first component (i.e. the adjuvant component comprising or consisting of at least one mRNA complexed with a cationic or polycationic compound) and the second component (i.e. the at least one free mRNA) may also be selected in the composition disclosed herein on the basis of the molar ratio of both mRNAs to each other, i.e. the mRNA of the adjuvant component, being complexed with a cationic or polycationic compound and the at least one free mRNA of the second component. Typically, the molar ratio of the mRNA of the adjuvant component to the at least one free mRNA of the second component may be selected such, that the molar ratio suffices the above (w/w) and/or N/P-definitions. More preferably, the molar ratio of the mRNA of the adjuvant component to the at least one free mRNA of the second component may be selected e.g. from a molar ratio of about 0.001:1, 0.01:1, 0.1:1, 0.2:1, 0.3:1, 0.4:1, 0.5:1, 0.6:1, 0.7:1, 0.8:1, 0.9:1, 1:1, 1:0.9, 1:0.8, 1:0.7, 1:0.6, 1:0.5, 1:0.4, 1:0.3, 1:0.2, 1:0.1, 1:0.01, 1:0.001, etc. or from any range formed by any two of the above values, e.g. a range selected from about 0.001:1 to 1:0.001, including a range of about 0.01:1 to 1:0.001, 0.1:1 to 1:0.001, 0.2:1 to 1:0.001, 0.3:1 to 1:0.001, 0.4:1 to 1:0.001, 0.5:1 to 1:0.001, 0.6:1 to 1:0.001, 0.7:1 to 1:0.001, 0.8:1 to 1:0.001, 0.9:1 to 1:0.001, 1:1 to 1:0.001, 1:0.9 to 1:0.001, 1:0.8 to 1:0.001, 1:0.7 to 1:0.001, 1:0.6 to 1:0.001, 1:0.5 to 1:0.001, 1:0.4 to 1:0.001, 1:0.3 to 1:0.001, 1:0.2 to 1:0.001, 1:0.1 to 1:0.001, 1:0.01 to 1:0.001, or a range of about 0.01:1 to 1:0.01, 0.1:1 to 1:0.01, 0.2:1 to 1:0.01, 0.3:1 to 1:0.01, 0.4:1 to 1:0.01, 0.5:1 to 1:0.01, 0.6:1 to 1:0.01, 0.7:1 to 1:0.01, 0.8:1 to 1:0.01, 0.9:1 to 1:0.01, 1:1 to 1:0.01, 1:0.9 to 1:0.01, 1:0.8 to 1:0.01, 1:0.7 to 1:0.01, 1:0.6 to 1:0.01, 1:0.5 to 1:0.01, 1:0.4 to 1:0.01, 1:0.3 to 1:0.01, 1:0.2 to 1:0.01, 1:0.1 to 1:0.01, 1:0.01 to 1:0.01, or including a range of about 0.001:1 to 1:0.01, 0.001:1 to 1:0.1, 0.001:1 to 1:0.2, 0.001:1 to 1:0.3, 0.001:1 to 1:0.4, 0.001:1 to 1:0.5, 0.001:1 to 1:0.6, 0.001:1 to 1:0.7, 0.001:1 to 1:0.8, 0.001:1 to 1:0.9, 0.001:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, or a range of about 0.01:1 to 1:0.01, 0.01:1 to 1:0.1, 0.01:1 to 1:0.2, 0.01:1 to 1:0.3, 0.01:1 to 1:0.4, 0.01:1 to 1:0.5, 0.01:1 to 1:0.6, 0.01:1 to 1:0.7, 0.01:1 to 1:0.8, 0.01:1 to 1:0.9, 0.01:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, etc.

Even more preferably, the molar ratio of the mRNA of the adjuvant component to the at least one free mRNA of the second component may be selected e.g. from a range of about 0.01:1 to 1:0.01. Most preferably, the molar ratio of the mRNA of the adjuvant component to the at least one free mRNA of the second component may be selected e.g. from a molar ratio of about 1:1. Any of the above definitions with regard to (w/w) and/or N/P ratio may also apply.

Suitable adjuvants may furthermore be selected from nucleic acids having the formula (IV): GlXmGn, wherein: G is guanosine, uridine or an analogue of guanosine or uridine; X is guanosine, uridine, adenosine, thymidine, cytidine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uridine or an analogue thereof, when m > 3 at least 3 successive uridines or analogues of uridine occur; n is an integer from 1 to 40, wherein when n = 1 G is guanosine or an analogue thereof, when n > 1 at least 50% of the nucleotides are guanosine or an analogue thereof.

Other suitable adjuvants may furthermore be selected from nucleic acids having the formula (V): ClXmCn, wherein: C is cytidine, uridine or an analogue of cytidine or uridine; X is guanosine, uridine, adenosine, thymidine, cytidine or an analogue of the above-mentioned nucleotides; l is an integer from 1 to 40, wherein when l = 1 C is cytidine or an analogue thereof, when l > 1 at least 50% of the nucleotides are cytidine or an analogue thereof; m is an integer and is at least 3; wherein when m = 3 X is uridine or an analogue thereof, when m > 3 at least 3 successive uridines or analogues of uridine occur; n is an integer from 1 to 40, wherein when n = 1 C is cytidine or an analogue thereof, when n > 1 at least 50% of the nucleotides are cytidine or an analogue thereof.

According to one further aspect, the present invention may provide a vaccine for use in the treatment of non-small cell lung cancer, wherein the treatment further comprises administration of a chemotherapeutic agent. The vaccine comprises a composition as defined by the claims. Preferably, the vaccine further comprises a pharmaceutically acceptable carrier. Accordingly, the vaccine comprises at least six distinct mRNA species as defined by the claims, encoding at least the above defined antigens 5T4, Suvivin, NY-ESO-1, MAGE-C1, MAGE-C2 and MUC-1. Accordingly, the vaccine described herein is based on the same components as the composition as defined above. Insofar, it may be referred to the above disclosure defining the composition. In non-claimed embodiments, the vaccine may, however, be provided in physically separate form and may be administered by separate administration steps. The vaccine disclosed herein may correspond to the composition disclosed herein, if the mRNA components are provided by one single composition. However, according to a non-claimed embodiment the vaccine disclosed herein may e.g. be provided physically separated. E.g., the mRNA species may be provided such that two separate compositions, which may contain at least one mRNA species each (e.g. three distinct mRNA species) encoding for three distinct antigens, are provided, which may or may not be combined (not claimed). Also, the vaccine disclosed herein may be a combination of three distinct compositions, each composition comprising at least one mRNA encoding two of the above six antigens (not claimed). Or, the vaccine may be provided as a combination of at least one mRNA, preferably six mRNAs, each encoding for one of the above defined six antigens (not claimed). The vaccine may be combined to provide one single composition prior to its use or it may be used such that more than one administration is required to administer the distinct mRNA species coding for the above defined six distinct antigens (not claimed). If the vaccine contains at least one mRNA molecule, typically at least two mRNA molecules, encoding for the above defined six antigens, it may e.g. be administered by one single administration (combining all mRNA species), by two separate administrations (e.g. each administration administering mRNA molecules encoding for three of the above six antigens), by three, four, five or six administrations (in case all of the mRNA species encode one of the above defined six antigens and are provided physically separate) (not claimed). Accordingly; any combination of mono-, bi- or multicistronic mRNAs encoding for the above defined six antigens (and optionally further antigens), provided as separate entitities (containing one mRNA species) or as combined entity (containing more than one mRNA species), is understood as a vaccine according to the present disclosure (not claimed). According to a non-claimed embodiment of the vaccine disclosed herein, each of the antigens defined herein is provided as an individual (monocistronic) mRNA, which is administered separately.

As with the composition disclosed herein, the entities of the vaccine may be provided in liquid and or in dry (e.g. lyophylized) form. They may contain further components, in particular further components allowing for its pharmaceutical use. The vaccine or the composition described herein may, e.g., additionally contain a pharmaceutically acceptable carrier and/or further auxiliary substances and additives and/or adjuvants.

The vaccine or composition disclosed herein typically comprises a safe and effective amount of the at least six mRNAs of the composition as defined above encoding the antigens as defined above. As used herein, "safe and effective amount" means an amount of the at least six mRNAs of the composition or the vaccine as defined above, that is sufficient to significantly induce a positive modification of lung cancer, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma. At the same time, however, a "safe and effective amount" is small enough to avoid serious side-effects, that is to say to permit a sensible relationship between advantage and risk. The determination of these limits typically lies within the scope of sensible medical judgment. In relation to the vaccine or composition disclosed herein, the expression "safe and effective amount" preferably means an amount of the mRNA (and thus of the encoded antigens) that is suitable for stimulating the adaptive immune system in such a manner that no excessive or damaging immune reactions are achieved but, preferably, also no such immune reactions below a measurable level. Such a "safe and effective amount" of the at least six mRNAs of the composition or vaccine as defined above may furthermore be selected in dependence of the type of mRNA, e.g. monocistronic, bi- or even multicistronic mRNA, since a bi- or even multicistronic mRNA may lead to a significantly higher expression of the encoded antigen(s) than use of an equal amount of a monocistronic mRNA. A "safe and effective amount" of the at least one mRNA of the composition or vaccine as defined above will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The vaccine or composition disclosed herein can be used according to the invention for human and also for veterinary medical purposes, as a pharmaceutical composition or as a vaccine.

In a preferred embodiment, the at least six mRNAs of the composition, vaccine or kit of parts for use according to the invention are provided in lyophilized form. Preferably, the at least six lyophilized mRNAs are reconstituted in a suitable buffer, advantageously based on an aqueous carrier, prior to administration, e.g. Ringer-Lactate solution, which is preferred, Ringer solution, a phosphate buffer solution. In a preferred embodiment, the composition, the vaccine or the kit of parts disclosed herein contains six mRNAs, which are provided separately in lyophilized form (optionally together with at least one further additive) and which are preferably reconstituted separately in a suitable buffer (such as Ringer-Lactate solution) prior to its use so as to allow individual administration of each of the six (monocistronic) mRNAs.

The vaccine or composition for use according to the invention may typically contain a pharmaceutically acceptable carrier. The expression "pharmaceutically acceptable carrier" as used herein preferably includes the liquid or non-liquid basis of the inventive vaccine. If the vaccine is provided in liquid form, the carrier will be water, typically pyrogen-free water; isotonic saline or buffered (aqueous) solutions, e.g phosphate, citrate etc. buffered solutions. Particularly for injection of the vaccine, water or preferably a buffer, more preferably an aqueous buffer, may be used, containing a sodium salt, preferably at least 50 mM of a sodium salt, a calcium salt, preferably at least 0.01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc.. Without being limited thereto, examples of sodium salts include e.g. NaCl, Nal, NaBr, Na₂CO₃, NaHCO₃, Na₂SO₄, examples of the optional potassium salts include e.g. KCl, Kl, KBr, K₂CO₃, KHCO₃, K₂SO₄, and examples of calcium salts include e.g. CaCl₂, CaI₂, CaBr₂, CaCO₃, CaSO₄, Ca(OH)₂. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl₂) and optionally potassium chloride (KCl), wherein further anions may be present additional to the chlorides. CaCl₂ can also be replaced by another salt like KCl. Typically, the salts in the injection buffer are present in a concentration of at least 50 mM sodium chloride (NaCl), at least 3 mM potassium chloride (KCl) and at least 0.01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in "in vivo" methods occurring liquids such as blood, lymph, cytosolic liquids, or other body liquids, or e.g. liquids, which may be used as reference media in "in vitro" methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well, which are suitable for administration to a person. The term "compatible" as used herein means that the constituents of the vaccine are capable of being mixed with the the at least six mRNAs of the composition, encoding at least six antigens as defined above, in such a manner that no interaction occurs which would substantially reduce the pharmaceutical effectiveness of the vaccine under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose, trehalose and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The choice of a pharmaceutically acceptable carrier is determined, in principle, by the manner in which the vaccine is administered. The vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, vaccines may be administered by an intradermal, subcutaneous, or intramuscular route, preferably by injection, which may be needle-free and/or needle injection. Compositions/vaccines are therefore preferably formulated in liquid or solid form. The suitable amount of the vaccine to be administered can be determined by routine experiments with animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The vaccine or composition can additionally contain one or more auxiliary substances in order to further increase the immunogenicity. A synergistic action of the at least six mRNAs of the composition or vaccine as defined above and of an auxiliary substance, which may be optionally be co-formulated (or separately formulated) with the vaccine or composition as described above, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CSF, which allow an immune response produced by the immune-stimulating adjuvant disclosed herein to be enhanced and/or influenced in a targeted manner. Particularly preferred auxiliary substances are cytokines, such as monokines, lymphokines, interleukins or chemokines, that - additional to induction of the adaptive immune response by the encoded at least six antigens - promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH. Preferably, such immunogenicity increasing agents or compounds are provided separately (not co-formulated with the vaccine or composition) and administered individually.

Further additives which may be included in the vaccine or composition are emulsifiers, such as, for example, Tween ; wetting agents, such as, for example, sodium lauryl sulfate; colouring agents; taste-imparting agents, pharmaceutical carriers; tablet-forming agents; stabilizers; antioxidants; preservatives.

The vaccine or composition can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an vaccine or composition in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytidine/guanine dinucleotide sequence(s) (CpG motif(s)). According to a first preferred alternative, at least one CpG motif contained in these sequences, that is to say the C (cytidine) and the G (guanine) of the CpG motif, is unmethylated. All further cytidines or guanines optionally contained in these sequences can be either methylated or unmethylated. According to a further preferred alternative, however, the C (cytidine) and the G (guanine) of the CpG motif can also be present in methylated form.

Preferably, the above compounds are formulated and administered separately from the above composition or vaccine containing the at least six mRNAs encoding at least the above defined six antigens.

According to a further preferred object of the present disclosure, the composition or vaccine disclosed herein may be used for the preparation of a medicament for the treatment of lung cancer and diseases or disorders related thereto, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

According to a further preferred object of the present invention, the vaccine or the composition containing the at least six mRNAs encoding the antigens as defined in the claims is provided for use in the treatment of non-small cell lung cancer, wherein the treatment further comprises administration of a chemotherapeutic agent.

Further disclosed herein is also the vaccine or composition described herein for use in the treatment of lung cancer and diseases or disorders related thereto, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

In this context, also included in the present disclosure are the composition or the vaccine for use in treating lung cancer, preferably non-small cell lung cancer, and diseases or disorders related thereto, preferably a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma, by administering to a subject in need thereof a pharmaceutically effective amount of an vaccine, or a pharmaceutically effective amount of an composition. Such a treatment typically comprises an optional first step of preparing the composition, or the vaccine, and a second step, comprising administering (a pharmaceutically effective amount of) said composition or said vaccine to a patient in need thereof. A subject in need thereof will typically be a mammal. In the context of the present disclosure, the mammal is preferably selected from the group comprising, without being limited thereto, e.g. goat, cattle, swine, dog, cat, donkey, monkey, ape, a rodent such as a mouse, hamster, rabbit and, particularly, human, wherein the mammal typically suffers from lung cancer, preferably of non-small cell lung cancer, and diseases or disorders related thereto, preferably a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

The present disclosure also relates to the composition or the at least six mRNAs encoding the antigens as defined herein for the preparation of a vaccine, preferably for eliciting an immune response in a mammal, preferably for the treatment of lung cancer, more preferably for the treatment of a non-small cell lung cancer (NSCLC) related condition as defined herein.

Preferably, the subject receiving the composition or vaccine is a patient with stage 0, I (IA and/or IB), II (IIA and/or IIB), III (IIIA and/or IIIB) or stage IV non-small cell lung cancer.

In a particular embodiment, the subject receiving the composition or vaccine is a patient with stage III or stage IV non-small cell lung cancer.

Additionally, the subject receiving the composition or vaccine may be a patient with non-small cell lung cancer receiving chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or PD1 pathway inhibitors, or a patient, who has achieved partial response or stable disease after having received one or more of the treatments specified above.

In this context, a PD-1 pathway inhibitor is preferably defined herein as a compound capable of impairing the PD-1 pathway signaling, preferably signaling mediated by the PD-1 receptor. Therefore, the PD-1 pathway inhibitor may be any inhibitor directed against any member of the PD-1 pathway capable of antagonizing PD-1 pathway signaling. In this context, the inhibitor may be an antagonistic antibody, targeting any member of the PD-1 pathway, preferably directed against PD-1 receptor, PD-L1 or PD-L2. This antagonistic antibody may also be encoded by a nucleic acid. Also, the PD-1 pathway inhibitor may be a fragment of the PD-1 receptor or the PD1-receptor blocking the activity of PD1 ligands. B7-1 or fragments thereof may act as PD1-inhibiting ligands as well. Furthermore, the PD-1 pathway inhibitor may be siRNA (small interfering RNA) or antisense RNA directed against a member of the PD-1 pathway, preferably PD-1, PD-L1 or PD-L2. Additionally, a PD-1 pathway inhibitor may be a protein comprising (or a nucleic acid coding for) an amino acid sequence capable of binding to PD-1 but preventing PD-1 signaling, e.g. by inhibiting PD-1 and B7-H1 or B7-DL interaction. Additionally, a PD-1 pathway inhibitor may be a small molecule inhibitor capable of inhibiting PD-1 pathway signaling, e.g. a PD-1 binding peptide or a small organic molecule.

Furthermore in this context, a tyrosine kinase inhibitor is preferably defined herein as a compound capable of impairing signalling of one or more tyrosine kinases, preferably of one or more growth factor tyrosine kinases. Preferably, a tyrosine kinase inhibitor, as used in this context, is an inhibitor of epidermal growth factor receptor (EGFR) tyrosine kinase. In a preferred embodiment, a tyrosine kinase inhibitor, as used herein, is an oral EGFR tyrosine kinase inhibitor. In a further preferred embodiment, a tyrosine kinase inhibitor is selected from the group of erlotinib, gefitinib or afatinib. In another embodiment, a tyrosine kinase inhibitor, as used in this context, is an ALK inhibitor, preferably crizotinib or ceritinib.

As used herein, an antibody used in antibody therapy is preferably directed against a growth factor or a growth factor recetor, which is preferably functionally linked with a tyrosine kinase. Preferably, an antibody, in this context, is directed against vascular endothelial growth factor (VEGF) or against epidermal growth factor receptor (EGFR). In a preferred embodiment, bevacizumab is used in an antibody therapy. In another embodiment, cetuximab is used in an antibody therapy.

In an embodiment disclosed herein, the subject receiving the composition or vaccine is a patient before or after surgery (e.g. lobectomy), wherein the patient preferably has NSCLC in stage I or II.

In a further disclosed embodiment, the subject receiving the composition or vaccine is a patient receiving radiotherapy, or a patient, who has achieved partial response (PR) or stable disease (SD) after radiotherapy wherein the patient preferably has NSCLC in stage I or II.

According to an embodiment disclosed herein, the subject receiving the composition or vaccine is a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel), or a patient, who has achieved partial response (PR) or stable disease (SD) after chemotherapy, wherein the patient preferably has NSCLC in stage III or IV.

In another embodiment disclosed herein, the subject receiving the composition or vaccine is a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel) in combination with radiotherapy (chemoradiation), or a patient, who has achieved partial response (PR) or stable disease (SD) after chemoradiation, wherein the patient preferably has NSCLC in stage III (preferably locally advanced) or IV.

According to a further disclosed embodiment, the subject receiving the inventive composition or vaccine is a patient receiving only one chemotherapy, preferably gemcitabine, taxanes, premetrexed, paclitaxel, vinorelbine, or etoposide, preferably as second-line or third line treatment, or a patient, who has achieved partial response (PR) or stable disease (SD) after such second-line or third-line treatment.

In an embodiment disclosed herein, the subject receiving the composition or vaccine is a patient with stage III or stage IV non-small cell lung cancer (NSCLC) after first-line and optional second-line chemotherapy (e.g. platinum-based chemotherapy or platinum-based combination chemotherapy (combination of platinum-based chemotherapy with at least one further chemotherapeutic agent)) or first-line and optional second-line chemoradiation, wherein the patient has preferably achieved partial response (PR) or stable disease (SD) after first-line and optional second-line chemotherapy.

According to an embodiment disclosed herein, the subject receiving the composition or vaccine is a patient with stage III or IV non-small cell lung cancer (NSCLC) and non-squamous histology, with or without activating epidermal growth factor receptor (EGFR) mutations.

According to another embodiment disclosed herein, the subject receiving the composition or vaccine is a patient with stage III or IV non-small cell lung cancer (NSCLC) and squamous histology, with or without activating epidermal growth factor receptor (EGFR) mutations.

In an embodiment disclosed herein, the subject receiving the composition or vaccine is a patient with stage III or IV non-small cell lung cancer (NSCLC) and non-squamous histology, preferably without activating epidermal growth factor receptor (EGFR) mutations, who has preferably achieved partial response (PR) or stable disease (SD) after first-line chemotherapy using platinum or platinum-based combination chemotherapy (e.g. platinum and pemetrexed).

In another embodiment disclosed herein, the subject receiving the composition or vaccine is a patient with stage III or IV NSCLC and squamous cell histology, who has preferably achieved partial response (PR) or stable disease (SD) after first-line chemotherapy using platinum or platinum-based combination chemotherapy (e.g. platinum and pemetrexed).

Even more preferably, the subject receiving the composition or vaccine is a patient suffering from metastatic lung cancer, preferably metastatic non-small cell lung cancer.

In a further disclosed embodiment, the subject receiving the composition or vaccine is a patient with stage III locoregionally advanced NSCLC, preferably receiving treatment with concomitant chemoradiation (e.g. as defined above), or has achieved a response or stable disease (non-progression) after chemoradiation (as defined herein).

In a further disclosed embodiment, the subject receiving the composition or vaccine is a patient with stage III or IV NSCLC, irrespective of histological or molecular subtype and is preferably receiving concomitant treatment with a PD-1 pathway inhibitor or has achieved a response or stable disease (non-progression) after treatment with a PD-1 pathway inhibitor.

Furthermore, according to a specific embodiment disclosed herein, the subject receiving the composition or vaccine is a patient receiving an antibody or a combination of chemotherapy and an antibody, preferably bevacizumab or a combination of bevacizumab with chemotherapy, preferably a platinum-based chemotherapy, or a patient, who has achieved a response or stable disease after this treatment.

According to another embodiment disclosed herein, the subject receiving the composition or vaccine is a patient receiving a tyrosine kinase inhibitor, preferably an EGFR tyrosine kinase inhibitor (e.g. erlotinib, gefitinib or afatinib), and preferably as second-line treatment after first-line chemotherapy, or a patient, who has achieved a response or stable disease after EGFR tyrosine kinase inhibitor therapy.

According to another embodiment disclosed herein, the subject receiving the composition or vaccine is a patient harboring an activating EGFR mutation and receiving a tyrosine kinase inhibitor, preferably an EGFR tyrosine kinase inhibitor (e.g. erlotinib, gefitinib or afatinib), or a patient, who has achieved a response or stable disease after EGFR tyrosine kinase inhibitor therapy.

In another embodiment disclosed herein, the subject receiving the composition or vaccine is a patient with stage III or IV NSCLC and preferably with a non-squamous histology, preferably harboring an activating EGFR mutation and is preferably receiving concomitant treatment with an EGFR tyrosine kinase inhibitor, or a patient, who has achieved a response or stable disease after receiving treatment with an EGFR tyrosine kinase inhibitor (e.g. erlotinib, gefitinib or afatinib or other EGFR tyrosine kinase inhibitors).

In another embodiment disclosed herein, the subject receiving the composition or vaccine is a patient receiving the tyrosine kinase inhibitor crizotinib or ceritinib or other ALK inhibitors.

Similarly, the present disclosure also relates to the vaccine per se or the at least six mRNAs encoding the antigens as defined herein for use in eliciting an adaptive immune response in a mammal, preferably for the treatment of lung cancer, more preferably for the treatment of a non-small cell lung cancer (NSCLC) related condition as defined herein.

Prevention or treatment of lung cancer in a patient in need thereof, preferably of a non-small cell lung cancer, and diseases or disorders related thereto, may be carried out by administering the composition and/or the vaccine at once or in a time staggered manner, e.g. as a kit of parts, each part containing at least one preferably different antigen. Preferably, each of the antigens is administered separately, i.e. each antigen is administered to a different part or region of the body of the subject to be treated, preferably simultaneously or within the same short time-frame, respectively. In a preferred embodiment, the individual mRNAs are administered distributed over the subject's four limbs (i.e. left/right arm and leg). Preferably, the administration (of all at least six mRNAs) occurs within an hour, more preferably within 30 minutes, even more preferably within 15, 10, 5, 4, 3, or 2 minutes or even within 1 minute.

For administration, preferably any of the administration routes may be used as defined above. In particular, an administration route is used, which is suitable for treating lung cancer, preferably non-small cell lung cancer, and diseases or disorders related thereto, by inducing or enhancing an adaptive immune response on the basis of the antigens encoded by the at least six mRNAs of the composition disclosed herein. Administering of the composition and/or the vaccine may then occur prior, concurrent and/or subsequent to administering another composition and/or vaccine as defined herein which may - in addition - contain another combination of mRNAs encoding different antigens, wherein each antigen encoded by the at least one mRNA of the inventive composition may preferably be suitable for the therapy of lung cancer, preferably of non-small cell lung cancer, and diseases or disorders related thereto. In this context, a therapy as defined herein may also comprise the modulation of a disease associated to lung cancer, preferably of non-small cell lung cancer, and of diseases or disorders related thereto.

According to one further aspect, the present disclosure furthermore comprises the composition or the at least six mRNAs encoding the antigens as defined herein (for the preparation of a vaccine) for use in modulating, preferably to induce or enhance, an immune response in a mammal as defined above, more preferably to treat and/or to support the treatment of lung cancer, preferably of non-small cell lung cancer, or of diseases or disorders related thereto. In this context, support of the treatment of lung cancer, may be any combination of a conventional lung cancer therapy method of such as surgery, radiation therapy, chemotherapy (e.g. first-line or second-line chemotherapy), chemoradiation, treatment with tyrosine kinase inhibitors, treatment with PD-1 pathway inhibitors , antibody therapy or some combination of these, and a therapy using the composition as defined herein. Support of the treatment of lung cancer may be also envisaged in any of the other embodiments defined herein. Accordingly, the composition or vaccine for any use in co-therapy with any of the above therapeutic approaches, in particular in combination with surgery, radiation therapy, chemotherapy, chemoradiation, and/or treatment with kinase inhibitors or antibodies is also disclosed herein.

In an embodiment disclosed herein, the composition or vaccine described herein is used in a treatment of lung cancer, which further comprises chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or PD1 pathway inhibitors, or a patient, who has achieved partial response or stable disease after having received one or more of the treatments specified above.

In this context, a PD-1 pathway inhibitor is preferably defined herein as a compound capable of impairing the PD-1 pathway signaling, preferably signaling mediated by the PD-1 receptor. Therefore, the PD-1 pathway inhibitor may be any inhibitor directed against any member of the PD-1 pathway capable of antagonizing PD-1 pathway signaling. In this context, the inhibitor may be an antagonistic antibody, targeting any member of the PD-1 pathway, preferably directed against PD-1 receptor, PD-L1 or PD-L2. This antagonistic antibody may also be encoded by a nucleic acid. Also, the PD-1 pathway inhibitor may be a fragment of the PD-1 receptor or the PD1-receptor blocking the activity of PD1 ligands. B7-1 or fragments thereof may act as PD1-inhibiting ligands as well. Furthermore, the PD-1 pathway inhibitor may be siRNA (small interfering RNA) or antisense RNA directed against a member of the PD-1 pathway, preferably PD-1, PD-L1 or PD-L2. Additionally, a PD-1 pathway inhibitor may be a protein comprising (or a nucleic acid coding for) an amino acid sequence capable of binding to PD-1 but preventing PD-1 signaling, e.g. by inhibiting PD-1 and B7-H1 or B7-DL interaction. Additionally, a PD-1 pathway inhibitor may be a small molecule inhibitor capable of inhibiting PD-1 pathway signaling, e.g. a PD-1 binding peptide or a small organic molecule.

As used herein, an antibody used in antibody therapy is preferably directed against a growth factor or a growth factor recetor, which is preferably functionally linked with a tyrosine kinase. Preferably, an antibody, in this context, is directed against vascular endothelial growth factor (VEGF) or against epidermal growth factor receptor (EGFR). In a preferred embodiment, bevacizumab is used in an antibody therapy. In another embodiment, cetuximab is used in an antibody therapy.

According to an embodiment disclosed herein, the composition or vaccine is provided for use in a treatment of lung cancer, which further comprises radiation therapy, wherein at least one tumor lesion is eligible for radiation. According to an embodiment disclosed herein, a tumor lesion eligible for radiation is selected from the group consisting of bone metastases, lymph nodes in the paraclavicular, axillary or cervical regions, skin or subcutaneous metastases and thoracic lesions (centrally located lung tumor, lymph nodes in the lung hilus or mediastinum). Preferably, radiation is administered in 4 daily fractions of 5 GY each to be administered within one week, preferably from Day 9-12.

In a further embodiment disclosed herein, the composition or vaccine is provided for use in a treatment of lung cancer, which further comprises administration of a kinase inhibitor. Therein, a kinase inhibitor is preferably a tyrosine kinase inhibitor, even more preferably a growth factor tyrosine kinase inhibitor, most preferably an oral EGFR tyrosine kinase inhibitor, such as, for instance, gefitinib, erlotinib or afatinib. Furthermore in this context, a tyrosine kinase inhibitor is preferably defined herein as a compound capable of impairing signalling of one or more tyrosine kinases, preferably of one or more growth factor tyrosine kinases. In another embodiment, a tyrosine kinase inhibitor, as used in this context, is an ALK inhibitor, preferably crizotinib or ceritinib.

In another embodiment disclosed herein, the composition or vaccine described herein is provided for use in a treatment of lung cancer, which further comprises administration of a chemotherapeutic agent, such as a platinum-based compound (e.g carboplatin, cisplatin), pemetrexed, gemcitabine, taxanes, vinorelbine, etoposide docetaxel or paclitaxel. Even more preferably, the composition or vaccine is provided for use in a subject receiving chemotherapy, preferably platinum-based combination chemotherapy preferably as defined above, which is preferably further combined with radiation therapy (chemoradiation).

Preferably, the composition or vaccine is provided for use in a combination treatment of lung cancer, wherein a patient has a stage 0, I (IA and/or IB), II (IIA and/or IIB), III (IIIA and/or IIIB) or stage IV non-small cell lung cancer.

In an embodiment disclosed herein, the composition or vaccine is provided for use in a treatment of lung cancer in a patient before or after surgery (e.g. lobectomy), wherein the patient preferably has NSCLC in stage I or II.

In a further disclosed embodiment, the composition or vaccine is provided for use in a treatment of lung cancer in a patient receiving radiotherapy, or in a patient, who has achieved partial response (PR) or stable disease (SD) after radiotherapy, wherein the patient preferably has NSCLC in stage I or II.

According to an embodiment disclosed herein, the composition or vaccine is provided for use in a treatment of lung cancer in a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel), or in a patient, who has achieved partial response (PR) or stable disease (SD) after chemotherapy, wherein the patient preferably has NSCLC in stage III or IV.

In another embodiment disclosed herein, the composition or vaccine is provided for use in a treatment of lung cancer in a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel) in combination with radiotherapy (chemoradiation), or in a patient, who has achieved partial response (PR) or stable disease (SD) after chemoradiation, wherein the patient preferably has NSCLC in stage III (preferably locally advanced) or IV.

According to a further disclosed embodiment, the composition or vaccine is provided for use in a treatment of lung cancer in a patient receiving only one chemotherapy, preferably gemcitabine, taxanes, premetrexed, paclitaxel, vinorelbine, or etoposide, preferably as second-line or third line treatment, or in a patient, who has achieved partial response (PR) or stable disease (SD) after such second-line or third-line treatment.

In an embodiment disclosed herein, the composition or vaccine is provided for use in a treatment of lung cancer in a patient with stage III or stage IV non-small cell lung cancer (NSCLC) after first-line and optional second-line chemotherapy (e.g. platinum-based chemotherapy or platinum-based combination chemotherapy (combination of platinum-based chemotherapy with at least one further chemotherapeutic agent)) or first-line and optional second-line chemoradiation, wherein the patient has preferably achieved partial response (PR) or stable disease (SD) after first-line and optional second-line chemotherapy.

According to an embodiment disclosed herein, the composition or vaccine is provided for use in a treatment of lung cancer in a patient with stage III or IV non-small cell lung cancer (NSCLC) and non-squamous histology, with or without activating epidermal growth factor receptor (EGFR) mutations.

According to another embodiment disclosed herein, the composition or vaccine is provided for use in a treatment of lung cancer in a patient with stage III or IV non-small cell lung cancer (NSCLC) and squamous histology, with or without activating epidermal growth factor receptor (EGFR) mutations.

In an embodiment disclosed herein, the composition or vaccine is provided for use in a treatment of lung cancer in a patient with stage III or IV non-small cell lung cancer (NSCLC) and non-squamous histology, preferably without activating epidermal growth factor receptor (EGFR) mutations, wherein the patient has preferably achieved partial response (PR) or stable disease (SD) after first-line chemotherapy using platinum or platinum-based combination chemotherapy (e.g. platinum and pemetrexed).

In another embodiment disclosed herein, the composition or vaccine is provided for use in a treatment of lung cancer in a patient with stage III or IV NSCLC and squamous cell histology, wherein the patient has preferably achieved partial response (PR) or stable disease (SD) after first-line chemotherapy using platinum or platinum-based combination chemotherapy (e.g. platinum and pemetrexed).

The present disclosure also comprises the composition or vaccine as described herein, which is provided for use in a treatment of lung cancer in a patient suffering from metastatic lung cancer, preferably metastatic non-small cell lung cancer.

In a further disclosed embodiment, the composition or vaccine is provided for use in a treatment of lung cancer in a patient with stage III locoregionally advanced NSCLC, wherein the patient preferably receives concomitant treatment with chemoradiation (e.g. as defined above), or wherein the patient has achieved a response or stable disease (non-progression) after chemoradiation (as defined herein).

In a further disclosed embodiment, the composition or vaccine is provided for use in a treatment of lung cancer in a patient with stage III or IV NSCLC, irrespective of histological or molecular subtype, wherein the patient is preferably receiving concomitant treatment with a PD-1 pathway inhibitor or has achieved a response or stable disease (non-progression) after treatment with a PD-1 pathway inhibitor.

Furthermore, according to an embodiment disclosed herein, the composition or vaccine is provided for use in a treatment of lung cancer in a patient receiving an antibody or a combination of chemotherapy and an antibody, preferably in a patient receiving bevacizumab or a combination of bevacizumab with chemotherapy, preferably a platinum-based chemotherapy, or in a patient, who has achieved a response or stable disease after this treatment.

According to another embodiment disclosed herein, the composition or vaccine is provided for use in a treatment of lung cancer in a patient receiving a tyrosine kinase inhibitor, preferably an EGFR tyrosine kinase inhibitor (e.g. erlotinib, gefitinib or afatinib), and preferably as second-line treatment after first-line chemotherapy, or in a patient, who has achieved a response or stable disease after EGFR tyrosine kinase inhibitor therapy.

According to another embodiment disclosed herein, the composition or vaccine is provided for use in a treatment of lung cancer in a patient harboring an activating EGFR mutation and receiving a tyrosine kinase inhibitor, preferably an EGFR tyrosine kinase inhibitor (e.g. erlotinib, gefitinib or afatinib), or in a patient, who has achieved a response or stable disease after EGFR tyrosine kinase inhibitor therapy.

In another embodiment disclosed herein, the composition or vaccine is provided for use in a treatment of lung cancer in a patient with stage III or IV NSCLC, preferably with a non-squamous histology, preferably harboring an activating EGFR mutation and wherein the patient is preferably receiving concomitant treatment with an EGFR tyrosine kinase inhibitor, or in a patient, who has achieved a response or stable disease after receiving treatment with an EGFR tyrosine kinase inhibitor (e.g. erlotinib, gefitinib or afatinib or other EGFR tyrosine kinase inhibitors).

In another embodiment disclosed herein, the composition or vaccine is provided for use in a treatment of lung cancer in a patient receiving the tyrosine kinase inhibitor crizotinib or ceritinib or other ALK inhibitors.

The immunization protocol for the immunization of a subject against the combination of at least six antigens as defined herein typically comprises a series of single doses or dosages of the composition or the vaccine. A single dosage, as used herin, refers to the initial/first dose, a second dose or any futher doses, respectively, which are preferably administered in order to "boost" the immune reaction. In this context, each single dosage comprises the administration of all of the at least six antigens as described herein, wherein the interval between the administration of two single dosages can vary from at least one day, preferably 2, 3, 4, 5, 6 or 7 days, to at least one week, preferably 2, 3, 4, 5, 6, 7 or 8 weeks. The intervals between single dosages may be constant or vary over the course of the immunization protocol, e.g. the intervals may be shorter in the beginning and longer towards the end of the protocol. Depending on the total number of single dosages and the interval between single dosages, the immunization protocol may extend over a period of time, which preferably lasts at least one week, more preferably several weeks (e.g. 2, 3, 4, 5, 6, 7, 8, 9, 10, 11 or 12 weeks), even more preferably several months (e.g. 3, 4, 5, 6, 7, 8, 9, 10, 11, 12, 18 or 24 months). Each single dosage encompasses the administration of all of the at least six antigens as defined herein and may therefore involve at least one, preferably 1, 2, 3, 4, 5, 6, 7, 8, 9, 10, 11 or 12 injections. In the case, where the composition disclosed herein is administered, a single dosage typically comprises one injection. In the case, where the vaccine comprises separate mRNA formulations encoding the respective antigens as described herein, the minimum number of injections carried out during the administration of a single dosage corresponds to the number of separate components of the vaccine. In certain embodiments, the administration of a single dosage may encompass more than one injection for each component of the vaccine (e.g. a specific mRNA formulation comprising a mRNA encoding, for instance, one of the six antigens as defined herein). For example, parts of the total volume of an individual component of the vaccine may be injected into different body parts, thus involving more than one injection. In a more specific example, a single dosage of a vaccine comprising six separate mRNA formulations, each of which is administered in two different body parts, comprises twelve injections. Typically, a single dosage comprises all injections required to administer all components of the vaccine, wherein a single component may involve more than one injection as outlined above. In the case, where the administration of a single dosage of the vaccine disclosed herein encompasses more than one injection, the injections are carried out essentially simultaneously or concurrently, i.e. typically in a time-staggered fashion within the time-frame that is required for the practitioner to carry out the single injection steps, one after the other. The administration of a single dosage therefore preferably extends over a time period of several minutes, e.g. 2, 3, 4, 5, 10, 15, 30 or 60 minutes.

Administration of the composition or the at least six mRNAs encoding the antigens as defined herein or the vaccine may be carried out in a time staggered treatment. A time staggered treatment may be e.g. administration of the composition or the at least six mRNAs encoding the antigens as defined herein or the vaccine prior, concurrent and/or subsequent to a conventional therapy of lung cancer, preferably of non-small cell lung cancer, and diseases or disorders related thereto, e.g. by administration of the medicament or the composition or vaccine prior, concurrent and/or subsequent to a therapy or an administration of a therapeutic suitable for the treatment of lung cancer, preferably of non-small cell lung cancer, and diseases or disorders related thereto. Such time staggered treatment may be carried out using e.g. a kit, preferably a kit of parts as defined below.

Time staggered treatment may additionally or alternatively also comprise an administration of the composition or vaccine disclosed herein, preferably of the at least one mRNA encoding the antigens as defined above, in a form, wherein the at least one mRNA encoding the antigens as defined above, preferably forming part of the composition or vaccine disclosed herein, is administered parallel, prior or subsequent to another at least one mRNA encoding the antigens as defined above, preferably forming part of the same composition or vaccine. Preferably, the administration (of all at least one mRNAs) occurs within an hour, more preferably within 30 minutes, even more preferably within 15, 10, 5, 4, 3, or 2 minutes or even within 1 minute. Such time staggered treatment may be carried out using e.g. a kit, preferably a kit of parts as defined below.

In an embodiment disclosed herein, the composition or vaccine is administered repeatedly, wherein each administration preferably comprises individual administration of the at least one mRNA as described herein. At each time point of administration, the at least one mRNA may be administered more than once (e.g. 2 or 3 times). In a further embodiment disclosed herein, six mRNAs (each encoding one of the antigens as defined above) are administered at each time point, wherein each mRNA is administered twice by injection, thus resulting in twelve injections distributed over the four limbs.

According to a final embodiment, the present invention also provides kits, particularly kits of parts, for use in the treatment of non-small cell lung cancer, wherein the treatment further comprises administration of a chemotherapeutic agent, the kits or kits of parts comprising the active (immunostimulatory) composition, and/or the vaccine disclosed herein, optionally a liquid vehicle for solubilising and optionally technical instructions with information on the administration and dosage of the composition and/or the vaccine. The technical instructions may contain information about administration and dosage of the composition, and/or the vaccine. Such kits, preferably kits of parts, may be applied e.g. for any of the above mentioned applications or uses, preferably for the use of the composition for the preparation of a medicament, preferably a vaccine, for the treatment of lung cancer, preferably of non-small cell lung cancer, and diseases or disorders related thereto. The kits may also be applied for the use of the composition for the preparation of an vaccine for the treatment of lung cancer, preferably of non-small cell lung cancer, and diseases or disorders related thereto, wherein the composition and/or the vaccine due to the encoded at least six antigens may be capable to induce or enhance an immune response in a mammal as defined above. Such kits may further be applied for the use of the composition for the preparation of a medicament, preferably a vaccine, for modulating, preferably for eliciting, e.g. to induce or enhance, an immune response in a mammal as defined above, and preferably to support treatment of lung cancer, preferably of non-small cell lung cancer, and diseases or disorders related thereto. Kits of parts, as a special form of kits, may contain one or more identical or different active (immunostimulatory) compositions and/or one or more identical or different vaccines in different parts of the kit. Kits of parts may also contain an (e.g. one) active composition, an (e.g. one) vaccine and/or the at least six mRNAs encoding at least six antigens as defined above in different parts of the kit, e.g. each part of the kit containing at least one mRNA encoding a preferably different antigen. Additionally, a combination of both types of kits of parts is possible. Kits of parts may be used, e.g. when a time staggered treatment is envisaged, e.g. when using different formulations and/or increasing concentrations of the composition, the vaccine and/or the at least six mRNAs encoding at least six antigens as defined above during the same treatment in vivo. Kits of parts may also be used when a separated formulation or administration of at least one of the antigens of the composition (i.e. in parts) is envisaged or necessary (e.g. for technical reasons), but e.g. a combined presence of the different antigens in vivo is still to be achieved. Particularly kits of parts as a special form of kits are envisaged, wherein each part of the kit contains at least one preferably different antigen as defined above, all parts of the kit of parts forming the composition or the vaccine as defined herein. Such specific kits of parts may particularly be suitable, e.g. if different antigens are formulated separately as different parts of the kits, but are then administered at once together or in a time staggered manner to the mammal in need thereof. In the latter case administration of all of the different parts of such a kit typically occurs within a short time limit, such that all antigens are present in the mammal at about the same time subsequent to administration of the last part of the kit. In an embodiment disclosed herein, the kit contains at least two parts containing the six mRNAs as described herein. Preferably, all six mRNAs are provided in separate parts of the kit, wherein the mRNAs are preferably lyophilised. More preferably, the kit further contains as a part a vehicle for solubilising the at least six mRNAs, the vehicle preferably being Ringer-lactate solution. Any of the above kits may be provided for use in a treatment as defined above.

### Advantages of the present invention

The present invention provides a composition for use in the the treatment of non-small cell lung cancer as defined in the claims, wherein the composition comprises at least six mRNAs as defiend in the claims, encoding at least six antigens capable of eliciting an (adaptive) immune response in a mammal wherein the antigens are selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1 B), MAGE-C1 (Melanoma antigen family C1), MAGE-C2 (Melanoma antigen family C2), and MUC1 (Mucin 1). Such a composition allows efficient treatment of non-small cell lung cancer, wherein the treatment further comprises administration of a chemotherapeutic agent, or supplementary treatment when using conventional therapies. It furthermore avoids the problem of uncontrolled propagation of the introduced DNA sequences by the use of RNA as an approach for curative methods. mRNA as used in the composition disclosed herein has additional considerable advantages over DNA expression systems e.g. in immune response, immunization or vaccination. These advantages include, inter alia, that RNA introduced into a cell is not integrated into the genome. This avoids the risk of mutation of this gene, which otherwise may be completely or partially inactivated or give rise to misinformation. It further avoids other risks of using DNA as an agent to induce an immune response (e.g. as a vaccine) such as the induction of pathogenic anti-DNA antibodies in the patient into whom the foreign DNA has been introduced, so bringing about a (possibly fatal) immune response. In contrast, no anti-RNA antibodies have yet been detected.

### Figures

The following Figures are intended to illustrate the invention further. They are not intended to limit the subject matter of the invention thereto.

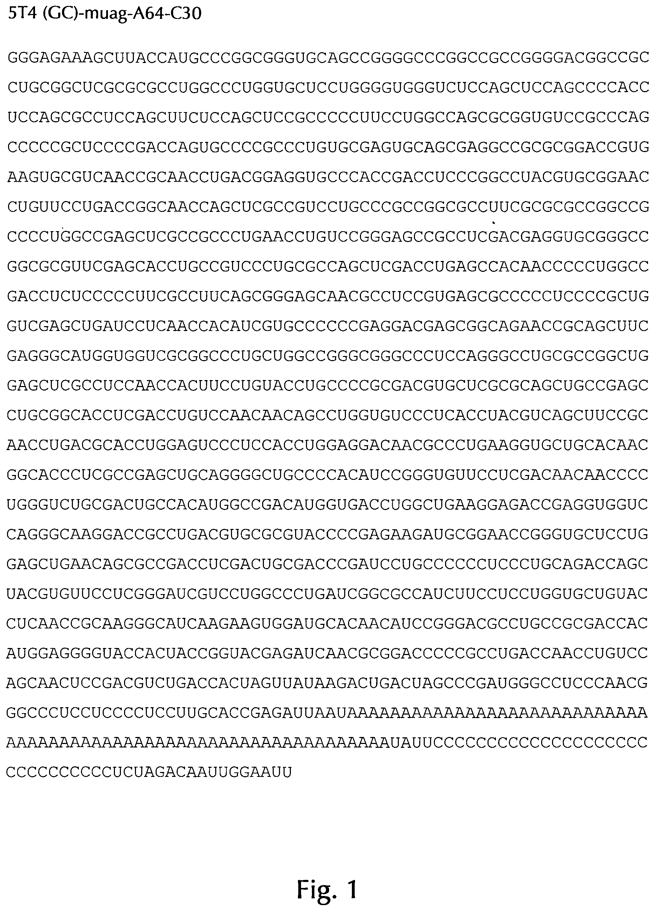

- Figure 1:: depicts the mRNA sequence 5T4 (GC)-muag-A64-C30 (SEQ ID NO: 1), encoding 5T4 (Trophoblast glycoprotein, TPBG). The mRNA contains following sequence elements: A 5'-CAP, a GC-optimized coding sequence for stabilization and a better codon usage encoding 5T4 according to SEQ ID No. 3, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail).
- Figure 2:: depicts the mRNA sequence 5T4 (GC)-muag-A64-C30-HistoneSL (SEQ ID NO: 19), encoding 5T4 (Trophoblast glycoprotein, TPBG). The mRNA contains following sequence elements: A 5'-CAP, a GC-optimized coding sequence for stabilization and a better codon usage encoding 5T4 according to SEQ ID No. 3, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail); and a histone stem-loop sequence according to SEQ ID No. 72.

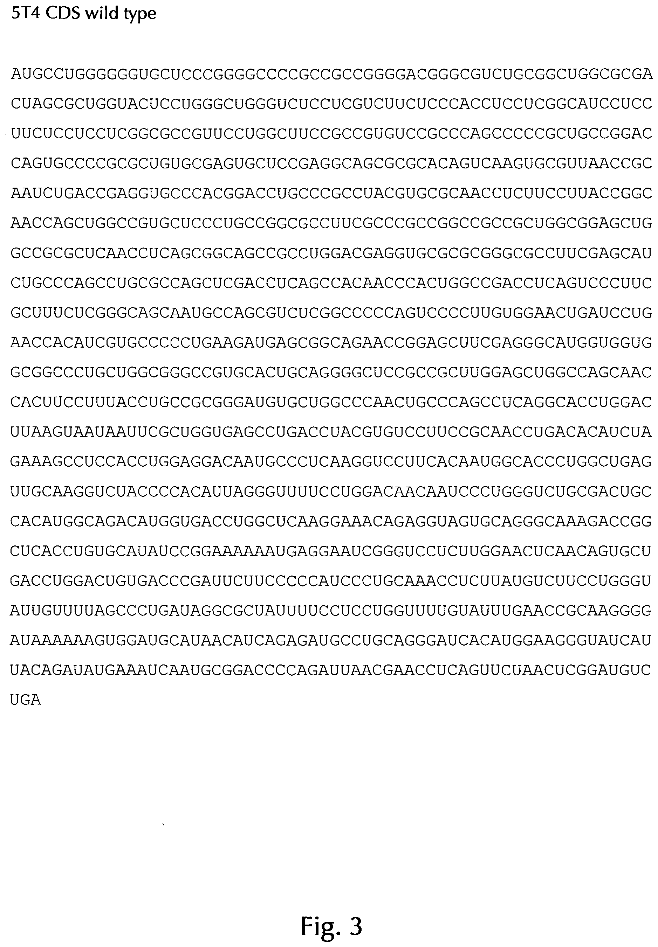

- Figure 3:: depicts the wildtype coding sequence, encoding 5T4 (Trophoblast glycoprotein, TPBG), according to SEQ ID NO: 2, i.e. the coding sequence (CDS) encoding 5T4 (Trophoblast glycoprotein, TPBG) without GC-optimized coding sequence.

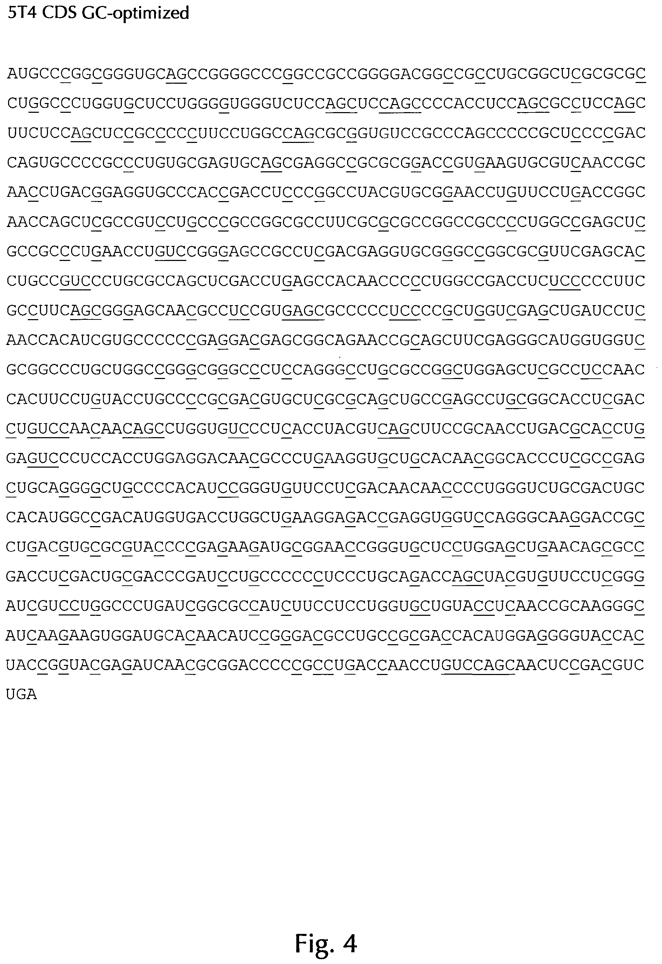

- Figure 4:: depicts the GC-optimized coding sequence encoding 5T4 (Trophoblast glycoprotein, TPBG) according to SEQ ID NO: 3.

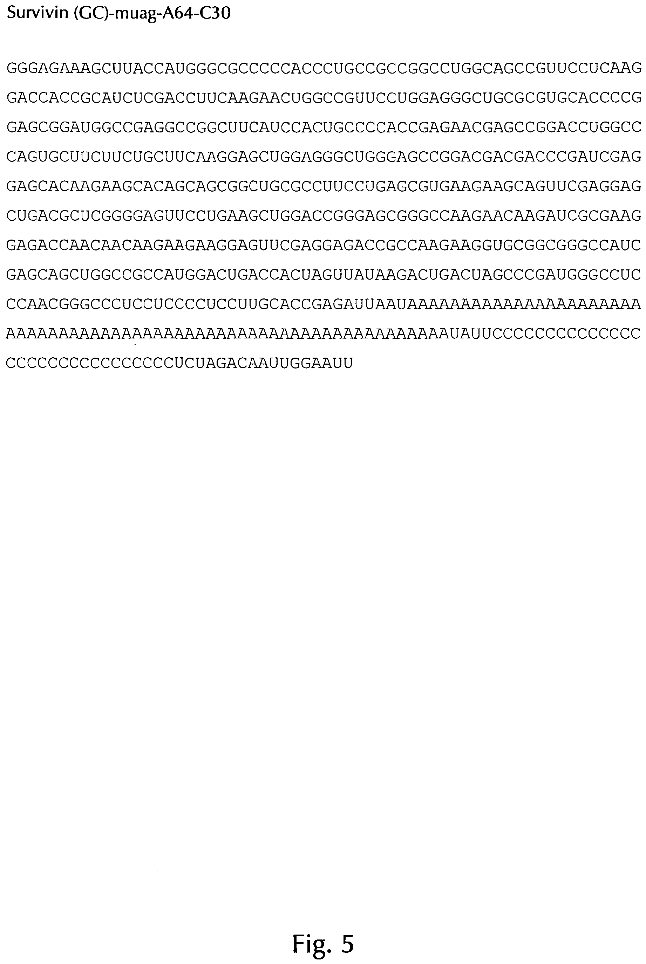

- Figure 5:: depicts the mRNA sequence Survivin (GC)-muag-A64-C30 (SEQ ID NO: 4), encoding Survivin (Baculoviral IAP repeat-containing protein 5; BIRC5). The mRNA contains following sequence elements: A 5'-CAP, a GC-optimized coding sequence for stabilization and a better codon usage encoding Survivin according to SEQ ID No. 6, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail).
- Figure 6:: depicts the mRNA sequence Survivin (GC)-muag-A64-C30-HistoneSL (SEQ ID NO: 20), encoding Survivin (Baculoviral IAP repeat-containing protein 5; BIRC5). The mRNA contains following sequence elements: A 5'-CAP, a GC-optimized coding sequence for stabilization and a better codon usage encoding Survivin according to SEQ ID No. 6, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail); and a histone stem-loop sequence according to SEQ ID No. 72.

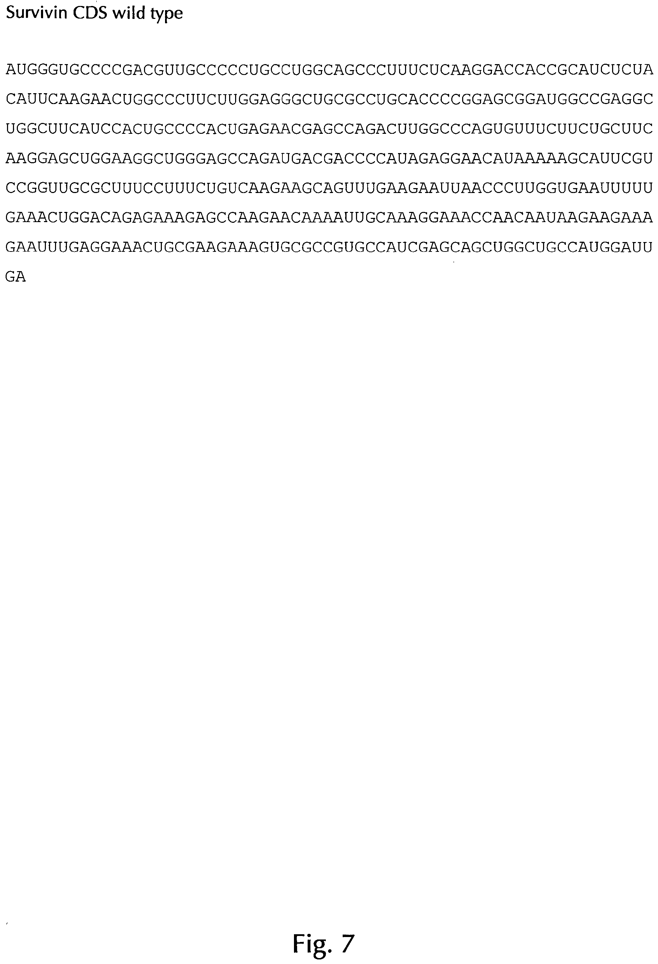

- Figure 7:: depicts the wildtype coding sequence, encoding Survivin, according to SEQ ID NO: 5, i.e. the coding sequence (CDS) encoding Survivin without GC-optimized coding sequence.

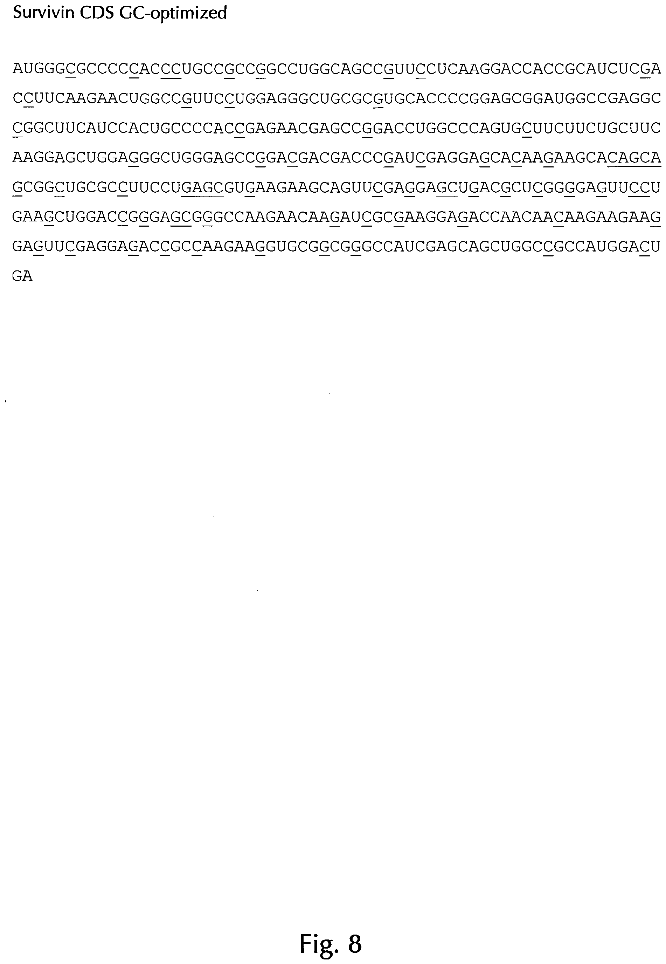

- Figure 8:: depicts the GC-optimized coding sequence encoding Survivin according to SEQ ID NO: 6.

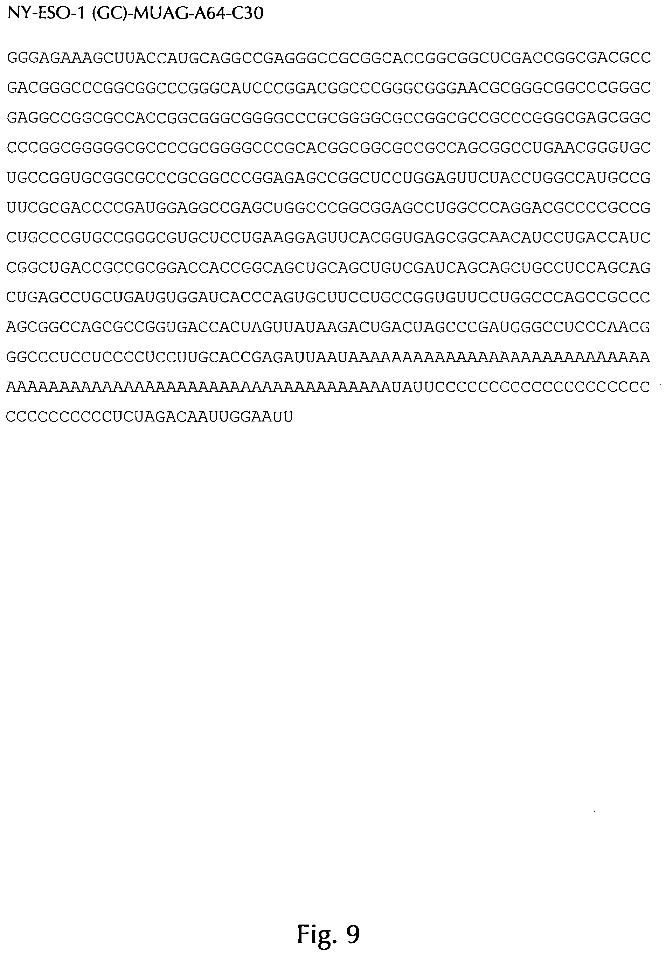

- Figure 9:: depicts the mRNA sequence NY-ESO-1 (GC)-muag-A64-C30 (SEQ ID NO: 7), encoding NY-ESO-1 (New York esophageal squamous cell carcinoma 1, CTAG1 B). The mRNA contains following sequence elements: A 5'-CAP, a GC-optimized coding sequence for stabilization and a better codon usage encoding NY-ESO-1 according to SEQ ID No. 9, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail).
- Figure 10:: depicts the mRNA sequence NY-ESO-1 (GC)-muag-A64-C30-HistoneSL (SEQ ID NO: 21), encoding NY-ESO-1 (New York esophageal squamous cell carcinoma 1, CTAG1B). The mRNA contains following sequence elements: A 5'-CAP, a GC-optimized coding sequence for stabilization and a better codon usage encoding NY-ESO-1 according to SEQ ID No. 9, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail); and a histone stem-loop sequence according to SEQ ID No. 72.

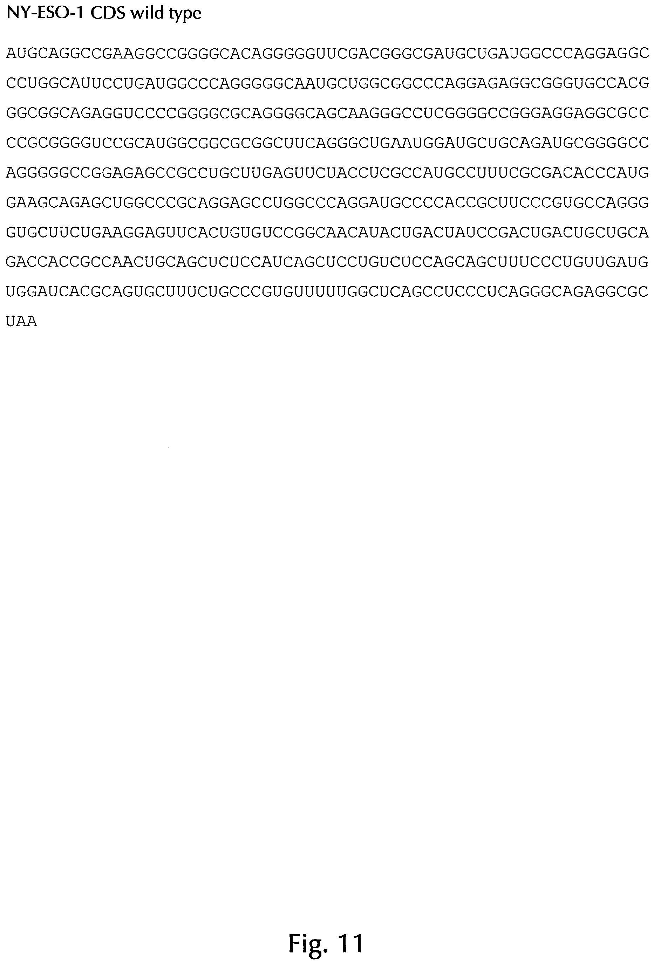

- Figure 11:: depicts the wildtype coding sequence, encoding NY-ESO-1 (New York esophageal squamous cell carcinoma 1, CTAG1B), according to SEQ ID NO: 8, i.e. the coding sequence (CDS) encoding NY-ESO-1 without GC-optimized coding sequence.

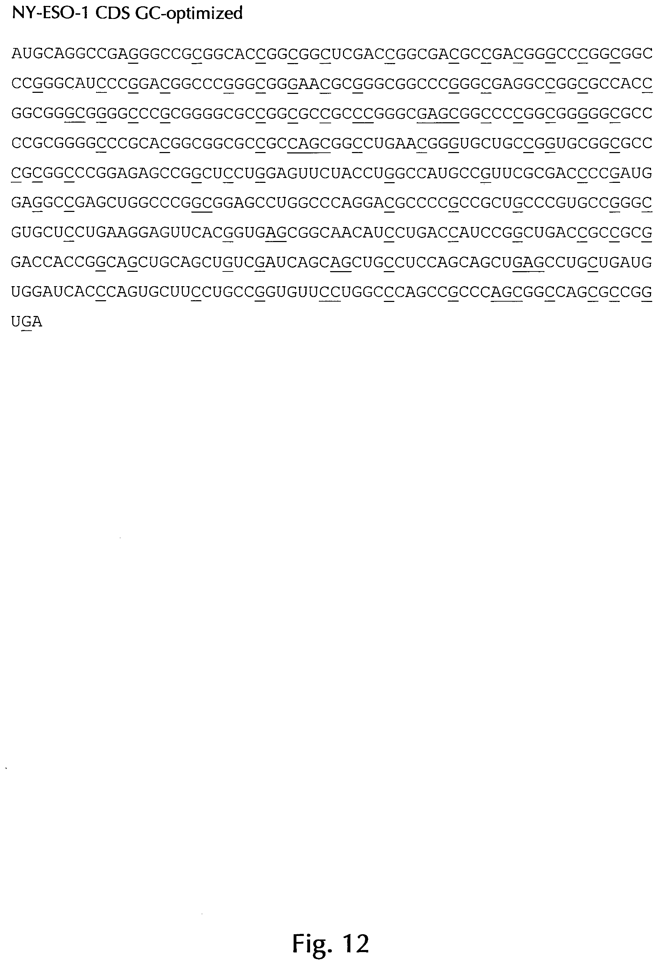

- Figure 12:: depicts the GC-optimized coding sequence encoding NY-ESO-1 (New York esophageal squamous cell carcinoma 1, CTAG1B) according to SEQ ID NO: 9.

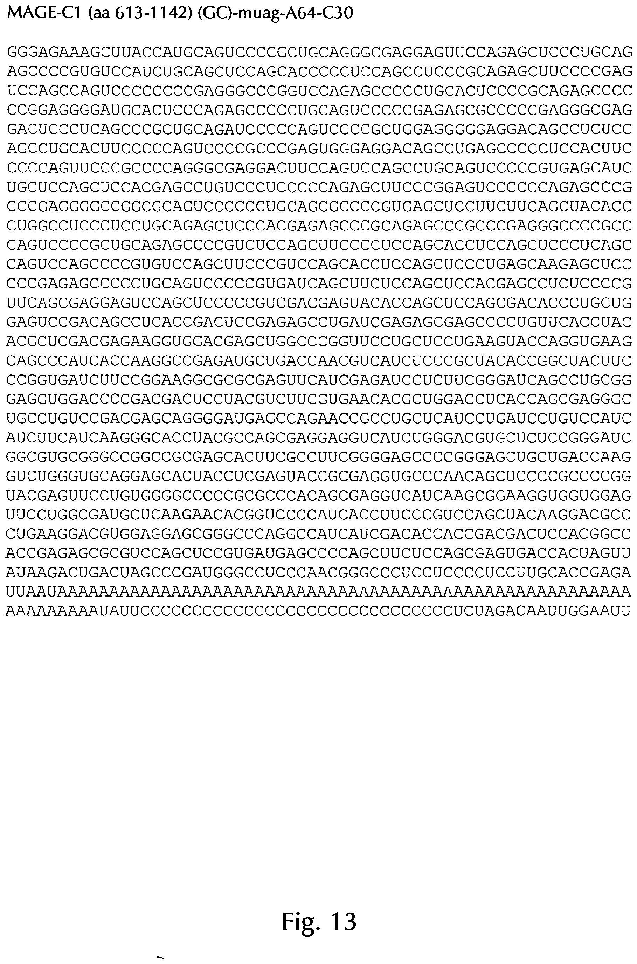

- Figure 13:: depicts the mRNA sequence MAGE-C1 (aa 613-1142) (GC)-muag-A64-C30 (SEQ ID NO: 10), encoding MAGE-C1 comprising the amino acid sequence aa 613-1142 of the wild type protein MAGE-C1. The mRNA contains following sequence elements: A 5'-CAP, a GC-optimized coding sequence for stabilization and a better codon usage encoding MAGE-C1 (aa 613-1142) according to SEQ ID No. 25, the stabilizing sequence "muag" in the 3'-UTR according to SEQ ID No.70, ∼ 64 Adenosin at the 3'-terminal end (poly-A-tail), ∼ 30 cytidines at the 3'- terminal end (poly-C-tail).
- Figure 14:: depicts the mRNA sequence MAGE-C1 (aa 613-1142) (GC)-muag-A64-C30-HistoneSL (SEQ ID NO: 22), encoding MAGE-C1 (aa 613-1142). The mRNA contains following sequence elements:
A 5'-CAP, a GC-optimized coding sequence for stabilization and a better codon usage encoding MAGE-C1 (aa 613-1142) according to SEQ ID No. 25, the stabilizing sequence "muag" in the 3'-UTR according to SEQ ID No.70, ∼ 64 Adenosin at the 3'-terminal end (poly-A-tail), ∼ 30 cytidines at the 3'- terminal end (poly-C-tail); and a histone stem-loop sequence according to SEQ ID No. 72.

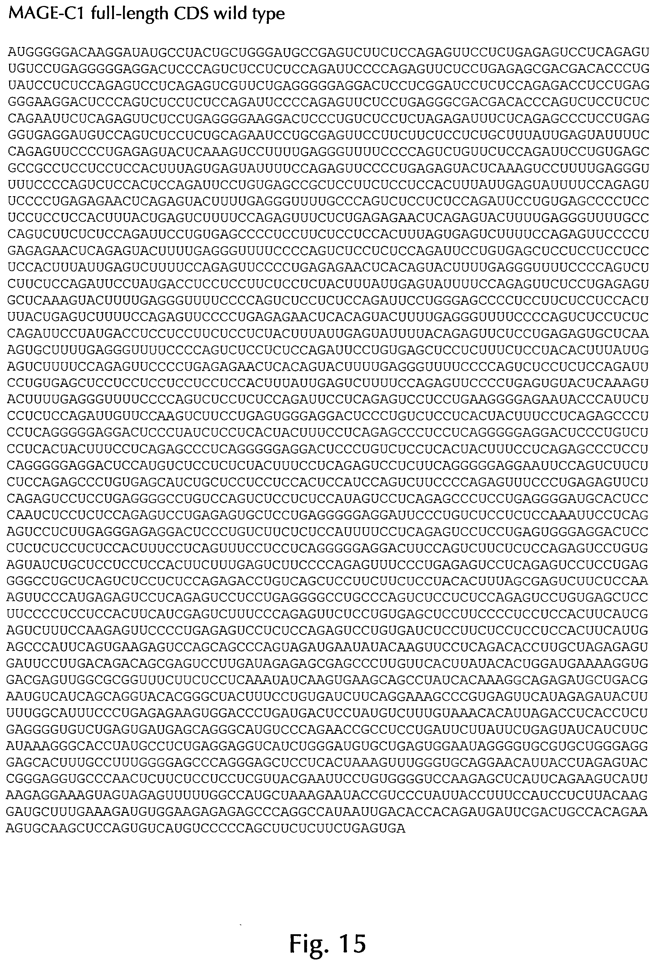

- Figure 15:: depicts the wildtype coding sequence, encoding the full-length protein of MAGE-C1 according to SEQ ID NO: 11, i.e. the coding sequence (CDS) encoding MAGE-C1 without GC-optimized coding sequence.

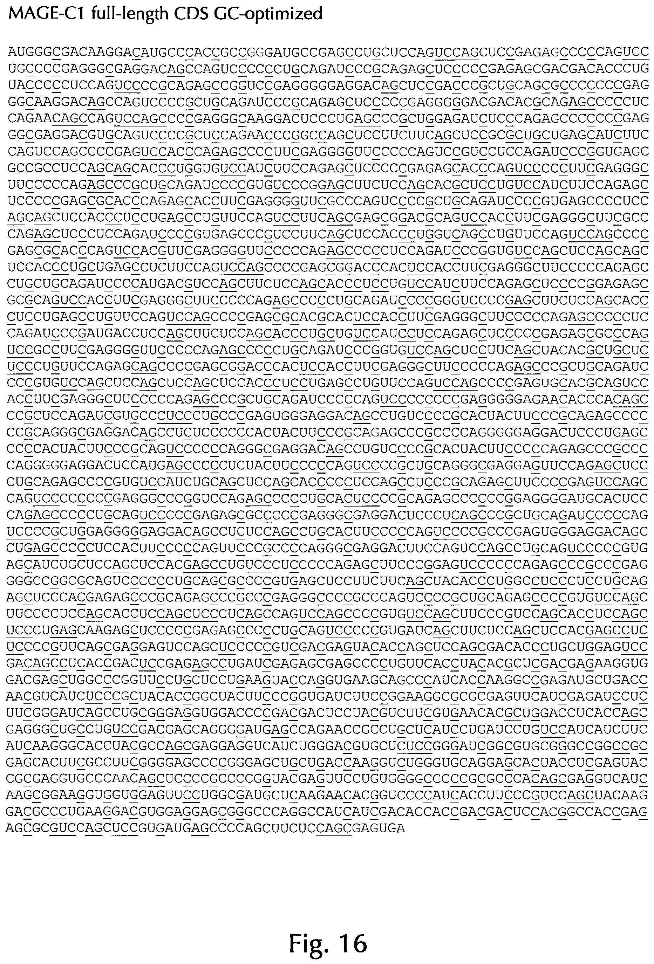

- Figure 16:: depicts the GC-optimized coding sequence encoding the full-length protein of MAGE-C1 according to SEQ ID NO: 12.

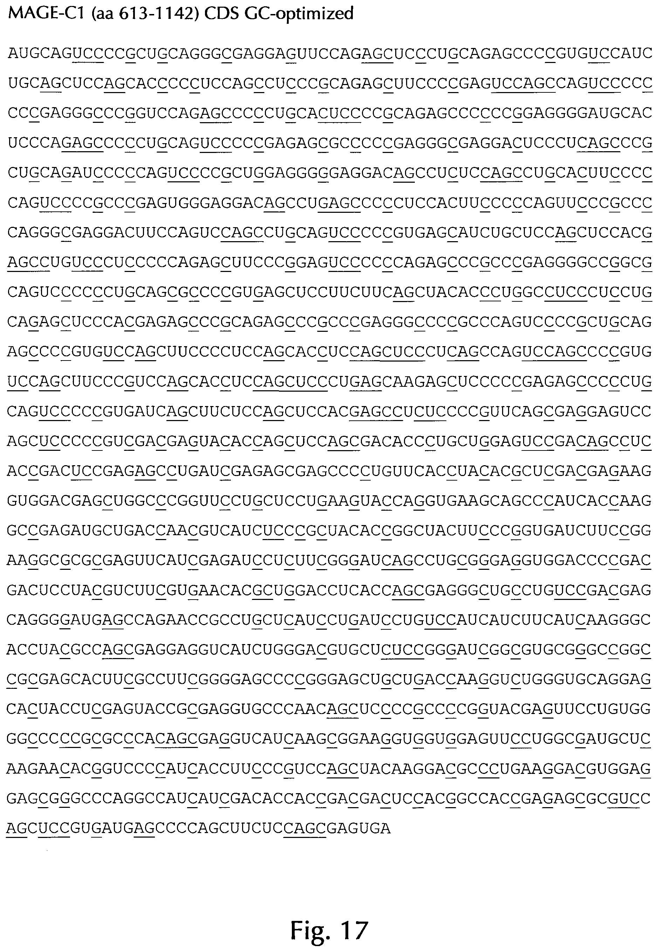

- Figure 17:: depicts the GC-optimized coding sequence encoding MAGE-C1 (aa 613-1142) according to SEQ ID NO: 25.

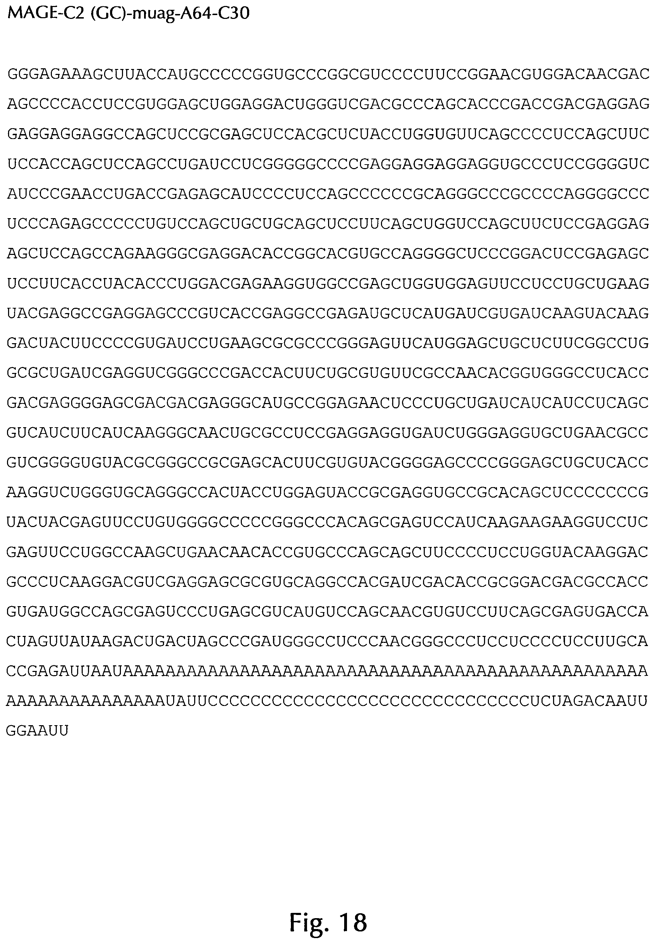

- Figure 18:: depicts the mRNA sequence MAGE-C2 (GC)-muag-A64-C30 (SEQ ID NO: 13), encoding MAGE-C2. The mRNA contains following sequence elements: A 5'-CAP, a GC-optimized coding sequence for stabilization and a better codon usage encoding MAGE-C2 according to SEQ ID No. 15, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail).
- Figure 19:: depicts the mRNA sequence MAGE-C2 (GC)-muag-A64-C30-HistoneSL (SEQ ID NO: 23), encoding MAGE-C2. The mRNA contains following sequence elements:
A 5'-CAP, a GC-optimized coding sequence for stabilization and a better codon usage encoding MAGE-C2 according to SEQ ID No. 9, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail); and a histone stem-loop sequence according to SEQ ID No. 72.

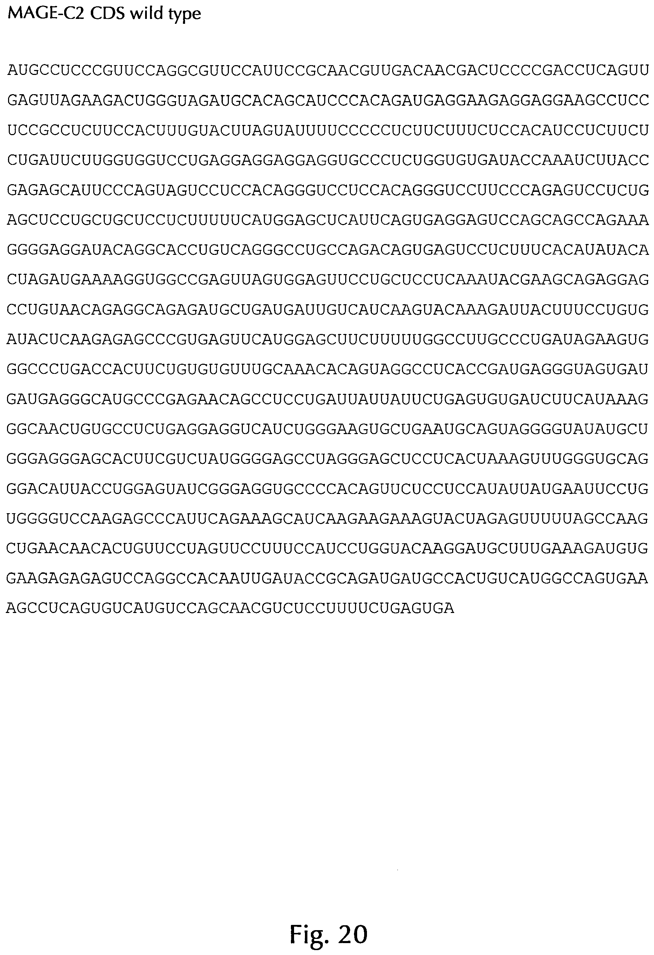

- Figure 20:: depicts the wildtype coding sequence, encoding MAGE-C2, according to SEQ ID NO: 14, i.e. the coding sequence (CDS) encoding MAGE-C2 without GC-optimized coding sequence.

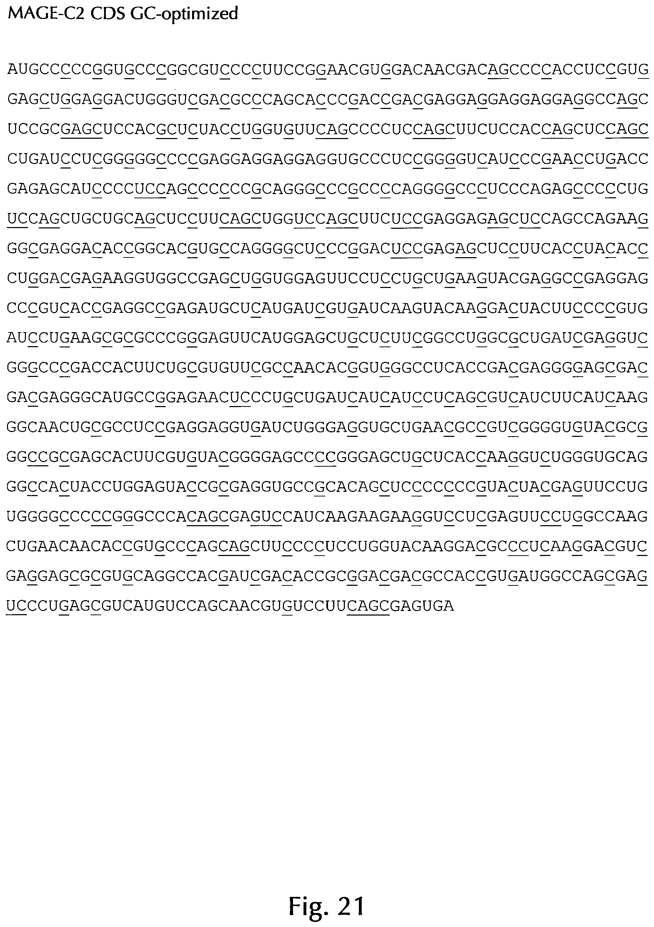

- Figure 21:: depicts the GC-optimized coding sequence encoding MAGE-C2 according to SEQ ID NO: 15.

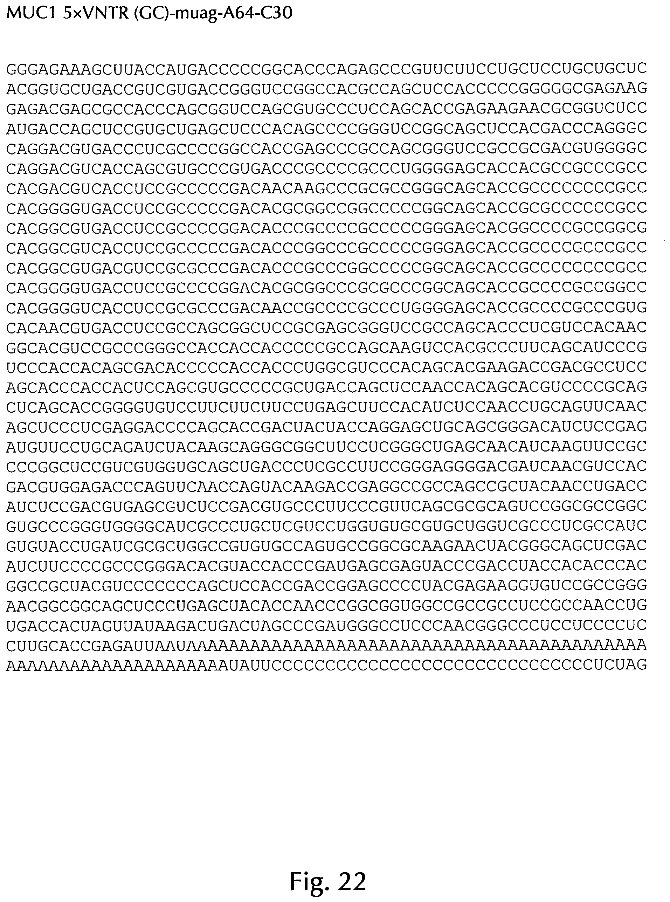

- Figure 22:: depicts the mRNA sequence MUC1 5xVNTR (GC)-muag-A64-C30 (SEQ ID NO: 16), encoding MUC1 (Mucin 1) comprising 5 tandem repeats. The mRNA contains following sequence elements:
A 5'-CAP, a GC-optimized coding sequence for stabilization and a better codon usage encoding MUC1 5xVNTR according to SEQ ID No. 18, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail).
- Figure 23:: depicts the mRNA sequence MUC1 5xVNTR (GC)-muag-A64-C30-HistoneSL (SEQ ID NO: 24), encoding MUC1 (Mucin 1) comprising 5 tandem repeats. The mRNA contains following sequence elements:
A 5'-CAP, a GC-optimized coding sequence for stabilization and a better codon usage encoding MUC1 5xVNTR according to SEQ ID No. 18, the stabilizing sequence "muag" in the 3'-UTR according to SEQ ID No.70, ∼ 64 Adenosin at the 3'-terminal end (poly-A-tail), ∼ 30 cytidines at the 3'-terminal end (poly-C-tail); and a histone stem-loop sequence according to SEQ ID No. 72.

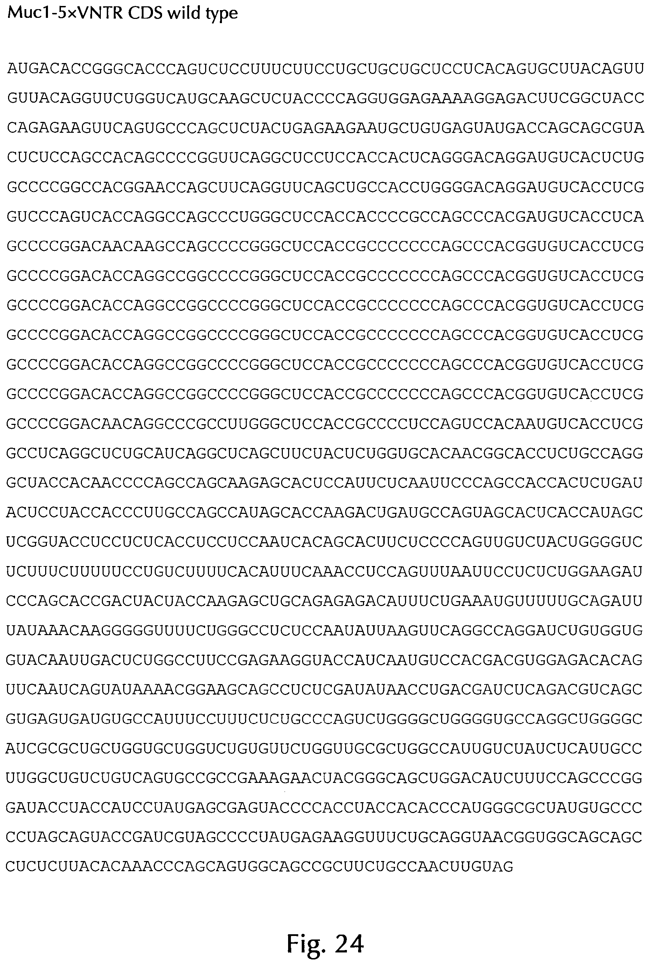

- Figure 24:: depicts the wildtype coding sequence, encoding MUC1 5xVNTR, according to SEQ ID NO: 17, i.e. the coding sequence (CDS) encoding MUC1 without GC-optimized coding sequence.

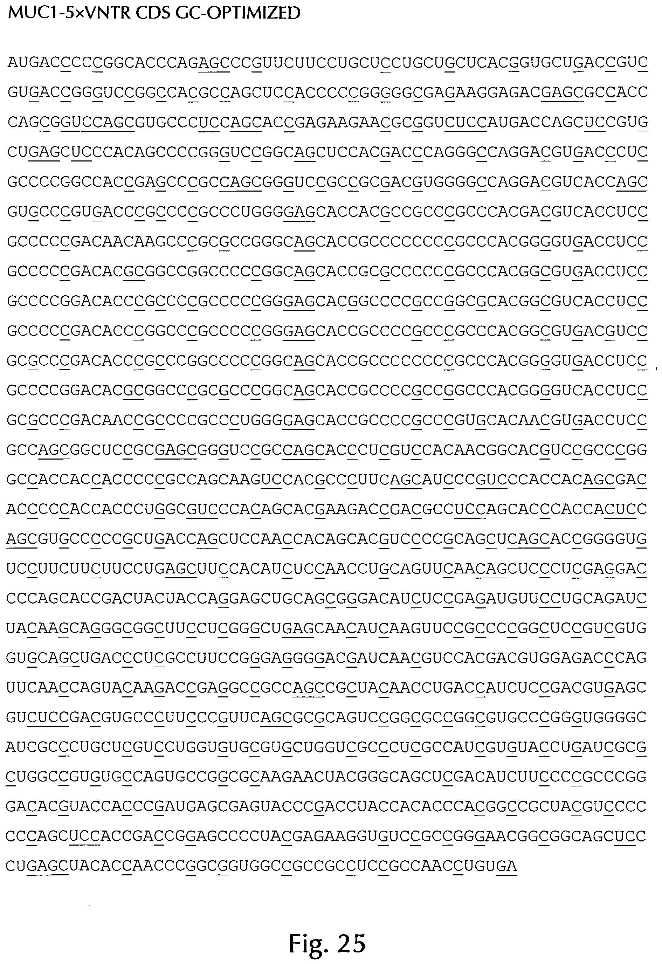

- Figure 25:: depicts the GC-optimized coding sequence encoding MUC1 5xVNTR according to SEQ ID NO: 18.

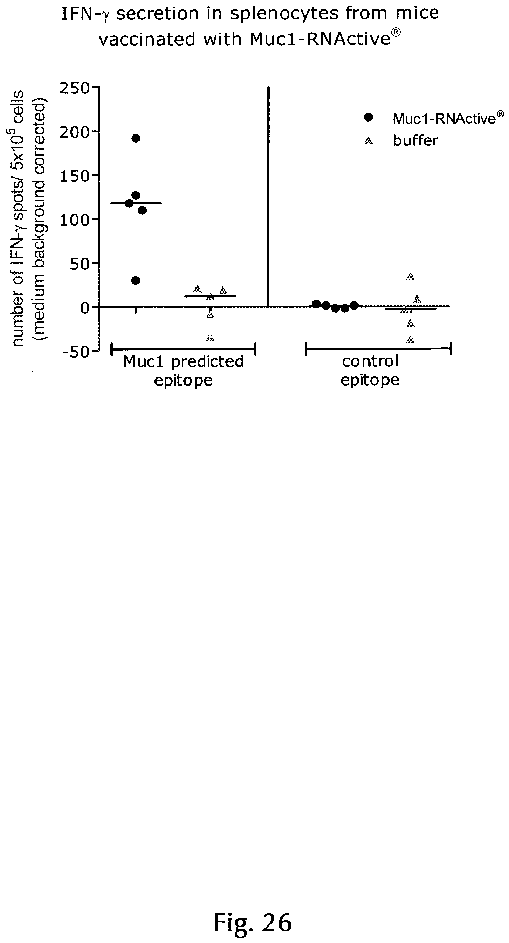

- Figure 26:: shows detection of a MUC1-specific cellular immune response by ELISPOT. C57BL/6 mice were vaccinated with 32µg MUC1-RNActive® (SEQ ID NO: 16) on days 1, 5, 8, 12 and 15. Ex vivo ELISpot analysis of the secretion of IFN-gamma in splenocytes from vaccinated and control mice was performed on day 6 after last vaccination. Cells were stimulated on the plate either with MUC1-derived peptide (predicted MHC-class I epitope) or with control peptide. The graph shows single data points for individual mice.

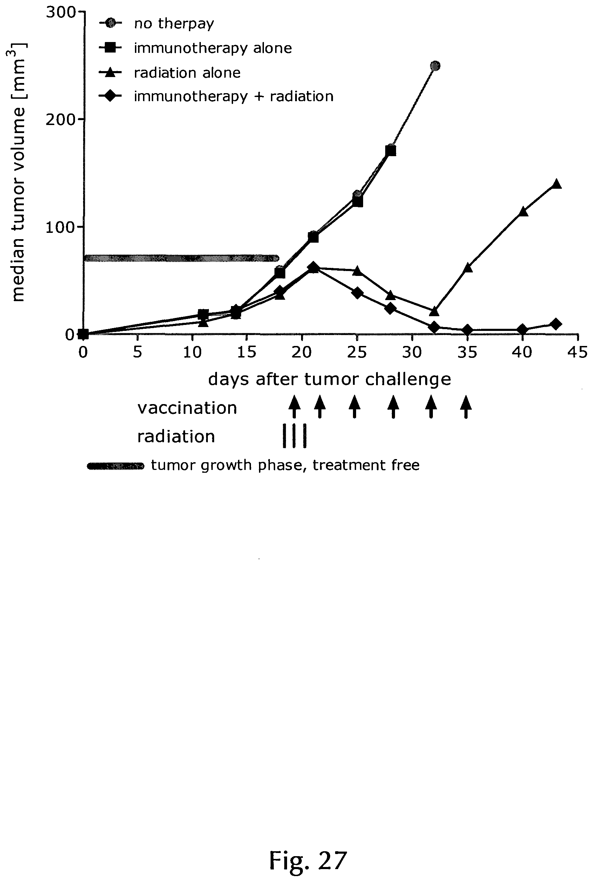

- Figure 27:: shows the effect of the combination of mRNA immunotherapy with radiation therapy in the treatment of low immunogenic and radioresistant Lewis Lung Carcinoma (LLC).

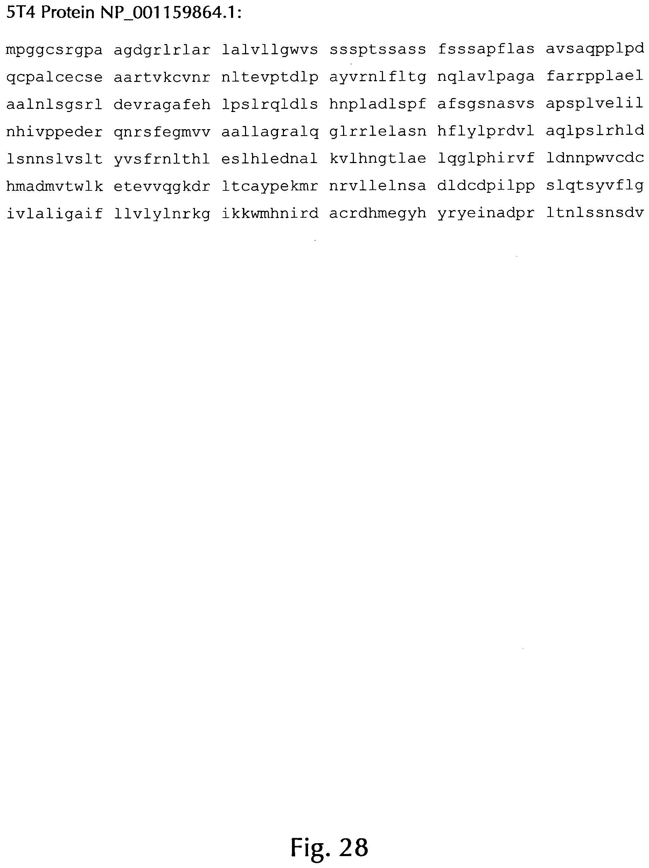

- Figure 28:: shows the protein sequence of 5T4 NP_001159864.1 according to SEQ ID NO: 75.

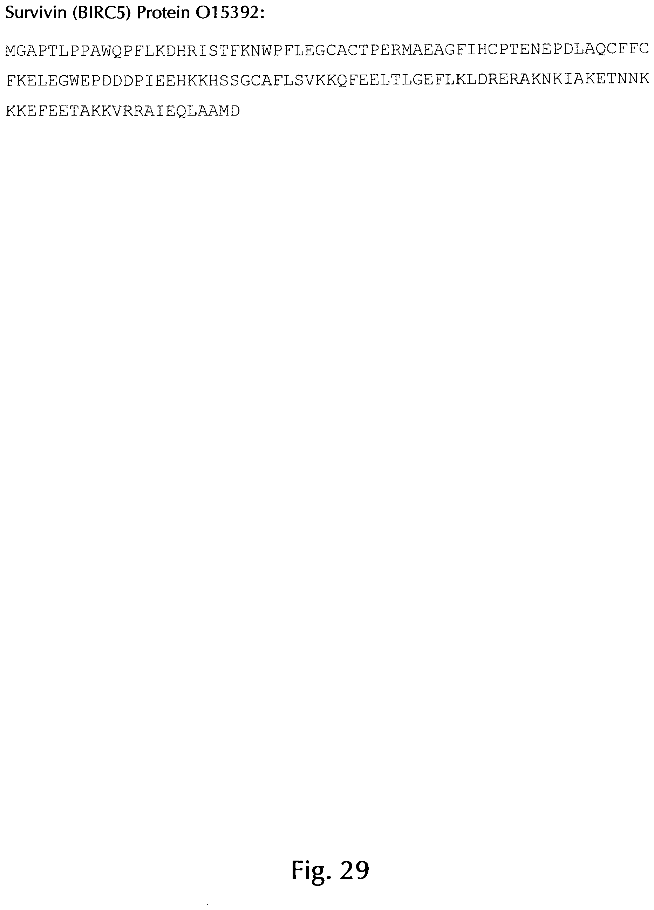

- Figure 29:: shows the protein sequence of Survivin (BIRC5) 015392 according to SEQ ID NO: 76.

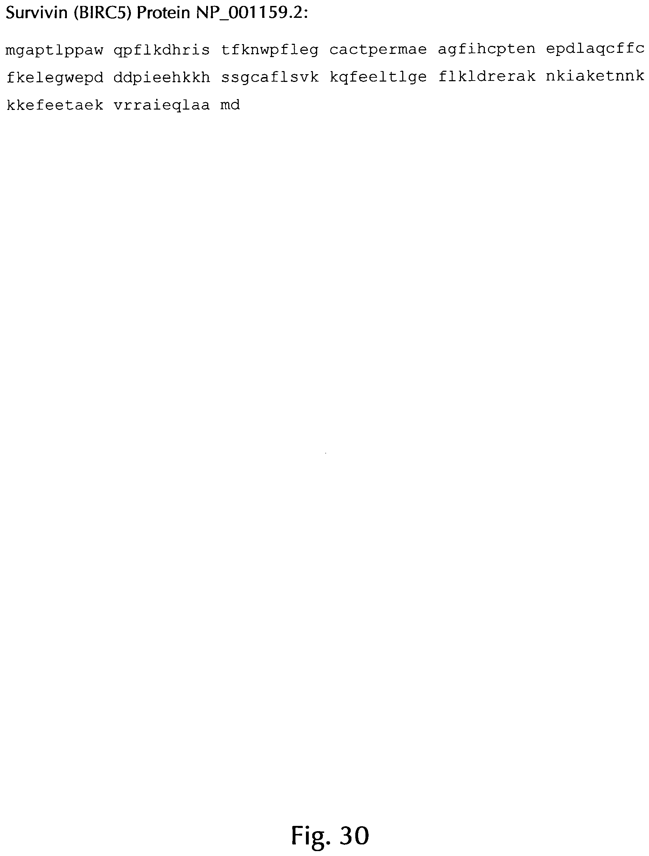

- Figure 30:: shows the protein sequence of Survivin (BIRC5) NP_001159.2 according to SEQ ID NO: 77.

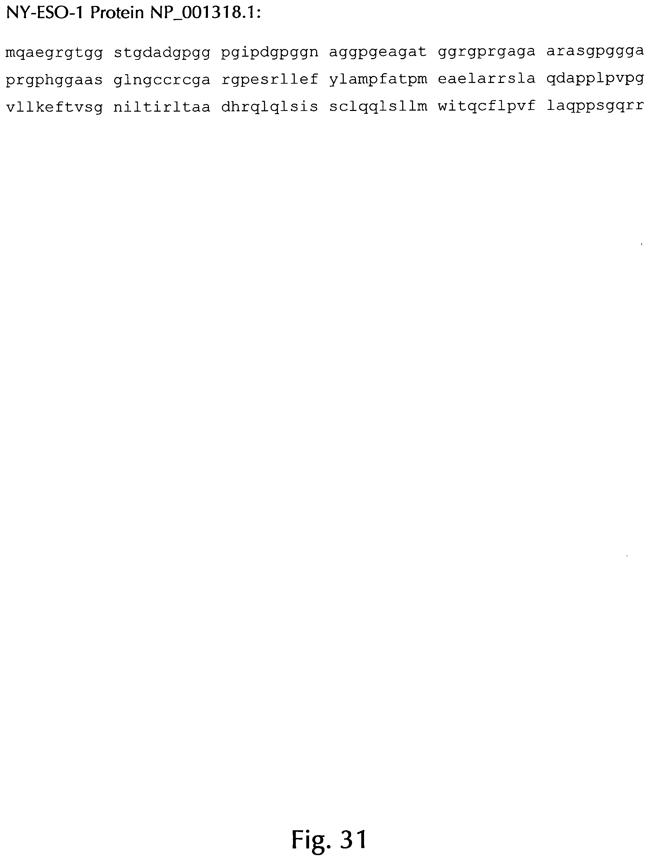

- Figure 31:: shows the protein sequence of NY-ESO-1 NP_001318.1 according to SEQ ID NO: 78.

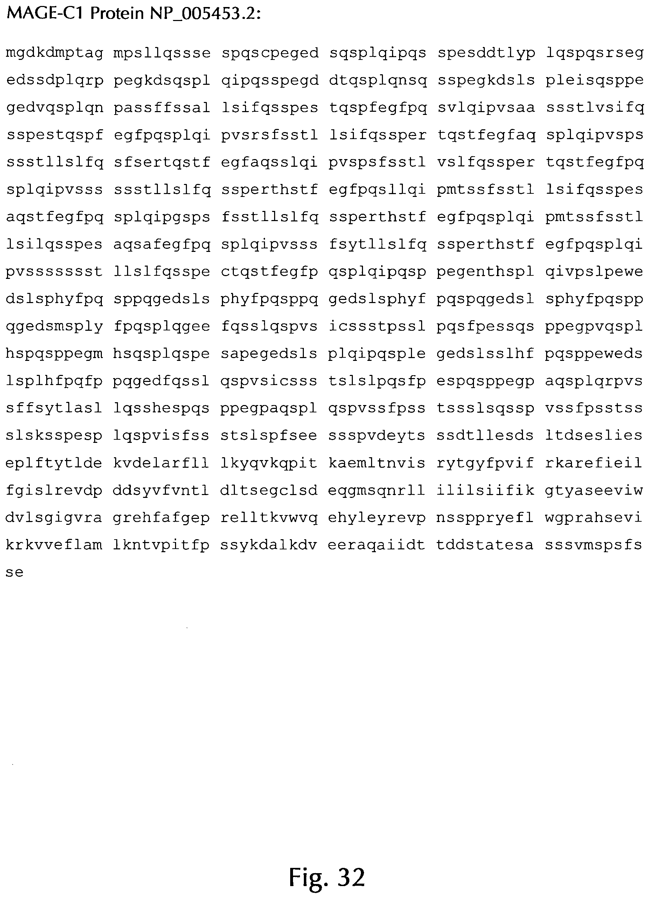

- Figure 32:: shows the protein sequence of MAGE-C1 NP_005453.2 according to SEQ ID NO: 79.

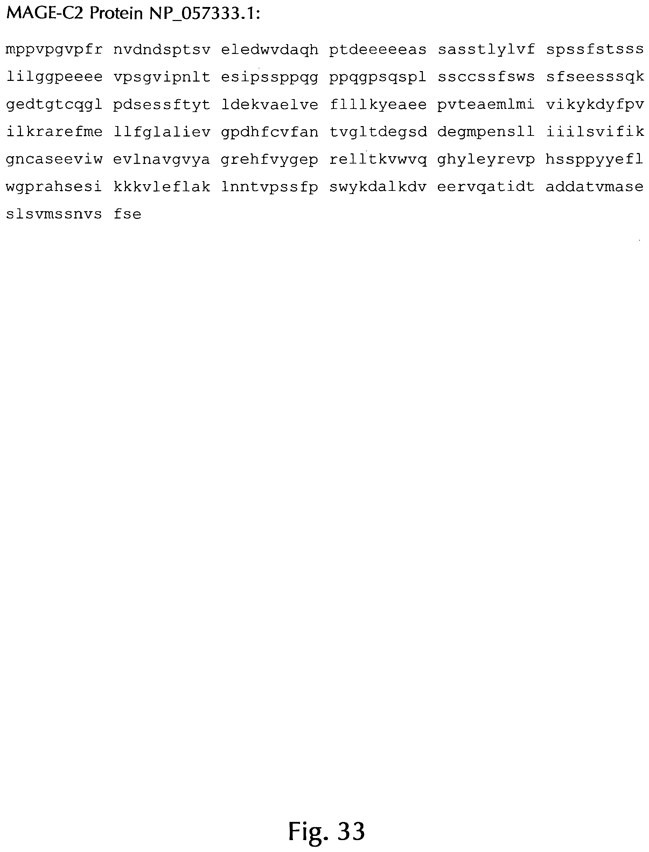

- Figure 33:: shows the protein sequence of MAGE-C2 NP_057333.1 according to SEQ ID NO: 80.

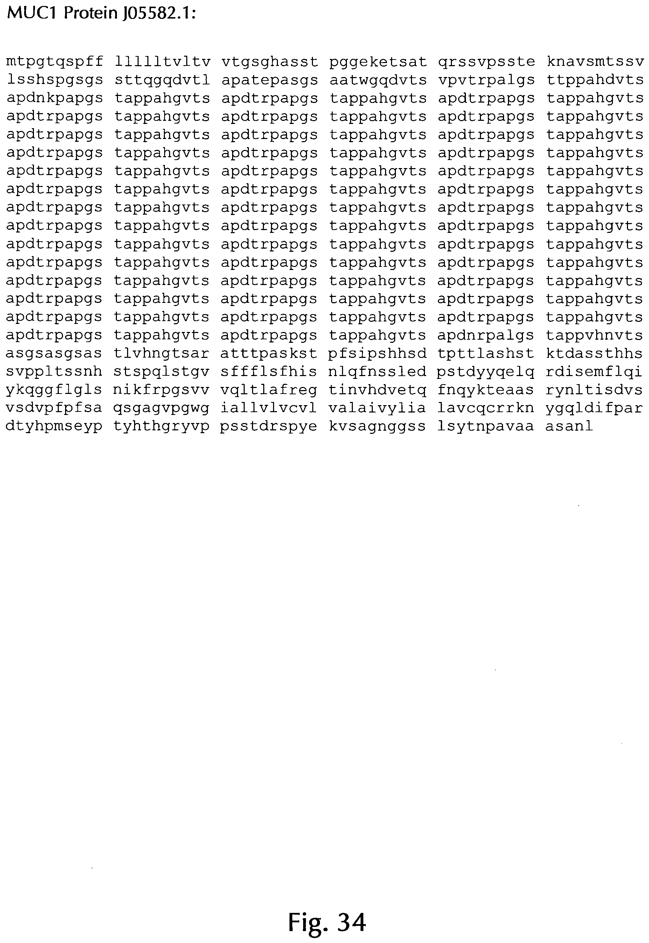

- Figure 34:: shows the protein sequence of MUC1 Protein J05582.1according to SEQ ID NO: 81.

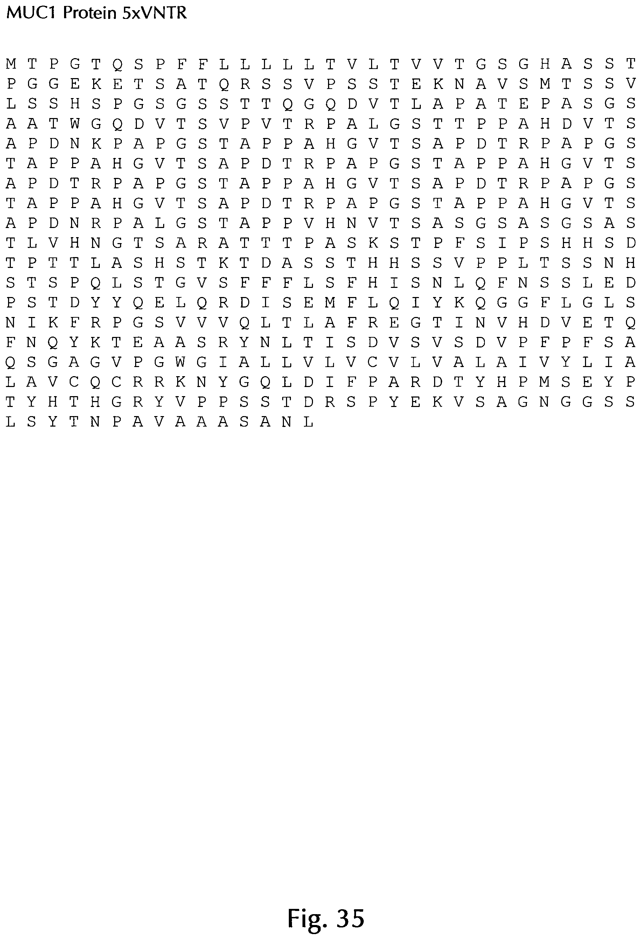

- Figure 35:: shows the protein sequence of MUC1 Protein 5xVNTR according to SEQ ID NO: 82.

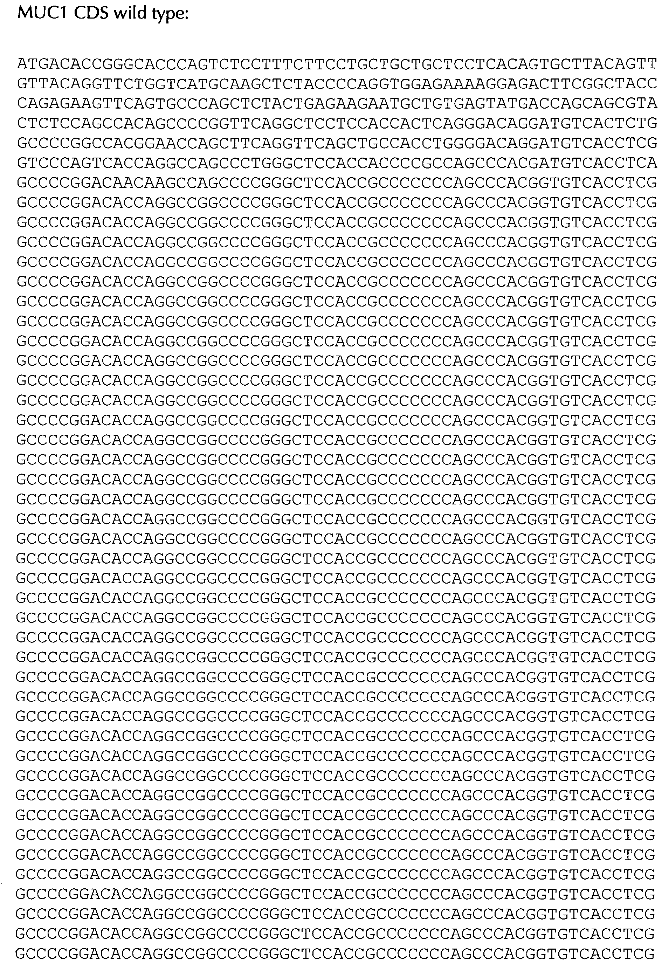

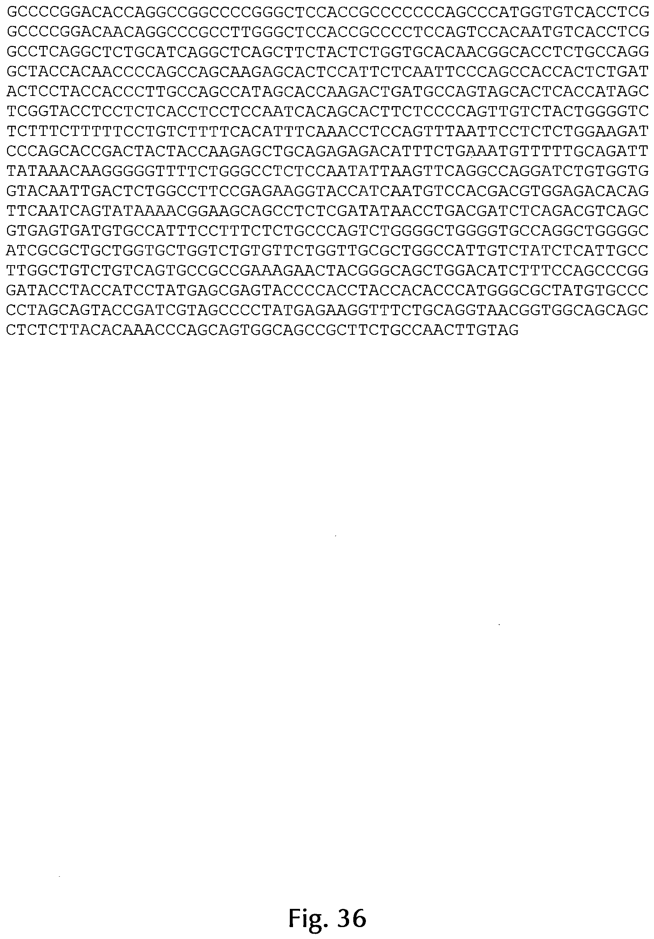

- Figure 36:: depicts the wildtype coding sequence, encoding MUC1, according to SEQ ID NO: 83, i.e. the full-length coding sequence (CDS) encoding MUC1 without GC-optimized coding sequence.

### Examples:

The following examples are intended to illustrate the invention further. They are not intended to limit the subject matter of the invention thereto.

### 1. Preparation of an mRNA vaccine based on MUC1 and induction of antigen-specific cytotoxic T-cells:

### 1.1 Preparation of an mRNA vaccine based on MUC1:

The mRNA vaccine consists of GC-optimized mRNAs coding for MUC1 (SEQ ID NO: 16). The mRNA was complexed with protamine by addition of protamine to the mRNA in the ratio (1:2) (w/w) (adjuvant component). After incubation for 10 min, the same amount of free mRNA used as antigen-providing mRNA was added.

The resulting composition was dissolved in 80% (v/v) Ringer-lactate solution.

### 1.2 Vaccination

C57BL/6 mice were vaccinated intradermally with 32 µg of one of the mRNA vaccines as described under 1.1 above. Control mice were treated injected intradermally with buffer (Ringer-lactate). Vaccination comprised five immunizations with 2 immunizations per week. The immune response was analysed 5 or 6 days after completion of the vaccination cycle.

### 1.3 ELISPOT - Detection of CTL (cytotoxic T cell) responses

For the detection of CTL (cytotoxic T cell) responses the analysis of IFN-gamma secretion in response to a specific stimulus can be visualized at a single cell level using the ELISPOT technique.

Splenocytes from mice vaccinated with the mRNA vaccine as described under 1.1 above and control mice were isolated 5 or 6 days after the last vaccination and then transferred into 96-well ELISPOT plates coated with an IFN-gamma capture antibody. The cells were then stimulated for 24 hours at 37°C using the following peptides:

| MUC1-derived peptide (SEQ ID NO:73) | Connexin-derived peptide (control) (SEQ ID NO: 74) |
|---|---|
| SAPDNRPAL | FEQNTAQP |

After the incubation period the cells were washed out of the plate and the IFN-gamma secreted by the cells was detected using a biotinylated secondary antibody against murine IFN-gamma, followed by streptavidin-AKP. Spots were visualized using BCIP/NBT substrate and counted using an automated ELISPOT reader (Immunospot Analyzer, CTL Analyzers LLC).

Intradermal vaccination with the MUC1-encoding mRNA led to the activation of antigen-specific CD8⁺ T-cells as demonstrated by the secretion of IFN-gamma in the ELISpot.

### 2. Combination of mRNA vaccination and irradiation in mice bearing low immunogenic (LLC) tumors

This study examined the efficacy of a vaccination and irradiation combination in C57BL/6 mice bearing low immunogenic (LLC) tumors.

On day 0 C57BL/6 mice (n = 10 per group) were challenged subcutaneously with syngeneic 3LL cells (LLC cells expressing tumor antigens EGFR and Connexin) in the right hind limb. Treatment was started when tumors reached 50 mm³. Mice were either vaccinated with mRNAs encoding EGFR and Connexin (twice a week) or tumors were radiated with 36 Gy distributed in 3 equal doses for 3 consecutive days, or mice were treated with combination therapy (with first vaccination given 6h before second radiation) as indicated in Fig. 28. Untreated mice served as control. Tumor growth was monitored by measuring the tumor size in 3 dimensions using calipers.

Due to the lack of MHC class I expression, immunotherapy alone was ineffective in inhibiting tumor growth and also radiation therapy resulted in only transient tumor control. On the other hand, mRNA vaccines combined with radiotherapy resulted in a strong synergistic anti-tumor effect.

### 3. Clinical study: mRNA-derived cancer vaccine and local radiation as treatment of NSCLC

### Study Drug:

A composition comprising an mRNA coding for 5T4 (Trophoblast glycoprotein, TPBG) according to SEQ ID No. 19, an mRNA coding for Survivin (Baculoviral IAP repeat-containing protein 5; BIRC5) according to SEQ ID No. 20, an mRNA coding for NY-ESO-1 (New York esophageal squamous cell carcinoma 1; CTAG1B) according to SEQ ID No. 21, an mRNA coding for MAGE-C1 (Melanoma antigen family C1) according to SEQ ID No. 22, an mRNA coding for MAGE-C2 (Melanoma antigen family C2) according to SEQ ID No. 23, and an mRNA coding for MUC1 (Mucin 1) according to SEQ ID No. 24 complexed with protamine according to example 1.1. is used as vaccine. Each antigen-providing mRNA is provided as a sterile lyophilizate. Reconstitution in Ringer-Lactate provides a solution for intradermal injection. The drug product used in the clinical trial is denominated CV9202. It is a vaccine comprising six drug product components each comprising a different antigen-providing mRNA.

### Study Population:

- Stratum 1: Patients with stage IV non-small cell lung cancer (NSCLC) and non-squamous histology, without activating epidermal growth factor receptor (EGFR) mutations, who have achieved partial response (PR) or stable disease (SD) after at least 4 cycles of platinum- and pemetrexed-based first-line chemotherapy, and an indication for maintenance therapy with pemetrexed.
- Stratum 2: Patients with stage IV NSCLC and squamous cell histology, who have achieved PR or SD after at least 4 cycles of platinum-based and non-platinum compound first-line chemotherapy (platinum-based combination chemotherapy).
- Stratum 3: Patients with stage IV NSCLC and non-squamous histology, harboring an activating EGFR mutation, who have achieved PR after up to 6 months of treatment with an EGFR tyrosine kinase inhibitor (TKI).

### Study population: Inclusion criteria

1. Patients: > 18 years of age with histologically or cytologically-confirmed stage IV NSCLC, and a confirmed EGFR mutation status in case of non-squamous cell histology
   - Stratum 1: Non-squamous NSCLC without activating EGFR mutation
   - Stratum 2: Squamous NSCLC
   - Stratum 3: Non-squamous NSCLC harboring an activating EGFR mutation
2. PR or SD according to RECIST Version 1.1 after first-line therapy which should have consisted of:
   Stratum 1: PR or SD after cisplatin or carboplatin and pemetrexed treatment (at least 4 cycles)
   Stratum 2: PR or SD after cisplatin or carboplatin and a non-platinum compound treatment (at least 4 cycles)
   Stratum 3: PR after gefitinib, erlotinib or afatinib treatment of up to 6 months
3. For patients in stratum 1, maintenance therapy with pemetrexed should be indicated as to the investigator's opinion
4. Presence of at least one tumor lesion that is eligible for radiation with 4 x 5 GY, and at least one additional measurable tumor lesion according to RECIST Version 1.1.
   Tumor lesions eligible for radiation are:
   Bone metastases
   Lymph nodes in the paraclavicular, axillary or cervical regions
   Skin or subcutaneous metastases
   For patients in strata 1 and 2 only: Thoracic lesions (centrally located lung tumor, lymph nodes in the lung hilus or mediastinum)
5. Performance Status: Eastern Cooperative Oncology Group (ECOG) 0 to 1
6. Adequate organ function:
   Bone marrow function: Hematologic values at the start of vaccination: hemoglobin ≥ 95 g/L, platelet count ≥ 75000/µL, white blood cell count ≥ 2000/µL, absolute neutrophil count ≥ 1000/µL, lymphocyte count ≥ 0.8 x 109/L
   Hepatic: ALT and AST ≤ 2.5 times ULN in patients without liver metastases and ≤ 5 times ULN in patients with liver metastases
   Renal: Serum creatinine ≤ 2 mg/dL, and creatinine clearance ≥ 45 mL/min according to MDRD formula
7. Use of highly effective contraception in patients of child-producing potential while enrolled in the study, and for 1 month after the last immunization
8. Written informed consent prior to conducting any study related procedure
   - Treatment duration: In each patient, the vaccine will be administered until disease progression and the need to start a subsequent systemic second-line treatment, or occurrence of unacceptable toxicity, whichever comes first.
   - Timing of screening: Patients will start screening 2 weeks after day 1 of the last cycle of their first-line chemotherapy (strata 1 and 2), or within 6 months after start of treatment with an EGFR TKI (erlotinib or gefitinib) (stratum 3).

### Method of administration

Every antigen-providing mRNA is applied individually on the same day as two intradermal injections of 200 µl each giving a total of 12 injections.

The first three vaccinations will be in a weekly interval (days 1, 8, 15), followed by intervals of 2 or 3 weeks until day 57, 3-weekly intervals until month 6 and 6-weekly thereafter as specified in the study protocol as specified in the study protocol.. Vaccinations will be administered until occurrence of unacceptable toxicity or tumor progression requiring the initiation of a systemic second line therapy.

In strata 1 and 3, CV9202 will be administered initially on Day 1, 8, 15, 36, and 57. During treatment with pemetrexed (stratum 1), vaccination will be administered 4-7 days before the next scheduled dose of pemetrexed. This schedule is chosen to avoid vaccination during the neutrophil nadir and interference with anti-inflammatory steroids.

In stratum 2, CV9202 will be administered initially on Day 1, 8, 15, 29, 43 and 57. This intensified schedule is chosen since patients have an expected shorter time to disease progression because they do not receive concomitant anticancer treatment.

In all strata, patients will continue vaccination after Day 57 in case no criterion for treatment discontinuation is met. After Day 57 vaccinations will be administered every 3 weeks until 6 months from the day of first vaccination. After the 6-month period, vaccination will be performed every 6 weeks until criteria for discontinuation are met:

### Criteria for treatment discontinuation:

1. Occurrence of toxicity requiring permanent discontinuation of treatment
2. Disease progression requiring the start of a subsequent systemic treatment

### Administration of

Vaccines: At every single vaccination time point, each of the 6 drug components will be administered separately on the same day. Two intradermal (i.d.) injections of 200 µL each will be performed per component, resulting in a total of 12 injections.

Radiation: Radiotherapy will be administered in 4 daily fractions of 5 GY each to be administered within one week, preferably from Day 9-12.

### Selection of tumor lesions for radiation

Lesions selected for radiation should measure at least 2 cm in the longest diameter, and the radiation oncologist at the trial site must confirm that the respective lesion is eligible for radiation in accordance with this protocol before the patient is enrolled.

The following lesions are potentially eligible and should be selected according to the following hierarchy:
First Preference: Bone metastases
Second Preference: Lymph nodes in the paraclavicular, axillary or cervical regions
Third Preference: Skin or subcutaneous metastases
Fourth preference (for patients in strata 1 and 2 only): Thoracic lesions (centrally located lung tumor, lymph nodes in the lung hilus or mediastinum)
In patients in stratum 3, no thoracic lesion should be selected for radiation since patients are at higher risk for radiation pneumonitis after thoracic radiation due to concomitant treatment with EGFR TKIs.

Irradiation of more than one lesion with the same schedule of 4 x 5 GY (e.g. multiple bone metastases) is permitted if clinically indicated as long as a measurable lesion remains for the evaluation of an abscopal response. Irradiation of more than one lesion should be performed within the same time window.

## Claims

1. Composition for use in the treatment of non-small cell lung cancer, the composition comprising six mRNAs, wherein each mRNA encodes a different antigen selected from the group consisting of:
• 5T4 (Trophoblast glycoprotein; TPBG);
• Survivin (Baculoviral IAP repeat-containing protein 5; BIRC5);
• NY-ESO-1 (New York esophageal squamous cell carcinoma 1; CTAG1B);
• MAGE-C1 (Melanoma antigen family C1);
• MAGE-C2 (Melanoma antigen family C2) and
• MUC1 (Mucin 1),
and wherein each mRNA is identical to a different RNA sequence selected from the RNA sequences according to SEQ ID NO: 19, 20, 21, 22, 23 or 24, wherein each mRNA is complexed with a cationic compound selected from the group consisting of a cationic peptide, a cationic protein, a cationic polysaccharide, a cationic polymer and a cationic lipid, and
wherein the treatment further comprises administration of a chemotherapeutic agent.

2. The composition for use according to claim 1, wherein the cationic compound is a polycation, preferably protamine or oligofectamine, most preferably protamine.

3. The composition for use according to claim 2, wherein the N/P ratio of at least one mRNA to the one or more polycations is in the range of about 0.1 to 10, including a range of about 0.3 to 4, of about 0.5 to 2, of about 0.7 to 2 and of about 0.7 to 1.5.

4. The composition for use according to any of claims 1 to 3 comprising at least one RNA, which is complexed with one or more polycations, and at least one free RNA, wherein the complexed RNA is preferably identical to the free RNA.

5. The composition for use according to any of claims 1 to 4, wherein the composition additionally comprises at least one adjuvant, wherein the at least one adjuvant is preferably selected from the group consisting of:
cationic or polycationic compounds, comprising cationic or polycationic peptides or proteins, including protamine, nucleoline, spermin or spermidine, poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones, cationic polysaccharides, including chitosan, polybrene, cationic polymers, including polyethyleneimine (PEI), cationic lipids, including DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglycylspermin, DIMRI: Dimyristooxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine / chloride, CLIP1: rac-[(2,3-dioctadecyloxypropyl)(2-hydroxyethyl)pdimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, including modified polyaminoacids, including β-aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1,4-butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, etc., silan backbone based polymers , such as PMOXA-PDMS copolymers, etc., Blockpolymers consisting of a combination of one or more cationic blocks selected of a cationic polymer as mentioned before, and of one or more hydrophilic- or hydrophobic blocks (e.g polyethyleneglycole);
or
cationic or polycationic proteins or peptides, selected from following proteins or peptides having the following total formula (I): (Arg)ₗ;(Lys)ₘ;(His)ₙ;(Orn)ₒ;(Xaa)ₓ, wherein l + m + n + o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide;
or
nucleic acids having the formula (II): GₗXₘGₙ, wherein: G is guanosine, uridine or an analogue of guanosine or uridine; X is guanosine, uridine, adenosine, thymidine, cytidine or an analogue of the above-mentioned nucleotides; I is an integer from 1 to 40, wherein when I = 1 G is guanosine or an analogue thereof, when I > 1 at least 50% of the nucleotides are guanosine or an analogue thereof; m is an integer and is at least 3; wherein when m = 3 X is uridine or an analogue thereof, when m > 3 at least 3 successive uridines or analogues of uridine occur; n is an integer from 1 to 40, wherein when n = 1 G is guanosine or an analogue thereof, when n > 1 at least 50% of the nucleotides are guanosine or an analogue thereof;
or
nucleic acids having the formula (III): CₗXₘCₙ, wherein: C is cytidine, uridine or an analogue of cytidine or uridine; X is guanosine, uridine, adenosine, thymidine, cytidine or an analogue of the above-mentioned nucleotides; l is an integer from 1 to 40, wherein when l = 1 C is cytidine or an analogue thereof, when I > 1 at least 50% of the nucleotides are cytidine or an analogue thereof; m is an integer and is at least 3; wherein when m = 3 X is uridine or an analogue thereof, when m > 3 at least 3 successive uridines or analogues of uridine occur; n is an integer from 1 to 40, wherein when n = 1 C is cytidine or an analogue thereof, when n > 1 at least 50% of the nucleotides are cytidine or an analogue thereof.

6. A vaccine for use in the treatment of non-small cell lung cancer, the vaccine comprising a composition as defined in any of claims 1 to 5, wherein the vaccine preferably further comprises a pharmaceutically acceptable carrier, wherein the treatment further comprises administration of a chemotherapeutic agent.

7. The composition for use according to any of claims 1 to 5, or the vaccine for use according to claim 6, wherein the chemotherapeutic agent is selected from the group consisting of a platinum-based compound, pemetrexed, gemcitabine, taxanes, vinorelbine, etoposide, docetaxel and paclitaxel.

8. The composition for use according to any of claims 1 to 5 or 7, or the vaccine according to claim 6 or 7, wherein the treatment further comprises radiation therapy.

9. The composition for use according to any of claims 1 to 5, 7 or 8, or the vaccine for use according to any of claims 6 to 8, wherein the composition or vaccine is used for treatment of a patient with stage III or stage IV non-small cell lung cancer.

10. The composition for use or the vaccine for use according to claim 9, wherein the non-small cell lung cancer is **characterized by** a non-squamous histology.

11. The composition for use according to any of claims 1 to 5 or 7 to 10, or the vaccine for use according to any of claims 6 to 10, wherein the subject is a patient receiving chemotherapy, preferably a platinum-based chemotherapy or a platinum-based combination chemotherapy, or a patient having achieved partial response or stable disease after chemotherapy.

12. Combination of six mRNAs for use in the treatment of non-small cell lung cancer, wherein each mRNA encodes one antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1) and wherein each mRNA is identical to a different RNA sequence selected from the RNA sequences according to SEQ ID NO: 19, 20, 21, 22, 23 or 24, and wherein each mRNA is complexed with a cationic compound selected from the group consisting of a cationic peptide, a cationic protein, a cationic polysaccharide, a cationic polymer and a cationic lipid, and
wherein the treatment further comprises administration of a chemotherapeutic agent.

13. Combination for use according to claim 12, wherein each of the six mRNAs is administered separately.

14. Combination for use according to claim 12 or 13, wherein the mRNAs are administered by intradermal injection.

15. Combination for use according to any of claims 12 to 14, wherein the treatment further comprises radiation therapy.

16. Combination for use according to any of claims 12 to 15, wherein the composition is used for treatment of a patient with stage III or stage IV non-small cell lung cancer.

17. Combination for use according to claim 16, wherein the non-small cell lung cancer is **characterized by** a non-squamous histology.

18. Combination for use according to any of claims 12 to 17, wherein the subject is a patient receiving chemotherapy, preferably a platinum-based chemotherapy or a platinum-based combination chemotherapy, or a patient having achieved partial response or stable disease after chemotherapy.

19. A kit, preferably kit of parts, for use in the treatment of non-small cell lung cancer, the kit comprising the composition for use as defined in any of claims 1 to 5 or 7 to 11, and/or a vaccine for use as defined in any of claims 6 to 11, and optionally a liquid vehicle for solubilising and optionally technical instructions with information on the administration and dosage of the active composition and/or the vaccine.

20. The kit for use according to claim 19, wherein the kit is a kit of parts and each part of the kit contains at least one mRNA preferably encoding a different antigen selected from the antigens defined in claim 1, all parts of the kit of parts forming the composition or the vaccine of the preceding claims.

21. The kit for use according to claim 19 or 20, wherein the kit contains at least two parts containing six mRNAs.

22. The kit for use according to any of claims 19 to 21, wherein all six mRNAs are provided in lyophilized form in separate parts.

23. The kit for use according to claim 22, wherein the kit contains as a part Ringer-Lactate solution.

24. The kit for use according to any of claims 19 to 23, wherein the kit contains six parts, each part containing one of the six mRNAs.

## Patentansprüche

1. Zusammensetzung zur Verwendung bei der Behandlung von nicht-kleinzelligem Lungenkrebs, wobei die Zusammensetzung sechs mRNAs umfasst, wobei jede mRNA für ein anderes Antigen kodiert, ausgewählt aus der Gruppe, bestehend aus:
• 5T4 (Trophoblast-Glykoprotein; TPBG);
• Survivin (Baculovirales IAP-Repeat-enthaltendes Protein 5; BIRC5);
• NY-ESO-1 (New York-Speiseröhren-Plattenepithelzellkarzinom 1; CTAG1B);
• MAGE-C1 (Melanom-Antigenfamilie C1);
• MAGE-C2 (Melanom-Antigenfamilie C2) und
• MUC1 (Mucin 1),
und wobei jede mRNA identisch ist mit einer anderen RNA-Sequenz, ausgewählt aus den RNA-Sequenzen gemäß SEQ ID NO: 19, 20, 21, 22, 23 oder 24, wobei jede mRNA mit einer kationischen Verbindung komplexiert ist, ausgewählt aus der Gruppe, bestehend aus einem kationischen Peptid, einem kationischen Protein, einem kationischen Polysaccharid, einem kationischen Polymer und einem kationischen Lipid, und
wobei die Behandlung ferner die Verabreichung eines chemotherapeutischen Mittels umfasst.

2. Zusammensetzung zur Verwendung nach Anspruch 1, wobei die kationische Verbindung ein Polykation, vorzugsweise Protamin oder Oligofectamin, am bevorzugtesten Protamin ist.

3. Zusammensetzung zur Verwendung nach Anspruch 2, wobei das N/P-Verhältnis von mindestens einer mRNA zu einer oder mehreren Polykationen im Bereich von etwa 0,1 bis 10 liegt, einschließlich eines Bereichs von etwa 0,3 bis 4, von etwa 0,5 bis 2, von etwa 0,7 bis 2 und von etwa 0,7 bis 1,5.

4. Zusammensetzung zur Verwendung nach einem der Ansprüche 1 bis 3, umfassend mindestens eine RNA, die mit einer oder mehreren Polykationen komplexiert ist, und mindestens eine freie RNA, wobei die komplexierte RNA vorzugsweise identisch mit der freien RNA ist.

5. Zusammensetzung zur Verwendung nach einem der Ansprüche 1 bis 4, wobei die Zusammensetzung zusätzlich mindestens ein Adjuvans umfasst, wobei das mindestens eine Adjuvans vorzugsweise ausgewählt ist aus der Gruppe, bestehend aus:
kationischen oder polykationischen Verbindungen, umfassend kationische oder polykationische Peptide oder Proteine, einschließlich Protamin, Nucleolin, Spermin oder Spermidin, Poly-L-Lysin (PLL), Poly-Arginin, basischen Polypeptiden, zellpenetrierenden Peptiden (CPPs), einschließlich HIV-bindender Peptide, Tat, HIV-1 Tat (HIV), von Tat abgeleiteter Peptide, Penetratin, von VP22 abgeleiteter oder analoger Peptide, HSV VP22 (Herpes simplex), MAP, KALA oder Proteintransduktionsdomänen (PTDs), PpT620, Prolinreichen Peptiden, Arginin-reichen Peptiden, Lysin-reichen Peptiden, MPG-Peptid(en), Pep-1, L-Oligomeren, Calcitonin-Peptide(n), von Antennapedia abgeleiteten Peptiden (insbesondere von Drosophila-Antennapedia), pAntp, plsl, FGF, Lactoferrin, Transportan, Buforin-2, Bac715-24, SynB, SynB(1), pVEC, von hCT abgeleiteten Peptiden, SAP, Protamin, Spermin, Spermidin oder Histonen, kationischen Polysacchariden, einschließlich Chitosan, Polybren, kationischen Polymeren, einschließlich Polyethylenimin (PEI), kationischen Lipiden, einschließlich DOTMA: [1-(2,3-Sioleyloxy)propyl)]-N,N,N-trimethylammoniumchlorid, DMRIE, di-C14-Amidin, DOTIM, SAINT, DC-Chol, BGTC, CTAP, DOPC, DODAP, DOPE: Dioleylphosphatidylethanol-amin, DOSPA, DODAB, DOIC, DMEPC, DOGS: Dioctadecylamidoglycylspermin, DIMRI: Dimyristooxypropyldimethylhydroxyethylammoniumbromid, DOTAP: Dioleoyloxy-3-(trimethylammonio)propan, DC-6-14: O,O-Ditetradecanoyl-N-(α-trimethylammonioacetyl)diethanolaminchlorid, CLIP1: rac-[(2,3-Dioctadecyloxypropyl)(2-hydroxyethyl)]-dimethylammoniumchlorid, CLIP6: rac-[2(2,3-Dihexadecyloxypropyl-oxymethyloxy)ethyl]trimethylammonium, CLIP9: rac-[2(2,3-Dihexadecyloxypropyl-oxysuccinyloxy)ethyl]-trimethylammonium, Oligofectamin oder kationischen oder polykationischen Polymeren, einschließlich modifizierter Polyaminosäuren, einschließlich β-Aminosäure-Polymere oder umgekehrter Polyamide, modifizierten Polyethylenen, einschließlich PVP (Poly(N-ethyl-4-vinylpyridiniumbromid)), modifizierten Acrylaten, einschließlich pDMAEMA (Poly(dimethylaminoethylmethylacrylat)), modifizierten Amidoaminen, einschließlich pAMAM (Poly(amidoamin)), modifizierten Polybetaaminoestern (PBAE), einschließlich Diamin-endmodifizierter 1,4-Butandioldiacrylat-co-5-amino-1-pentanol-Polymere, Dendrimeren, einschließlich Polypropylamin-Dendrimere oder pAMAM-basierter Dendrimere, Polyimin(en), einschließlich PEI: Poly(ethylenimin), Poly(propylenimin), Polyallylamin, Polymeren auf Zuckergrundgerüstbasis, einschließlich Polymere auf Cyclodextrinbasis, Polymeren auf Dextranbasis, Chitosan, etc., Polymeren auf Silangrundgerüstbasis, wie PMOXA-PDMS-Copolymeren usw., Blockpolymeren, bestehend aus einer Kombination aus einem oder mehreren kationischen Blöcken, ausgewählt aus einem kationischen Polymer, wie vorstehend erwähnt, und einem oder mehreren hydrophilen oder hydrophoben Blöcken (z.B. Polyethylenglykol);
oder
kationischen oder polykationischen Proteinen oder Peptiden, ausgewählt aus folgenden Proteinen oder Peptiden mit der folgenden Gesamtformel (I): (Arg)ₗ;(Lys)ₘ;(His)ₙ;(Orn)ₒ;(Xaa)ₓ, wobei l + m + m + n + o + x = 8-15, und l, m, n oder o unabhängig voneinander jede Zahl sein kann, die aus 0, 1, 2, 3, 4, 5, 6, 7, 8, 9, 10, 11, 12, 13, 14 oder 15 ausgewählt ist, vorausgesetzt, dass der Gesamtgehalt von Arg, Lys, His und Orn mindestens 50% aller Aminosäuren des Oligopeptids ausmacht; und Xaa jede Aminosäure sein kann, ausgewählt aus nativen (= natürlich vorkommenden) oder nichtnativen Aminosäuren mit Ausnahme von Arg, Lys, His oder Orn; und x jede Zahl ausgewählt aus 0, 1, 2, 3 oder 4 sein kann, vorausgesetzt, dass der Gesamtgehalt von Xaa 50 % aller Aminosäuren des Oligopeptids nicht übersteigt;
oder
Nukleinsäuren mit der Formel (II): GₗXₘGₙ, wobei: G Guanosin, Uridin oder ein Analogon von Guanosin oder Uridin ist; X Guanosin, Uridin, Adenosin, Thymidin, Cytidin oder ein Analogon der oben genannten Nukleotide ist; l eine ganze Zahl von 1 bis 40 ist, wobei, wenn l = 1, G Guanosin oder ein Analogon davon ist, wenn l > 1, mindestens 50% der Nukleotide Guanosin oder ein Analogon davon sind; m eine ganze Zahl ist und mindestens 3 ist; wobei, wenn m = 3 ist, X Uridin oder ein Analogon davon ist, wenn m > 3, mindestens 3 aufeinanderfolgende Uridine oder Analoga von Uridin auftreten; n eine ganze Zahl von 1 bis 40 ist, wobei, wenn n = 1, G Guanosin oder ein Analogon davon ist, wenn n > 1, mindestens 50% der Nucleotide Guanosin oder ein Analogon davon sind;
oder
Nukleinsäuren mit der Formel (III): CₗXₘCₙ, wobei: C Cytidin, Uridin oder ein Analogon von Cytidin oder Uridin ist; X Guanosin, Uridin, Adenosin, Thymidin, Cytidin oder ein Analogon der oben genannten Nukleotide ist; l eine ganze Zahl von 1 bis 40 ist, wobei, wenn l = 1, C Cytidin oder ein Analogon davon ist, wenn l > 1, mindestens 50% der Nukleotide Cytidin oder ein Analogon davon sind; m eine ganze Zahl ist und mindestens 3 ist; wobei, wenn m = 3 ist, X Uridin oder ein Analogon davon ist, wenn m > 3, mindestens 3 aufeinanderfolgende Uridine oder Analoga von Uridin auftreten; n eine ganze Zahl von 1 bis 40 ist, wobei, wenn n = 1, C Cytidin oder ein Analogon davon ist, wenn n > 1 mindestens 50% der Nukleotide Cytidin oder ein Analogon davon sind.

6. Impfstoff zur Verwendung bei der Behandlung von nicht-kleinzelligem Lungenkrebs, wobei der Impfstoff eine Zusammensetzung, wie durch einen der Ansprüche 1 bis 5 definiert, umfasst, wobei der Impfstoff vorzugsweise weiterhin einen pharmazeutisch akzeptablen Träger umfasst, wobei die Behandlung ferner die Verabreichung eines chemotherapeutischen Mittels umfasst.

7. Zusammensetzung zur Verwendung nach einem der Ansprüche 1 bis 5 oder Impfstoff zur Verwendung nach Anspruch 6, wobei das chemotherapeutische Mittel ausgewählt ist aus der Gruppe, bestehend aus einer Verbindung auf Platinbasis, Pemetrexed, Gemcitabin, Taxanen, Vinorelbin, Etoposid, Docetaxel und Paclitaxel.

8. Zusammensetzung zur Verwendung nach einem der Ansprüche 1 bis 5 oder 7 oder Impfstoff nach Anspruch 6 oder 7, wobei die Behandlung ferner eine Strahlentherapie umfasst.

9. Zusammensetzung zur Verwendung nach einem der Ansprüche 1 bis 5, 7 oder 8 oder Impfstoff zur Verwendung nach einem der Ansprüche 6 bis 8, wobei die Zusammensetzung oder der Impfstoff zur Behandlung eines Patienten mit nicht-kleinzelligem Lungenkrebs mit Stadium (stage) III oder Stadium (stage) IV verwendet wird.

10. Zusammensetzung zur Verwendung oder Impfstoff zur Verwendung nach Anspruch 9, wobei der nicht-kleinzellige Lungenkrebs durch eine nicht-squamöse Histologie gekennzeichnet ist.

11. Zusammensetzung zur Verwendung nach einem der Ansprüche 1 bis 5 oder 7 bis 10 oder Impfstoff zur Verwendung nach einem der Ansprüche 6 bis 10, wobei das Subjekt ein Patient ist, der eine Chemotherapie erhält, vorzugsweise eine Platin-basierte Chemotherapie oder eine Platin-basierte Kombinationschemotherapie, oder ein Patient, der ein partielles Ansprechen oder eine stabile Erkrankung nach der Chemotherapie aufweist.

12. Kombination von sechs mRNAs zur Verwendung bei der Behandlung von nicht-kleinzelligem Lungenkrebs, wobei jede mRNA für ein Antigen kodiert, ausgewählt aus der Gruppe bestehend aus 5T4 (Trophoblast-Glykoprotein, TPBG), Survivin (Baculovirales IAP-Repeat-enthaltendes Protein 5; BIRC5), NY-ESO-1 (New York-Speiseröhren-Plattenepithelzellkarzinom 1, CTAG1B), MAGE-C1 (Melanom-Antigenfamilie C1), MAGE-C2 (Melanom-Antigenfamilie C2) und MUC1 (Mucin 1) und wobei jede mRNA mit einer anderen RNA-Sequenz identisch ist, ausgewählt aus den RNA-Sequenzen gemäß SEQ ID NO: 19, 20, 21, 22, 23 oder 24, und wobei jede mRNA mit einer kationischen Verbindung komplexiert ist, ausgewählt aus der Gruppe, bestehend aus einem kationischen Peptid, einem kationischen Protein, einem kationischen Polysaccharid, einem kationischen Polymer und einem kationischen Lipid, und
wobei die Behandlung ferner die Verabreichung eines chemotherapeutischen Mittels umfasst.

13. Kombination zur Verwendung nach Anspruch 12, wobei jede der sechs mRNAs separat verabreicht wird.

14. Kombination zur Verwendung nach Anspruch 12 oder 13, wobei die mRNAs durch intradermale Injektion verabreicht werden.

15. Kombination zur Verwendung nach einem der Ansprüche 12 bis 14, wobei die Behandlung ferner Strahlentherapie umfasst.

16. Kombination zur Verwendung nach einem der Ansprüche 12 bis 15, wobei die Zusammensetzung zur Behandlung eines Patienten mit nicht-kleinzelligem Lungenkrebs mit Stadium (stage) III oder Stadium (stage) IV verwendet wird.

17. Kombination zur Verwendung nach Anspruch 16, wobei der nicht-kleinzellige Lungenkrebs durch eine nicht-squamöse Histologie gekennzeichnet ist.

18. Kombination zur Verwendung nach einem der Ansprüche 12 bis 17, wobei das Subjekt ein Patient ist, der eine Chemotherapie erhält, vorzugsweise eine Platin-basierte Chemotherapie oder eine Platin-basierte Kombinations-Chemotherapie, oder ein Patient, der ein partielles Ansprechen oder eine stabile Erkrankung nach der Chemotherapie aufweist.

19. Ein Kit, vorzugsweise ein Kit von Teilen (kit of parts), zur Verwendung bei der Behandlung von nicht-kleinzelligem Lungenkrebs, wobei der Kit die Zusammensetzung zur Verwendung, wie durch einen der Ansprüche 1 bis 5 oder 7 bis 11 definiert, und/oder einen Impfstoff zur Verwendung, wie durch einen der Ansprüche 6 bis 11 definiert, umfasst, und optional ein flüssiges Vehikel zum Löslichmachen und optional technische Anweisungen mit Informationen über die Verabreichung und Dosierung der aktiven Zusammensetzung und/oder des Impfstoffs.

20. Kit zur Verwendung nach Anspruch 19, wobei der Kit ein Kit von Teilen ist und jeder Teil des Kits mindestens eine mRNA enthält, die vorzugsweise für ein anderes Antigen kodiert, ausgewählt aus den in Anspruch 1 definierten Antigenen, wobei alle Teile des Kits von Teilen die Zusammensetzung oder den Impfstoff der vorhergehenden Ansprüche bilden.

21. Kit zur Verwendung nach Anspruch 19 oder 20, wobei der Kit mindestens zwei Teile (parts) mit sechs mRNAs enthält.

22. Kit zur Verwendung nach einem der Ansprüche 19 bis 21, wobei alle sechs mRNAs in lyophilisierter Form in separaten Teilen (parts) bereitgestellt werden.

23. Kit zur Verwendung nach Anspruch 22, wobei der Kit als Teil (part) Ringer-Lactat-Lösung enthält.

24. Kit zur Verwendung nach einem der Ansprüche 19 bis 23, wobei der Kit sechs Teile (parts) enthält, wobei jeder Teil eine der sechs mRNAs enthält.

## Revendications

1. Composition destinée à être utilisée dans le traitement du cancer du poumon non à petites cellules, la composition comprenant six ARNm, où chaque ARNm code un antigène différent choisi dans le groupe consistant en:
• 5T4 (glycoprotéine des trophoblastique; TPBG);
• survivine (protéine contenant des répétitions IAP baculovirale 5; BIRC5) ;
• NY-ESO-1 (carcinome épidermoïde de l'oesophage de New York 1; CTAG1B);
• MAGE-C1 (famille d'antigènes du mélanome C1);
• MAGE-C2 (famille d'antigènes du mélanome C2) et
• MUC1 (mucine 1),
et où chaque ARNm est identique à une séquence d'ARN différente choisie parmi les séquences d'ARN selon SEQ ID NO: 19, 20, 21, 22, 23 ou 24, où chaque ARNm est complexé avec un composé cationique choisi dans le groupe consistant en un peptide cationique, une protéine cationique, un polysaccharide cationique, un polymère cationique et un lipide cationique, et
où le traitement comprend en outre l'administration d'un agent chimiothérapeutique.

2. Composition destinée à être utilisée selon la revendication 1, où le composé cationique est un polycation, de préférence la protamine ou l'oligofectamine, de manière particulièrement préférable la protamine.

3. Composition destinée à être utilisée selon la revendication 2, où le rapport N/P d'au moins un ARNm aux un ou plusieurs polycations est dans la plage d'environ 0,1 à 10, incluant une plage d'environ 0,3 à 4, d'environ 0,5 à 2, d'environ 0,7 à 2 et d'environ 0,7 à 1,5.

4. Composition destinée à être utilisée selon l'une quelconque des revendications 1 à 3 comprenant au moins un ARN, qui est complexé avec un ou plusieurs polycations, et au moins un ARN libre, où l'ARN complexé est de préférence identique à l'ARN libre.

5. Composition destinée à être utilisée selon l'une quelconque des revendications 1 à 4, où la composition comprend en outre au moins un adjuvant, où le au moins un adjuvant est de préférence choisi dans le groupe consistant en:
les composés cationiques ou polycationiques, comprenant les peptides ou protéines cationiques ou polycationiques, incluant la protamine, la nucléoline, la spermine ou la spermidine, la poly-L-lysine (PLL), la poly-arginine, les polypeptides basiques, les peptides pénétrant dans les cellules (CPP), incluant les peptides liant VIH, Tat, Tat de VIH-1 (VIH), les peptides dérivés de Tat, la pénétratine, les peptides dérivés ou analogues de VP22, VP22 de VHS (herpes simplex), les domaines de transduction de MAP, KALA ou de protéine (les PTD), PpT620, les peptides riches en proline, les peptides riches en arginine, les peptides riches en lysine, un ou des peptides de MPG, Pep-1, les oligomères L, un ou des peptides de calcitonine, les peptides dérivés de Antennapedia (en particulier de Drosophila antennapedia), pAntp, plsl, FGF, la lactoferrine, le transportan, la buforine-2, Bac715-24, SynB, SynB(1), pVEC, les peptides dérivés de hCT, SAP, la protamine, la spermine, la spermidine, ou les histones, les polysaccharides cationiques, incluant le chitosane, le polybrène, les polymères cationiques, incluant la polyéthylèneimine (PEI), les lipides cationiques, incluant DOTMA: le chlorure de [1-(2,3-sioléyloxy)propyl)]-N,N,N-triméthylammonium, DMRIE, la di-C14-amidine, DOTIM, SAINT, DC-Chol, BGTC, CTAP, DOPC, DODAP, DOPE: dioléyl phosphatidyléthanol-amine, DOSPA, DODAB, DOIC, DMEPC, DOGS: dioctadécylamidoglicylspermine, DIMRI: bromure de dimyristooxypropyldiméthylhydroxyéthylammonium, DOTAP: dioléoyloxy-3-(triméthylammonio)propane, DC-6-14: chlorure de O,O-ditétradécanoyl-N-(-triméthylammonioacétyl)diéthanolamine, CLIP1: chlorure de rac-[(2,3-dioctadécyloxypropyl)(2-hydroxyéthyl)]-diméthyl-ammonium, CLIP6: rac-[2(2,3-dihexadécyloxypropyl-oxyméthyloxy)-éthyl]triméthylammonium, CLIP9: rac-[2(2,3-dihexadécyloxypropyloxysuccinyloxy)éthyl]-triméthylammonium, l'oligofectamine, ou les polymères cationiques ou polycationiques, incluant les polyaminoacides modifiés, incluant les polymères de β-aminoacides ou les polyamides inversés, les polyéthylènes modifiés, incluant PVP (poly(bromure de N-éthyl-4-vinylpyridinium)), les acrylates modifiés, incluant pDMAEMA (poly(méthacrylate de diméthylaminoéthyle)), les amidoamines modifiées incluant pAMAM (poly(amidoamine)), un polybêtaaminoester modifié (PBAE), incluant les copolymères de diacrylate de 1,4-butanediol et de 5-amino-1-pentanol modifiés par diamine aux extrémités, les dendrimères, incluant les dendrimères de polypropylamine ou les dendrimères à base de pAMAM, une ou des polyimine(s), incluant PEI: poly(éthylèneimine), poly(propylèneimine), polyallylamine, les polymères à base de squelette de sucre, incluant les polymères à base de cyclodextrine, les polymères à base de dextrane, le chitosane, etc., les polymères à base de squelette de silane, comme les copolymères PMOXA-PDMS, etc., les polymères séquencés consistant en une combinaison d'une ou plusieurs séquences cationiques choisies parmi un polymère cationique comme mentionné précédemment, et d'une ou plusieurs séquences hydrophiles ou hydrophobes (par exemple polyéthylèneglycol) ;
ou
les protéines ou les peptides cationiques ou polycationiques, choisis parmi les protéines ou peptides suivants ayant la formule brute (1) suivante: (Arg)ₗ;(Lys)ₘ;(His)ₙ;(Orn)ₒ;(Xaa)ₓ, où l + m + n + o + x = 8-15, et l, m, n ou o indépendamment les uns des autres peuvent être tout nombre choisi parmi 0, 1, 2, 3, 4, 5, 6, 7, 8, 9, 10, 11, 12, 13, 14 ou 15, à condition que la teneur totale de Arg, Lys, His et Orn représente au moins 50% de tous les aminoacides de l'oligopeptide; et Xaa peut être tout aminoacide choisi parmi les aminoacides natifs (= naturels) ou non natifs à l'exception de Arg, Lys, His ou Orn; et x peut être tout nombre choisi parmi 0, 1, 2, 3 ou 4, à condition que la teneur totale de Xaa ne dépasse pas 50 % de tous les aminoacides de l'oligopeptide ; ou
les acides nucléiques ayant la formule (II) : GₗXₘGₙ, où: G est la guanosine, l'uridine ou un analogue de la guanosine ou de l'uridine; X est la guanosine, l'uridine, l'adénosine, la thymidine, la cytidine ou un analogue des nucléotides mentionnés ci-dessus ; l est un entier de 1 à 40, où quand l = 1 G est la guanosine ou un analogue de celle-ci, quand l > 1 au moins 50 % des nucléotides sont la guanosine ou un analogue de celle-ci ; m est un entier et est au moins 3; où quand m = 3 X est l'uridine ou un analogue de celui-ci, quand m > 3 au moins 3 uridines ou analogues de l'uridine successifs existent ; n est un entier de 1 à 40, où quand n = 1 G est la guanosine ou un analogue de celle-ci, quand n > 1 au moins 50% des nucléotides sont la guanosine ou un analogue de celle-ci ;
ou
les acides nucléiques ayant la formule (III): CₗXₘCₙ, où: C est la cytidine, l'uridine ou un analogue de la cytidine ou de l'uridine; X est la guanosine, l'uridine, l'adénosine, la thymidine, la cytidine ou un analogue des nucléotides mentionnés ci-dessus ; l est un entier de 1 à 40, où quand l = 1 C est la cytidine ou un analogue de celle-ci, quand l > 1 au moins 50% des nucléotides sont la cytidine ou un analogue de celle-ci; m est un entier et est au moins 3; où quand m = 3 X est l'uridine ou un analogue de celui-ci, quand m > 3 au moins 3 uridines ou analogues de l'uridine successifs existent ; n est un entier de 1 à 40, où quand n = 1 C est la cytidine ou un analogue de celle-ci, quand n > 1 au moins 50% des nucléotides sont la cytidine ou un analogue de celle-ci.

6. Vaccin destiné à être utilisé dans le traitement du cancer du poumon non à petites cellules, le vaccin comprenant une composition telle que définie dans l'une quelconque des revendications 1 à 5, où le vaccin comprend en outre de préférence un vecteur pharmaceutiquement acceptable, où le traitement comprend en outre l'administration d'un agent chimiothérapeutique.

7. Composition destinée à être utilisée selon l'une quelconque des revendications 1 à 5, ou vaccin destiné à être utilisé selon la revendication 6, où l'agent chimiothérapeutique est choisi dans le groupe consistant en un composé à base de platine, le pemetrexed, la gemcitabine, les taxanes, la vinorelbine, l'étoposide, le docétaxel et le paclitaxel.

8. Composition destinée à être utilisée selon l'une quelconque des revendications 1 à 5 ou 7, ou vaccin selon la revendication 6 ou 7, où le traitement comprend en outre une radiothérapie.

9. Composition destinée à être utilisée selon l'une quelconque des revendications 1 à 5, 7 ou 8, ou vaccin destiné à être utilisé selon l'une quelconque des revendications 6 à 8, où la composition ou vaccin est utilisée pour le traitement d'un patient ayant un cancer du poumon non à petites cellules au stade III ou au stade IV.

10. Composition destinée à être utilisée ou vaccin destiné à être utilisé selon la revendication 9, où le cancer du poumon non à petites cellules est **caractérisé par** une histologie non squameuse.

11. Composition destinée à être utilisée selon l'une quelconque des revendications 1 à 5 ou 7 à 10, ou vaccin destiné à être utilisé selon l'une quelconque des revendications 6 à 10, où le sujet est un patient recevant une chimiothérapie, de préférence une chimiothérapie à base de platine ou une chimiothérapie de combinaison à base de platine, ou un patient ayant réalisé une réponse partielle ou une maladie stable après une chimiothérapie.

12. Combinaison de six ARNm destinée à être utilisée dans le traitement du cancer du poumon non à petites cellules, où chaque ARNm code un antigène choisi dans le groupe consistant en 5T4 (glycoprotéine des trophoblastes, TPBG), survivine (protéine contenant des répétitions IAP baculovirale 5; BIRC5), NY-ESO-1 (carcinome épidermoïde de l'oesophage de New York 1; CTAG1B), MAGE-C1 (famille d'antigènes du mélanome C1), MAGE-C2 (famille d'antigènes du mélanome C2) et MUC1 (mucine 1) et où chaque ARNm est identique à une séquence d'ARN différente choisie parmi les séquences d'ARN selon SEQ ID NO: 19, 20, 21, 22, 23 ou 24, et où chaque ARNm est complexé avec un composé cationique choisi dans le groupe consistant en un peptide cationique, une protéine cationique, un polysaccharide cationique, un polymère cationique et un lipide cationique, et
où le traitement comprend en outre l'administration d'un agent chimiothérapeutique.

13. Combinaison destinée à être utilisée selon la revendication 12, où chacun des six ARNm est administré séparément.

14. Combinaison destinée à être utilisée selon la revendication 12 ou 13, où les ARNm sont administrés par injection intradermique.

15. Combinaison destinée à être utilisée selon l'une quelconque des revendications 12 à 14, où le traitement comprend en outre une radiothérapie.

16. Combinaison destinée à être utilisée selon l'une quelconque des revendications 12 à 15, où la composition est utilisée pour le traitement d'un patient ayant un cancer du poumon non à petites cellules au stade III ou au stade IV.

17. Combinaison destinée à être utilisée selon la revendication 16, où le cancer du poumon non à petites cellules est **caractérisé par** une histologie non squameuse.

18. Combinaison destinée à être utilisée selon l'une quelconque des revendications 12 à 17, où le sujet est un patient recevant une chimiothérapie, de préférence une chimiothérapie à base de platine ou une chimiothérapie de combinaison à base de platine, ou un patient ayant réalisé une réponse partielle ou une maladie stable après une chimiothérapie.

19. Kit, de préférence kit de parties, destiné à être utilisé dans le traitement du cancer du poumon non à petites cellules, le kit comprenant la composition destinée à être utilisée telle que définie dans l'une quelconque des revendications 1 à 5 ou 7 à 11, et/ou un vaccin destiné à être utilisé tel que défini dans l'une quelconque des revendications 6 à 11, et éventuellement un véhicule liquide pour dissoudre et éventuellement des instructions techniques avec des informations sur l'administration et le dosage de la composition active et/ou du vaccin.

20. Kit destiné à être utilisé selon la revendication 19, où le kit est un kit de parties et chaque partie du kit contient au moins un ARNm de préférence codant un antigène différent choisi parmi les antigènes définis dans la revendication 1, toutes les parties du kit de parties formant la composition ou le vaccin des revendications précédentes.

21. Kit destiné à être utilisé selon la revendication 19 ou 20, où le kit contient au moins deux parties contenant six ARNm.

22. Kit destiné à être utilisé selon l'une quelconque des revendications 19 à 2, où les six ARNm sont tous fournis sous forme lyophilisée dans des parties séparées.

23. Kit destiné à être utilisé selon la revendication 22, où le kit contient une solution Ringer-lactate comme partie.

24. Kit destiné à être utilisé selon l'une quelconque des revendications 19 à 23, où le kit contient six parties, chaque partie contenant l'un des six ARNm.
